# EUROPEAN PATENT APPLICATION

(11) **EP 4 501 928 A1**
(43) Date of publication of application: **05.02.2025**
(21) Application number: 23775276.1
(22) Date of filing: 21.03.2023
(51) Int. Cl.: C07D 417/12, A61K 31/4439, A61K 31/444, A61P 35/00, A23L 33/10, C07D 417/14, C07D 413/14

(54) **THIAZOLE DERIVATIVE COMPOUND AND USES THEREOF**

(30) Priority: 22.03.2022 KR 20220035430
(71) Applicant: Autotelic Bio Inc., Cheongju-si, Chungcheongbuk-do 28160 (KR)
(72) Inventor: PARK, Chang Hee, Hwaseong-si Gyeonggi-do 18477 (KR); KIM, Tae Hun, Sejong 30043 (KR); CHOI, So Won, Suwon-si Gyeonggi-do 16698 (KR); KIM, Jaehyun, Seongnam-si Gyeonggi-do 13208 (KR); SEO, Yujin, Yongin-si Gyeonggi-do 16944 (KR)
(74) Representative: Mewburn Ellis LLP
(86) International application number: PCT/KR2023/003739
(87) International publication number: WO 2023/182780

(57) **Abstract**

The present invention relates to a novel thiazole derivative compound, a compound selected from pharmaceutically acceptable salts thereof, solvates thereof or stereoisomers thereof, and a medical use thereof. The present invention provides a TGFβR1 (ALK5) inhibitor that selectively inhibits only TGFβR1 without interfering with normal TGFβ signaling, and the novel TGFβR1 inhibitor may be used for various indications such as anticancer drugs.

## Description

### Technical Field

The present disclosure relates to a thiazole derivative compound which is a novel TGFβR1 (ALK5) inhibitor and a medical use thereof.

### Background Art

A transforming growth factor β (TGFβ) superfamily of growth factors is involved in inhibition of cell growth, tissue homeostasis, extracellular matrix (ECM) remodeling, endothelial-to-mesenchymal transition (EMT), cell migration and invasion, and immunoregulation as well as numerous signaling pathways that regulate diverse biological processes, including mesenchymal-to-epithelial transition.

In regard to ECM remodeling, TGFβ signaling may increase fibroblast populations and ECM deposits, while TGFβ ligands are capable of regulating T-regulatory cell functions, growth of immune precursor cells, and maintenance of homeostasis in the immune system.

In normal epithelial cells, TGFβ is a potent growth inhibitor and promoter of cell differentiation, but with the development and progression of tumor, TGFβ may become a promoter of tumorigenesis by stimulating angiogenesis, altering the stromal environment, and inducing local and systemic immunosuppression.

Therefore, TGFβ is known to be a therapeutic target for a number of clinical indications, but no safe and effective TGFβ therapeutics have been developed despite the efforts by many groups for the development of TGFβ therapeutics.

Therefore, it is necessary to develop therapeutics for various indications such as immuno-oncology drugs through the development of selective inhibitors of low molecular weight TGFβR1 (activin receptor-like kinase 5 (ALK5)) that is effective with few side effects among TGFβ therapeutics, which are emerging as therapeutic targets for various clinical indications.

### Disclosure of the Invention

### Technical Goals

An object of the present disclosure is to provide a compound selected from a novel thiazole derivative compound, a pharmaceutically acceptable salt thereof, a solvate thereof, or a stereoisomer thereof.

Another object of the present disclosure is to provide a pharmaceutical composition for treating or preventing a cancer disease, including a compound selected from the thiazole derivative compound, a pharmaceutically acceptable salt thereof, a solvate thereof, or a stereoisomer thereof as an active ingredient.

Another object of the present disclosure is to provide a health functional food composition for ameliorating or preventing a cancer disease, including a compound selected from the thiazole derivative compound, a pharmaceutically acceptable salt thereof, a solvate thereof, or a stereoisomer thereof as an active ingredient.

Another object of the present disclosure is to provide a reagent composition for suppressing expression of transforming growth factor beta (TGFβ), including a compound selected from the thiazole derivative compound, a pharmaceutically acceptable salt thereof, a solvate thereof, or a stereoisomer thereof as an active ingredient.

### Technical Solutions

To achieve the above objects, the present disclosure provides a compound selected from a thiazole derivative compound represented by the following Chemical Formula 1, a pharmaceutically acceptable salt thereof, a solvate thereof, or a stereoisomer thereof.

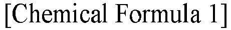

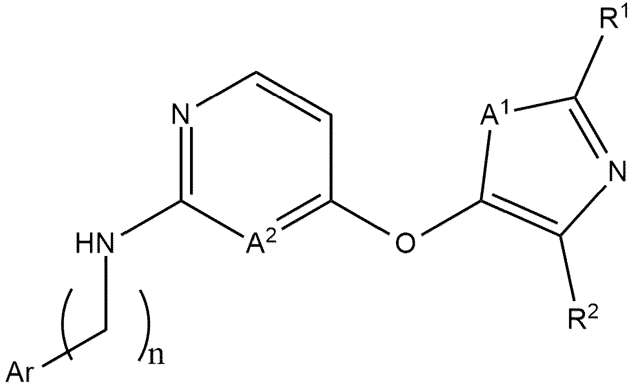

In the Chemical Formula 1, A¹ is O or S, A² is CH or N, R¹ and R² may be the same or different and are each one of (C1-C4)alkyl, (C1-C4)alkylene, (C1-C4)alkoxy, trifluoromethyl (CF₃), halo, NR¹¹R¹², unsubstituted or substituted (C3-C8)cycloalkyl, unsubstituted or substituted (C5-C10)aryl, and unsubstituted or substituted heteroaryl of 5 to 10 atoms including one or more heteroatoms selected from the group consisting of N, O, and S, the R¹¹ and R¹² may be the same or different and are each any one of hydrogen, (C1-C5)alkyl, and (C3-C8)cycloalkyl, the substituted (C3-C8)cycloalkyl, substituted (C5-C10)aryl, or substituted heteroaryl of 5 to 10 atoms is substituted with (C1-C5)alkyl, n is one of 0 to 3, Ar is unsubstituted or substituted (C5-C10)aryl or unsubstituted or substituted heteroaryl of 5 to 10 atoms including one or more heteroatoms selected from the group consisting of N, O, and S, the substituted (C5-C10)aryl or substituted heteroaryl of 5 to 10 atoms is substituted with R³, the R³ is -R³⁻¹ or -(C1-C5)alkyl-R³⁻¹, the R³⁻¹ is any one of hydrogen, halogen, hydroxy, cyano, (C1-C5)alkyl, (C1-C5)alkoxy, -CONR³¹⁻¹R³¹⁻², -COOR³², -NHSO₂R³³, -SO₂R³⁴, - SO₂NR³⁵⁻¹R³¹⁻², -COR³⁶, -NH-(C1-C3)alkyl-R³⁷, 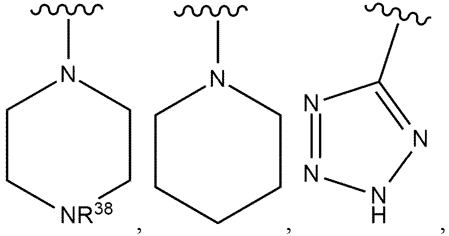 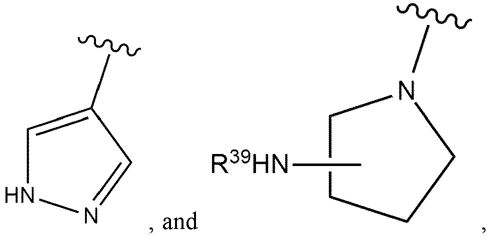 the R³¹⁻¹, R³¹⁻², R³², R³³, R³⁴ , R³⁵⁻¹, and R³⁵⁻² may be the same or different and are each hydrogen, hydroxy, trifluoromethyl, (C1-C5)alkyl, (C3-C8)cycloalkyl, -SO₂-(C1-C3)alkyl, -(C1-C3)alkyl-R³⁰¹, and 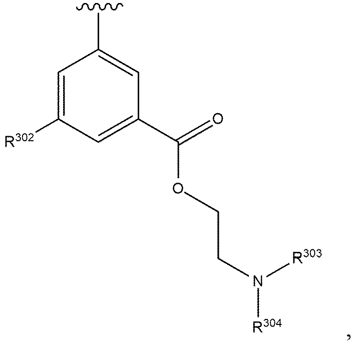 the R³⁰¹ is any one of (C1-C4)alkylamino, di[(C1∼C4)alkyl]amino, (C3-C8)cycloalkyl, 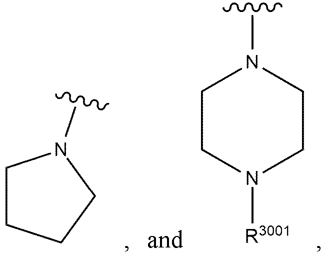 the R³⁰⁰¹ is hydrogen or (C1-C5)alkyl, the R³⁰² is halogen,

the R³⁰³ and R³⁰⁴ may be the same or different and are each hydrogen or (C1-C3)alkyl, the R³⁶ and R³⁷ may be the same or different and are each 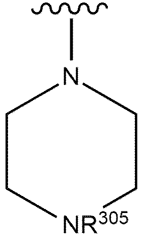 or 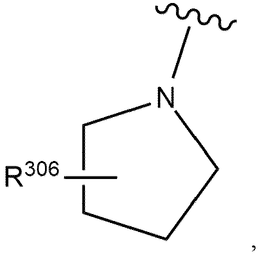 the R³⁰⁵ is hydrogen or (C1-C5)alkyl, the R³⁰⁶ is any one of hydrogen, amino, (C1~C4)alkylamino, and di[(C1~C4)alkyl]amino, and the R³⁸ and R³⁹ may be the same or different and are each any one of hydrogen, (C1-C3)alkyl, and -(C1-C3)alkyl-(C 1-C3)alkoxy.

In addition, the present disclosure provides a pharmaceutical composition for treating or preventing a cancer disease, including a compound selected from the thiazole derivative compound, a pharmaceutically acceptable salt thereof, a solvate thereof, or a stereoisomer thereof as an active ingredient.

In addition, the present disclosure provides a health functional food composition for ameliorating or preventing a cancer disease, including a compound selected from the thiazole derivative compound, a pharmaceutically acceptable salt thereof, a solvate thereof, or a stereoisomer thereof as an active ingredient.

In addition, the present disclosure provides a reagent composition for suppressing expression of transforming growth factor beta (TGFβ), including a compound selected from the thiazole derivative compound, a pharmaceutically acceptable salt thereof, a solvate thereof, or a stereoisomer thereof as an active ingredient.

### Advantageous Effects

The present disclosure relates to a compound selected from a novel thiazole derivative compound, a pharmaceutically acceptable salt thereof, a solvate thereof, or a stereoisomer thereof, and a use thereof, wherein the compound selectively inhibits only TGFβR1 without interfering with normal TGFβ signaling, and has a therapeutic effect on various related cancer diseases, and therefore it may be widely used as a treatment method in medical institutions such as hospitals.

### Brief Description of Drawings

FIGS. 1 to 32 show structures of novel synthesized thiazole derivative compounds.

### Best Mode for Carrying Out the Invention

Hereinafter the present disclosure will be described in detail.

The present disclosure provides a compound selected from a thiazole derivative compound represented by the following Chemical Formula 1, a pharmaceutically acceptable salt thereof, a solvate thereof, or a stereoisomer thereof.

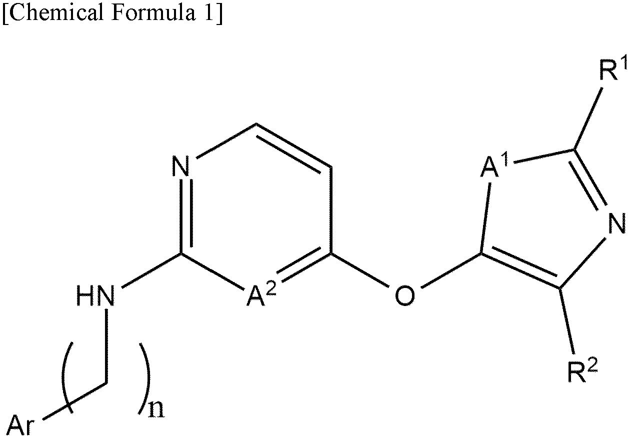

In the Chemical Formula 1, A¹ is O or S, A² is CH or N, R¹ and R² may be the same or different and are each one of (C1-C4)alkyl, (C1-C4)alkylene, (C1-C4)alkoxy, trifluoromethyl (CF₃), halo, NR¹¹R¹², unsubstituted or substituted (C3-C8)cycloalkyl, unsubstituted or substituted (C5-C10)aryl, and unsubstituted or substituted heteroaryl of 5 to 10 atoms including one or more heteroatoms selected from the group consisting of N, O, and S, the R¹¹ and R¹² may be the same or different and are each any one of hydrogen, (C1-C5)alkyl, and (C3-C8)cycloalkyl, the substituted (C3-C8)cycloalkyl, substituted (C5-C10)aryl, or substituted heteroaryl of 5 to 10 atoms is substituted with (C1-C5)alkyl, n is one of 0 to 3, Ar is unsubstituted or substituted (C5-C10)aryl or unsubstituted or substituted heteroaryl of 5 to 10 atoms including one or more heteroatoms selected from the group consisting of N, O, and S, the substituted (C5-C10)aryl or substituted heteroaryl of 5 to 10 atoms is substituted with R³, the R³ is -R³⁻¹ or -(C1-C5)alkyl-R³⁻¹, the R³⁻¹ is any one of hydrogen, halogen, hydroxy, cyano, (C1-C5)alkyl, (C1-C5)alkoxy, -CONR³¹⁻¹R³¹⁻², -COOR³², -NHSO₂R₃³, -SO₂R³⁴, - SO₂NR³⁵⁻¹R³⁵⁻², -COR³⁶, -NH-(C1-C3)alkyl-R³⁷, 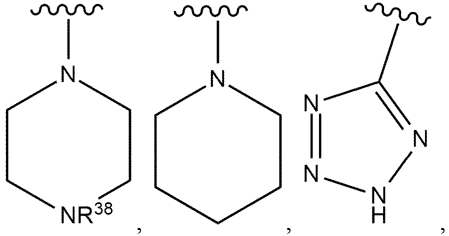 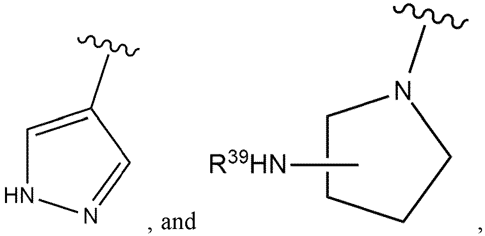 the R³¹⁻¹, R³¹⁻², R³², R³³, R³⁴, R³⁵⁻¹, and R³⁵⁻² may be the same or different and are each hydrogen, hydroxy, trifluoromethyl, (C1-C5)alkyl, (C3-C8)cycloalkyl, -SO₂-(C1-C3)alkyl, -(C1-C3)alkyl-R³⁰¹, and 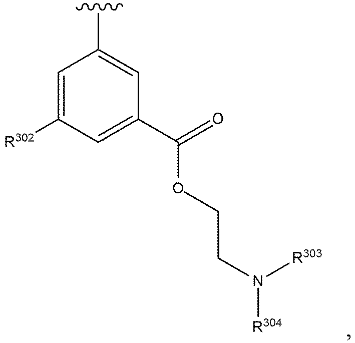 the R³⁰¹ is any one of (C1-C4)alkylamino, di[(C1~C4)alkyl]amino, (C3-C8)cycloalkyl, 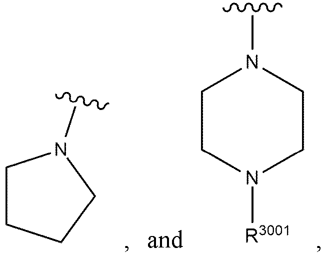 the R³⁰⁰¹ is hydrogen or (C1-C5)alkyl, the R³⁰² is halogen,
the R³⁰³ and R³⁰⁴ may be the same or different and are each hydrogen or (C1-C3)alkyl, the R³⁶ and R³⁷ may be the same or different and are each 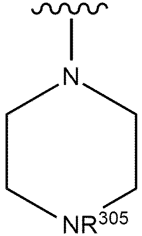 or 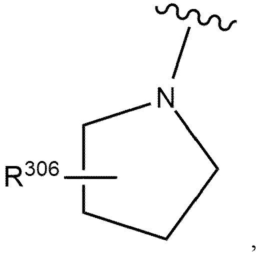 the R³⁰⁵ is hydrogen or (C1-C5)alkyl, the R³⁰⁶ is any one of hydrogen, amino, (C1-C4)alkylamino, and di[(C1-C4)alkyl]amino, and the R³⁸ and R³⁹ may be the same or different and are each any one of hydrogen, (C1-C3)alkyl, and -(C1-C3)alkyl-(C 1-C3)alkoxy.

In the Chemical Formula 1, R¹ is any one of (C1-C3)alkyl, (C1-C3)alkylene, methoxy, trifluoromethyl (CF₃), bromine (Br), (C3-C6)cycloalkyl, and NR¹¹R¹², the R¹¹ and R¹² may be the same or different and are each any one of the hydrogen, (C1-C3)alkyl, and cyclopropyl, R² is 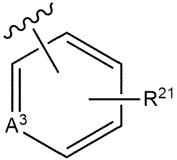 or naphthyl, the A³ is CH or N, the R²¹ is hydrogen or methyl, n is one of 0 to 2, Ar is any one of 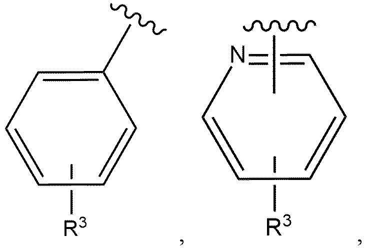 and 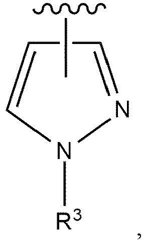 the R³ is -R³⁻¹ or -(C1-C3)alkyl-R³⁻¹, the R³⁻¹ is any one of hydrogen, fluorine, chlorine, hydroxy, cyano, (C1-C3)alkyl, (C1-C3)alkoxy, -CONR³¹⁻¹R³¹⁻², -COOR³², - NHSO₂R₃³, -SO₂R³⁴, -SO₂NR³⁵⁻¹R³⁵⁻², -COR³⁶, -NH-(C1-C3)alkyl-R³⁷, 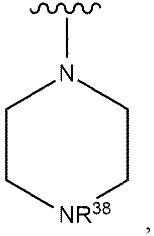 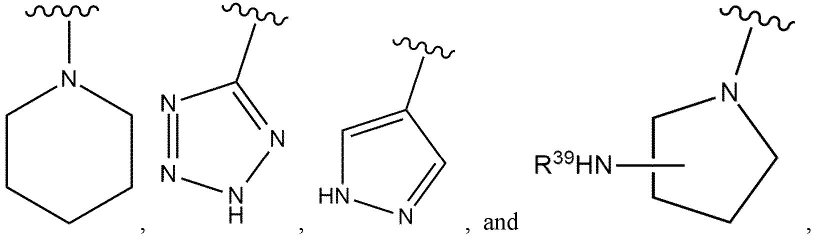 R³¹⁻¹, R³¹⁻², R³², R³³, R³⁴, R³⁵⁻¹, and R³⁵⁻² may be the same or different and are each hydrogen, hydroxy, trifluoromethyl, (C1-C3)alkyl, (C3-C6)cycloalkyl, -SO₂CH₃, -(C1-C3)alkyl-R³⁰¹, and 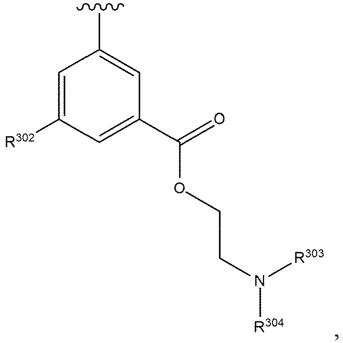 the R³⁰¹ is any one of methylamino, dimethylamino, cyclopropyl, 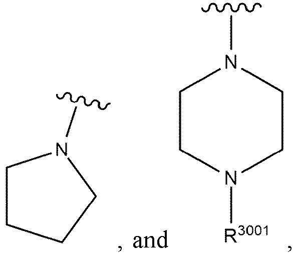 the R³⁰⁰¹ is hydrogen or (C1-C3)alkyl, the R³⁰² is fluorine, the R³⁰³ and R³⁰⁴ may be the same or different and are each hydrogen or methyl, the R³⁶ and R³⁷ may be the same or different and are each 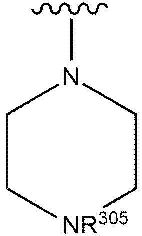 or 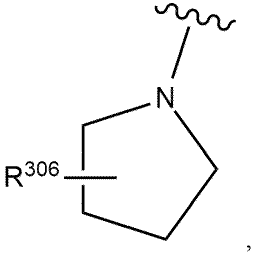 the R³⁰⁵ is (C1-C3)alkyl, the R³⁰⁶ is any one of hydrogen, amino, and methylamino, and the R³⁸ and R³⁹ may be the same or different and are each any one of hydrogen, (C1-C3)alkyl, and -(C1-C3)alkyl-methoxy.

In the Chemical Formula 1, R¹ is any one of methyl, ethyl, isopropyl, ethylene, methoxy, trifluoromethyl (CF₃), bromine (Br), cyclopropyl, cyclopentyl, cyclohexyl, and NR¹¹R¹², the R¹¹ and R¹² may be the same or different and are each any one of hydrogen, methyl, and cyclopropyl, R² is one of 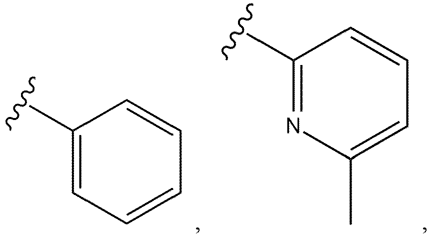 and 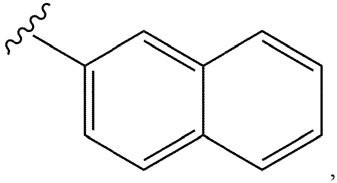 n is one of 0 to 2, Ar is any one of 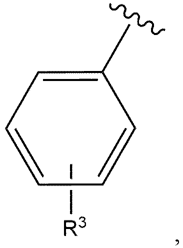 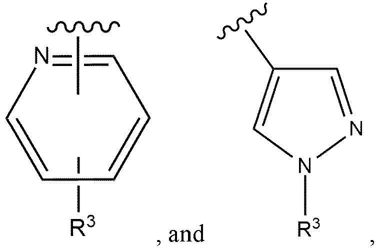 the R³ is -R³⁻¹ or -(C1-C3)alkyl-R³⁻¹, the R³⁻¹ is any one of hydrogen, fluorine, chlorine, hydroxy, cyano, methyl, methoxy, -CONR³¹⁻¹R³¹⁻², -COOR³², -NHSO₂R³³, -SO₂R³⁴, -SO₂NR³⁵⁻¹R³⁵⁻², -COR³⁶, -NH-(C1-C2)alkyl-R³⁷, 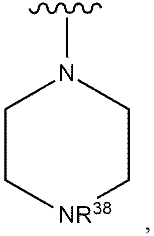 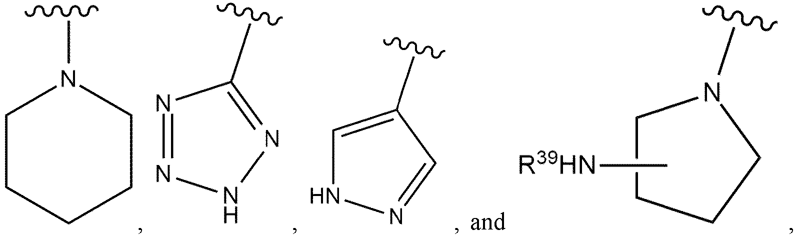 the R³¹⁻¹ and R³¹⁻² may be the same or different and are each hydrogen, methyl, isopropyl, hydroxy, - SO₂CH₃, -(C1-C2)alkyl-R³⁰¹, and 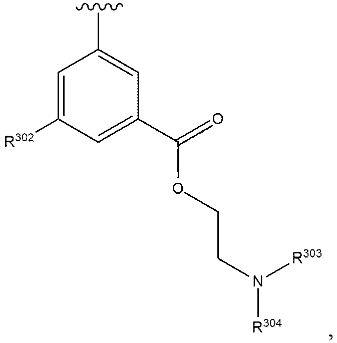 the R³⁰¹ is any one of dimethylamino, cyclopropyl, 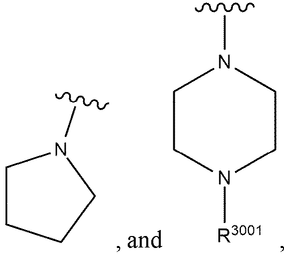 the R³⁰⁰¹ is one of methyl, ethyl, and isopropyl, the R³⁰² is fluorine, the R³⁰³ and R³⁰⁴ are methyl, the R³² is one of hydrogen, methyl, and -(C1-C2)alkyl-dimethylamino, the R³³ is one of methyl, ethyl, cyclopropyl, and trifluoromethyl, the R³⁴ is one of hydroxy, methyl, ethyl, cyclopropyl, and trifluoromethyl, the R³⁵⁻¹ and R³⁵⁻² may be the same or different and are each one of hydrogen, methyl, and cyclopropyl, the R³⁶ is 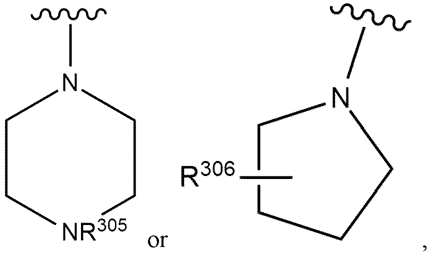 the R³⁰⁵ is methyl, the R³⁰⁶ is amino or methylamino, the R³⁷ is 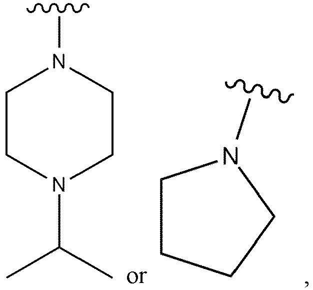 the R³⁸ is methyl or - CH₂CH₂OCH₃, and the R³⁹ may be hydrogen or methyl.

The compound represented by Chemical Formula 1 may be selected from the group of compounds below.
(1) 3-((4-((2-Amino-4-phenylthiazol-5-yl)oxy)pyridin-2-yl)amino)benzenesulfonamide
(2) 3-((4-((2-Amino-4-phenylthiazol-5-yl)oxy)pyridin-2-yl)amino)benzamide
(3) Methyl 3-((4-((2-amino-4-phenylthiazol-5-yl)oxy)pyridin-2-yl)amino)benzoate
(4) 4-((4-((2-Amino-4-phenylthiazol-5-yl)oxy)pyridin-2-yl)amino)benzenesulfonamide
(5) 4-((4-((2-Amino-4-phenylthiazol-5-yl)oxy)pyridin-2-yl)amino)benzamide
(6) 4-Phenyl-5-((2-(pyridin-3-ylamino)pyridin-4-yl)oxy)thiazol-2-amine
(7) 5-((2-((6-Fluoropyridin-3-yl)amino)pyridin-4-yl)oxy)-4-phenylthiazol-2-amine
(8) 4-Phenyl-5-((2-(pyridin-4-ylamino)pyridin-4-yl)oxy)thiazol-2-amine
(9) 6-((4-((2-Amino-4-phenylthiazol-5-yl)oxy)pyridin-2-yl)amino)nicotinamide
(10) 3-((4-((2-amino-4-phenylthiazol-5-yl)oxy)pyridin-2-yl)amino)-N,N-dimethylbenzamide
(11) 5-((4-((2-Amino-4-phenylthiazol-5-yl)oxy)pyridin-2-yl)amino)picolinamide
(12) 3-((4-((2-Amino-4-phenylthiazol-5-yl)oxy)pyridin-2-yl)amino)-A-methylbenzenesulfonamide
(13) 3-((4-((2-Amino-4-phenylthiazol-5-yl)oxy)pyridin-2-yl)amino)-*N*,*N-*dimethylbenzenesulfonamide
(14) *N*-(3-((4-((2-Amino-4-phenylthiazol-5-yl)oxy)pyridin-2-yl)amino)phenyl)methanesulfonamide
(15) 5-((2-((3-(Methylsulfonyl)phenyl)amino)pyridin-4-yl)oxy)-4-phenylthiazol-2-amine
(16) *N*-(3-((4-((2-Amino-4-phenylthiazol-5-yl)oxy)pyridin-2-yl)amino)phenyl)cyclopropanesulfonamide
(17) 4-Phenyl-5-((2-((3-((trifluoromethyl)sulfonyl)phenyl)amino)pyridin-4-yl)oxy)thiazol-2-amine
(18) 3-((4-((2-Amino-4-phenylthiazol-5-yl)oxy)pyridin-2-yl)amino)benzenesulfonic acid
(19) 5-((2-((3-((Methylsulfonyl)methyl)phenyl)amino)pyridin-4-yl)oxy)-4-phenylthiazol-2-amine
(20) 3-((4-((2-Amino-4-phenylthiazol-5-yl)oxy)pyridin-2-yl)amino)-A-cyclopropylbenzenesulfonamide
(21) 5-((2-((3-Fluorobenzyl)amino)pyridin-4-yl)oxy)-4-phenylthiazol-2-amine
(22) 2-((4-((2-Amino-4-phenylthiazol-5-yl)oxy)pyridin-2-yl)amino)isonicotinamide
(23) 3-((4-((2-Amino-4-phenylthiazol-5-yl)oxy)pyridin-2-yl)amino)-A-methylbenzamide
(24) 5-((2-((2-Methoxypyridin-4-yl)amino)pyridin-4-yl)oxy)-4-phenylthiazol-2-amine
(25) 5-((2-(Benzylamino)pyridin-4-yl)oxy)-4-phenylthiazol-2-amine
(26) 4-Phenyl-5-((2-((pyridin-4-ylmethyl)amino)pyridin-4-yl)oxy)thiazol-2-amine
(27) 5-((2-((2-Chloropyridin-4-yl)amino)pyridin-4-yl)oxy)-4-phenylthiazol-2-amine
(28) 4-Phenyl-5-((2-((pyridin-3-ylmethyl)amino)pyridin-4-yl)oxy)thiazol-2-amine
(29) 3-((4-((2-Amino-4-phenylthiazol-5-yl)oxy)pyridin-2-yl)amino)phenyl)methanesulfonamide
(30) 5-((2-((2-Fluoropyridin-4-yl)amino)pyridin-4-yl)oxy)-4-phenylthiazol-2-amine
(31) 5-((2-((6-Methoxypyridin-3-yl)amino)pyridin-4-yl)oxy)-4-phenylthiazol-2-amine
(32) 5-((4-((2-Amino-4-phenylthiazol-5-yl)oxy)pyridin-2-yl)amino)nicotinamide
(33) 6-((4-((2-Amino-4-phenylthiazol-5-yl)oxy)pyridin-2-yl)amino)picolinamide
(34) 5-((2-((3-(Ethylsulfonyl)phenyl)amino)pyridin-4-yl)oxy)-4-phenylthiazol-2-amine
(35) 5-((2-((6-Chloropyridin-3-yl)amino)pyridin-4-yl)oxy)-4-phenylthiazol-2-amine
(36)*N-*(3-((4-((2-Amino-4-phenylthiazol-5-yl)oxy)pyridin-2-yl)amino)phenyl)-1,1,1-trifluoromethanesulfonamide
(37) 5-((2-((1-Methyl-1*H*-pyrazol-4-yl)amino)pyridin-4-yl)oxy)-4-phenylthiazol-2-amine
(38) 5-((2-((1*H*-Pyrazol-4-yl)amino)pyridin-4-yl)oxy)-4-phenylthiazol-2-amine
(39) 4-((4-((2-amino-4-phenylthiazol-5-yl)oxy)pyridin-2-yl)amino)benzoic acid
(40) 3-((4-((2-Amino-4-phenylthiazol-5-yl)oxy)pyridin-2-yl)amino)benzoic acid
(41) 2-(3-((4-((2-amino-4-phenylthiazol-5-yl)oxy)pyridin-2-yl)amino)phenyl)propan-2-ol
(42) 2-(4-((4-((2-Amino-4-phenylthiazol-5-yl)oxy)pyridin-2-yl)amino)pyridin-2-yl)propan-2-ol
(43) 2-(5-((4-((2-Amino-4-phenylthiazol-5-yl)oxy)pyridin-2-yl)amino)pyridin-2-yl)propan-2-ol
(44) 2-(3-((4-((2-(Cyclopropylamino)-4-phenylthiazol-5-yl)oxy)pyridin-2-yl)amino)phenyl)propan-2-ol
(45) 3-((4-((2-(Cyclopropylamino)-4-phenylthiazol-5-yl)oxy)pyridin-2-yl)amino)benzenesulfonamide
(46) 3-((4-((2-(Cyclopropylamino)-4-phenylthiazol-5-yl)oxy)pyridin-2-yl)amino)benzamide
(47) 4-((4-((2-(Cyclopropylamino)-4-phenylthiazol-5-yl)oxy)pyridin-2-yl)amino)benzamide
(48) 3-((4-((2-(Cyclopropylamino)-4-phenylthiazol-5-yl)oxy)pyridin-2-yl)amino)benzoic acid
(49) 4-((4-((2-(Cyclopropylamino)-4-phenylthiazol-5-yl)oxy)pyridin-2-yl)amino)benzoic acid
(50) 2-(3-((4-((2-(Methylamino)-4-phenylthiazol-5-yl)oxy)pyridin-2-yl)amino)phenyl)propan-2-ol
(51) 3-((4-((2-(Methylamino)-4-phenylthiazol-5-yl)oxy)pyridin-2-yl)amino)benzenesulfonamide
(52) 3-((4-((2-(Methylamino)-4-phenylthiazol-5-yl)oxy)pyridin-2-yl)amino)benzamide
(53) 4-((4-((2-(Methylamino)-4-phenylthiazol-5-yl)oxy)pyridin-2-yl)amino)benzenesulfonamide
(54) 2-(4-((4-((2-(Methylamino)-4-phenylthiazol-5-yl)oxy)pyridin-2-yl)amino)pyridin-2-yl)propan-2-ol
(55) 2-(5-((4-((2-(Methylamino)-4-phenylthiazol-5-yl)oxy)pyridin-2-yl)amino)pyridin-2-yl)propan-2-ol
(56) 4-((4-((2-(Methylamino)-4-phenylthiazol-5-yl)oxy)pyridin-2-yl)amino)benzoic acid
(57) 3-((4-((2-(Methylamino)-4-phenylthiazol-5-yl)oxy)pyridin-2-yl)amino)benzoic acid
(58) 3-((4-((2-Methyl-4-phenylthiazol-5-yl)oxy)pyridin-2-yl)amino)benzenesulfonamide
(59) 2-(4-((4-((2-Methyl-4-phenylthiazol-5-yl)oxy)pyridin-2-yl)amino)pyridin-2-yl)propan-2-ol
(60) 3-((4-((2-Methyl-4-phenylthiazol-5-yl)oxy)pyridin-2-yl)amino)benzamide
(61) 2-(3-((4-((2-Methyl-4-phenylthiazol-5-yl)oxy)pyridin-2-yl)amino)phenyl)propan-2-ol
(62) 5-((2-((1-(Methylsulfonyl)-1*H*-pyrazol-4-yl)amino)pyridin-4-yl)oxy)-4-phenylthiazol-2-amine
(63) 4-((4-((2-Methyl-4-phenylthiazol-5-yl)oxy)pyridin-2-yl)amino)benzamide
(64) 4-((4-((2-Methyl-4-phenylthiazol-5-yl)oxy)pyridin-2-yl)amino)benzenesulfonamide
(65) 2-(5-((4-((2-Methyl-4-phenylthiazol-5-yl)oxy)pyridin-2-yl)amino)pyridin-2-yl)propan-2-ol
(66) 2-Methyl-2-(3-((4-((2-methyl-4-phenylthiazol-5-yl)oxy)pyridin-2-yl)amino)phenyl)propanoic acid
(67) *N*,*N*-Dimethyl-3-((4-((2-methyl-4-phenylthiazol-5-yl)oxy)pyridin-2-yl)amino)benzamide
(68) 3-((4-((2-Methyl-4-phenylthiazol-5-yl)oxy)pyridin-2-yl)amino)benzenesulfonic acid
(69) *N-*Methyl-3-((4-((2-methyl-4-phenylthiazol-5-yl)oxy)pyridin-2-yl)amino)benzamide
(70) 4-((2-Methyl-4-phenylthiazol-5-yl)oxy)-*N-*(3-(methylsulfonyl)phenyl)pyridin-2-amine
(71) *N-*(3-((4-((2-Methyl-4-phenylthiazol-5-yl)oxy)pyridin-2-yl)amino)phenyl)methanesulfonamide
(72) 4-((2-Methyl-4-phenylthiazol-5-yl)oxy)-*N*-(3-((trifluoromethyl)sulfonyl)phenyl)pyridin-2-amine
(73) *N-*Methyl-3-((4-((2-methyl-4-phenylthiazol-5-yl)oxy)pyridin-2-yl)amino)benzenesulfonamide
(74) *N*,*N*-Dimethyl-3-((4-((2-methyl-4-phenylthiazol-5-yl)oxy)pyridin-2-yl)amino)benzenesulfonamide
(75) *N-*(3-((4-((2-Methyl-4-phenylthiazol-5-yl)oxy)pyridin-2-yl)amino)phenyl)cyclopropanesulfonamide
(76) *N-*Cyclopropyl-3-((4-((2-methyl-4-phenylthiazol-5-yl)oxy)pyridin-2-yl)amino)benzenesulfonamide
(77) *N-*(3-(Ethylsulfonyl)phenyl)-4-((2-methyl-4-phenylthiazol-5-yl-yl)oxy)pyridin-2-amine
(78) 3-((4-((2-Methyl-4-phenylthiazol-5-yl)oxy)pyridin-2-yl)amino)phenyl)methanesulfonamide
(79) 4-((2-Methyl-4-phenylthiazol-5-yl)oxy)-*N*-(3-((methylsulfonyl)methyl)phenyl)pyridin-2-amine
(80) 4-((2-Methyl-4-phenylthiazol-5-yl)oxy)-*N-*(pyridin-4-ylmethyl)pyridin-2-amine
(81) 4-((2-Methyl-4-phenylthiazol-5-yl)oxy)-*N-*(pyridin-3-ylmethyl)pyridin-2-amine
(82) 4-((2-Methyl-4-phenylthiazol-5-yl)oxy)-*N*-(pyridin-3-yl)pyridin-2-amine
(83) *N-*(6-Fluoropyridin-3-yl)-4-((2-methyl-4-phenylthiazol-5-yl)oxy)pyridin-2-amine
(84) *N-*(6-Chloropyridin-3-yl)-4-((2-methyl-4-phenylthiazol-5-yl)oxy)pyridin-2-amine
(85) *N-*(6-Methoxypyridin-3-yl)-4-((2-methyl-4-phenylthiazol-5-yl)oxy)pyridin-2-amine
(86) 4-((2-Methyl-4-phenylthiazol-5-yl)oxy)-*N-*(pyridin-4-yl)pyridin-2-amine
(87) *N-*(2-Fluoropyridin-4-yl)-4-((2-methyl-4-phenylthiazol-5-yl)oxy)pyridin-2-amine
(88) *N-*(2-Methoxypyridin-4-yl)-4-((2-methyl-4-phenylthiazol-5-yl)oxy)pyridin-2-amine
(89) *N*-(2-Chloropyridin-4-yl)-4-((2-methyl-4-phenylthiazol-5-yl)oxy)pyridin-2-amine
(90) 6-((4-((2-Methyl-4-phenylthiazol-5-yl)oxy)pyridin-2-yl)amino)nicotinamide
(91) 5-((4-((2-Methyl-4-phenylthiazol-5-yl)oxy)pyridin-2-yl)amino)picolinamide
(92) 5-((4-((2-Methyl-4-phenylthiazol-5-yl)oxy)pyridin-2-yl)amino)nicotinamide
(93) 2-((4-((2-Methyl-4-phenylthiazol-5-yl)oxy)pyridin-2-yl)amino)isonicotinamide
(94)*N-*Benzyl-4-((2-methyl-4-phenylthiazol-5-yl)oxy)pyridin-2-amine
(95)*N*-(4-Fluorobenzyl)-4-((2-methyl-4-phenylthiazol-5-yl)oxy)pyridin-2-amine
(96) *N-*(3-Fluorobenzyl)-4-((2-methyl-4-phenylthiazol-5-yl)oxy)pyridin-2-amine
(97) 4-((2-Methyl-4-phenylthiazol-5-yl)oxy)-*N*-(1-(methylsulfonyl)-1*H*-pyrazol-4-yl)pyridin-2-amine
(98) *N*-(1-(Cyclopropylsulfonyl)-1*H*-pyrazol-4-yl)-4-((2-methyl-4-phenylthiazol-5-yl)oxy)pyridin-2-amine
(99) *N*-(1-Methyl-1*H*-pyrazol-4-yl)-4-((2-methyl-4-phenylthiazol-5-yl)oxy)pyridin-2-amine
(100) 4-((2-Methyl-4-phenylthiazol-5-yl)oxy)-*N-*(1*H*-pyrazol-4-yl)pyridin-2-amine
(101) 6-((4-((2-Methyl-4-phenylthiazol-5-yl)oxy)pyridin-2-yl)amino)picolinamide
(102) 4-((4-((2-Methyl-4-phenylthiazol-5-yl)oxy)pyridin-2-yl)amino)picolinamide
(103) *N-*(4-Methoxyphenyl)-4-((2-methyl-4-phenylthiazol-5-yl)oxy)pyridin-2-amine
(104) 4-((2-Methyl-4-phenylthiazol-5-yl)oxy)-*N*-(4-(piperazin-1-yl)phenyl)pyridin-2-amine
(105) 4-((2-Methyl-4-phenylthiazol-5-yl)oxy)-*N*-(4-(2-(piperidin-1-yl)ethoxy)phenyl)pyridin-2-amine
(106) *N*-(3-Methoxy-4-(4-methylpiperazin-1-yl)phenyl)-4-((2-methyl-4-phenylthiazol-5-yl)oxy)pyridin-2-amine
(107) 1,1,1-Trifluoro-*N*-(3-((4-((2-methyl-4-phenylthiazol-5-yl)oxy)pyridin-2-yl)amino)phenyl)methanesulfonamide
(108) *N*-(4-(4-(2-Methoxyethyl)piperazin-1-yl)phenyl)-4-((2-methyl-4-phenylthiazol-5-yl)oxy)pyridin-2-amine
(109) 3-((4-((2-Methyl-4-phenylthiazol-5-yl)oxy)pyridin-2-yl)amino)benzoic acid
(110) 4-((4-((2-Methyl-4-phenylthiazol-5-yl)oxy)pyridin-2-yl)amino)benzoic acid
(111) 2-(3-((4-((2-Methyl-4-phenylthiazol-5-yl)oxy)pyridin-2-yl)amino)phenyl)acetonitrile
(112) *N*-(3-((1*H*-tetrazol-5-yl)methyl)phenyl)-4-((2-methyl-4-phenylthiazol-5-yl)oxy)pyridin-2-amine
(113) 3-(3-((4-((2-Methyl-4-phenylthiazol-5-yl)oxy)pyridin-2-yl)amino)phenyl)propanenitrile
(114) *N*-(3-(2-(2*H*-Tetrazol-5-yl)ethyl)phenyl)-4-((2-methyl-4-phenylthiazol-5-yl)oxy)pyridin-2-amine
(115) 2-(3-((4-((2-Cyclopropyl-4-phenylthiazol-5-yl)oxy)pyridin-2-yl)amino)phenyl)propan-2-ol
(116) 3-((4-((2-Cyclopropyl-4-phenylthiazol-5-yl)oxy)pyridin-2-yl)amino)benzenesulfonamide
(117) 3-((4-((2-Cyclopropyl-4-phenylthiazol-5-yl)oxy)pyridin-2-yl)amino)benzamide
(118) 4-((4-((2-Cyclopropyl-4-phenylthiazol-5-yl)oxy)pyridin-2-yl)amino)benzenesulfonamide
(119) 4-((4-((2-Cyclopropyl-4-phenylthiazol-5-yl)oxy)pyridin-2-yl)amino)benzamide
(120) 2-(4-((4-((2-Cyclopropyl-4-phenylthiazol-5-yl)oxy)pyridin-2-yl)amino)pyridin-2-yl)propan-2-ol
(121) 2-(5-((4-((2-Cyclopropyl-4-phenylthiazol-5-yl)oxy)pyridin-2-yl)amino)pyridin-2-yl)propan-2-ol
(122) *N*-(3-((4-((2-Cyclopropyl-4-phenylthiazol-5-yl)oxy)pyridin-2-yl)amino)phenyl)ethanesulfonamide
(123) *N*-(3-((4-((2-Cyclopropyl-4-phenylthiazol-5-yl)oxy)pyridin-2-yl)amino)phenyl)-1,1,1-trifluoromethanesulfonamide
(124) 6-((4-((2-Cyclopropyl-4-phenylthiazol-5-yl)oxy)pyridin-2-yl)amino)picolinamide
(125) 6-((4-((2-Cyclopropyl-4-phenylthiazol-5-yl)oxy)pyridin-2-yl)amino)-*N-*methylpicolinamide
(126) 4-((4-((2-Cyclopropyl-4-phenylthiazol-5-yl)oxy)pyridin-2-yl)amino)-*N-*methylbenzamide
(127) *N*-(6-((4-((2-Cyclopropyl-4-phenylthiazol-5-yl)oxy)pyridin-2-yl)amino)pyridin-3-yl)methanesulfonamide
(128) *N*-(4-((4-((2-Cyclopropyl-4-phenylthiazol-5-yl)oxy)pyridin-2-yl)amino)phenyl)methanesulfonamide
(129) 6-((4-((2-Cyclopropyl-4-phenylthiazol-5-yl)oxy)pyridin-2-yl)amino)nicotinamide
(130) Methyl 6-((4-((2-cyclopropyl-4-phenylthiazol-5-yl)oxy)pyridin-2-yl)amino)nicotinate
(131) Methyl 6-((4-((2-cyclopropyl-4-phenylthiazol-5-yl)oxy)pyridin-2-yl)amino)picolinate
(132) 6-((4-((2-Cyclopropyl-4-phenylthiazol-5-yl)oxy)pyridin-2-yl)amino)nicotinic acid
(133) 6-((4-((2-Cyclopropyl-4-phenylthiazol-5-yl)oxy)pyridin-2-yl)amino)-*N-*methylnicotinamide
(134) 6-((4-((2-Cyclopropyl-4-phenylthiazol-5-yl)oxy)pyridin-2-yl)amino)picolinic acid
(135) 3-((4-((2-Cyclopropyl-4-phenylthiazol-5-yl)oxy)pyridin-2-yl)amino)benzoic acid
(136) 4-((4-((2-Cyclopropyl-4-phenylthiazol-5-yl)oxy)pyridin-2-yl)amino)benzoic acid
(137) 6-((4-((2-Cyclopropyl-4-phenylthiazol-5-yl)oxy)pyridin-2-yl)amino)picolinic acid
(138) 6-((4-((2-Cyclopropyl-4-phenylthiazol-5-yl)oxy)pyridin-2-yl)amino)-N-hydroxynicotinamide
(139) 4-((4-((2-Cyclopropyl-4-phenylthiazol-5-yl)oxy)pyridin-2-yl)amino)-*N-*hydroxybenzamide
(140) 6-((4-((2-Cyclopropyl-4-phenylthiazol-5-yl)oxy)pyridin-2-yl)amino)-N-(methylsulfonyl)nicotinamide
(141) 2-(3-((4-((2-Methoxy-4-phenylthiazol-5-yl)oxy)pyridin-2-yl)amino)phenyl)propan-2-ol
(142) 3-((4-((2-Methoxy-4-phenylthiazol-5-yl)oxy)pyridin-2-yl)amino)benzenesulfonamide
(143) 3-((4-((2-Methoxy-4-phenylthiazol-5-yl)oxy)pyridin-2-yl)amino)benzamide
(144) 3-((4-((2-Methoxy-4-phenylthiazol-5-yl)oxy)pyridin-2-yl)amino)benzoic acid
(145) 3-((4-((4-Phenyl-2-(trifluoromethyl)thiazol-5-yl)oxy)pyridin-2-yl)amino)benzenesulfonamide
(146) 6-((4-((4-Phenyl-2-(trifluoromethyl)thiazol-5-yl)oxy)pyridin-2-yl)amino)nicotinamide
(147) 4-((4-((4-Phenyl-2-(trifluoromethyl)thiazol-5-yl)oxy)pyridin-2-yl)amino)benzamide
(148) N Methyl-4-((4-((4-phenyl-2-(trifluoromethyl)thiazol-5-yl)oxy)pyridin-2-yl)amino)benzamide
(149) 6-((4-((4-Phenyl-2-(trifluoromethyl)thiazol-5-yl)oxy)pyridin-2-yl)amino)nicotinic acid
(150) *N-*Methyl-6-((4-((4-phenyl-2-(trifluoromethyl)thiazol-5-yl)oxy)pyridin-2-yl)amino)nicotinamide
(151) 3-((4-((4-Phenyl-2-(trifluoromethyl)thiazol-5-yl)oxy)pyridin-2-yl)amino)benzoic acid
(152) 4-((4-((4-Phenyl-2-(trifluoromethyl)thiazol-5-yl)oxy)pyridin-2-yl)amino)benzoic acid
(153) 2-(3-((4-((2-(Dimethylamino)-4-phenylthiazol-5-yl)oxy)pyridin-2-yl)amino)phenyl)propan-2-ol
(154) 3-((4-((2-(Dimethylamino)-4-phenylthiazol-5-yl)oxy)pyridin-2-yl)amino)benzenesulfonamide
(155) 3-((4-((2-(Dimethylamino)-4-phenylthiazol-5-yl)oxy)pyridin-2-yl)amino)benzamide
(156) 4-((4-((2-(Dimethylamino)-4-phenylthiazol-5-yl)oxy)pyridin-2-yl)amino)benzenesulfonamide
(157) 4-((4-((2-(Dimethylamino)-4-phenylthiazol-5-yl)oxy)pyridin-2-yl)amino)benzamide
(158) 2-(4-((4-((2-(Dimethylamino)-4-phenylthiazol-5-yl)oxy)pyridin-2-yl)amino)pyridin-2-yl)propan-2-ol
(159) 2-(5-((4-((2-(Dimethylamino)-4-phenylthiazol-5-yl)oxy)pyridin-2-yl)amino)pyridin-2-yl)propan-2-ol
(160) 6-((4-((2-(Dimethylamino)-4-phenylthiazol-5-yl)oxy)pyridin-2-yl)amino)picolinamide
(161) 6-((4-((2-(Dimethylamino)-4-phenylthiazol-5-yl)oxy)pyridin-2-yl)amino)-*N-*methylpicolinamide
(162) Methyl 6-((4-((2-(Dimethylamino)-4-phenylthiazol-5-yl)oxy)pyridin-2-yl)amino)picolinate
(163) 6-((4-((2-(Dimethylamino)-4-phenylthiazol-5-yl)oxy)pyridin-2-yl)amino)nicotinamide
(164) 4-((4-((2-(Dimethylamino)-4-phenylthiazol-5-yl)oxy)pyridin-2-yl)amino)-*N-*methylbenzamide
(165) 6-((4-((2-(Dimethylamino)-4-phenylthiazol-5-yl)oxy)pyridin-2-yl)amino)nicotinic acid
(166) 6-((4-((2-(Dimethylamino)-4-phenylthiazol-5-yl)oxy)pyridin-2-yl)amino)-*N-*methylnicotinamide
(167) 3-((4-((2-(Dimethylamino)-4-phenylthiazol-5-yl)oxy)pyridin-2-yl)amino)benzoic acid
(168) 4-((4-((2-(Dimethylamino)-4-phenylthiazol-5-yl)oxy)pyridin-2-yl)amino)benzoic acid
(169) 6-((4-((2-(Dimethylamino)-4-phenylthiazol-5-yl)oxy)pyridin-2-yl)amino)picolinic acid
(170) Methyl 3-((4-((2-bromo-4-phenylthiazol-5-yl)oxy)pyridin-2-yl)amino)benzoate
(171) 3-((4-((2-Bromo-4-phenylthiazol-5-yl)oxy)pyridin-2-yl)amino)benzoic acid
(172) 3-((4-((2-Bromo-4-phenylthiazol-5-yl)oxy)pyridin-2-yl)amino)benzamide
(173) 6-((4-((2-Cyclopentyl-4-phenylthiazol-5-yl)oxy)pyridin-2-yl)amino)nicotinamide
(174) 6-((4-((2-Cyclopentyl-4-phenylthiazol-5-yl)oxy)pyridin-2-yl)amino)nicotinic acid
(175) 4-((4-((2-Cyclopentyl-4-phenylthiazol-5-yl)oxy)pyridin-2-yl)amino)benzoic acid
(176) 6-((4-((2-Cyclopentyl-4-phenylthiazol-5-yl)oxy)pyridin-2-yl)amino)-*N-*methylnicotinamide
(177) 6-((4-((2-Cyclohexyl-4-phenylthiazol-5-yl)oxy)pyridin-2-yl)amino)nicotinamide
(178) 6-((4-((2-Cyclohexyl-4-phenylthiazol-5-yl)oxy)pyridin-2-yl)amino)nicotinic acid
(179) 4-((4-((2-Cyclohexyl-4-phenylthiazol-5-yl)oxy)pyridin-2-yl)amino)benzoic acid
(180) 6-((4-((2-Cyclohexyl-4-phenylthiazol-5-yl)oxy)pyridin-2-yl)amino)-*N-*methylnicotinamide
(181) 3-((4-((2-Ethyl-4-phenylthiazol-5-yl)oxy)pyridin-2-yl)amino)benzenesulfonamide
(182) 2-(3-((4-((2-Ethyl-4-phenylthiazol-5-yl)oxy)pyridin-2-yl)amino)phenyl)propan-2-ol
(183) 3-((4-((2-Ethyl-4-phenylthiazol-5-yl)oxy)pyridin-2-yl)amino)benzamide
(184) 4-((4-((2-Ethyl-4-phenylthiazol-5-yl)oxy)pyridin-2-yl)amino)benzenesulfonamide
(185) 4-((4-((2-Ethyl-4-phenylthiazol-5-yl)oxy)pyridin-2-yl)amino)benzamide
(186) 2-(4-((4-((2-Ethyl-4-phenylthiazol-5-yl)oxy)pyridin-2-yl)amino)pyridin-2-yl)propan-2-ol
(187) 2-(5-((4-((2-Ethyl-4-phenylthiazol-5-yl)oxy)pyridin-2-yl)amino)pyridin-2-yl)propan-2-ol
(188) *N*-(3-((4-((2-Ethyl-4-phenylthiazol-5-yl)oxy)pyridin-2-yl)amino)phenyl)ethanesulfonamide
(189) 4-((4-((2-Ethyl-4-phenylthiazol-5-yl)oxy)pyridin-2-yl)amino)-*N-*methylbenzamide
(190) 6-((4-((2-Ethyl-4-phenylthiazol-5-yl)oxy)pyridin-2-yl)amino)nicotinamide
(191) 6-((4-((2-Ethyl-4-phenylthiazol-5-yl)oxy)pyridin-2-yl)amino)nicotinate
(192) 6-((4-((2-Ethyl-4-phenylthiazol-5-yl)oxy)pyridin-2-yl)amino)nicotinic acid
(193) 6-((4-((2-Ethyl-4-phenylthiazol-5-yl)oxy)pyridin-2-yl)amino)-*N-*methylnicotinamide
(194) 3-((4-((2-Ethyl-4-phenylthiazol-5-yl)oxy)pyridin-2-yl)amino)benzoic acid
(195) 4-((4-((2-Ethyl-4-phenylthiazol-5-yl)oxy)pyridin-2-yl)amino)benzoic acid
(196) 6-((4-((2-Ethyl-4-phenylthiazol-5-yl)oxy)pyridin-2-yl)amino)-*N-*isopropylnicotinamide
(197) 6-((4-((2-Ethyl-4-phenylthiazol-5-yl)oxy)pyridin-2-yl)amino)pyridin-3-yl)(4-methylpiperazin-1-yl)methanone
(198) 3-Aminopyrrolidin-1-yl)(6-(4-(2-ethyl-4-phenylthiazol-5-yloxy) pyridin-2-ylamino)pyridin-3-yl)methanone
(199) 6-((4-((2-Ethyl-4-phenylthiazol-5-yl)oxy)pyridin-2-yl)amino)-*N-*(2-(pyrrolidin-1-yl)ethyl)nicotinamide
(200) 6-((4-((2-Ethyl-4-phenylthiazol-5-yl)oxy)pyridin-2-yl)amino)pyridin-3-yl)(3-(methylamino)pyrrolidin-1-yl)methanone
(201) 6-((4-((2-Ethyl-4-phenylthiazol-5-yl)oxy)pyridin-2-yl)amino)-*N*-(2-(4-methylpiperazin-1-yl)ethyl)nicotinamide
(202) 6-((4-((2-Ethyl-4-phenylthiazol-5-yl)oxy)pyridin-2-yl)amino)-*N*-(2-(4-i sopropylpiperazin-1-yl)ethyl)nicotinamide
(203) 6-((4-((2-Ethyl-4-phenylthiazol-5-yl)oxy)pyridin-2-yl)amino)-*N*-(2-(4-ethylpiperazin-1-yl)ethyl)nicotinamide
(204) 5-(3-Aminopyrrolidin-1-yl)-*N*-(4-((2-ethyl-4-phenylthiazol-5-yl)oxy)pyridin-2-yl)pyridin-2-amine
(205) *N*¹-(4-((2-Ethyl-4-phenylthiazol-5-yl)oxy)pyridin-2-yl)-*N*⁴-(2-(4-isopropylpiperazin-1-yl)ethyl)benzene-1,4-diamine
(206) *N*¹-(4-((2-Ethyl-4-phenylthiazol-5-yl)oxy)pyridin-2-yl)-*N*⁴-(2-(4-methylpiperazin-1-yl)ethyl)benzene-1,4-diamine
(207) *N*¹-(4-((2-Ethyl-4-phenylthiazol-5-yl)oxy)pyridin-2-yl)-*N*⁴-(2-(4-ethylpiperazin-1-yl)ethyl)benzene-1,4-diamine
(208) *N*¹-(4-((2-Ethyl-4-phenylthiazol-5-yl)oxy)pyridin-2-yl)-*N*⁴-(2-(pyrrolidin-1-yl)ethyl)benzene-1,4-diamine
(209) *N*²-(4-((2-Ethyl-4-phenylthiazol-5-yl)oxy)pyridin-2-yl)-*N*⁵-(2-(4-methylpiperazin-1-yl)ethyl)pyridine-2, 5-diamine
(210) *N*²-(4-((2-Ethyl-4-phenylthiazol-5-yl)oxy)pyridin-2-yl)-*N*⁵-(2-(pyrrolidin-1-yl)ethyl)pyridine-2,5-diamine
(211) *N*²-(4-((2-Ethyl-4-phenylthiazol-5-yl)oxy)pyridin-2-yl)-*N*⁵-(2-(4-ethylpiperazin-1-yl)ethyl)pyridine-2,5-diamine
(212) *N*²-(4-((2-Ethyl-4-phenylthiazol-5-yl)oxy)pyridin-2-yl)-*N*⁵-(2-(4-isopropylpiperazin-1-yl)ethyl)pyridine-2, 5-diamine
(213) *N*-(5-(1*H*-Pyrazol-4-yl)pyridin-2-yl)-4-((2-ethyl-4-phenylthiazol-5-yl)oxy)pyridin-2-amine
(214) *N*-(4-(3-Aminopyrrolidin-1-yl)phenyl)-4-((2-ethyl-4-phenylthiazol-5-yl)oxy)pyridin-2-amine
(215) 4-((2-Ethyl-4-phenylthiazol-5-yl)oxy)-*N*-(4-(3-(methylamino)pyrrolidin-1-yl)phenyl)pyridin-2-amine
(216) Methyl 6-((4-((2-isopropyl-4-phenylthiazol-5-yl)oxy)pyridin-2-yl)amino)picolinate
(217) 6-((4-((2-Isopropyl-4-phenylthiazol-5-yl)oxy)pyridin-2-yl)amino)picolinic acid
(218) 3-((4-((2-Isopropyl-4-phenylthiazol-5-yl)oxy)pyridin-2-yl)amino)benzenesulfonamide
(219) 2-(3-((4-((2-Isopropyl-4-phenylthiazol-5-yl)oxy)pyridin-2-yl)amino)phenyl)propan-2-ol
(220) 3-((4-((2-Isopropyl-4-phenylthiazol-5-yl)oxy)pyridin-2-yl)amino)benzamide
(221) 4-((4-((2-Isopropyl-4-phenylthiazol-5-yl)oxy)pyridin-2-yl)amino)benzenesulfonamide
(222) 4-((4-((2-Isopropyl-4-phenylthiazol-5-yl)oxy)pyridin-2-yl)amino)benzamide
(223) 2-(4-((4-((2-Isopropyl-4-phenylthiazol-5-yl)oxy)pyridin-2-yl)amino)pyridin-2-yl)propan-2-ol
(224) 2-(5-((4-((2-Isopropyl-4-phenylthiazol-5-yl)oxy)pyridin-2-yl)amino)pyridin-2-yl)propan-2-ol
(225) 6-((4-((2-Isopropyl-4-phenylthiazol-5-yl)oxy)pyridin-2-yl)amino)-A-methylpicolinamide
(226) 6-((4-((2-Isopropyl-4-phenylthiazol-5-yl)oxy)pyridin-2-yl)amino)picolinamide
(227) 6-((4-((2-Isopropyl-4-phenylthiazol-5-yl)oxy)pyridin-2-yl)amino)nicotinamide
(228) 6-((4-((2-Isopropyl-4-phenylthiazol-5-yl)oxy)pyridin-2-yl)amino)-A-methylnicotinamide
(229) 4-((4-((2-Isopropyl-4-phenylthiazol-5-yl)oxy)pyridin-2-yl)amino)-A-methylbenzamide
(230) 3-((4-((2-Isopropyl-4-phenylthiazol-5-yl)oxy)pyridin-2-yl)amino)benzoic acid
(231) 4-((4-((2-Isopropyl-4-phenylthiazol-5-yl)oxy)pyridin-2-yl)amino)benzoic acid
(232) 6-((4-((2-Isopropyl-4-phenylthiazol-5-yl)oxy)pyridin-2-yl)amino)nicotinic acid
(233) 3-((4-((2-(Methylamino)-4-(naphthalen-2-yl)thiazol-5-yl)oxy)pyridin-2-yl)amino)benzenesulfonamide
(234) 3-((4-((2-Amino-4-(naphthalen-2-yl)thiazol-5-yl)oxy)pyridin-2-yl)amino)benzenesulfonamide
(235) 3-((4-((2-(Dimethylamino)-4-(naphthalen-2-yl)thiazol-5-yl)oxy)pyridin-2-yl)amino)benzenesulfonamide
(236) 3-((6-((2-Amino-4-phenylthiazol-5-yl)oxy)pyrimidin-4-yl)amino)benzenesulfonamide
(237) 3-((6-((2-Methyl-4-phenylthiazol-5-yl)oxy)pyrimidin-4-yl)amino)benzenesulfonamide
(238) 3-((4-((2-Methyl-4-phenylthiazol-5-yl)oxy)pyrimidin-2-yl)amino)benzenesulfonamide
(239) 3-((6-((2-Amino-4-phenylthiazol-5-yl)oxy)pyrimidin-4-yl)amino)benzenesulfonamide
(240) 3-((6-((2-Amino-4-phenylthiazol-5-yl)oxy)pyrimidin-4-yl)amino)benzenesulfonamide
(241) 6-((4-((2-Ethyl-4-phenylthiazol-5-yl)oxy)pyrimidin-2-yl)amino)picolinamide
(242) 2-((4-((2-Ethyl-4-phenylthiazol-5-yl)oxy)pyrimidin-2-yl)amino)isonicotinamide
(243) 3-((4-((2-Ethyl-4-phenylthiazol-5-yl)oxy)pyrimidin-2-yl)amino)benzamide
(244) 6-((4-((2-Ethyl-4-phenylthiazol-5-yl)oxy)pyrimidin-2-yl)amino)nicotinic acid
(245) 6-((4-((2-Ethyl-4-phenylthiazol-5-yl)oxy)pyrimidin-2-yl)amino)nicotinamide
(246) 4-((4-((2-Ethyl-4-phenylthiazol-5-yl)oxy)pyrimidin-2-yl)amino)benzoic acid
(247) 3-((4-((2-Amino-4-(6-methylpyridin-2-yl)thiazol-5-yl)oxy)pyridin-2-yl)amino)benzenesulfonamide
(248) 4-((4-((2-Amino-4-(6-methylpyridin-2-yl)thiazol-5-yl)oxy)pyridin-2-yl)amino)benzoic acid
(249) 3-((4-((2-Amino-4-(6-methylpyridin-2-yl)thiazol-5-yl)oxy)pyridin-2-yl)amino)benzoic acid
(250) 3-((4-((2-Ethyl-4-(6-methylpyridin-2-yl)thiazol-5-yl)oxy)pyridin-2-yl)amino)benzenesulfonamide
(251) 6-((4-((2-Ethyl-4-(6-methylpyridin-2-yl)thiazol-5-yl)oxy)pyridin-2-yl)amino)nicotinamide
(252) 6-((4-((2-Ethyl-4-(6-methylpyridin-2-yl)thiazol-5-yl)oxy)pyridin-2-yl)amino)nicotinic acid
(253) 6-((4-((2-Ethyl-4-(6-methylpyridin-2-yl)thiazol-5-yl)oxy)pyridin-2-yl)amino)-N-methylnicotinamide
(254) 6-((4-((2-Ethyl-4-(6-methylpyridin-2-yl)thiazol-5-yl)oxy)pyridin-2-yl)amino)picolinic acid
(255) 6-((4-((2-Ethyl-4-(6-methylpyridin-2-yl)thiazol-5-yl)oxy)pyridin-2-yl)amino)picolinamide
(256) 6-((4-((2-Ethyl-4-(6-methylpyridin-2-yl)thiazol-5-yl)oxy)pyridin-2-yl)amino)-N-methylpicolinamide
(257) 3-((4-((2-Ethyl-4-(6-methylpyridin-2-yl)thiazol-5-yl)oxy)pyridin-2-yl)amino)benzoic acid
(258) 3-((4-((2-Ethyl-4-(6-methylpyridin-2-yl)thiazol-5-yl)oxy)pyridin-2-yl)amino)benzamide
(259) 3-((4-((2-Ethyl-4-(6-methylpyridin-2-yl)thiazol-5-yl)oxy)pyridin-2-yl)amino)-*N*-methylbenzamide
(260) 5-((4-((2-Ethyl-4-(6-methylpyridin-2-yl)thiazol-5-yl)oxy)pyridin-2-yl)amino)nicotinic acid
(261) 5-((4-((2-Ethyl-4-(6-methylpyridin-2-yl)thiazol-5-yl)oxy)pyridin-2-yl)amino)nicotinamide
(262) 5-((4-((2-Ethyl-4-(6-methylpyridin-2-yl)thiazol-5-yl)oxy)pyridin-2-yl)amino)-*N*-methylnicotinamide
(263) 4-((4-((2-Ethyl-4-(6-methylpyridin-2-yl)thiazol-5-yl)oxy)pyridin-2-yl)amino)picolinic acid
(264) 4-((4-((2-Ethyl-4-(6-methylpyridin-2-yl)thiazol-5-yl)oxy)pyridin-2-yl)amino)picolinamide
(265) 4-((4-((2-Ethyl-4-(6-methylpyridin-2-yl)thiazol-5-yl)oxy)pyridin-2-yl)amino)-*N*-methylpicolinamide
(266) 2-((4-((2-Ethyl-4-(6-methylpyridin-2-yl)thiazol-5-yl)oxy)pyridin-2-yl)amino)isonicotinic acid
(267) 2-((4-((2-Ethyl-4-(6-methylpyridin-2-yl)thiazol-5-yl)oxy)pyridin-2-yl)amino)isonicotinamide
(268) 2-((4-((2-Ethyl-4-(6-methylpyridin-2-yl)thiazol-5-yl)oxy)pyridin-2-yl)amino)-*N*-methylisonicotinamide
(269)*N-*(2-(Dimethylamino)ethyl)-6-((4-((2-ethyl-4-(6-methylpyridin-2-yl)thiazol-5-yl)oxy)pyridin-2-yl)amino)nicotinamide
(270) *N-*(Cyclopropylmethyl)-6-((4-((2-ethyl-4-(6-methylpyridin-2-yl)thiazol-5-yl)oxy)pyridin-2-yl)amino)nicotinamide
(271) 2-(Dimethylamino)ethyl6-((4-((2-ethyl-4-(6-methylpyridin-2-yl)thiazol-5-yl)oxy)pyridin-2-yl)amino)nicotinate
(272) *N*-(3-((4-((2-ethyl-4-(6-methylpyridin-2-yl)thiazol-5-yl)oxy)pyridin-2-yl)amino)phenyl)methanesulfonamide
(273) 2-(Dimethylamino)ethyl3-(6-((4-((2-ethyl-4-(6-methylpyridin-2-yl)thiazol-5-yl)oxy)pyridin-2-yl)amino)nicotinamido)-5-fluorobenzoate
(274) 3-((4-((2-Cyclopropyl-4-(6-methylpyridin-2-yl)thiazol-5-yl)oxy)pyridin-2-yl)amino)benzamide
(275) 6-((4-((2-Ethyl-4-(6-methylpyridin-2-yl)thiazol-5-yl)oxy)pyridin-2-yl)amino)nicotinamide
(276) 2-((4-((2-Cyclopropyl-4-(6-methylpyridin-2-yl)thiazol-5-yl)oxy)pyridin-2-yl)amino)isonicotinamide
(277) 3-((4-((2-Cyclopropyl-4-(6-methylpyridin-2-yl)thiazol-5-yl)oxy)pyridin-2-yl)amino)benzenesulfonamide
(278) 6-((4-((2-Cyclopropyl-4-(6-methylpyridin-2-yl)thiazol-5-yl)oxy)pyridin-2-yl)amino)picolinamide
(279) 6-((4-((2-Cyclopropyl-4-(6-methylpyridin-2-yl)thiazol-5-yl)oxy)pyridin-2-yl)amino)-*N*-methylpicolinamide
(280) 2-((4-((2-Cyclopropyl-4-(6-methylpyridin-2-yl)thiazol-5-yl)oxy)pyridin-2-yl)amino)-*N*-methylisonicotinamide
(281) 3-((4-((2-Cyclopropyl-4-(6-methylpyridin-2-yl)thiazol-5-yl)oxy)pyridin-2-yl)amino)-N-methylbenzamide
(282) 6-((4-((2-Cyclopropyl-4-(6-methylpyridin-2-yl)thiazol-5-yl)oxy)pyridin-2-yl)amino)-*N*-methylnicotinamide
(283) 3-((4-((2-(Dimethylamino)-4-(6-methylpyridin-2-yl)thiazol-5-yl)oxy)pyridin-2-yl)amino)benzenesulfonamide
(284) 6-((4-((2-(Dimethylamino)-4-(6-methylpyridin-2-yl)thiazol-5-yl)oxy)pyridin-2-yl)amino)nicotinamide
(285) 6-((4-((2-(Dimethylamino)-4-(6-methylpyridin-2-yl)thiazol-5-yl)oxy)pyridin-2-yl)amino)nicotinic acid
(286) 6-((4-((2-(Dimethylamino)-4-(6-methylpyridin-2-yl)thiazol-5-yl)oxy)pyridin-2-yl)amino)-*N*-methylnicotinamide
(287) 3-((4-((2-Isopropyl-4-(6-methylpyridin-2-yl)thiazol-5-yl)oxy)pyridin-2-yl)amino)benzenesulfonamide
(288) 6-((4-((2-Isopropyl-4-(6-methylpyridin-2-yl)thiazol-5-yl)oxy)pyridin-2-yl)amino)nicotinamide
(289) 3-((4-((4-(6-Methylpyridin-2-yl)-2-(trifluoromethyl)thiazol-5-yl)oxy)pyridin-2-yl)amino)benzenesulfonamide
(290) 6-((4-((2-Isopropyl-4-(6-methylpyridin-2-yl)thiazol-5-yl)oxy)pyridin-2-yl)amino)nicotinamide
(291) 3-((4-((4-(6-Methylpyridin-2-yl)-2-(trifluoromethyl)thiazol-5-yl)oxy)pyridin-2-yl)amino)benzamide
(292) 3-((4-((2-(Methylamino)-4-(6-methylpyridin-2-yl)thiazol-5-yl)oxy)pyridin-2-yl)amino)benzenesulfonamide
(293) 6-((4-((2-Cyclopropyl-4-phenyloxazol-5-yl)oxy)pyridin-2-yl)amino)nicotinic acid
(294) 6-((4-((2-Cyclopropyl-4-phenyloxazol-5-yl)oxy)pyridin-2-yl)amino)nicotinamide
(295) 4-((4-((2-Cyclopropyl-4-phenyloxazol-5-yl)oxy)pyridin-2-yl)amino)benzoic acid
(296) 3-((4-((2-Cyclopropyl-4-phenyloxazol-5-yl)oxy)pyridin-2-yl)amino)benzamide
(297) 3-((4-((2-Cyclopropyl-4-phenyloxazol-5-yl)oxy)pyridin-2-yl)amino)-*N-*methylbenzamide
(298) 3-((4-((2-Cyclopropyl-4-phenyloxazol-5-yl)oxy)pyridin-2-yl)amino)benzenesulfonamide
(299) 6-((4-((2-Cyclopropyl-4-phenyloxazol-5-yl)oxy)pyridin-2-yl)amino)picolinamide
(300) 3-((4-((2-Cyclopropyl-4-phenyloxazol-5-yl)oxy)pyridin-2-yl)amino)benzoic acid
(301) 6-((4-((2-Cyclopropyl-4-phenyloxazol-5-yl)oxy)pyridin-2-yl)amino)picolinic acid
(302) 6-((4-((2-Cyclopropyl-4-phenyloxazol-5-yl)oxy)pyridin-2-yl)amino)-*N-*methylpicolinamide
(303) 3-((4-((2-Methyl-4-(6-methylpyridin-2-yl)thiazol-5-yl)oxy)pyridin-2-yl)amino)benzenesulfonamide
(304) 2-(3-((4-((2-Methyl-4-phenylthiazol-5-yl)oxy)pyridin-2-yl)amino)phenyl)acetonitrile
(305) *N*-(3-((1*H*-Tetrazol-5-yl)methyl)phenyl)-4-((2-methyl-4-(6-methylpyridin-2-yl)thiazol-5-yl)oxy)pyridin-2-amine
(306) 3-(3-((4-((2-Methyl-4-(6-methylpyridin-2-yl)thiazol-5-yl)oxy)pyridin-2-yl)amino)phenyl)propanenitrile
(307) *N*-(3-(2-(2*H*-Tetrazol-5-yl)ethyl)phenyl)-4-((2-methyl-4-(6-methylpyridin-2-yl)thiazol-5-yl)oxy)pyridin-2-amine
(308) Methyl 2-methyl-2-(3-((4-((2-methyl-4-(6-methylpyridin-2-yl)thiazol-5-yl)oxy)pyridin-2-yl)amino)phenyl)propanoate
(309) 2-Methyl-2-(3-((4-((2-methyl-4-(6-methylpyridin-2-yl)thiazol-5-yl)oxy)pyridin-2-yl)amino)phenyl)propanoic acid
(310) 4-((4-((2-(Methylamino)-4-phenylthiazol-5-yl)oxy)pyridin-2-yl)amino)benzamide
(311)*N-*(3-((4-((2-Ethyl-4-phenylthiazol-5-yl)oxy)pyridin-2-yl)amino)phenyl)-1,1,1-trifluoromethanesulfonamide and
(312) 4-((2-Ethyl-4-phenylthiazol-5-yl)oxy)-*N*-(5-(3-(methylamino)pyrrolidin-1-yl)pyridin-2-yl)pyridin-2-amine.

In addition, the present disclosure provides a pharmaceutical composition for treating or preventing a cancer disease, including a compound selected from the thiazole derivative compound, a pharmaceutically acceptable salt thereof, a solvate thereof, or a stereoisomer thereof as an active ingredient.

The cancer disease may be selected from the group consisting of, but is not limited to, lung cancer, breast cancer, ovarian cancer, uterine cancer, pancreatic cancer, lung cancer, stomach cancer, liver cancer, colorectal cancer, skin cancer, head or neck cancer, brain cancer, laryngeal cancer, prostate cancer, bladder cancer, esophageal cancer, thyroid cancer, kidney cancer, and rectal cancer.

The cancer disease may be a transforming growth factor beta (TGFβ)-related cancer disease.

The pharmaceutical composition may selectively inhibit transforming growth factor beta receptor 1 (TGFβR1).

In another example embodiment of the present disclosure, the pharmaceutical composition may further include one or more additives selected from the group consisting of suitable carriers, excipients, disintegrating agents, sweeteners, coating agents, swelling agents, lubricants, glydents, flavoring agents, antioxidants, buffers, bacteriostatics, diluents, dispersing agents, surfactants, binders, and antifrictions that are commonly used in the manufacture of pharmaceutical compositions.

Specifically, as carriers, excipients, and diluents, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil may be used, and solid preparations for oral administration include tablets, pills, acids, granules, and capsules, wherein these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. For oral liquid preparations, suspensions, liquids, emulsions, and syrups may be used, and various excipients, such as humectants, sweeteners, fragrances, and preservatives, may be included in addition to the simple diluents that are commonly used, such as water and liquid paraffin. Preparations for parenteral administration include sterile aqueous solutions, non-aqueous solvents, suspensions, emulsions, lyophilized agents, and suppositories. As non-aqueous solvents and suspensions, propylene glycol, polyethylene glycol, vegetable oils such as olive oil, and injectable esters such as ethyl oleate may be used. As a base of suppositories, witepsol, macrogol, tween 61, cacao butter, laurin butter, and glycerogelatin may be used.

According to an example embodiment of the present disclosure, the pharmaceutical composition may be administered to a subject in a conventional manner through the intravenous, intra-arterial, intraperitoneal, intramuscular, intra-arterial, intraperitoneal, intrasternal, percutaneous, nasal, inhaled, topical, rectal, oral, intraocular, or intradermal routes.

The dosage of the active ingredient according to the present disclosure may vary depending on the condition and weight of the subject, the type and severity of disease, the drug form, and the route and duration of administration and may be appropriately selected by those skilled in the art, and the daily dosage may be 0.01 mg/kg to 200 mg/kg, preferably 0.1 mg/kg to 200 mg/kg, more preferably 0.1 mg/kg to 100 mg/kg. The administration may be conducted once a day or divided into several doses, but the scope of the present disclosure is not limited thereby.

In addition, the present disclosure provides a health functional food composition for ameliorating or preventing a cancer disease, including a compound selected from the thiazole derivative compound, a pharmaceutically acceptable salt thereof, a solvate thereof, or a stereoisomer thereof as an active ingredient.

The cancer disease may be selected from the group consisting of, but is not limited to, lung cancer, breast cancer, ovarian cancer, uterine cancer, pancreatic cancer, lung cancer, stomach cancer, liver cancer, colorectal cancer, skin cancer, head or neck cancer, brain cancer, laryngeal cancer, prostate cancer, bladder cancer, esophageal cancer, thyroid cancer, kidney cancer, and rectal cancer.

The health functional food may contain various nutritional supplements, vitamins, minerals (electrolytes), flavor agents such as synthetic flavor agents and natural flavor agents, coloring agents and fillers (cheese, chocolate, etc.), pectic acid and salts thereof, alginate and salts thereof, organic acids, protective colloidal thickeners, pH regulators, stabilizers, preservatives, glycerin, alcohol, and carbonating agents used in carbonated beverages.

Moreover, it may contain pulp for the manufacture of natural fruit juices, synthetic fruit juices, and vegetable drinks. These ingredients may be used independently or in combination. In addition, the health functional food composition may be in any one form of meat, sausage, bread, chocolate, candy, snacks, confectionery, pizza, instant noodles, chewing gum, ice cream, soups, beverages, teas, functional waters, drinks, alcohol, and vitamin complexes.

In addition, the health functional food may further include food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

Here, the content of the active ingredient added to the food in the process of manufacturing the health functional food may be appropriately adjusted if needed, and preferably the active ingredient may be added to be included in an amount of 1 part by weight to 90 parts by weight in 100 parts by weight of the food.

In addition, the present disclosure provides a reagent composition for suppressing expression of transforming growth factor beta (TGFβ), including a compound selected from the thiazole derivative compound, a pharmaceutically acceptable salt thereof, a solvate thereof, or a stereoisomer thereof as an active ingredient.

The reagent composition may selectively inhibit transforming growth factor beta receptor 1 (TGFβR1).

### Modes for Carrying Out the Invention

Hereinafter, the present disclosure will be described in detail through examples to help understanding of the present disclosure. However, examples as described below are merely intended to illustrate the present invention, and the scope of the present disclosure is not limited to the following examples. Examples of the present disclosure are provided to more completely explain the present disclosure to those skilled in the art.

### [Synthesis Example 1] NMR data of novel thiazole derivative compounds

### (1) A-1 : 3-((4-((2-Amino-4-phenylthiazol-5-yl)oxy)pyiridin-2-yl)amino)benzenesulfonamide

¹H-NMR (400 MHz, MeOD-*d₄*): *δ* 8.20-8.21 (1H, m), 8.07 (1H, d, *J =* 5.6 Hz), 7.70-7.72 (2H, m), 7.60-7.64 (1H, m), 7.36-7.44 (2H, m), 7.31 (2H, t, *J=* 7.8 Hz), 7.23-7.24 (1H, m), 6.60 (1H, dd, *J =* 6.0 and 2.4 Hz), 6.49 (1H, d, *J =* 2.4 Hz), 6.54 (1H, d, *J =* 2.0 Hz). * Five protons from NH and two NH₂ were not observed.MS Calcd.: 439.51; MS Found: 440.1 (M+H⁺).

### (2) A-2 : 3-((4-((2-Amino-4-phenylthiazol-5-yl)oxy)pyiridin-2-yl)amino)benzamide

¹H-NMR (400 MHz, DMSO-*d*₆): *δ* 9.26 (1H, s), 8.11 (1H, d, *J =* 6.0 Hz), 8.03 (1H, s), 7.85-7.83 (2H, m), 7.72 (2H, d, *J=* 8.0 Hz), 7.37-7.35 (3H, m), 7.33-7.22 (3H, m), 7.17 (2H, s), 6.63 (1H, dd, *J =* 6.0, 2.4 Hz), 6.52 (1H, d, *J =* 2.4 Hz). MS Calcd.: 403.46; MS Found: 404.1 (M+H⁺).

### (3) A-3 : Methyl 3-((4-((2-amino-4-phenylthiazol-5-yl)oxy)pyridin-2-yl)amino)benzoate

¹H-NMR (400 MHz, MeOD-d4): *δ* 8.16 (1H, t, *J =* 1.8 Hz), 8.04 (1H, d, *J=* 6.0 Hz), 7.72-7.69 (3H, m), 7.58 (1H, d, *J=* 7.6 Hz), 7.35-7.29 (3H, m), 7.25-7.23 (1H, m), 6.58 (1H, dd, *J =* 6.2, 2.2 Hz), 6.48 (1H, d, *J =* 2.4 Hz), 3.88 (3H, s). * Three protons from NH and NH₂ were not observed. MS Calcd.: 418.47; MS Found: 419.1 (M+H⁺).

### (4) A-4 : 4-((4-((2-Amino-4-phenylthiazol-5-yl)oxy)pyiridin-2-yl)amino)benzenesulfonamide

¹H-NMR (400 MHz, DMSO-*d*₆): *δ* 9.53 (1H, s), 8.14 (1H, d, *J =* 6.0 Hz), 7.77 (2H, d, *J =* 8.4 Hz), 7.70-7.64 (4H, m), 7.32 (2H, t, *J =* 7.6 Hz), 7.25-7.19 (1H, m), 7.16 (2H, s), 7.11 (2H, s), 6.70 (1H, dd, *J =* 5.8, 2.2 Hz), 6.56 (1H, d, *J=* 2.4 Hz). MS Calcd.: 439.51; MS Found: 440.1 (M+H⁺).

### (5) A-5 : 4-((4-((2-Amino-4-phenylthiazol-5-yl)oxy)pyridin-2-yl)amino)benzamide

¹H-NMR (400 MHz, DMSO-*d*₆): *δ* 9.41 (1H, s), 8.15 (1H, d, *J =* 6.0 Hz), 7.77-7.67 (7H, m), 7.34 (2H, t, *J =* 7.6 Hz), 7.23 (1H, t, *J =* 7.2 Hz), 7.17 (2H, s), 7.10 (1H, s), 6.68 (1H, dd, *J* = 2.4 Hz, 5.6 Hz), 6.55 (1H, d, *J=* 2.0 Hz). MS Calcd.: 403.46; MS Found: 404.1 (M+H⁺).

### (6) A-6 : 4-Phenyl-5-((2-(pyridin-3-ylamino)pyridin-4-yl)oxy)thiazol-2-amine

¹H-NMR (400 MHz, DMSO-*d*₆): *δ* 9.30 (1H, s), 8.69 (1H, d, *J=* 2.8 Hz), 8.23 (1H, d, *J =* 9.2 Hz ), 8.10 (1H, d, *J =*2.0 Hz) 8.06 (1H, d, *J =*3.6 Hz) 7.70 (2H, d, *J =* 7.6 Hz), 7.34 (2H, t, *J =* 8.0 Hz), 7.24-7.21 (2H, m), 7.15 (2H, s) 6.66 (1H, dd, *J =* 6.0, 2.4 Hz), 6.51 (1H, d, *J=* 2.4 Hz). MS Calcd.: 361.42; Found: 362.1 (M+H⁺).

### (7) A-7 : 5-((2-((6-Fluoropyridin-3-yl)amino)pyridin-4-yl)oxy)-4-phenylthiazol-2-amine

¹H-NMR (400 MHz, DMSO-*d*₆): *δ* 9.37 (1H, s), 8.41 (1H, s), 8.26 (1H, t, *J=* 8.8 Hz), 8.11 (1H, d, *J=* 6.0 Hz), 7.71 (2H, d, *J=* 7.6 Hz), 7.36 (2H, t, *J =* 7.6 Hz), 7.26 (1H, m), 7.22 (2H, s), 7.07 (1H, dd, *J* - 4.4 Hz), 6.68 (1H, dd, *J* - 2.0 Hz), 6.28 (1H, d, *J* - 2.0 Hz). MS Calcd.: 379.41; Found: 380.1 (M+H⁺).

### (8) A-8 : 4-Phenyl-5-((2-(pyridin-4-ylamino)pyridin-4-yl)oxy)thiazol-2-amine

¹H-NMR (400 MHz, DMSO-*d*₆): *δ* 9.57 (1H, s), 8.26 (2H, d, *J =* 6.4 Hz), 8.18 (1H, d, *J =* 6.0 Hz ), 7.69 (2H, d, *J =* 7.2 Hz), 7.59 (2H, d, *J =* 6.4 Hz), 7.34 (2H, t, *J =* 8.0 Hz ), 7.23-7.19 (1H, t, *J =* =7.2 Hz), 7.17(s, 2H), 6.75 (1H, dd, *J =* 6.0, 3.6 Hz), 6.58 (1H, d, *J =* 3.6 Hz). MS Calcd.: 361.42; Found: 362.1 (M+H⁺).

### (9) A-9 : 6-((4-((2-Amino-4-phenylthiazol-5-yl)oxy)pyiridin-2-yl)amino)nicotinamide

¹H-NMR (400 MHz, DMSO-*d*₆): *δ* 10.1 (1H, s), 8.66 (1H, d , *J =* 2.4 Hz), 8.17 (1H, d, *J =* 6.0 Hz), 8.07 (1H, dd, *J =* 8.8, 2.4 Hz), 7.88 (1H, brs), 7.75 (1H, d, *J =* 8.8 Hz), 7.69 (2H, d, *J =* 7.2 Hz), 7.59 (1H, s), 7.33-7.29 (3H, m), 7.22 (1H, t, *J =* 7.6 Hz), 7.13 (2H, s), 6.71 (1H, dd, *J=* 6.0, 2.4 Hz). MS Calcd.: 404.45; Found: 405.1 (M+H⁺).

### (10) A-10 : 3-((4-((2-Amino-4-phenylthiazol-5-yl)oxy)pyridin-2-yl)amino)-N,N-dimethylbenzamide

¹H-NMR (400 MHz, MeOH-D₄): *δ* 8.04 (1H, d, *J* = 6.0 Hz), 7.70 (3H, d, *J* = 7.6 Hz), 7.63 (1H, s), 7.50 (1H, d, *J* =8.0 Hz), 7.32 (4H, t, *J =* 8.4 Hz), 7.24 (1H, d, *J =* 7.2 Hz), 6.95 (1H, d, *J=* 7.6 Hz), 6.57 (1H, dd, *J =* 10.0, 2.4 Hz), 6.47 (1H, d, *J =* 2,0 Hz), 3.08 (3H, s), 3.00 (3H, s). *one protons from NH was not observed. MS Calcd.: 431.51; Found: 432.2 (M+H⁺).

### (11) A-11 : 5-((4-((2-Amino-5-phenylthiazol-5-yl)oxy)pyiridin-2-yl)amino)picolinamide

¹H-NMR (400 MHz, DMSO-*d*₆): *δ* 9.66 (1H, s), 8.71 (1H, d, *J=* 2.0 Hz), 8.36 (1H, d, *J =* 8.8 Hz), 8.17 (1H, d, *J =* 5.6 Hz), 7.91 (1H, d, *J =* 8.8 Hz), 7.85 (1H, s), 7.69 (2H, d, *J =* 7.6 Hz), 7.37 (1H, s), 7.34 (2H, t, *J =* 7.2 Hz), 7.23 (1H, d, *J =* 7.2 Hz), 7.17 (2H, s), 7.74 (1H, d, *J =* 8.0 Hz), 6.57 (1H, s). MS Calcd.: 404.45; Found: 405.1 (M+H⁺).

### (12) A-12 : 3-((4-((2-Amino-4-phenylthiazol-5-yl)oxy)pyridin-2-yl)amino)-N-methylbenzenesulfonamide

¹H-NMR (400 MHz, DMSO-*d*₆): *δ* 9.48 (1H, s), 8.14 (1H, s), 8.12 (1H, d, *J =* 5.6 Hz), 7.85 (1H, d, *J=* 7.6 Hz), 7.69 (2H, d, *J =* 8.0 Hz), 7.44 (1H, t, *J =* 8.0 Hz), 7.36-7.30 (3H, m), 7.23 (2H, d, *J=* 7.2 Hz), 7.16 (2H, s), 6.68(1H, dd, *J =* 5.6, 2.0 Hz), 6.51 (1H, d, *J =* 2.0 Hz), 2.39 (3H, d, *J=* 5.2 Hz). MS Calcd.: 453.54; Found: 454.1 (M+H⁺).

### (13) A-13 : 3-((4-((2-Amino-4-phenylthiazol-5-yl)oxy)pyridin-2-yl)amino)-N,N-dimethylbenzenesulfonamide

¹ H-NMR (400 MHz, DMSO-*d*₆): *δ* 9.52 (1H, s), 8.12-8.10 (2H, m), 7.93 (1H, d, *J =* 10.0 Hz), 7.69 (2H, d, *J=* 7.2 Hz), 7.48 (1H, t, *J=* 8.0 Hz), 7.34 (2H, t, *J =* 7.2 Hz), 7.23 (2H, t, *J=* 7.6 Hz), 7.16 (2H, s), 6.68 (1H, dd, *J =* 5.6, 2.0 Hz), 6.50 (1H, d, *J =* 2.4 Hz), 2,58 (6H, s). MS Calcd.: 467.56; Found: 468.1 (M+H⁺).

### (14) A-14 : N-(3-((4-((2-Amino-4-phenylthiazol-5-yl)oxy)pyiridin-2-yl)amino)phenyl)methanesulfonamide

¹H-NMR (400 MHz, DMSO-*d*₆): *δ* 9.63 (1H, s), 9.18 (1H, s), 8.06 (1H, d, *J =* 6.0 Hz), 7.69 (2H, d, *J =* 7.2 Hz), 7.46-7.44 (2H, m), 7.34 (2H, t, *J* =7.2 Hz ), 7.23 (1H, t, *J =* 7.6 Hz), 7.15-7.11 (3H, m), 6.70 (1H, d, *J =* 9.2 Hz), 6.61 (1H, dd, *J =* 6.0, 2.4 Hz), 6.50 (1H, d, *J =* 1.6 Hz), 2.94 (3H, s). MS Calcd.: 453.54; Found: 454.1 (M+H⁺).

### (15) A-15 : 5-((2-((3-(Methylsulfonyl)phenyl)amino)pyridin-4-yl)oxy)-4-phenylthiazol-2-amine

¹H-NMR (400 MHz, DMSO-*d*₆): *δ* 9.55 (1H, s), 8.27 (1H, s), 8.14 (1H, d, *J =* 6.0 Hz), 7.91 (1H,d, *J=* 9.2 Hz), 7.69 (2H, d, *J =* 7.2 Hz), 7.50 (1H, t, *J =* 8.0 Hz), 7.38-7.30 (3H, m), 7.23 (1H, t, *J=* 7.6 Hz), 7.16 (2H, s), 6.69 (1H, dd, *J =* 6.0, 2.4 Hz), 6.52 (1H, d, *J=* 2.0 Hz), 3.14 (3H, s). MS Calcd.: 438.52; Found: 439.1 (M+H⁺).

### (16) A-16 : N-(3-((4-((2-Amino-4-phenylthiazol-5-yl)oxy)pyiridin-2-yl)amino)phenyl)cyclopropanesulfonamide

¹H-NMR (400 MHz, DMSO-d₆): *δ* 9.60 (1H, s), 9.17 (1H, s), 8.06 (1H, d, *J* = 5.6 Hz), 7.69 (2H, d, *J =* 7.2 Hz), 7.52 (1H, s), 7.44 (1H, d, *J =* 8.4 Hz), 7.34 (2H, t, *J =* 7.2 Hz), 7.23 (1H, t, *J=* 7.6 Hz), 7.14-7.10 (3H, m), 6.72 (1H, d, *J=* 9.2 Hz), 6.60 (1H, dd, *J =* 5.6, 2.0 Hz), 6.50 (1H, d, *J =* 1.6 Hz), 2.57 (1H, q, *J =* 4.8 Hz), 0.93-0.83 (4H, m). MS Calcd.: 479.57; Found: 480.1 (M+H⁺).

### (17) A-17 : 4-Phenyl-5-((2-((3-((trifluoromethyl)sulfonyl)phenyl)amino)pyridin-4-yl)oxy)thiazol-2-amine

¹H-NMR (400 MHz, DMSO-*d*₆): *δ* 9.79 (1H, s), 8.64 (1H, s), 8.18 (1H, d, *J =* 6.0 Hz), 8.07 (1H, d, *J=* 7.2 Hz), 7.69-7.65 (3H, m), 7.55 (1H, d, *J=* 7.2 Hz), 7.34 (2H, t, *J* =7.6 Hz), 7.23 (1H, d, *J =* 7.2 Hz), 7.18 (2H, s), 6.74 (1H, dd, *J =*6.0*,* 4.0 Hz), 6.53 (1H, d, *J =* 5.6 Hz). MS Calcd.: 492.49; Found: 493.0 (M+H⁺).

### (18) A-18 : 3-((4-((2-Amino-4-phenylthiazol-5-yl)oxy)pyiridin-2-yl)amino)benzenesulfonic acid

¹H-NMR (400 MHz, DMSO-*d*₆): *δ* 9.56 (1H, brs), 8.03 (1H, d, *J =* 6.0 Hz), 7.68 (3H, d, *J=* 6.8 Hz), 7.54 (1H, brs), 7.36 (2H, t, *J =* 7.6 Hz), 7.25-7.23 (4H, m), 6.70 (1H, d, *J =* 4.8 Hz), 6.56 (1H, s). * Two protons from OH and NH were no observed. MS Calcd.: 440.50; Found: 441.1 (M+H⁺).

### (19)A-19 : 5-((2-((3-((Methylsulfonyl)methyl)phenyl)amino)pyridin-4-yl)oxy)-4-phenylthiazol-2-amine

¹H-NMR (400 MHz, DMSO-*d*₆): *δ* 9.21 (1H, s), 8.07 (1H, d, *J=* 5.2 Hz), 7.69 (3H, d, *J =* 7.6 Hz), 7.57 (1H, s), 7.34 (2H, t, *J =* 7.2 Hz), 7.21-7.25 (2H, m), 7.14 (2H, s), 6.90 (1H, d, *J =* 8.0 Hz), 6.61 (1H, dd, *J =* 6.0, 3.6 Hz), 4.38 (2H, s), 2.88 (3H, s). MS Calcd.: 452.5; Found: 453.1 (M+H⁺).

### (20) A-20 : 3-((4-((2-Amino-4-phenylthiazol-5-yl)oxy)pyiridin-2-yl)amino)-N-cyclopropylbenzenesulfonamide

¹H-NMR (400 MHz, DMSO-*d*₆): *δ* 9.48 (1H, s), 8.19 (1H, s), 8.12 (1H, d, *J =* 5.6 Hz), 7.84 (2H, m), 7.69 (2H, d, *J =* 7.6 Hz), 7.42 (1H, t, *J =* 7.6 Hz), 7.34 (2H, t, *J =* 7.6 Hz), 7.27-7.21 (2H, m), 7.16 (2H, s), 6.67 (1H, d, *J =* 3.6 Hz), 6.51 (1H, s), 2.06 (1H, m), 0.45-0,44 (2H, m), 0.37 (2H, m). MS Calcd.: 479.11; Found: 480.1 (M+H⁺).

### (21) A-21 : 5-((2-((3-Fluorobenzyl)amino)pyridin-4-yl)oxy)-4-phenylthiazol-2-amine

¹H-NMR (400 MHz, DMSO-*d*₆): *δ* 7.84 (1H, d, *J =* 6.0 Hz), 7.62 (2H, d, *J =* 7.2 Hz), 7.30-7.22 (4H, m), 7.07 (1H, d, *J=* 7.6 Hz), 7.00 (1H, d, *J=* 9.6 Hz), 6.92 (1H, t, *J=* 7.6 Hz), 6.39 (1H ,dd, *J=* 6.0, 2.0 Hz), 6.12 (1H, d, *J=* 2.0 Hz), 4.15 (2H, s). *Three protons from NH and NH₂ were not observed. MS Calcd.:392.45; Found:393.1(M+H⁺).

### (22) A-22 : 2-((4-((2-Amino-4-phenylthiazol-5-yl)oxy)pyiidin-2-yl)amino)isonicotinamide

¹H-NMR (400 MHz, DMSO-*d*₆): *δ* 9.92 (1H, s), 8.24 (1H, d, *J=* 5.2 Hz), 8.15 (1H, d, *J =* 5.6 Hz), 8.06 (2H, d, *J =* 6.4Hz), 7.69 (2H, d, *J =* 7.2Hz), 7.60 (1H, s), 7.55 (1H, s), 7.33 (2H, t, *J =* 7.6Hz), 7.22 (2H, t, *J =* 8.0 Hz), 7.12 (2H, s), 6.69 (1H, dd, *J =* 8.4, 2.4 Hz). MS Calcd.: 404.45; Found: 405.1 (M+H⁺).

### (23) A-23 : 3-((4-((2-Amino-4-phenylthiazol-5-yl)oxy)pyiridin-2-yl)amino)-N-methylbenzamide

¹H-NMR (400 MHz, DMSO-*d*₆): *δ* 9.25 (1H, s), 8.31-8.28 (1H, m), 8.09 (1H, d, *J* = 6.0 Hz), 7.98 (1H, s), 7.81 (1H, s), 7.69 (2H, d, *J=* 7.2 Hz), 7.32 (2H, t, *J=* 7.6 Hz), 7.27 (2H, d, *J =* 5.2 Hz), 7.22 (1H, t, *J =* 7.4 Hz), 7.15 (2H, s), 6.61 (1H, dd, *J =* 6.0, 2.4 Hz), 6.50 (1H, d, *J=* 2.0 Hz), 2.73 (3H, d, *J =* 4.4 Hz). MS Calcd.: 417.48; MS Found: 418.1 (M+H⁺).

### (24) A-24 : 5-((2-((2-Methoxypyridin-4-yl)amino)pyridin-4-yl)oxy)-4-phenylthiazol-2-amine

¹H-NMR (400 MHz, DMSO-*d*₆): *δ* 9.53 (1H, s), 8.17 (1H, d, *J=* 6.0 Hz), 7.86 (1H, d, *J =* 5.6 Hz), 7.68 (2H, d, *J =* 7.6 Hz), 7.32 (3H, t, *J =* 7.6 Hz), 7.22 (1H, t, *J =* 7.4 Hz), 7.17 (2H, s), 6.98 (1H, dd, *J =* 5.2, 2.0 Hz), 6.74 (1H, dd, *J =* 6.0, 2.0 Hz), 6.56 (1H, d, *J =* 2.4 Hz). 3.76 (3H, s). MS Calcd.: 391.45; MS Found: 392.1 (M+H⁺).

### (25) A-25 : 5-((2-(Benzylamino)pyridin-4-yl)oxy)-4-phenylthiazol-2-amine

¹H-NMR (400 MHz, MeOD): *δ* 7.83(1H, d, *J=* 6.0 Hz), 7.64 (2H, d, *J=* 7.2 Hz), 7.34 (1H, d, *J=* 4.4 Hz), 7.28-7.23 (5H, m), 7.21-7.20 (2H, m), 6.36 (1H, d, *J=* 6.4 Hz), 6.14 (1H, d, *J=* 2.0 Hz), 4.39 (2H, d, *J=* 8.0 Hz). *Three protons from NH and NHBoc were not observed MS Calcd.: 374.46; Found: 375.1 (M+H⁺).

### (26) A-26 : 4-Phenyl-5-((2-((pyridin-4-ylmethyl)amino)pyridin-4-yl)oxy)thiazol-2-amine

¹H-NMR (400 MHz, DMSO-*d*₆): *δ* 8.43 (2H, d, *J =* 5.6 Hz), 7.85 (1H, d, *J =* 5.6 Hz), 7.67 (2H, d, *J=* 7.2 Hz), 7.33-7.29 (3H, m), 7.23-7.21 (3H, m), 7.09 (2H, s), 6.34 (1H, dd, *J =* 5.6 2.0 Hz), 6.22 (1H, d, *J =* 2.0 Hz), 4.44 (2H, d, *J =* 6.0 Hz). MS Calcd.: 375.45; Found: 376.1 (M+H⁺).

### (27) A-27 : 5-((2-((2-Chloropyridin-4-yl)amino)pyridin-4-yl)oxy)-4-phenylthiazol-2-amine

¹H-NMR (400 MHz, MeOH-d₄): *δ* 8.20 (1H, d, *J=* 5.6 Hz), 8.00 (1H, d, *J=* 6.0 Hz), 7.94 (1H, d, *J =* 1.6 Hz), 7.70 (2H, d, *J =* 8.0 Hz), 7.43 (1H, dd, *J =* 6.0, 2.0 Hz), 7.30 (2H, t, *J* = 7.6 Hz), 7.22 (1H, t, *J =* 7.6 Hz), 6.74 (1H, dd, *J =* 6.2, 2.2 Hz), 6.56 (1H, d, *J =* 2.0 Hz). * Three protons from NH and NH₂ were not observed. MS Calcd.: 395.87; MS Found: 396.1 (M+H⁺).

### (28) A-28 : 4-Phenyl-5-((2-((pyridin-3-ylmethyl)amino)pyridin-4-yl)oxy)thiazol-2-amine

¹H-NMR (400 MHz, MeOH-d₄): *δ* 8.46 (1H, s), 8.38 (1H, d, *J =* 4.4 Hz), 7.85 (1H, d, *J =* 5.6 Hz), 7.75 (1H, d, *J =* 7.6 Hz), 7.65 (2H, d, *J =* 7.6 Hz), 7.35 (1H, dd, *J =* 8.0, 5.2 Hz), 7.31 (2H, t, *J =* 8.8 Hz), 7.24 (1H, t, *J =* 10.0 Hz), 6.38 (1H, dd, *J =* 6.0, 2.4 Hz), 6.17 (1H, d, *J =* 2.4 Hz), 4.50 (2H, s). * Three protons from NH and NH₂ were not observed. MS Calcd.: 375.45; MS Found: 376.1 (M+H⁺).

### (29) A-29 : 3-((4-((2-Amino-4-phenylthiazol-5-yl)oxy)pyiridin-2-yl)amino)phenyl)methanesulfonamide

¹H-NMR (400 MHz, DMSO-*d*₆): *δ* 9.21 (1H, brs), 8.06 (1H, d, *J=* 6.0 Hz), 7.69 (3H, d, *J =* 7.6 Hz), 7.51 (1H, s), 7.34 (2H, t, *J =* 7.2 Hz), 7.23-7.19 (2H, m), 7.14 (2H, brs), 6.87 (1H, d, *J=* 7.6 Hz), 6.80 (2H, s), 6.60 (1H, d, *J=* 6.0 Hz), 6.52 (1H, d, *J=* 2.0 Hz), 4.16 (2H, s). MS Calcd.: 453.54; Found: 454.1 (M+H⁺).

### (30) A-30 : 5-((2-((2-Fluoropyridin-4-yl)amino)pyridin-4-yl)oxy)-4-phenylthiazol-2-amine

¹H-NMR (400 MHz, DMSO-*d*₆): *δ* 9.91 (1H, s), 8.23 (1H, d, *J=* 6.0 Hz), 7.91 (1H, d, *J=* 5.6 Hz), 7.68 (2H, d, *J=* 7.6 Hz), 7.61 (1H, s), 7.33 (2H, t, *J=* 7.2 Hz), 7.23 (2H, d, *J=* 7.2 Hz), 7.17 (2H, s), 6.80 (1H, d, *J=* 6.0 Hz), 6.60 (1H, d, *J=* 2.4 Hz). MS Calcd.: 379.41; Found: 380.1 (M+H⁺).

### (31) A-31 : 5-((2-((6-Methoxypyridin-3-yl)amino)pyridin-4-yl)oxy)-4-phenylthiazol-2-amine

¹H-NMR (400 MHz, DMSO-*d*₆): *δ* 9.01 (1H, s), 8.31 (1H, d, *J=* 2.8 Hz), 8.02 (1H, d, *J =* 6.0 Hz), 7.94 (1H, dd, *J =* 8.8, 2.4 Hz), 7.69 (2H, d, *J =* 7.2 Hz), 7.34 (2H, t, *J =* 7.2 Hz), 7.21 (1H, t, *J =* 7.2 Hz), 7.13 (2H, s), 6.72 (1H, d, *J =* 8.8 Hz), 6.56 (1H, d, *J =* 6.0 Hz), 6.39 (1H, s), 3.76 (3H, s). MS Calcd.: 391.45; Found: 392.1 (M+H⁺).

### (32) A-32 : 5-((4-((2-Amino-4-phenylthiazol-5-yl)oxy)pyiridin-2-yl)amino)nicotinamide

¹H-NMR (400 MHz, DMSO-*d*₆): *δ* 9.46 (1H, s), 8.82 (1H, d, *J=* 2.4 Hz), 8.53 (2H, d, *J=* 9.2 Hz), 8.14 (1H, d, *J=* 6.0 Hz), 8.04 (1H, s), 7.70 (2H, d, *J=* 8.0 Hz), 7.51 (1H, s), 7.34 (2H, t, *J =* 7.2 Hz), 7.23 (1H, t, *J =* 7.6 Hz), 7.17 (2H, s), 6.69 (1H, dd, *J =*6.0, 2.4 Hz), 6.53 (1H, d, *J=* 2.0 Hz). MS Calcd.: 404.45; Found: 405.1 (M+H⁺).

### (33) A-33 : 6-((4-((2-Amino-4-phenylthiazol-5-yl)oxy)pyiridin-2-yl)amino)picolinamide

¹H-NMR (400 MHz, DMSO-*d*₆): *δ* 9.89 (1H, s), 8.16 (1H, d, *J=* 5.6 Hz), 7.96 (1H, d, *J=* 8.4 Hz), 7.82 (1H, t, *J=* 7.2 Hz), 7.69 (2H, d, *J=* 7.6 Hz), 7.52-7.45 (3H, m), 7.33-7.27 (3H, m), 7.22 (1H, t, *J* = 7.6 Hz), 7.12 (2H, s), 6.72 (1H, dd, *J =* 4.2, 2.4 Hz). MS Calcd.: 404.45; Found: 405.1 (M+H⁺).

### (34) A-34 : 5-((2-((3-(Ethylsulfonyl)phenyl)amino)pyridin-4-yl)oxy)-4-phenylthiazol-2-amine

¹H-NMR (400 MHz, DMSO-*d*₆): *δ* 9.55 (1H, s), 8.22 (1H, s), 8.14 (1H, d, *J =* 6.4 Hz), 7.93 (1H, d, *J=* 8.0 Hz), 7.69 (2H, d, *J=* 7.2 Hz), 7.50 (1H, t, *J=* 8.0 Hz), 7.34 (3H, t, *J=* 7.6 Hz), 7.23 (1H, t, *J=* 7.6 Hz), 7.15 (2H, s), 6.69 (1H, dd, *J=* 5.6, 2.4 Hz), 6.52 (1H, d, *J=* 2.0 Hz), 3.22 (2H, q, *J =* 7.6 Hz), 1.90 (3H, t, *J =* 7.2 Hz). MS Calcd.: 425.55; Found: 453.1 (M+H⁺).

### (35) A-35 : 5-((2-((6-Chloropyridin-3-yl)amino)pyridin-4-yl)oxy)-4-phenylthiazol-2-amine

¹H-NMR (400 MHz, DMSO-*d*₆): *δ* 9.46 (1H, s), 8.57 (1H, d, *J=* 2.8 Hz), 8.22 (1H, d, *J=* 8.4 Hz), 8.12, (1H, d, *J=* 5.6 Hz), 7.69 (2H, d, *J=* 7.2 Hz), 7.36-7.30 (3H, m), 7.21 (1H, m), 7.16 (2H, s), 6.70 (1H, dd, *J=* 8.4, 2.4 Hz), 6.50 (1H, d, *J=* 1.6 Hz). MS Calcd.: 395.87; Found: 396.0 (M+H⁺).

### (36) A-36 : N-(3-((4-((2-Amino-4-phenylthiazol-5-yl)oxy)pyridin-2-yl)amino)phenyl)-1,1,1-trifluoromethanesulfonamide

¹H-NMR (400 MHz, DMSO-*d*₆): *δ* 9.35 (1H, brs), 8.08 (1H, d, *J=* 5.6 Hz), 7.68 (2H, d, *J=* 7.2 Hz), 7.63 (1H, s), 7.54 (1H, d, *J=* 8.0 Hz), 7.33 (2H, t, *J=* 7.8 Hz), 7.22-7.26 (3H, m), 6.76 (1H, d, *J =* 8.0 Hz), 6.65 (1H, d, *J =* 6.0 Hz), 6.52 (1H, s). *Two protons from NH was not observed. MS Calcd.: 507.51; MS Found: 508.1 (M+H⁺).

### (37) A-37 : 5-((2-((1-Methyl-1H-pyrazol-4-yl)amino)pyridin-4-yl)oxy)-4-phenylthiazol-2-amine

¹H-NMR (400 MHz, DMSO-*d*₆): *δ* 8.85 (1H, s), 8,01 (1H, d, *J=* 6.0 Hz), 7.84 (1H, s), 7.68 (2H, d, *J =* 7.2 Hz), 7.33-7.28 (3H, m), 7.22 (1H, t, *J =* 7.2 Hz), 7.12 (2H, s), 6.45 (1H, dd, *J =* 5.6, 2.0 Hz), 6.28 (1H, d, *J =* 2.4 Hz), 3.74 (3H, s). MS Calcd.: 364.42; Found: 365.1 (M+H⁺).

### (38) A-38 : 5-((2-((1H-Pyrazol-4-yl)amino)pyridin-4-yl)oxy)-4-phenylthiazol-2-amine

¹H-NMR (400 MHz, DMSO-*d*₆): *δ* 12.4 (1H, brs), 8.94 (1H, s), 8.00 (1H, d, *J =* 6.0 Hz), 7.68 (3H, d, *J =* 7.2 Hz), 7.32 (3H, t, *J =* 7.6 Hz), 7.21 (1H, t, *J =* 7.2 Hz), 7.12 (2H, s), 6.44 (1H, dd, *J=* 6.0, 2.0 Hz), 6.28 (1H, d, *J=* 1.6 Hz). MS Calcd.: 350.40; MS Found: 351.1 (M+H⁺).

### (39) A-39 : 4-((4-((2-Amino-4-phenylthiazol-5-yl)oxy)pyiridin-2-yl)amino)benzoic acid

¹H-NMR (400 MHz, DMSO-*d*₆): *δ* 12.4 (1H, brs), 9.55 (1H, s), 8.17 (1H, d, *J =* 5.6 Hz), 7.82 (2H, d, *J =* 8.4 Hz), 7.76-7.70 (4H, m), 7.35 (2H, t, *J =* 7.6 Hz), 7.26-7.22 (1H, m), 7.18 (2H, s), 6.72 (1H, dd, *J =* 6.0, 2.4 Hz), 6.59 (1H, d, *J =* 2.4 Hz). MS Calcd.: 404.44; MS Found: 405.1 (M+H⁺).

### (40) A-40 : 3-((4-((2-Amino-4-phenylthiazol-5-yl)oxy)pyridin-2-yl)amino)benzoic acid

¹H-NMR (400 MHz, MeOD-d₄): *δ* 9.14 (1H, s), 8.09 (1H, d, *J =* 5.6 Hz), 7.78 (1H, d, *J =* 8.8 Hz), 7.71 (2H, d, *J =* 8.0 Hz), 7.40-7.33 (3H, m), 7.24 (2H, t, *J =* 7.6 Hz), 7.16 (2H, s), 6.59 (1H, d, *J =* 4.0 Hz), 6.51 (1H, d, *J =* 2.4 Hz). * Two protons from NH and OH were not observed. MS Calcd.: 404.44; MS Found: 405.1 (M+H⁺).

### (41) A-41 : 2-(3-((4-((2-Amino-4-phenylthiazol-5-yl)oxy)pyiridin-2-yl)amino)phenyl)propan-2-ol

¹H-NMR (400 MHz, CDCl₃): *δ* 8.08 (1H, d, *J =* 6.0 Hz), 7.74 (2H, d, *J=* 8.0 Hz), 7.38 (1H, s), 7.34-7.29 (3H, m), 7.26-7.25 (1H, m), 7.13 (1H, d, *J =* 8.0 Hz), 7.09 (1H, d, *J =* 8.0 Hz), 6.70 (1H, s), 6.59 (1H, d, *J =* 1.6 Hz), 6.54 (1H, dd, *J =* 5.4, 2.2 Hz), 4.83 (1H, s), 1.55 (6H, s). * A proton from OH was not observed. MS Calcd.: 418.51; MS Found: 419.2(M+H⁺).

### (42) A-42 : 2-(4-((4-((2-Amino-4-phenylthiazol-5-yl)oxy)pyiridin-2-yl)amino)pyiridin-2-yl)propan-2-ol

¹H-NMR (400 MHz, DMSO-*d*₆): *δ* 9.59 (1H, s), 8.21-8.19 (2H, m), 7.71-7.67 (4H, m), 7.34 (2H, t, *J =* 7.6 Hz), 7.24 (1H, t, *J =* 7.6 Hz), 7.18 (2H, s), 6.74 (1H, dd, *J =* 5.6, 2.0 Hz), 6.62 (1H, d, *J* = 2.4 Hz), 5.10 (1H, s), 1.40 (6H, s). MS Calcd.: 419.50; MS Found: 420.2(M+H⁺).

### (43) A-43 : 2-(5-((4-((2-Amino-4-phenylthiazol-5-yl)oxy)pyridin-2-yl)amino)pyridin-2-yl)propan-2-ol

¹H-NMR (400 MHz, DMSO-*d*₆): *δ* 9.21 (1H, s), 8.58 (1H, d, *J =* 2.0 Hz), 8.07 (2H, dd, *J =* 8.4, 2.8 Hz), 7.69 (2H, d, *J =* 8.4 Hz), 7.49 (1H, d, *J =* 8.8 Hz), 7.33 (2H, t, *J =* 7.6 Hz), 7.22 (1H, t, *J =* 7.4 Hz), 7.14 (2H, s), 6.62 (1H, dd, *J =* 6.0, 2.4 Hz), 6.48 (1H, dd, *J =* 2.0 Hz), 5.04 (1H, s), 1.38 (6H, s). MS Calcd.: 419.50; MS Found: 420.2(M+H⁺).

### (44) A-44 : 2-(3-((4-((2-(Cyclopropylamino)-4-phenylthiazol-5-yl)oxy)pyridin-2-yl)amino)phenyl)propan-2-ol

¹H-NMR (400 MHz, DMSO-*d*₆): *δ* 9.06 (1H, s), 8.17 (1H, d, *J=* 1.2 Hz), 8.08 (1H, d, *J =* 5.6 Hz), 7.73 (2H, dd, *J =* 8.4, 1.2 Hz), 7.64-7.62 (1H, m), 7.51 (1H, t, *J=* 2.0 Hz), 7.36 (2H, t, *J* = 7.6 Hz), 7.27-7.22 (1H, m), 7.15 (1H, t, *J =* 8.0 Hz), 6.97 (1H, d, *J =* 8.4 Hz), 6.59 (1H, dd, *J =* 5.8, 2.2 Hz), 6.51 (1H, d, *J =* 2.4 Hz), 4.92 (1H, s), 2.60-2.52 (1H, m), 0.75-0.73 (2H, m), 0.57-0.55 (2H, m). MS Calcd.: 458.58; MS Found: 459.2 (M+H⁺).

### (45) A-45 : 3-((4-((2-(Cyclopropylamino)-4-phenylthiazol-5-yl)oxy)pyridin-2-yl)amino)benzenesulfonamide

¹H-NMR (400 MHz, CDCl₃): *δ* 9.14 (1H, d, *J=* 5.6 Hz), 7.93 (1H, t, *J =* 1.6 Hz), 7.72 (2H, d, *J=* 8.0 Hz), 7.52-7.43 (1H, m), 7.42-7.39 (2H, m), 7.33-7.29 (2H, m), 7.26-7.23 (1H, m), 6.78 (1H, brs), 6.66 (1H, dd, *J =* 6.0, 2.0 Hz), 6.53 (1H, d, *J =* 2.0 Hz), 5.66 (1H, s), 4.94 (2H, s), 2.68-2.61 (1H, m), 0.82-0.73 (2H, m), 0.74-0.71 (2H, m). MS Calcd.: 479.57; MS Found: 480.1 (M+H⁺).

### (46) A-46 : 3-((4-((2-(Cyclopropylamino)-4-phenylthiazol-5-yl)oxy)pyridin-2-yl)amino)benzamide

¹H-NMR (400 MHz, DMSO-*d*₆): *δ* 9.24 (1H, s), 8.18 (1H, s), 8.12 (1H, d, *J=* 6.0 Hz), 8.02 (1H, s), 7.84-7.82 (2H, m), 7.73 (2H, d, *J=* 7.2 Hz), 7.38-7.34 (3H, m), 7.31-7.25 (3H, m), 6.64 (1H, dd, *J=* 5.6, 2.4 Hz), 6.52 (1H, d, *J=* 2.0 Hz), 2.60-2.52 (1H, m), 0.77-0.73 (2H, m), 0.58-0.54 (2H, m). MS Calcd.: 443.52; MS Found: 444.2 (M+H⁺).

### (47) A-47 : 4-((4-((2-(Cyclopropylamino)-4-phenylthiazol-5-yl)oxy)pyridin-2-yl)amino)benzamide

¹H-NMR (400 MHz, DMSO-*d*₆): *δ* 9.40 (1H, s), 8.19 (1H, d, *J=* 1.6 Hz), 8.16 (1H, d, *J=* 6.0 Hz), 7.80-7.73 (5H, m), 7.69 (2H, d, *J=* 8.4 Hz), 7.36 (2H, t, *J=* 7.4 Hz), 7.28-7.24 (1H, m), 7.11 (1H, brs), 6.70 (1H, dd, *J =* 5.8, 2.2 Hz), 6.56 (1H, d, *J =* 2.4 Hz), 2.60-2.55 (1H, m), 0.77-0.72 (2H, m), 0.58-0.55 (2H, m). MS Calcd.: 443.52; MS Found: 444.2 (M+H⁺).

### (48) A-48 : 3-((4-((2-(Cyclopropylamino)-4-phenylthiazol-5-yl)oxy)pyridin-2-yl)amino)benzoic acid

¹H-NMR (400 MHz, DMSO-*d*₆): *δ* 9.33 (1H, s), 8.24 (1H, s), 8.18 (1H, s), 8.14 (1H, d, *J=* 6.0 Hz), 7.89 (1H, d, *J=* 9.2 Hz), 7.73 (1H, d, *J=* 8.0 Hz), 7.45 (1H, d, *J=* 8.0 Hz), 7.38-7.33 (2H, m), 7.28-7.24 (1H, m), 6.67 (1H, dd, *J=* 7.8, 1.0 Hz), 6.52 (1H, d, *J=* 1.6 Hz), 2.69-2.64 (1H, m), 0.77-0.71 (2H, m), 0.58-0.54 (2H, m). * Three protons from NH and OH were not observed. MS Calcd.: 444.51; MS Found: 445.1 (M+H⁺).

### (49) A-49 : 4-((4-((2-(Cyclopropylamino)-4-phenylthiazol-5-yl)oxy)pyridin-2-yl)amino)benzoic acid

¹H-NMR (400 MHz, MeOH-d4): *δ* 7.96 (1H, d, *J =* 5.6 Hz), 7.78-7.75 (3H, m), 7.63 (1H, d, *J=* 8.4 Hz), 7.36 (2H, d, *J=* 8.8 Hz), 7.22 (2H, t, *J =* 7.4 Hz), 7.16-7.14 (1H, m), 6.49 (1H, dd, *J=* 6.0, 2.4 Hz), 6.41 (1H, d, *J=* 2.4 Hz), 2.53-2.47 (1H, m), 0.71-0.69 (2H, m), 0.57-0.54 (2H, m). * Four protons from NH₂ and OH were not observed. MS Calcd.: 444.51; MS Found: 445.2 (M+H⁺).

### (50) A-50 : 2-(3-((4-((2-(Methylamino)-4-phenylthiazol-5-yl)oxy)pyridin-2-yl)amino)phenyl)propan-2-ol

¹H-NMR (400 MHz, CDCl₃): *δ* 8.08 (1H, d, *J=* 5.2 Hz), 7.76 (2H, d, *J=* 8.0 Hz), 7.37-7.36 (1H, m), 7.34-7.28 (2H, m), 7.25-7.21 (1H, m), 7.14-7.10 (2H, m), 6.56 (1H, s), 6.58 (1H, d, *J=* 2.0 Hz), 6.56-6.54 (1H, m), 5.04 (1H, d, *J=* 4.4 Hz), 2.98 (3H, d, *J=* 4.4 Hz), 1.54 (6H, s). * Two protons from OH and NH peak were not observed. MS Calcd.: 432.54; MS Found: 433.2 (M+H⁺).

### (51) A-51 : 3-((4-((2-(Methylamino)-4-phenylthiazol-5-yl)oxy)pyridin-2-yl)amino)benzenesulfonamide

¹H-NMR (400 MHz, DMSO-*d*₆): *δ* 9.45 (1H, s), 8.20 (1H, t, *J =2.0* Hz), 8.13 (1H, d, *J =* 6.0 Hz), 7.81 (1H, d, *J =* 8.0 Hz), 7.77-7.74 (2H, m), 7.69 (1H, d, *J =* 4.4 Hz), 7.43-7.36 (4H, m), 7.34-7.25 (2H, m), 6.69-6.67 (1H, m), 6.53 (1H, d, *J =* 2.4 Hz), 2.89 (3H, d, *J =* 4.8 Hz). * A proton from NH was not observed. MS Calcd.: 453.54; MS Found: 454.1 (M+H⁺).

### (52) A-52 : 3-((4-((2-(Methylamino)-4-phenylthiazol-5-yl)oxy)pyridin-2-yl)amino)benzamide

¹H-NMR (400 MHz, DMSO-*d*₆): *δ* 9.24 (s, 1H), 8.11 (d, *J* = 6.0 Hz, 1H), 8.02 (s, 1H), 7.83-7.81 (m, 2H), 7.76 (d, *J=* 7.2 Hz, 2H), 7.68-7.67 (m, 1H), 7.37-7.32 (m, 3H), 7.28-7.25 (m, 3H), 6.63 (dd, *J=* 1.6 Hz, 5.6 Hz, 1H), 6.51 (d, *J=* 2.0 Hz, 1H), 2.88 (d, *J=* 5.2 Hz, 3H). MS Calcd.: 417.48; MS Found: 418.2 (M+H⁺).

### (53) A-53 : 4-((4-((2-(Methylamino)-4-phenylthiazol-5-yl)oxy)pyridin-2-yl)amino)benzenesulfonamide

¹H-NMR (400 MHz, DMSO-*d*₆): *δ* 9.53 (1H, s), 8.16 (1H, d, *J=* 5.6 Hz), 7.80-7.74 (4H, m), 7.69-7.65 (3H, s), 7.36 (2H, t, *J=* 7.2 Hz), 7.26-7.24 (1H, m), 7.12 (2H, m), 6.73-6.72 (1H, m), 6.57 (1H, s), 2.89 (3H, d, *J* = 4.8 Hz). MS Calcd.: 453.54; MS Found: 454.1 (M+H⁺).

### (54) A-54 : 2-(4-((4-((2-(Methylamino)-4-phenylthiazol-5-yl)oxy)pyridin-2-yl)amino)pyridin-2-yl)propan-2-ol

¹H-NMR (400 MHz, DMSO-*d*₆): *δ* 9.53 (1H, s), 8.17 (2H, dd, *J=* 1.6 Hz, 5.8 Hz), 7.72 (2H, d, *J=* 6.8 Hz), 7.68-7.62 (3H, m), 7.33 (2H, t, *J=* 7.6 Hz), 7.22 (1H, t, *J* = 7.2 Hz), 6.71 (1H, dd, *J=* 2.4 Hz, 5.6 Hz), 6.59 (1H, d, *J =* 2.0 Hz), 5.06 (1H, s), 2.86 (3H, d, *J =* 4.8 Hz), 1.36 (6H, s). MS Calcd.: 433.53; MS Found: 434.2 (M+H⁺).

### (55) A-55 : 2-(5-((4-((2-(Methylamino)-4-phenylthiazol-5-yl)oxy)pyridin-2-yl)amino)pyridin-2-yl)propan-2-ol

¹H-NMR (400 MHz, DMSO-*d*₆): *δ* 9.19 (1H, s), 8.57 (1H, d, *J=* 2.8 Hz), 8.07-8.05 (2H, m), 7.73 (2H, d, *J=* 7.6 Hz), 7.65-7.64 (1H, m), 7.47 (1H, d, *J=* 8.8 Hz), 7.33 (2H, t, *J=* 8.0 Hz), 7.23 (1H, d, *J=* 7.2 Hz), 6.61 (1H, dd, *J=* 2.4 Hz, 7.2 Hz), 6.47 (1H, S), 5.08 (1H, s), 2.86 (3H, d, *J=* 4.8 Hz), 1.37 (6H, s). MS Calcd.: 433.53; MS Found: 434.1 (M+H⁺).

### (56) A-56 : 4-((4-((2-(Methylamino)-4-phenylthiazol-5-yl)oxy)pyridin-2-yl)amino)benzoic acid

¹H-NMR (400 MHz, DMSO-d₆): *δ* 12.43 (1H, s), 9.51 (1H, s), 8.17 (1H, d, *J =* 5.6 Hz), 7.80 (2H, d, *J=* 8.8 Hz), 7.76-7.70 (4H, m), 7.69-7.67 (1H, m), 7.35 (2H, t, *J=* 7.6 Hz), 7.25 (1H, t, *J =* 7.6 Hz), 6.72 (1H, dd, *J =* 2.4 Hz, 5.8 Hz), 6.58 (1H, d, *J =* 2.4 Hz), 2.89 (3H, d, *J =* 4.8 Hz). MS Calcd.: 418.47; MS Found: 419.1 (M+H⁺).

### (57) A-57 : 3-((4-((2-(Methylamino)-4-phenylthiazol-5-yl)oxy)pyridin-2-yl)amino)benzoic acid

¹H-NMR (400 MHz, DMSO-*d*₆): *δ* 9.32 (1H, s), 8.25 (1H, s), 8.13 (1H, d, *J=* 5.2 Hz), 7.88 (1H, d, *J=* 8.4 Hz), 7.75 (2H, d, *J=* 7.6 Hz), 7.68 (1H, d, *J=* 4.8 Hz), 7.44 (1H, d, *J=* 8.0 Hz), 7.37-7.26 (3H, m), 7.25 (1H, t, *J=* 7.4 Hz), 6.66 (1H, dd, *J=* 2.4 Hz, 6.0 Hz), 6.51 (1H, d, *J =* 2.0 Hz), 2.89 (3H, d, *J =* 4.8 Hz). *A proton from OH was not observed. MS Calcd.: 418.47; MS Found: 419.1 (M+H⁺).

### (58) A-58 : 3-((4-((2-Methyl-4-phenylthiazol-5-yl)oxy)pyirdin-2-yl)amino)benzenesulfonamide

¹H-NMR (400 MHz, DMSO-*d*₆): *δ* 9.45 (1H, s), 8.18 (1H, s), 8.16 (1H, d, *J =* 6.0 Hz), 7.84-7.79 (3H, m), 7.42 (3H, t, *J =* 7.8 Hz), 7.33 (2H, d, *J =* 7.6 Hz), 7.30 (2H, s), 6.70 (1H, dd, *J =* 6.0, 2.4 Hz), 6.47 (1H, d, *J =* 1.6 Hz), 2.71 (3H, s). MS Calcd.: 438.52; MS Found: 439.1 (M+H⁺).

### (59) A-59 : 2-(4-((4-((2-Methyl-4-phenylthiazol-5-yl)oxy)pyiridin-2_ yl)amino)pyridin-2-yl)propan-2-ol

¹H-NMR (400 MHz, CDCl3): *δ* 8.29 (1H, d, *J* = 5.6 Hz), 8.21 (1H, d, *J* = 6.0 Hz), 7.85 (2H, d, *J =* 7.6 Hz), 7.36 (2H, t, *J =* 7.6 Hz), 7.30-7.29 (2H, m), 7.17-7.16 (1H, m), 6.81 (1H, brs), 6.68 (1H, dd, *J =* 5.6, 2.0 Hz), 6.52 (1H, s), 2.70 (3H, s), 1.49 (6H, s). * A proton from OH was not observed. MS Calcd.: 418.51; MS Found: 419.2 (M+H⁺).

### (60) A-60 : 3-((4-((2-Methyl-4-phenylthiazol-5-yl)oxy)pyridin-2-yl)amino)benzamide

¹H-NMR (400 MHz, DMSO-*d*₆): *δ* 9.23 (1H, s), 8.13 (1H, d, *J=* 5.2 Hz), 8.02 (1H, s), 7.85-7.79 (4H, m), 7.42 (2H, t, *J=* 7.6 Hz), 7.35 (1H, t, *J=* 7.8 Hz), 7.33-7.27 (3H, m), 6.65 (1H, dd, *J =* 5.8, 2.2 Hz), 6.45 (1H, d, *J=* 2.0 Hz), 2.70 (3H, s). MS Calcd.: 402.47; MS Found: 403.1 (M+H⁺).

### (61) A-61 : 2-(3-((4-((2-Methyl-4-phenylthiazol-5-yl)oxy)pyridin-2-yl)amino)phenyl)propan-2-ol

¹H-NMR (400 MHz, DMSO-*d*₆): *δ* 9.05 (1H, s), 8.10 (1H, d, *J=* 6.0 Hz), 7.82 (2H, d, *J =* 8.0 Hz), 7.59 (1H, d, *J =* 8.4 Hz), 7.50 (1H, s), 7.41 (2H, t, *J =* 7.6 Hz), 7.31 (1H, t, *J =* 7.4 Hz), 7.15 (1H, t, *J =* 7.8 Hz), 6.98 (1H, d, *J =* 8.0 Hz), 6.60 (1H, dd, *J =* 5.8, 2.2 Hz), 6.44 (1H, d, *J =* 2.4 Hz), 4.92 (1H, s), 2.69 (3H, s), 1.38 (6H, s). MS Calcd.: 418.2; MS Found: 439.1 (M+H⁺).

### (62) A-62 : 5-((2-((1-(Methylsulfonyl)-1H-pyrazol-4-yl)amino)pyridin-4-yl)oxy)-4-phenylthiazol-2-amine

¹H-NMR (400 MHz, MeOH-*d*₄): *δ* 8.46(1H, s), 8.10 (1H, d, *J* = 6.0 Hz), 7.82 (1H, s), 7.70 (2H, d, *J=* 7.6 Hz), 7.30 (2H, t, *J =* 8.0 Hz), 7.22.7.20 (1H, m), 6.55 (1H, dd, *J =* 5.6, 2.0 Hz), 6.36 (1H, d, *J =* 2.0 Hz), 3.28 (3H, s). *Three protons from NH and NHBoc were not observed. MS Calcd.: 428.49 Found: 429.1 (M+H⁺).

### (63) A-63 : 4-((4-((2-Methyl-4-phenylthiazol-5-yl)oxy)pyridin-2-yl)amino)benzamide)

¹H-NMR (400 MHz, CDCl₃): *δ* 9.38 (1H, s), 8.17 (1H, d, *J=* 5.6 Hz), 7.82 (2H, d, *J =* 6.8 Hz), 7.77-7.75 (3H, m), 7.67 (2H, d, *J=* 8.8 Hz), 7.41 (2H, t, *J=* 7.2 Hz), 7.33-7.29 (1H, m), 7.11 (1H, s), 6.53 (1H, dd, *J=* 2.4 Hz, 6.0 Hz), 6.48 (1H, d, *J=* 2.4 Hz), 2.70 (3H, s). MS Calcd.: 402.47; MS Found: 403.1 (M+H⁺).

### (64) A-64 : 4-((4-((2-Methyl-4-phenylthiazol-5-yl)oxy)pyridin-2-yl)amino)benzenesulfonamide

¹H-NMR (400 MHz, DMSO-*d*₆): *δ* 9.52 (1H, s), 8.19 (1H, d, *J=* 6.0 Hz), 7.82 (2H, d, *J =* 6.8 Hz), 7.77 (2H, d, *J =* 8.8 Hz), 7.67 (2H, d, *J =* 9.2 Hz), 7.41 (2H, t, *J =* 7.2 Hz), 7.33-7.31 (1H, m), 7.13 (2H, s), 6.74 (1H, dd, *J =* 2.4 Hz, 6.0 Hz), 6.50 (1H, d, *J =* 2.4 Hz), 2.70 (3H, s). MS Calcd.: 438.52; MS Found: 439.1 (M+H⁺).

### (65) A-65 : 2-(5-((4-((2-Methyl-4-phenylthiazol-5-yl)oxy)pyiridin-2-yl)amino)pyridin-2-yl)propan-2-ol

¹H-NMR (400 MHz, DMSO-*d*₆): *δ* 9.21 (1H, s), 8.59 (1H, d, *J=* 2.4 Hz), 8.11 (1H, d, *J =* 6.0 Hz), 8.08 (1H, dd, *J =* 8.6, 2.6 Hz), 7.83 (2H, d, *J =* 8.4 Hz), 7.51 (1H, d, *J =* 8.8 Hz), 7.42 (2H, t, *J =* 7.6 Hz), 7.32 (1H, t, *J =* 7.2 Hz), 6.66 (1H, dd, *J =* 5.8, 2.2 Hz), 6.43 (1H, d, *J* = 2.4 Hz), 5.07 (1H, s), 2.70 (3H, s), 1.40 (6H, s). MS Calcd.: 418.51; MS Found: 419.2 (M+H⁺).

### (66) A-66 : 2-Methyl-2-(3-((4-((2-methyl-4-phenylthiazol-5-yl)oxy)pyridin-2-yl)amino)phenyl)propanoic acid

¹H-NMR (400 MHz, DMSO-*d*₆): *δ* 9.14 (1H, s), 8.10 (1H, d, *J=* 6.0 Hz), 7.81 (2H, d, *J=* 7.2 Hz), 7.65 (1H, d, *J=* 7.2 Hz), 7.42-7.39 (3H, m), 7.33-7.31 (1H, m), 7.18 (1H, t, *J =* 12.0 Hz), 6.87 (1H, d, *J=* 8.0 Hz), 6.63 (1H, dd, *J=* 2.4 Hz, 6.0 Hz), 6.43 (1H, d, *J=* 2.4 Hz), 2.69 (3H, s), 1.43 (6H, s). A proton from OH was not observed. MS Calcd.: 445.53; MS Found: 446.2 (M+H⁺).

### (67) A-67 : N,N-Dimethyl-3-((4-((2-methyl-4-phenylthiazol-5-yl)oxy)pyridin-2-yl)amino)benzamide

¹H-NMR (400 MHz, DMSO-*d*₆): *δ* 9.22 (1H, s), 8.12 (1H, d, *J=* 5.6 Hz), 7.80 (2H, d, *J=* 6.8 Hz), 7.74 (1H, s), 7.54 (1H, d, *J=* 7.6 Hz), 7.39 (2H, t, *J=* 7.8 Hz), 7.31-7.24 (2H, m), 6.85 (1H, d, *J =* 7.2 Hz), 6.65 (1H, dd, *J =* 6.2, 2.2 Hz), 6.41 (1H, d, *J =* 2.0 Hz), 2.94 (3H, s), 2.88 (3H, s), 2.68 (3H, s). MS Calcd.: 430.52; MS Found: 431.1 (M+H⁺).

### (68) A-68 : 3-((4-((2-Methyl-4-phenylthiazol-5-yl)oxy)pyridin-2-yl)amino)benzenesulfonic acid

¹H-NMR (400 MHz, DMSO-*d*₆): *δ* 9.15 (1H, s), 8.10 (1H, d, *J =* 6.0 Hz), 7.81-7.78 (3H, m), 7.63 (1H, d, *J=* 8.4 Hz), 7.40 (2H, t, *J=* 7.8 Hz), 7.29 (1H, t, *J=* 7.4 Hz), 7.17-7.09 (2H, m), 6.60 (1H, dd, *J=* 6.0, 2.4 Hz), 6.42 (1H, d, *J=* 2.4 Hz), 2.68 (3H, s). * A proton from OH was not observed. MS Calcd.: 439.51; MS Found: 440.1 (M+H⁺).

### (69) A-69 : N-Methyl-3-((4-((2-methyl-4-phenylthiazol-5-yl)oxy)pyridin-2-yl)amino)benzamide

¹H-NMR (400 MHz, DMSO-*d*₆): *δ* 9.24 (1H, s), 8.32-8.28 (1H, m), 8.11 (1H, d, *J* = 5.2 Hz), 7.98 (1H, s), 7.81-7.78 (3H, m), 7.39 (2H, t, *J=* 7.8 Hz), 7.31-7.27 (3H, m), 6.63 (1H, dd, *J=* 5.6, 2.0 Hz), 6.43 (1H, d, *J=* 2.0 Hz), 2.73 (3H, d, *J=* 4.8 Hz), 2.68 (3H, m). MS Calcd.: 416.50; MS Found: 417.1 (M+H⁺).

### (70) A-70 : 4-((2-Methyl-4-phenylthiazol-5-yl)oxy)-N-(3-(methylsulfonyl)phenyl)pyridin-2-amine

¹H-NMR (400 MHz, DMSO-*d*₆): *δ* 9.53 (1H, s), 8.26 (1H, s), 8.17 (1H, d, *J =* 6.0 Hz), 7.88 (1H, d, *J=* 9.2 Hz), 7.80 (2H, d, *J=* 7.2 Hz), 7.49 (1H, d, *J=* 8.0 Hz), 7.41-7.37 (3H, m), 7.30 (1H, t, *J=* 7.4 Hz), 6.71 (1H, dd, *J =* 5.6, 2.0 Hz), 6.45 (1H, d, *J=* 2.4 Hz), 3.14 (3H, s), 2.69 (3H, s). MS Calcd.: 437.53; MS Found: 438.0 (M+H⁺).

### (71) A-71 : N-(3-((4-((2-Methyl-4-phenylthiazol-5-yl)oxy)pyridin-2-yl)amino)phenyl)methanesulfonamide

¹H-NMR (400 MHz, DMSO-*d*₆): *δ* 9.64 (1H, s), 9.16 (1H, s), 8.09 (1H, d, *J =* 6.0 Hz), 7.80 (2H, d, *J =* 7.2 Hz), 7.45-7.41 (2H, m), 7.39 (2H, t, *J =* 7.8 Hz), 7.30 (1H, t, *J =* 5.2 Hz), 7.14 (1H, t, *J =* 8.4 Hz), 6.70 (1H, d, *J =* 8.0 Hz), 6.62 (1H, dd, *J =* 3.8, 1.8 Hz), 6.43 (1H, d, *J* = 2.4 Hz), 2.94 (3H, s), 2.68 (3H, s). MS Calcd.: 452.55; MS Found: 453.1 (M+H⁺).

### (72) A-72 : 4-((2-Methyl-4-phenylthiazol-5-yl)oxy)-N-(3-((trifluorornethyl)sulfonyl)phenyl)pyridin-2-amine

¹H-NMR (400 MHz, DMSO-*d*₆): *δ* 9.97 (1H, s), 8.83 (1H, s), 8.20 (1H, d, *J=* 5.6 Hz), 8.04 (1H, d, *J =* 8.8 Hz), 7.80 (2H, d, *J =* 8.0 Hz), 7.68 (1H, t, *J =* 8.0 Hz), 7.56 (1H, d, *J =* 7.6 Hz), 7.39 (2H, t, *J =* 7.8 Hz), 7.17 (1H, t, *J =* 7.8 Hz), 6.77 (1H, dd, *J =* 5.6, 2.4 Hz), 6.46 (1H, d, *J =* 2.0 Hz), 2.69 (3H, s). MS Calcd.: 491.51; MS Found: 492.0 (M+H⁺).

### (73) A-73 : N-Methyl-3-((4-((2-methyl-4-phenylthiazol-5-yl)oxy)pyridin-2-yl)amino)benzenesulfonamide

¹H-NMR (400 MHz, DMSO-*d*₆): *δ* 9.46 (1H, s), 8.15-8.14 (2H, m), 7.83-7.79 (3H, m), 7.45-7.35 (4H, m), 7.31 (1H, t, *J=* 3.8 Hz), 7.23 (1H, d, *J=* 8.4 Hz), 6.69 (1H, d, *J=* 6.0 Hz), 6.44 (1H, d, *J=* 2.4 Hz), 2.68 (3H, s), 2.39 (3H, d, *J=* 4.8 Hz). MS Calcd.: 452.55; MS Found: 453.1 (M+H⁺).

### (74) A-74 : N,N-Dimethyl-3-((4-((2-methyl-4-phenylthiazol-5-yl)oxy)pyridin-2-yl)amino)benzenesulfonamide

¹H-NMR (400 MHz, DMSO-*d*₆): *δ* 9.50 (1H, s), 8.15 (1H, d, *J=* 5.6 Hz), 8.09 (1H, s), 7.90 (1H, d, *J=* 7.6 Hz), 7.80 (2H, d, *J=* 7.6 Hz), 7.47 (1H, t, *J=* 8.0 Hz), 7.39 (2H, t, *J=* 7.8 Hz), 7.30 (1H, t, *J=* 5.8 Hz), 7.19 (1H, d, *J=* 8.0 Hz), 6.70 (1H, dd, *J* = 8.2, 2.2 Hz), 6.43 (1H, s), 2.68 (3H, s), 2.58 (6H, s). MS Calcd.: 466.58; MS Found: 467.1 (M+H⁺).

### (75) A-75 : N-(3-((4-((2-Methyl-4-phenylthiazol-5-yl)oxy)pyridin-2-yl)amino)phenyl)cyclopropanesulfonamide

¹H-NMR (400 MHz, DMSO-*d*₆): *δ* 9.61 (1H, s), 9.15 (1H, s), 8.09 (1H, d, *J=* 6.0 Hz), 7.80 (2H, d, *J=* 7.6 Hz), 7.50 (1H, s), 7.44-7.37 (3H, m), 7.29 (1H, t, *J=* 7.4 Hz), 7.13 (1H, t, *J =* 8.2 Hz), 6.72 (1H, d, *J =* 8.4 Hz), 6.62 (1H, dd, *J =* 5.8, 2.2 Hz), 6.43 (1H, d, *J =* 2.4 Hz), 2.68 (3H, s), 2.58-2.54 (1H, m), 0.93-0.89 (4H, m). MS Calcd.: 478.59; MS Found: 479.1 (M+H⁺).

### (76) A-76 : N-Cyclopropyl-3-((4-((2-methyl-4-phenylthiazol-5-yl)oxy)pyridin-2-yl)amino)benzenesulfonamide

¹H-NMR (400 MHz, DMSO-*d*₆): *δ* 9.47 (1H, s), 8.18 (1H, s), 8.14 (1H, d, *J =* 6.0 Hz), 7.84-7.79 (3H, m), 7.45-7.37 (3H, m), 7.31-7.26 (2H, m), 6.69 (1H, dd, *J* = 6.2, 2.2 Hz), 6.44 (1H, d, *J =* 2.0 Hz), 2.68 (3H, s), 2.10-2.06 (1H, m), 0.46-2.43 (2H, m), 0.39-0.37 (2H, m). * A proton from NH was not observed. MS Calcd.: 478.59; MS Found: 479.1 (M+H⁺).

### (77)A-77 : N-(3-(Ethylsulfonyl)-4-((2-methyl-4-phenylthiazol-5-yl)oxy)pyridin-2-amine

¹H-NMR (400 MHz, DMSO-*d*₆): *δ* 9.60 (1H, s), 8.38 (1H, s), 8.16 (1H, d, *J=* 5.2 Hz), 7.90 (1H, d, *J=* 9.6 Hz), 7.80 (2H, d, *J=* 7.2 Hz), 7.49 (1H, t, *J=* 8.0 Hz), 7.39 (2H, t, *J=* 7.4 Hz), 7.32 (2H, t, *J =* 9.2 Hz), 6.71 (1H, d, *J =* 5.6 Hz), 6.45 (1H, s), 3.22 (2H, q, *J =* 5.9 Hz), 2.69 (3H, s), 1.08 (3H, t, *J=* 7.4 Hz). MS Calcd.: 438.52; Found: 439.1 (M+H⁺).

### (78) A-78 : 3-((4-((2-Methyl-4-phenylthiazol-5-yl)oxy)pyridin-2-yl)amino)phenyl)methanesulfonamide

¹H-NMR (400 MHz, DMSO-*d*₆): *δ* 9.16 (1H, s), 8.09 (1H, d, *J=* 7.6 Hz), 7.80 (2H, d, *J =* 7.6 Hz), 7.68 (1H, d, *J =* 8.4 Hz), 7.55 (1H, s), 7.39 (2H, t, *J =* 7.8 Hz), 7.30 (1H, t, *J =* 6.0 Hz), 7.21 (1H, t, *J =* 7.8 Hz), 6.90 (1H, d, *J =* 7.6 Hz), 6.62 (1H, dd, *J =* 6.0, 2.4 Hz), 6.44 (1H, d, *J =* 2.4 Hz), 4.17 (2H, s), 2.68 (3H, s). * Two protons from NH₂ were not observed. MS Calcd.: 452.55; MS Found: 453.1 (M+H⁺).

### (79) A-79 : 4-((2-Methyl-4-phenylthiazol-5-yl)oxy)-N-(3-((methylsulfonyl)methyl)phenyl)pyridin-2-amine

¹H-NMR (400 MHz, DMSO-*d*₆): *δ* 9.19 (1H, s), 8.09 (1H, d, *J=* 6.0 Hz), 7.80 (2H, d, *J =* 7.6 Hz), 7.67 (1H, d, *J =* 8.0 Hz), 7.59 (1H, s), 7.39 (2H, t, *J =* 7.8 Hz), 7.29 (1H, t, *J =* 7.8 Hz), 7.23 (1H, t, *J =* 7.8 Hz), 6.90 (1H, d, *J =* 7.6 Hz), 6.62 (1H, dd, *J =* 6.0, 2.4 Hz), 6.44 (1H, d, *J=* 2.4 Hz), 4.39 (2H, s), 2.89 (3H, s), 2.68 (3H, s). MS Calcd.: 451.56; MS Found: 452.1 (M+H⁺).

### (80) A-80 : 4-((2-Methyl-4-phenylthiazol-5-yl)oxy)-N-(pyridin-4-ylmethyl)pyridin-2-amine

¹H-NMR (400 MHz, DMSO-*d*₆): *δ* 8.43 (2H, d, *J=* 5.6 Hz), 7.87 (1H, d, *J=* 6.0 Hz), 7.78 (2H, d, *J =* 7.2 Hz), 7.38 (2H, t, *J =* 7.4 Hz), 7.32-7.28 (2H, m), 7.22 (2H, d, *J =* 5.6 Hz), 6.35 (1H, dd, *J =* 6.0, 2.4 Hz), 6.16 (1H, d, *J =* 2.0 Hz), 4.44 (2H, d, *J =* 6.0 Hz), 2.65 (3H, s). MS Calcd.: 374.46; MS Found: 375.1 (M+H⁺).

### (81) A-81 : 4-((2-Methyl-4-phenylthiazol-5-yl)oxy)-N-(pyridin-3-ylmethyl)pyridin-2-amine

¹H-NMR (400 MHz, DMSO-*d*₆): *δ* 8.47 (1H, s), 8.40 (1H, d, *J=* 3.2 Hz), 7.91 (1H, d, *J =* 5.6 Hz), 7.78 (2H, d, *J =* 7.2 Hz), 7.64 (1H, d, *J =* 7.6 Hz), 7.38 (2H, t, *J =* 7.4 Hz), 7.29 (2H, t, *J =* 6.6 Hz), 7.24 (1H, t, *J =* 5.8 Hz), 6.35 (1H, dd, *J =* 6.0, 2.4 Hz), 6.14 (1H, d, *J =* 2.4 Hz), 4.43 (2H, d, *J=* 6.0 Hz), 2.65 (3H, s). MS Calcd.: 374.46; MS Found: 375.1 (M+H⁺).

### (82) A-82 : 4-((2-Methyl-4-phenylthiazol-5-yl)oxy)-N-(pyridin-3-yl)pyridin-2-amine

¹H-NMR (400 MHz, DMSO-*d*₆): *δ* 9.28 (1H, s), 8.69 (1H, s), 8.15-7.12 (2H, m), 8.07 (1H, s), 7.80 (2H, d, *J =* 7.2 Hz), 7.39 (2H, t, *J =* 7.8 Hz), 7.30 (1H, t, *J =* 7.4 Hz), 6.64 (1H, dd, *J=* 5.6, 2.0 Hz), 6.68 (1H, dd, *J=* 5.6, 2.0 Hz), 6.43 (1H, d, *J=* 2.4 Hz), 2.68 (3H, s). MS Calcd.: 360.43; MS Found: 361.1 (M+H⁺).

### (83) A-83 : N-(6-Fluoropyridin-3-yl)-4-((2-methyl-4-phenylthiazol-5-yl)oxy)pyridin-2-amine

¹H-NMR (400 MHz, DMSO-*d*₆): *δ* 9.33 (1H, s), 8.39 (1H, s), 8.21 (1H, t, *J =* 6.6 Hz), 8.11 (1H, d, *J =* 5.2 Hz), 7.80 (2H, d, *J =* 7.6 Hz), 7.39 (2H, t, *J =* 7.8 Hz), 7.30 (1H, t, *J =* 7.8 Hz), 7.06 (1H, dd, *J =* 9.2, 3.2 Hz), 6.68 (1H, dd, *J =* 5.8, 2.2 Hz), 6.39 (1H, d, *J =* 2.0 Hz), 2.68 (3H, s). MS Calcd.: 378.42; MS Found: 379.1 (M+H⁺).

### (84) A-84 : N-(6-Chloropyridin-3-yl)-4-((2-methyl-4-phenylthiazol-5-yl)oxy)pyridin-2-amine

¹H-NMR (400 MHz, DMSO-*d*₆): *δ* 9.45 (1H, s), 8.57 (1H, d, *J =* 3.2 Hz), 8.19 (1H, dd, *J* = 8.6, 2.6 Hz), 8.14 (1H, d, *J =* 6.0 Hz), 7.80 (2H, d, *J =* 8.0 Hz), 7.41-7.34 (3H, m), 7.30 (1H, t, *J =* 7.6 Hz), 6.72 (1H, dd, *J =* 5.2, 2.4 Hz), 6.43 (1H, d, *J =* 2.0 Hz), 2.68 (3H, s). MS Calcd.: 394.88; MS Found: 395.1 (M+H⁺).

### (85) A-85 : N-(6-Methoxypyridin-3-yl)-4-((2-methyl-4-phenylthiazol-5-yl)oxy)pyridin-2-amine

¹H-NMR (400 MHz, DMSO-*d*₆): *δ* 9.00 (1H, s), 8.30 (1H, d, *J =* 2.4 Hz), 8.04 (1H, d, *J =* 6.4 Hz), 7.93 (1H, dd, *J =* 9.0, 2.2 Hz), 7.80 (2H, d, *J =* 8.0 Hz), 7.40 (2H, t, *J =* 7.8 Hz), 7.30 (1H, t, *J =* 7.4 Hz), 6.72 (1H, d, *J =* 8.8 Hz), 6.58 (1H, d, *J =* 5.6 Hz), 6.32 (1H, d, *J =* 1.6 Hz), 3.77 (3H, s), 2.67 (3H, s). MS Calcd.: 390.46; MS Found: 391.1 (M+H⁺).

### (86) A-86 : 4-((2-Methyl-4-phenylthiazol-5-yl)oxy)-N-(pyridin-4-yl)pyridin-2-amine

¹H-NMR (400 MHz, DMSO-*d*₆): *δ* 9.56 (1H, s), 8.26 (2H, brs), 8.21 (1H, d, *J =* 5.6 Hz), 7.79 (2H, d, *J =* 7.6 Hz), 7.59 (2H, s), 7.39 (2H, t, *J =* 7.6 Hz), 7.29 (1H, t, *J =* 7.4 Hz), 6.78 (1H, dd, *J =* 5.8, 2.6 Hz), 6.50 (1H, d, *J =* 2.0 Hz), 2.68 (3H, s). MS Calcd.: 360.43; MS Found: 361.1 (M+H⁺).

### (87) A-87 : N-(2-Fluoropyridin-4-yl)-4-((2-methyl-4-phenylthiazol-5-yl)oxy)pyridin-2-amine

¹H-NMR (400 MHz, DMSO-*d*₆): *δ* 9.89 (1H, s), 8.25 (1H, d, *J =* 5.6 Hz), 7.92 (1H, d, *J =* 6.0 Hz), 7.79 (2H, d, *J =* 8.0 Hz), 7.61 (1H, d, *J =* 1.6 Hz), 7.39 (2H, t, *J =* 7.6 Hz), 7.30 (1H, t, *J =* 8.0 Hz), 7.22 (1H, d, *J =* 6.0 Hz), 6.84 (1H, dd, *J =* 5.8, 2.2 Hz), 6.52 (1H, d, *J =* 2.0 Hz), 2.69 (3H, s). MS Calcd.: 378.42; MS Found: 379.1 (M+H⁺).

### (88) A-88 : N-(2-Methoxypyridin-4-yl)-4-((2-methyl-4-phenylthiazol-5-yl)oxy)pyridin-2-amine

¹H-NMR (400 MHz, DMSO-*d*₆): *δ* 9.51 (1H, s), 8.20 (1H, d, *J =* 5.6 Hz), 7.86 (1H, d, *J =* 6.0 Hz), 7.79 (2H, d, *J =* 7.6 Hz), 7.39 (2H, t, *J* =7.6 Hz), 7.31-7.28 (2H, m), 6.97 (1H, d, *J =* 5.6 Hz), 6.76 (1H, d, *J =* 6.0 Hz), 6.48 (1H, d, *J =* 2.0 Hz), 3.76 (3H, s), 2.68 (3H, s). MS Calcd.: 390.46; MS Found: 391.1 (M+H⁺).

### (89)A-89 : N-(2-Chloropyridin-4-yl)-4-((2-methyl-4-phenylthiazol-5-yl)oxy)pyridin-2-amine

¹H-NMR (400 MHz, DMSO-*d*₆): *δ* 9.83 (1H, s), 8.26 (1H, d, *J =* 6.0 Hz), 8.07 (1H, d, *J =* 5.2 Hz), 7.94 (1H, d, *J =* 1.6 Hz), 7.79 (2H, d, *J =* 7.6 Hz), 7.41-7.34 (3H, m), 7.30 (1H, t, *J =* 7.4 Hz), 6.84 (1H, dd, *J =* 5.8, 2.2 Hz), 6.51 (1H, d, *J=* 2.4 Hz), 2.69 (3H, s). MS Calcd.: 394.88; MS Found: 395.1 (M+H⁺).

### (90) A-90 _: 6-((4-((2-Methyl-4-phenylthiazol-5-yl)oxy)pyridin-2-yl)amino)nicotinamide

¹H-NMR (400 MHz, DMSO-*d*₆): *δ* 10.1 (1H, s), 8.65 (1H, d, *J =* 2.4 Hz), 8.19 (1H, d, *J =* 6.0 Hz), 8.06 (1H, dd, *J =* 9.0, 2.2 Hz), 7.89 (1H, s), 7.80 (2H, d, *J =* 7.2 Hz), 7.69 (1H, d, *J =* 8.8 Hz), 7.62 (1H, d, *J =* 2.4 Hz), 7.39 (2H, t, *J =* 7.6 Hz), 7.30-7.29 (2H, m), 6.71 (1H, dd, *J =* 5.8, 2.2 Hz), 2.69 (3H, s). MS Calcd.: 403.46; MS Found: 404.1 (M+H⁺).

### (91) A-91 : 5-((4-((2-Methyl-4-phenylthiazol-5-yl)oxy)pyridin-2-yl)amino)picolinamide

¹H-NMR (400 MHz, DMSO-*d*₆): *δ* 9.64 (1H, s), 7.70 (1H, d, *J =* 2.8 Hz), 8.35 (1H, dd, *J =* 8.8, 2.4 Hz), 8.19 (1H, d, *J =* 6.0 Hz), 7.90 (1H, d, *J =* 8.8 Hz), 7.86 (1H, s), 7.80 (2H, d, *J =* 7.2 Hz), 7.41-7.38 (3H, m), 7.31 (1H, d, *J =* 7.2 Hz), 6.76 (1H, dd, *J =* 7.4, 1.8 Hz), 6.49 (1H, d, *J =* 2.4 Hz), 2.69 (3H, s). MS Calcd.: 403.46; MS Found: 404.1 (M+H⁺).

### (92) A-92 : 5-((4-((2-Methyl-4-phenylthiazol-5-yl)oxy)pyridin-2-yl)amino)nicotinamide

¹H-NMR (400 MHz, DMSO-*d*₆): *δ* 9.56 (1H, s), 8.80 (1H, d, *J =* 1.6 Hz), 8.52 (2H, s), 8.16 (1H, d, *J =* 5.2 Hz), 8.05 (1H, s), 7.80 (2H, d, *J =* 7.2 Hz), 7.52 (1H, s), 7.39 (2H, t, *J =* 7.8 Hz), 7.30 (1H, t, *J =* 7.2 Hz), 6.71 (1H, dd, *J =* 5.6, 2.0 Hz), 6.46 (1H, d, *J =* 1.6 Hz), 2.69 (3H, s). MS Calcd.: 403.46; MS Found: 404.1 (M+H⁺).

### (93) A-93 : 2-((4-((2-Methyl-4-phenylthiazol-5-yl)oxy)pyridin-2-yl)amino)isonicotinamide

¹H-NMR (400 MHz, DMSO-*d*₆): *δ* 9.94 (1H, s), 8.24 (1H, d, *J =* 4.8 Hz), 8.17 (1H, d, *J =* 6.0 Hz), 8.08 (1H, s), 8.01 (1H, s), 7.80 (2H, d, *J =* 6.8 Hz), 7.61-7.59 (2H, m), 7.39 (2H, t, *J =* 7.6 Hz), 7.28 (1H, t, *J =* 7.4 Hz), 7.20 (1H, d, *J =* 5.2 Hz), 6.67 (1H, dd, *J =* 5.6, 2.4 Hz), 2.68 (3H, s). MS Calcd.: 403.46; MS Found: 404.1 (M+H⁺).

### (94) A-94 : N-Benzyl-4-((2-methyl-4-phenylthiazol-5-yl)oxy)pyridin-2-amine

¹H-NMR (400 MHz, DMSO-*d*₆): *δ* 7.89 (1H, d, *J =* 5.6 Hz), 7.77 (2H, d, *J =* 7.2 Hz), 7.38 (2H, t, *J =* 7.6 Hz), 7.31-7.23 (5H, m), 7.20-7.17 (2H, m), 6.32 (1H, dd, *J =* 5.6, 2.0 Hz), 6.12 (1H, d, *J* = 2.4 Hz), 4.40 (2H, d, *J =* 6.0 Hz), 2.64 (3H, s). MS Calcd.: 373.47; MS Found: 374.1 (M+H⁺).

### (95) A-95 : N-(4-Fluorobenzyl)-4-((2-methyl-4-phenylthiazol-5-yl)oxy)pyridin-2-amine

¹H-NMR (400 MHz, DMSO-*d*₆): *δ* 7.24 (1H, d, *J =* 5.6 Hz), 7.77 (2H, d, *J =* 7.6 Hz), 7.38 (2H, t, *J =* 7.6 Hz), 7.31-7.26 (3H, m), 7.19 (1H, t, *J =* 6.0 Hz), 7.08 (2H, d, *J =* 9.0 Hz), 6.33 (1H, dd, *J =* 5.6, 2.4 Hz), 6.11 (1H, d, *J =* 2.0 Hz), 4.37 (2H, d, *J =* 6.4 Hz), 2.65 (3H, s). MS Calcd.: 391.46; MS Found: 392.1 (M+H⁺).

### (96) A-96 : N-(3-Fluorobenzyl)-4-((2-methyl-4-phenylthiazol-5-yl)oxy)pyridin-2-amine

¹H-NMR (400 MHz, DMSO-*d*₆): *δ* 8.00 (1H, d, *J=* 8.0 Hz), 7.90 (2H, d, *J=* 5.6 Hz), 7.39 (2H, t, *J =* 7.6 Hz), 7.33-7.27 (3H, m), 7.09-6.99 (3H, m), 6.35 (1H, d, *J =* 4.8 Hz), 6.14 (1H, s), 4.42 (2H, d, *J=* 6.0 Hz), 2.69 (3H, s). MS Calcd.: 394.46; MS Found: 392.1 (M+H⁺).

### (97) A-97 : 4-((2-Methyl-4-phenylthiazol-5-yl)oxy)-N-(1-(methylsulfonyl)-1H-pyrazol-4-yl)pyridin-2-amine

¹H-NMR (400 MHz, DMSO-*d*₆): *δ* 9.40 (1H, s), 8.43 (1H, s), 8.16 (1H, d, *J=* 5.6 Hz), 7.84 (1H, s), 7.79 (2H, d, *J =* 8.0 Hz), 7.39 (2H, t, *J =* 7.8 Hz), 7.29 (1H, t, *J =* 5.8 Hz), 6.62 (1H, dd, *J =* 7.6, 2.0 Hz), 6.33 (1H, d, *J =* 2.4 Hz), 3.41 (3H, s), 2.68 (3H, s). MS Calcd.: 427.50; MS Found: 428.1 (M+H⁺).

### (98) A-98 : N-(1-(Cyclopropylsulfonyl)-1H-pyrazol-4-yl)-4-((2-methyl-4-phenylthiazol-5-yl)oxy)pyridin-2-amine

¹H-NMR (400 MHz, DMSO-*d*₆): *δ* 9.40 (1H, s), 8.42 (1H, s), 8.16 (1H, d, *J =* 6.0 Hz), 7.85 (1H, s), 7.79 (2H, d, *J =* 7.6 Hz), 7.39 (2H, t, *J =* 7.6 Hz), 7.29 (1H, d, *J =* 7.4 Hz), 6.62 (1H, dd, *J =* 6.0, 2.0 Hz), 6.33 (1H, d, *J =* 2.0 Hz), 3.03-2.99 (1H, m), 2.68 (3H, s), 1.17-1.14 (2H, m), 1.12-1.10 (2H, m). MS Calcd.: 453.54; MS Found: 454.1 (M+H⁺).

### (99) A-99 : N-(1-Methyl-1H-pyrazol-4-yl)-4-((2-methyl-4-phenylthiazol-5-yl)oxy)pyridin-2-amine

¹H-NMR (400 MHz, DMSO-*d*₆): *δ* 8.85 (1H, s), 8.03 (1H, d, *J=* 5.2 Hz), 7.85 (1H, s), 7.79 (2H, d, *J =* 7.6 Hz), 7.39 (2H, t, *J =* 7.6 Hz), 7.31-7.27 (2H, m), 6.46 (1H, dd, *J =* 5.6, 2.0 Hz), 6.22 (1H, d, *J=* 2.4 Hz), 3.75 (3H, s), 2.66 (3H, s). MS Calcd.: 363.44; MS Found: 364.1 (M+H⁺).

### (100) A-100 : 4-((2-Methyl-4-phenylthiazol-5-yl)oxy)-N-(1H-pyrazol-4-yl)pyridin-2-amine

¹H-NMR (400 MHz, DMSO-*d*₆): *δ* 12.4 (1H, brs), 8.82 (1H, s), 8.02 (1H, d, *J =* 5.6 Hz), 7.83 (1H, s), 7.79 (2H, d, *J =* 7.6 Hz), 7.39 (3H, t, *J =* 7.6 Hz), 7.29 (1H, t, *J =* 7.8 Hz), 6.45 (1H, dd, *J* = 5.8, 2.2 Hz), 6.22 (1H, d, *J* = 2.0 Hz), 2.66 (3H, s). MS Calcd.: 349.41; MS Found: 350.1 (M+H⁺).

### (101) A-101 : 6-((4-((2-Methyl-4-phenylthiazol-5-yl)oxy)pyridin-2-yl)amino)picolinamide

¹H-NMR (400 MHz, DMSO-*d*₆): *δ* 9.89 (1H, s), 8.18 (1H, d, *J =* 5.6 Hz), 7.91 (1H, d, *J* = 8.8 Hz), 7.83-7.79 (3H, m), 7.63 (1H, s), 7.49 (1H, d, *J =* 6.8 Hz), 7.40 (1H, s), 7.38 (2H, t, *J =* 7.4 Hz), 7.30-7.28 (2H, m), 6.71 (1H, dd, *J =* 6.4, 3.4 Hz), 2.68 (3H, s). MS Calcd.: 403.46; MS Found: 404.1 (M+H⁺).

### (102) A-102 : 4-((4-((2-Methyl-4-phenylthiazol-5-yl)oxy)pyridin-2-yl)amino)picolinamide

¹H-NMR (400 MHz, DMSO-*d*₆): *δ* 9.79 (1H, s), 8.31 (1H, d, *J =* 5.2 Hz), 8.24-8.22 (2H, m), 7.99 (1H, s), 7.81-7.79 (3H, m), 7.54 (1H, s), 7.38 (2H, t, *J =* 6.8 Hz), 7.30 (1H, d, *J* = 6.8 Hz), 6.81 (1H, s), 6.53 (1H, d, *J =* 2.4 Hz), 2.69 (3H, s). MS Calcd.: 403.46; MS Found: 404.1 (M+H⁺).

### (103) A-103 : N-(4-Methoxyphenyl)-4-((2-methyl-4-phenylthiazol-5-yl)oxy)pyridin-2-amine

¹H-NMR (400 MHz, DMSO-*d*₆): *δ* 8.86 (1H, s), 8.03 (1H, d, *J =* 5.6 Hz), 7.80 (2H, d, *J =* 7.6 Hz), 7.44 (2H, d, *J =* 9.6 Hz), 7.39 (2H, t, *J =* 7.6 Hz), 7.29 (1H, t, *J =* 7.4 Hz), 6.81 (2H, d, *J =* 8.8 Hz), 6.53 (1H, dd, *J* = 5.8, 2.2 Hz), 6.31 (1H, d, *J* = 2.4 Hz), 3.67 (3H, s), 2.67 (3H, s). MS Calcd.: 389.47; MS Found: 390.1 (M+H⁺).

### (104) A-104 : 4-((2-Methyl-4-phenylthiazol-5-yl)oxy)-N-(4-(piperazin-1-yl)phenyl)pyridin-2-amine

¹H-NMR (400 MHz, DMSO-*d*₆): *δ* 8.80 (1H, s), 8.01 (1H, d, *J =* 6.0 Hz), 7.80 (2H, d, *J =* 7.2 Hz), 7.41-7.37 (4H, m), 7.30 (1H, d, *J =* 7.6 Hz), 6.81 (2H, d, *J =* 8.8 Hz), 6.50 (1H, dd, *J =* 5.6, 2.4 Hz), 6.30 (1H, d, *J =* 2.4 Hz), 2.98-2.93 (4H, m), 2.87-2.83 (4H, m), 2.67 (3H, s). * A proton from NH was not observed. MS Calcd.: 443.56; MS Found: 444.2 (M+H⁺).

### (105) A-105 : 4-((2-Methyl-4-phenylthiazol-5-yl)oxy)-N-(4-(2-(piperidin-1-yl)ethoxy)phenyl)pyridin-2-amine

¹H-NMR (400 MHz, DMSO-*d*₆): *δ* 8.86 (1H, s), 8.03 (1H, d, *J =* 5.6 Hz), 7.80 (2H, d, *J =* 7.6 Hz), 7.44-7.37 (4H, m), 7.30 (1H, d, *J =* 7.2 Hz), 6.81 (2H, d, *J =* 9.2 Hz), 6.53 (1H, dd, *J =* 5.8, 2.2 Hz), 6.31 (1H, d, *J =* 2.4 Hz), 3.97 (2H, t, *J =* 5.8 Hz), 2.67 (3H, s), 2.62-2.58 (2H, m), 2.39 (4H, s), 1.49-1.44 (4H, m), 1.38-1.32 (2H, m). MS Calcd.: 486.63; MS Found: 487.2 (M+H⁺).

### (106) A-106 : N-(3-Methoxy-4-(4-methylpiperazin-1-yl)phenyl)-4-((2-methyl-4-phenylthiazol-5-yl)oxy)pyridin-2-amine

¹H-NMR (400 MHz, DMSO-*d*₆): *δ* 8.90 (1H, s), 8.05 (1H, d, *J=* 6.0 Hz), 7.80 (2H, d, *J =* 7.6 Hz), 7.39 (2H, t, *J =* 7.6 Hz), 7.29 (1H, t, *J =* 7.4 Hz), 7.18 (1H, d, *J =* 2.4 Hz), 7.06 (1H, dd, *J =* 8.6, 2.2 Hz), 6.74 (1H, d, *J =* 8.8 Hz), 6.54 (1H, dd, *J =* 5.8, 2.2 Hz), 6.34 (1H, d, *J =* 2.0 Hz), 3.69 (3H, s), 2.85 (4H, brs), 2.67 (3H, s), 2.40 (4H, brs), 2.18 (3H, s). MS Calcd.: 487.62; MS Found: 488.2 (M+H⁺).

### (107) A-107 : 1,1,1-Trifluoro-N-(3-((4-((2-methyl-4-phenylthiazol-5-yl)oxy)pyridin-2-yl)amino)phenyl)methanesulfonamide

¹H-NMR (400 MHz, DMSO-*d*₆): *δ* 9.25 (1H, brs), 8.11 (1H, d, *J =* 5.6 Hz), 7.80 (2H, d, *J =* 8.0 Hz), 7.62 (1H, brs), 7.53 (1H, d, *J =* 8.4 Hz), 7.40 (2H, t, *J =* 7.6 Hz), 7.29-7.31 (1H, m), 7.22 (1H, t, *J =* 8.8 Hz), 6.74 (1H, d, *J =* 8.4 Hz), 6.45 (1H, d, *J =* 6.4 Hz), 6.43 (1H, s), 2.68 (3H, s). * A proton from NH was not observed. MS Calcd.: 506.07; MS Found: 507.1 (M+H⁺).

### (108) A-108 : N-(4-(4-(2-Methoxyethyl)piperazin-1-yl)phenyl)-4-((2-methyl-4-phenylthiazol-5-yl)oxy)pyridin-2-amine

¹H-NMR (400 MHz, DMSO-*d*₆): *δ* 8.79 (1H, s), 8.01 (1H, d, *J=* 6.0 Hz), 7.80 (2H, d, *J=* 7.6 Hz), 7.41-7.36 (4H, m), 7.29 (1H, t, *J=* 7.2 Hz), 6.81 (2H, d, *J=* 8.8 Hz), 6.48 (1H, dd, *J* = 8.4, 2.4 Hz), 6.30 (1H, d, *J =* 2.0 Hz), 3.44 (2H, t, *J =* 5.6 Hz), 3.22 (3H, s), 3.32-2.98 (4H, m), 2.67 (3H, s), 2.56-2.48 (6H, m). MS Calcd.: 501.64; MS Found: 502.2 (M+H⁺).

### (109) A-109 : 3-((4-((2-Methyl-4-phenylthiazol-5-yl)oxy)pyridin-2-yl)amino)benzoic acid

¹H-NMR (400 MHz, DMSO-*d*₆): *δ* 12.8 (1H, brs), 9.31 (1H, s), 8.24 (1H, s), 8.16 (1H, d, *J =* 6.0 Hz), 7.87 (1H, d, *J =* 7.6 Hz), 7.83 (2H, d, *J =* 7.2 Hz), 7.47-7.40 (3H, m), 7.37-7.30 (2H, m), 6.68 (1H, dd, *J =* 6.0, 2.0 Hz), 6.45 (1H, d, *J =* 2.0 Hz), 2.70 (3H, s). MS Calcd.: 403.45; MS Found: 404.1 (M+H⁺).

### (110) A-110 : 4-((4-((2-Methyl-4-phenylthiazol-5-yl)oxy)pyridin-2-yl)amino)benzoic acid

¹H-NMR (400 MHz, DMSO-*d*₆): *δ* 9.50 (1H, s), 8.19 (1H, d, *J =* 6.0 Hz), 7.83-7.82 (4H, m), 7.72 (2H, d, *J =* 8.8 Hz), 7.41 (2H, t, *J =* 6.8 Hz), 7.33-7.29 (1H, m), 6.74 (1H, dd, *J* = 1.6 Hz, 5.4 Hz), 6.51 (1H, d, *J =* 1.6 Hz), 2.70 (3H, s). *A proton from OH was not observed. MS Calcd.: 403.65; MS Found: 404.1 (M+H⁺).

### (111) A-111 : 2-(3-((4-((2-Methyl-4-phenylthiazol-5-yl)oxy)pyridin-2-yl)amino)phenyl)acetonitrile

¹H-NMR (400 MHz, CDCl₃): *δ* 8.11 (1H, d, *J =* 8.0 Hz), 7.85 (2H, d, *J =* 8.0 Hz), 7.49-7.13 (3H, m), 7.32-7.27 (2H, m), 7.20 (1H, d, *J =* 16.0 Hz), 6.98 (1H, d, *J =* 8.0 Hz), 6.57 (1H, s), 6.55 (1H, dd, *J =* 8.4, 4.4 Hz), 6.45 (1H, d, *J =* 4.0 Hz), 3.70 (2H, s), 2.70 (3H, s).

### (112) A-112 : N-(3-((1H-Tetrazol-5-yl)methyl)phenyl)-4-((2-methyl-4-phenylthiazol-5-yl)oxy)pyridin-2-amine

¹H-NMR (400 MHz, DMSO-*d*₆): *δ* 9.09(1H,s),8.08(1H,d,*J* = 5.6 Hz), 7.79 (2H, d, *J =* 7.6 Hz), 7.57 (1H, d, *J =* 8.8 Hz), 7.41-7.37 (3H, m), 7.29 (1H, t, *J =* 7.6 Hz), 7.17 (1H, t, *J =* 8.0 Hz), 6.75 (1H, d, *J =* 7.6 Hz), 6.61 (1H, dd, *J =* 6.0, 2.4 Hz), 6.40 (1H, d, *J =* 2.0 Hz), 4.20 (2H, s), 2.67 (3H, s). * A proton from NH was not observed. MS Calcd.: 441.51;MS Found: 442.2 (M+H⁺).

### (113) A-113 : 3-(3-((4-((2-Methyl-4-phenylthiazol-5-yl)oxy)pyridin-2-yl)amino)phenyl)propanenitrile

¹H-NMR (400 MHz, DMSO-*d*₆): *δ* 9.08 (1H, s), 8.09 (1H, d, *J =* 5.6 Hz), 7.80 (2H, d, *J =* 7.6 Hz), 7.51 (1H, d, *J =* 8.0 Hz), 7.43-7.37 (3H, m), 7.30 (1H, t, *J =* 4.8 Hz), 7.17 (1H, t, *J =* 7.8 Hz), 6.80 (1H, d, *J =* 8.0 Hz), 6.61 (1H, dd, *J =* 6.0, 2.4 Hz), 6.43 (1H, d, *J =* 2.4 Hz), 2.79-2.74 (4H, m), 2.68 (3H, s). MS Calcd.: 412.5; MS Found: 413.2 (M+H⁺).

### (114) A-114 : N-(3-(2-(2H-Tetrazol-5-yl)ethyl)phenyl)-4-((2-methyl-4-phenylthiazol-5-yl)oxy)pyridin-2-amine

¹H-NMR (400 MHz, DMSO-*d*₆): *δ* 9.03 (1H, s), 8.09 (1H, d, *J =* 6.0 Hz), 7.80 (2H, d, *J =* 7.6 Hz), 7.44-7.37 (4H, m), 7.30 (1H, t, *J =* 6.0 Hz), 7.11 (1H, t, *J =* 7.4 Hz), 6.72 (1H, d, *J =* 8.4 Hz), 6.60 (1H, dd, *J =* 6.0, 2.4 Hz), 6.41 (1H, s), 3.18-3.07 (2H, m), 2.96-2.91 (2H, m), 2.67 (3H, s). * A proton from NH was not observed. MS Calcd.: 455.53; MS Found: 456.2 (M+H⁺).

### (115) A-115 : 2-(3-((4-((2-Cyclopropyl-4-phenylthiazol-5-yl)oxy)pyridin-2-yl)amino)phenyl)propan-2-ol

¹H-NMR (400 MHz, CDCl₃): *δ* 8.09 (1H, dd, *J =* 5.0, 1.0 Hz), 7.83 (2H, dd, *J =* 8.2, 1.4 Hz), 7.37-7.33 (3H, m), 7.30-7.25 (2H, m), 7.17-7.11 (2H, m), 6.59 (1H, brs), 6.51-6.49 (2H, m), 2.24-2.20 (1H, m), 1.55 (6H, s), 1.14-1.10 (4H, m). * A proton from NH was not observed. MS Calcd.: 443.56; MS Found: 444.2 (M+H⁺).

### (116) A-116 : 3-((4-((2-Cyclopropyl-4-phenylthiazol-5-yl)oxy)pyridin-2-yl)amino)benzenesulfonamide

¹H-NMR (400 MHz, CDCl₃): *δ* 8.12 (1H, d, *J =* 4.0 Hz), 7.95 (1H, s), 7.83 (2H, d, *J =* 8.8 Hz), 7.52-7.47 (2H, m), 7.41-7.31 (3H, m), 7.28-7.27 (1H, m), 6.80 (1H, brs), 6.59 (1H, dd, *J =* 2.4 Hz, 5.4 Hz), 6.42-6.40 (1H, m), 4.89 (2H, brs), 2.29-2.22 (1H, m), 1.17-1.12 (4H, m). MS Calcd.: 464.56; MS Found: 465.1 (M+H⁺).

### (117) A-117 : 3-((4-((2-Cyclopropyl-4-phenylthiazol-5-yl)oxy)pyridin-2-yl)amino)benzamide

¹H-NMR (400 MHz, CDCl₃): *δ* 8.12 (1H, s), 7.86-7.84 (3H, m), 7.49-7.47 (1H, m), 7.43-7.41 (1H, m), 7.39-7.34 (3H, m), 7.29-7.26 (2H, m), 6.54-6.60 (2H, m), 6.45 (1H, s), 2.21-2.30 (1H, m), 1.56-1.13 (4H, m). * A proton from NH was not observed. MS Calcd.: 428.51; MS Found: 429.2 (M+H⁺).

### (118) A-118 : 4-((4-((2-Cyclopropyl-4-phenylthiazol-5-yl)oxy)pyridin-2-yl)amino)benzenesulfonamide

¹H-NMR (400 MHz, DMSO-*d*₆): *δ* 9.53 (1H, s), 8.19 (1H, d, *J =* 5.6 Hz), 7.81-7.77 (4H, m), 7.67 (2H, d, *J =* 9.2 Hz), 7.41 (2H, t, *J =* 8.0 Hz), 7.33-7.30 (1H, m), 7.14 (2H, s), 6.75 (1H, dd, *J* = 5.8, 2.2 Hz), 6.49 (1H, d, *J* = 2.4 Hz), 2.48-2.42 (1H, m), 1.18-1.15 (2H, m), 1.10-1.07 (2H, m). MS Calcd.: 464.56; MS Found: 465.1 (M+H⁺).

### (119) A-119 : 4-((4-((2-Cyclopropyl-4-phenylthiazol-5-yl)oxy)pyridin-2-yl)amino)benzamide

¹H-NMR (400 MHz, DMSO-*d*₆): *δ* 9.39 (1H, s), 8.17 (1H, d, *J =* 5.6 Hz), 7.81-7.75 (5H, m), 7.68 (2H, d, *J =* 8.8 Hz), 7.41 (2H, t, *J =* 7.6 Hz), 7.31 (1H, t, *J =* 7.4 Hz), 7.12 (1H, brs), 6.71 (1H, dd, *J =* 6.0, 2.4 Hz), 6.48 (1H, d, *J =* 2.0 Hz), 2.50-2.42 (1H, m), 1.20-1.15 (2H, m), 1.10-1.07 (2H, m). MS Calcd.: 428.51; MS Found: 429.2 (M+H⁺).

### (120) A-120 : 2-(4-((4-((2-Cyclopropyl-4-phenylthiazol-5-yl)oxy)pyridin-2-yl)amino)pyridin-2-yl)propan-2-ol

¹H-NMR (400 MHz, DMSO-*d*₆): *δ* 9.52 (1H, s), 8.20-8.18 (2H, m), 7.77 (2H, d, *J =* 7.6 Hz), 7.67-7.63 (2H, m), 7.38 (2H, t, *J =* 7.6 Hz), 7.29 (1H, t, *J =* 7.4 Hz), 6.74 (1H, dd, *J =* 6.0, 2.4 Hz), 6.52 (1H, dd, *J =* 2.4 Hz), 5.07 (1H, s), 2.43-7.40 (1H, m), 1.39 (6H, s), 1.17-1.12 (2H, m), 1.08-1.05 (2H, m). MS Calcd.: 444.55; MS Found: 445.2(M+H⁺).

### (121) A-121 : 2-(5-((4-((2-Cyclopropyl-4-phenylthiazol-5-yl)oxy)pyridin-2-yl)amino)pyridin-2-yl)propan-2-ol

¹H-NMR (400 MHz, DMSO-*d*₆): *δ* 9.19 (1H, s), 8.56 (1H, dd, *J =* 2.8 Hz), 8.09 (1H, d, *J =* 6.0 Hz), 8.06 (1H, dd, *J* = 8.8, 2.8 Hz), 7.78 (2H, d, *J =* 7.2 Hz), 7.48 (1H, d, *J =* 8.8 Hz), 7.39 (2H, t, *J =* 7.6 Hz), 7.29 (1H, t, *J =* 7.4 Hz), 6.64 (1H, dd, *J =* 5.8, 2.2 Hz), 6.39 (1H, d, *J* = 2.4 Hz), 5.05 (1H, s), 2.44-2.38 (1H, m), 1.38 (6H, s), 1.17-1.12 (2H, m), 1.07-1.03 (2H, m). MS Calcd.: 444.55; MS Found: 445.2 (M+H⁺).

### (122) A-122 : N-(3-((4-((2-Cyclopropyl-4-phenylthiazol-5-yl)oxy)pyridin-2-yl)amino)phenyl)ethanesulfonamide

¹H-NMR (400 MHz, DMSO-*d*₆): *δ* 9.67 (1H, bs), 9.14 (1H, s), 8.08 (1H, d, *J =* 5.6 Hz), 7.77 (2H, d, *J =* 8.0 Hz), 7.44-7.45 (2H, m), 7.38 (2H, t, *J =* 7.4 Hz), 7.26-7.30 (1H, m), 7.13 (1H, t, *J =* 8.2 Hz), 6.70 (1H, d, *J =* 8.8 Hz), 6.61 (1H, dd, *J =* 8.5 and 2.2 Hz), 6.42 (1H, s), 3.04 (2H, q, *J =* 7.1 Hz), 2.37-2.42 (1H, m), 1.15-1.18 (3H, m), 1.12-1.14 (2H, m), 1.04-1.06 (2H, m). MS Calcd.: 492.13; MS Found: 493.2 (M+H⁺).

### (123) A-123 : N-(3-((4-((2-Cyclopropyl-4-phenylthiazol-5-yl)oxy)pyridin-2-yl)amino)phenyl)-1,1,1-trifluoromethanesulfonamide

¹H-NMR (400 MHz, DMSO-*d*₆): *δ* 11.7 (1H, bs), 9.25 (1H, s), 8.10 (1H, d, *J =* 6.0 Hz), 7.77 (2H, d, *J =* 7.6 Hz), 7.61 (1H, s), 7.53 (1H, d, *J =* 8.4 Hz), 7.38 (2H, t, *J =* 7.6 Hz), 7.29 (1H, t, *J =* 7.0 Hz), 7.21 (1H, t, *J =* 8.2 Hz), 6.73 (1H, d, *J =* 8.0 Hz), 6.34-6.66 (1H, m), 6.42 (1H, s), 2.39-2.45 (1H, m), 1.04-1.24 (4H, m). MS Calcd.: 532.09; MS Found: 533.1 (M+H⁺).

### (124) A-124 : 6-((4-((2-Cyclopropyl-4-phenylthiazol-5-yl)oxy)pyridin-2-yl)amino)picolinamide

¹H-NMR (400 MHz, DMSO-*d*₆): *δ* 9.86 (1H, s), 8.18 (1H, d, *J =* 6.0 Hz), 7.98 (1H, d, *J =* 8.0 Hz), 7.83-7.77 (3H, m), 7.59 (1H, s), 7.50 (1H, d, *J =* 7.6 Hz), 7.46 (1H, s), 7.39 (2H, t, *J =* 7.2 Hz), 7.29 (1H, t, *J =* 6.8 Hz), 7.21 (1H, d, *J =* 2.0 Hz), 6.71 (1H, dd, *J =* 6.0, 4.0 Hz), 2.40 (1H ,m), 1.13-1.11 (2H, m), 1.06-1.05 (2H, m). MS Calcd.: 429.49; MS Found: 430.1 (M+H⁺).

### (125) A-125 : 6-((4-((2-Cyclopropyl-4-phenylthiazol-5-yl)oxy)pyridin-2-yl)amino)-N-methylpicolinamide

¹H-NMR (400 MHz, DMSO-*d*₆): *δ* 9.85 (1H, s), 8.18 (1H, d, *J =* 5.6 Hz), 8.02 (1H, d, *J =* 4.2 Hz), 7.95 (1H, d, *J =* 8.0 Hz), 7.82-7.77 (3H, m), 7.47 (1H, d, *J =* 7.2 Hz), 7.39 (2H, t, *J =* 7. 6 Hz), 7.30-7.25 (2H, m), 6.74 (1H, dd, *J =* 6.0, 2.4 Hz), 2.86 (3H, d, *J =* 5.2 Hz), 2.38-2.37 (1H, m), 1.14-1.10 (2H, m), 1.05-1.02 (2H, m). MS Calcd.: 443.52; MS Found: 444.2 (M+H⁺).

### (126) A-126 : 4-((4-((2-Cyclopropyl-4-phenylthiazol-5-yl)oxy)pyridin-2-yl)amino)-N-methylbenzamide

¹H-NMR (400 MHz, DMSO-*d*₆): *δ* 9.34 (1H, s), 8.15 (2H, d, *J =* 6.0 Hz), 7.78 (2H, d, *J =* 8.0 Hz), 7.71 (4H, q, , *J =* 9.2 Hz), 7.40 (2H, t, *J =* 7.6 Hz), 7.30 (1H, t, *J =* 7.6 Hz), 6.68 (1H, d, *J =* 6.0 Hz), 6.45 (1H, s), 2.73 (3H, d, *J =* 4.0), 2.40 (1H, m), 1.15-1.13 (2H, m), 1.05 (2H, m). MS Calcd.: 442.53; MS Found: 443.2 (M+H⁺).

### (127) A-127 : N-(6-((4-((2-Cyclopropyl-4-phenylthiazol-5-yl)oxy)pyridin-2-yl)amino)pyridin-3-yl)methanesulfonamide

¹H-NMR (400 MHz, DMSO-*d*₆): *δ* 9.79 (1H, s), 9.46 (1H, s), 8.13 (1H, d, *J =* 6.0 Hz), 8.02 (1H, d, *J =* 2.4 Hz), 7.77 (2H, d, *J =* 7.2 Hz), 7.67 (1H, d, *J =* 9.2 Hz), 7.53-7.49 (2H, m), 7.37 (2H, t, *J =* 7.6 Hz), 7.28 (1H, t, *J =* 7.2 Hz), 6.64 (1H, dd, *J =* 6.0, 2.4 Hz), 2.93 (3H, s), 2.41-2.27 (1H, m), 1.15-1.13 (2H, m), 1.06-1.04 (2H, m). MS Calcd.: 479.57; MS Found: 480.2 (M+H⁺).

### (128) A-128 : N-(4-((4-((2-Cyclopropyl-4-phenylthiazol-5-yl)oxy)pyridin-2-yl)amino)phenyl)methanesulfonamide

¹H-NMR (400 MHz, DMSO-*d*₆): *δ* 9.33 (1H, s), 9.07 (1H, s), 8.07 (1H, d, *J =* 5.6 Hz), 7.78 (2H, d, *J =* 7.6 Hz), 7.54 (2H, d, *J =* 8.8 Hz), 7.40 (2H, t, *J =* 7.2 Hz), 7.30 (1H, t, *J =* 7.6 Hz), 7.08 (2H, d, *J =* 8.8 Hz), 6.60 (1H, dd, *J=* 6.0, 2.4 Hz), 6.36 (1H, d, *J =* 2.0 Hz), 2.86 (3H, s), 2.40 (1H, m), 1.14-1.12 (2H, m), 1.14 (2H, m). MS Calcd.: 478.59; MS Found: 479.2 (M+H⁺).

### (129) A-129 : 6-((4-((2-Cyclopropyl-4-phenylthiazol-5-yl)oxy)pyridin-2-yl)amino)nicotinamide

¹H-NMR (400 MHz, DMSO-*d*₆): *δ* 10.09 (1H, s), 8.65 (1H, s), 8.19 (1H, d, *J =* 5.6 Hz), 8.07 (1H, d, *J =* 9.2 Hz), 7.78 (1H, brs), 7.76 (2H, d, *J =* 3.2 Hz), 7.73 (1H, d, *J =* 8.4 Hz), 7.56 (1H, s), 7.39 (2H, t, *J =* 7.6 Hz), 7.92 (2H, t, *J =* 7.2 Hz), 6.71 (1H, dd, *J =* 5.6, 2.4 Hz), 2.42 (1H, m), 1.15-1.11 (2H, m), 1.05-1.04 (2H, m). MS Calcd.: 429.49; MS Found: 430.2 (M+H⁺).

### (130)A-130 : Methyl 6-((4-((2-cyclopropyl-4-phenylthiazol-5-yl)oxy)pyridin-2-yl)amino)nicotinate

¹H-NMR (400 MHz, DMSO-*d*₆): *δ* 10.32 (1H, brs), 8.69 (1H, s), 8.21 (1H, d, *J =* 5.6 Hz), 8.10-8.11 (1H, m), 7.78 (3H, t, *J =* 8.2 Hz), 7.58 (1H, s), 7.37 (2H, t, *J =* 7.6 Hz), 7.28 (1H, t, *J =* 7.2 Hz), 6.75-6.77 (1H, m), 3.81 (3H, s), 2.38-2.43 (1H, m), 1.15-1.21 (2H, m), 1.05-1.11 (2H, m). MS Calcd.: 444.13; MS Found: 445.1 (M+H⁺).

### (131) A-131 : Methyl 6-((4-((2-cyclopropyl-4-phenylthiazol-5-yl)oxy)pyridin-2-yl)amino)picolinate

¹H-NMR (400 MHz, DMSO-*d*₆): *δ* 10.16 (1H, s), 8.18 (1H, d, *J =* 5.6 Hz), 7.91 (1H, d, *J =* 8.8 Hz), 7.84-7.77 (3H, m), 7.72 (1H, s), 7.55 (1H, d, *J =* 7.2 Hz), 7.39 (2H, *J =* 7.6 Hz), 7.30 (1H, t, *J =* 7.2 Hz), 6.71 (1H, d, *J =* 5.6 Hz), 3.79 (3H, s), 2.39-2.38 (1H, m), 1.14-1.11 (2H, m), 1.10-1.03 (2H, m). MS Calcd.: 444.51; MS Found: 445.2 (M+H⁺).

### (132) A-132 : 6-((4-((2-Cyclopropyl-4-phenylthiazol-5-yl)oxy)pyridin-2-yl)amino)nicotinic acid

¹H-NMR (400 MHz, DMSO-*d*₆): *δ* 10.65 (1H, brs), 8.71 (1H, s), 8.25 (1H, d, *J =* 6.0 Hz), 8.14 (1H, d, *J =* 8.4 Hz), 7.76 (2H, d, *J =* 6.8 Hz), 7.61-7.63 (1H, m), 7.44 (1H, brs), 7.38 (2H, t, *J =* 7.6 Hz), 7.27-7.31 (1H, m), 6.87 (1H, brs), 2.34-2.44 (1H, m), 1.14-1.16 (2H, m), 1.06-1.14 (2H, m). * A proton from NH was not observed. MS Calcd.: 430.48; MS Found: 431.1 (M+H⁺).

### (133) A-133 : 6-((4-((2-Cyclopropyl-4-phenylthiazol-5-yl)oxy)pyridin-2-yl)amino)-N-methylnicotinamide

¹H-NMR (400 MHz, DMSO-*d*₆): *δ* 10.08 (1H, s), 8.60 (1H, s), 8.32-8.33 (1H, m), 8.17 (1H, d, *J =* 6.0 Hz), 8.00-8.02 (1H, m), 7.71-7.77 (3H, m), 7.54 (1H, s), 7.36 (2H, t, *J =* 7.6 Hz), 7.26 (1H, t, *J =* 7.0 Hz), 6.70 (1H, dd, *J =* 6.0 and 2.4 Hz), 2.74 (3H, d, *J =* 6.0 Hz), 2.30-2.46 (1H, m), 1.12-1.14 (2H, m), 1.03-1.04 (2H, m). MS Calcd.: 443.53; MS Found: 444.1 (M+H⁺).

### (134) A-134 : 6-((4-((2-Cyclopropyl-4-phenylthiazol-5-yl)oxy)pyridin-2-yl)amino)picolinic acid

¹H-NMR (400 MHz, DMSO-*d*₆): *δ* 10.06 (1H, s), 8.16 (1H, d, *J =* 5.6 Hz), 7.89 (1H, d, *J =* 8.0 Hz), 7.81-7.74 (4H, m), 7.53 (1H, d, *J =* 7.6 Hz), 7.39 (2H, t, *J =* 7.2 Hz), 7.29 (1H, t, *J =* 7.2 Hz), 6.64 (1H, d, *J =* 3.6 Hz), 2.39-2.37 (1H, m), 1.13-1.10 (2H, m), 1.05-1.04 (2H, m). * A proton from OH was not observed. MS Calcd.: 430.48; MS Found: 431.1 (M+H⁺).

### (135) A-135 : 3-((4-((2-Cyclopropyl-4-phenylthiazol-5-yl)oxy)pyridin-2-yl)amino)benzoic acid

¹H-NMR (400 MHz, DMSO-*d*₆): *δ* 12.9 (1H, brs), 9.35 (1H, s), 8.23 (1H, t, *J =* 1.8 Hz), 8.15 (1H, d, *J =* 6.0 Hz), 7.87 (1H, dd, *J =* 7.6, 1.6 Hz), 7.80 (2H, d, *J =* 5.6 Hz), 4.47 (1H, d, *J =* 8.0 Hz), 7.43-7.31 (4H, m), 6.69 (1H, dd, *J =* 5.8, 2.2 Hz), 6.44 (1H, d, *J =* 2.4 Hz), 2.47-2.41 (1H, m), 1.17-1.13 (2H, m), 1.10-1.17 (2H, m). MS Calcd.: 429.49; MS Found: 430.2 (M+H⁺).

### (136) A-136 : 4-((4-((2-Cyclopropyl-4-phenylthiazol-5-yl)oxy)pyridin-2-yl)amino)benzoic acid

¹H-NMR (400 MHz, DMSO-*d*₆): *δ* 9.52 (1H, s), 8.19 (1H, d, *J =* 6.0 Hz), 7.81 (3H, t, *J =* 9.0 Hz), 7.74 (2H, d, *J =* 8.4 Hz), 7.41 (2H, t, *J =* 7.6 Hz), 7.31 (1H, t, *J =* 8.6 Hz), 6.85 (1H, dd, *J =* 7.6, 2.4 Hz), 6.51 (1H, d, *J =* 2.4 Hz), 2.49-2.42 (1H, m), 1.19-1.14 (2H, m), 1.09-1.05 (2H, m). * Two protons from NH and OH was not observed. MS Calcd.: 429.49; MS Found: 430.2 (M+H⁺).

### (137) A-137 : 6-((4-((2-Cyclopropyl-4-phenylthiazol-5-yl)oxy)pyridin-2-yl)amino)picolinic acid

¹H-NMR (400 MHz, DMSO-*d*₆): *δ* 9.40 (1H, s), 8.41 (1H, s), 8.14 (1H, d, *J =* 6.0 Hz), 7.83 (1H, s), 7.68 (2H, d, *J =* 7.6 Hz), 7.33 (2H, t, *J =* 7.2 Hz), 7.23 (1H, t, *J =* 8.0 Hz), 7.14 (2H, s), 6.61 (1H, dd, *J =* 6.0, 2.4 Hz), 6.40 (1H, d, *J* = 2.4 Hz), 3.01 (1H, q, *J* = 3.6 Hz), 1.17-1.10 (4H, m). MS Calcd.: 454.53; MS Found: 455.1 (M+H⁺).

### (138) A-138 : 6-((4-((2-Cyclopropyl-4-phenylthiazol-5-yl)oxy)pyridin-2-yl)amino)-N-hydroxynicotinamide

¹H-NMR (400 MHz, DMSO-*d*₆): *δ* 11.1 (1H, s), 10.2 (1H, brs), 9.01 (1H, brs), 8.53 (1H, s), 8.19 (1H, d, *J =* 6.4 Hz), 7.97 (1H, d, *J =* 9.2 Hz), 7.77 (2H, d, *J =* 7.2 Hz), 7.70 (1H, d, *J =* 8.0 Hz), 7.54 (1H, s), 7.38 (2H, t, *J =* 7.4 Hz), 7.28 (1H, t, *J =* 8.0 Hz), 6.74 (1H, s), 2.48-2.38 (1H, m), 1.18-1.11 (2H, m), 1.09-1.02 (2H, m). MS Calcd.: 445.49; MS Found: 446.2 (M+H⁺).

### (139) A-139 : 4-((4-((2-Cyclopropyl-4-phenylthiazol-5-yl)oxy)pyridin-2-yl)amino)-N-hydroxybenzamide

¹H-NMR (400 MHz, DMSO-*d*₆): *δ* 11.0 (1H, s), 9.36 (1H, s), 8.82 (1H, s), 8.15 (1H, d, *J =* 6.0 Hz), 7.77 (2H, d, *J =* 7.6 Hz), 7.64 (3H, q, *J =* 8.1 Hz), 7.38 (2H, t, *J =* 7.6 Hz), 7.29 (1H, t, *J =* 7.4 Hz), 6.69 (1H, dd, *J =* 8.4, 2.4 Hz), 6.45 (1H, d, *J =* 2.4 Hz), 2.44-2.38 (1H, m), 1.18-1.12 (2H, m), 1.08-1.03 (2H, m). * A proton from OH was not observed. MS Calcd.: 444.51; MS Found: 445.2 (M+H⁺).

### (140) A-140 : 6-((4-((2-Cyclopropyl-4-phenylthiazol-5-yl)oxy)pyridin-2-yl)amino)-N-(methylsulfonyl)nicotinamide

¹H-NMR (400 MHz, DMSO-*d*₆): *δ* 9.97 (1H, s), 8.45 (1H, s), 8.16 (1H, d, *J =* 5.2 Hz), 8.05 (1H, dd, *J* = 8.8, 2.0 Hz), 7.78 (2H, d, *J =* 7.6 Hz), 7.62 (1H, d, *J =* 8.8 Hz), 7.58 (1H, s), 7.38 (2H, t, *J =* 7.6 Hz), 7.27 (1H, t, *J =* 7.4 Hz), 6.84 (1H, d, *J =* 8.4 Hz), 6.68 (1H, d, *J =* 3.2 Hz), 2.86 (3H, s), 2.46-2.37 (1H, m), 1.18-1.12 (2H, m), 1.09-1.02 (2H, m). MS Calcd.: 507.58; MS Found: 508.1 (M+H⁺).

### (141) A-141 : 2-(3-((4-((2-Methoxy-4-phenylthiazol-5-yl)oxy)pyridin-2-yl)amino)phenyl)propan-2-ol

¹H-NMR (400 MHz, CDCl₃): *δ* 8.10 (1H, d, *J =* 6.0 Hz), 7.82-7.80 (2H, m), 7.36-7.33 (3H, m), 7.30-7.26 (2H, m), 7.16 (1H, dd, *J* = 8.4, 1.2 Hz), 7.11 (1H, dd, *J* = 8.4, 1.2 Hz), 6.68 (1H, brs), 6.57 (1H, d, *J =* 2.4 Hz), 6.55 (1H, dd, *J =* 5.8, 2.2 Hz), 4.12 (3H, s), 1.55 (6H, s). * A proton from NH was not observed. MS Calcd.: 433.52; MS Found: 434.1 (M+H⁺).

### (142) A-142 : 3-((4-((2-Methoxy-4-phenylthiazol-5-yl)oxy)pyridin-2-yl)amino)benzenesulfonamide

¹H-NMR (400 MHz, CDCl₃): *δ* 8.16 (1H, d, *J =* 6.0 Hz), 8.02-7.98 (1H, m), 7.82 (2H, d, *J =* 7.6 Hz), 7.55-7.52 (1H, m), 7.49-7.40 (2H, m), 7.37-7.34 (2H, m), 7.29-7.26 (1H, m), 6.64 (2H, dd, *J =* 5.6, 2.0 Hz), 6.49 (1H, d, *J =* 2.0 Hz), 4.75 (2H, s), 4.15 (3H, s). MS Calcd.: 454.52; MS Found: 455.1 (M+H⁺).

### (143) A-143 : 3-((4-((2-Methoxy-4-phenylthiazol-5-yl)oxy)pyridin-2-yl)amino)benzamide

¹H-NMR (400 MHz, DMSO-*d*₆): *δ* 9.25 (1H, s), 8.14 (1H, d, *J =* 6.0 Hz), 8.02 (1H, t, *J =* 2.0 Hz), 7.86-7.77 (4H, m), 7.43-7.27 (6H, m), 6.67 (1H, dd, *J =* 6.0, 2.4 Hz), 6.52 (1H, d, *J =* 2.4 Hz), 4.13 (3H, s). MS Calcd.: 418.47; MS Found: 419.1 (M+H⁺).

### (144) A-144 : 3-((4-((2-Methoxy-4-phenylthiazol-5-yl)oxy)pyridin-2-yl)amino)benzoic acid

¹H-NMR (400 MHz, DMSO-*d*₆): *δ* 12.8 (1H, brs), 9.39 (1H, s), 8.23 (1H, s), 8.16 (1H, d, *J =* 6.0 Hz), 7.87 (1H, d, *J =* 6.8 Hz), 7.78 (2H, d, *J =* 7.6 Hz), 7.48 (1H, d, *J =* 7.2 Hz), 7.44-7.30 (4H, m), 6.71 (1H, d, *J =* 6.0 Hz), 6.53 (1H, s), 4.13 (3H, s). MS Calcd.: 419.45; MS Found: 420.1 (M+H⁺).

### (145) A-145 : 3-((4-((4-Phenyl-2-(trifluoromethyl)thiazol-5-yl)oxy)pyridin-2-yl)amino)benzenesulfonamide

¹H-NMR (400 MHz, DMSO-*d*₆): *δ* 9.46 (1H, s), 8.20 (1H, d, *J =* 6.0 Hz), 8.17 (1H, s), 7.87 (2H, d, *J =* 7.2 Hz), 7.81 (1H, d, *J =* 8.4 Hz), 7.49 (2H, t, *J =* 7.4 Hz), 7.45-7.41 (2H, m), 7.34 (1H, d, *J =* 7.6 Hz), 7.30 (2H, s), 6.80 (1H, dd, *J =* 5.8, 2.2 Hz), 6.52 (1H, d, *J =* 2.4 Hz). MS Calcd.: 492.49; MS Found: 493.1 (M+H⁺).

### (146) A-146 : 6-((4-((4-Phenyl-2-(trifluoromethyl)thiazol-5-yl)oxy)pyridin-2-yl)amino)nicotinamide

¹H-NMR (400 MHz, DMSO-*d*₆): *δ* 10.15 (s, 1H), 8.66 (d, *J =* 2.1 Hz, 1H), 8.23 (d, *J* = 6.0 Hz, 1H), 8.07 (dd, *J* = 8.8, 2.4 Hz, 1H), 7.88-7.83 (m, 3H), 7.71 (d, *J =* 8.8 Hz, 1H), 7.64 (d, *J =* 2.4 Hz, 1H), 7.46 (t, *J =* 7.2 Hz, 2H), 7.39 (t, *J =* 7.6 Hz, 1H), 7.29 (s, 1H), 6.85 (dd, *J* = 5.2, 1.6 Hz, 1H). MS Calcd.: 457.43; Found: 458.15.(M+H⁺).

### (147) A-147 : 4-((4-((4-Phenyl-2-(trifluoromethyl)thiazol-5-yl)oxy)pyridin-2-yl)amino)benzamide

¹H-NMR (400 MHz, DMSO-*d*₆): *δ* 9.37 (s, 1H), 8.19 (d, *J* = 5.6 Hz, 1H), 7.84 (d, *J* = 7.2 Hz, 2H), 7.77-7.75 (m, 3H), 7.66 (d, *J* = 9.2 Hz, 2H), 7.47 (t, *J* = 7.2 Hz, 1H), 7.10 (s, 1H), 6.78 (dd, *J =* 5.6, 2.4 Hz, 1H), 6.51 (d, *J =* 2.4 Hz, 1H). MS Calcd.: 456.44; MS Found: 457.22 (M+H⁺).

### (148) A-148 : N-Methyl-4-((4-((4-phenyl-2-(trifluoromethyl)thiazol-5-yl)oxy)pyridin-2-yl)amino)benzamide

¹H-NMR (400 MHz, DMSO-*d*₆): *δ* 9.36 (s, 1H), 8.20-8.17 (m, 2H), 7.84 (d, *J =* 7.6 Hz, 2H), 7.71 (d, *J =* 8.0, 4.0 Hz, 2H), 7.66 (d, *J =* 9.2 Hz, 2H), 7.47 (t, *J =* 7.6 Hz, 2H), 7.40 (t, *J =* 6.8 Hz, 1H), 6.78 (dd, *J =* 6.0, 2.4 Hz, 1H), 6.51 (d, *J =* 2.4 Hz, 1H), 2.73 (d, *J =* 4.8 Hz, 3H). MS Calcd.: 470.47; MS Found: 471.22 (M+H⁺).

### (149) A-149 : 6-((4-((4-Phenyl-2-(trifluoromethyl)thiazol-5-yl)oxy)pyridin-2-yl)amino)nicotinic acid

¹H-NMR (400 MHz, DMSO-*d*₆): *δ* 11.50 (br s, 1H), 8.73 (s, 1H), 8.31 (d, *J =* 6.4 Hz, 1H), 8.19 (d, *J* = 8.8 Hz, 1H), 7.83 (d, *J =* 8.0 Hz, 2H), 7.58 (d, *J =* 8.8 Hz, 1H), 7.50-7.44 (m, 1H), 7.05 (d, *J=* 4.0 Hz, 1H). MS Calcd.: 458.42; MS Found: 459.20 (M+H⁺)

### (150) A-150 : N-Methyl-6-((4-((4-phenyl-2-(trifluoromethyl)thiazol-5-yl)oxy)pyridin-2-yl)amino)nicotinamide

¹H-NMR (400 MHz, DMSO-*d*₆): *δ* 10.15 (s, 1H), 8.61 (s, 1H), 8.34 (br s, 1H), 8.23 (d, *J =* 6.0 Hz, 1H), 8.03 (dd, *J =* 8.0, 1.6 Hz, 1H), 7.84 (d, *J =* 8.0 Hz, 1H), 7.84 (d, *J =* 8.0 Hz, 1H), 7.73 (d, *J =* 8.4 Hz, 1H), 7.62 (d, *J =* 2.0 Hz, 1H), 7.46 (t, *J =* 7.6 Hz, 2H), 7.40-7.39 (m, 1H), 6.85 (dd, *J =* 5.6, 2.8 Hz, 1H), 2.75 (d, *J =* 4.0 Hz, 3H). MS Calcd.: 471.46; MS Found: 472.21 (M+H⁺).

### (151) A-151 : 3-((4-((4-Phenyl-2-(trifluoromethyl)thiazol-5-yl)oxy)pyridin-2-yl)amino)benzoic acid

¹H-NMR (400 MHz, DMSO-*d*₆): *δ* 9.34 (1H, s), 8.19 (2H, d, *J =* 6.0 Hz), 7.87 (2H, d, *J =* 6.8 Hz), 7.49-7.42 (4H, m), 7.36-7.30 (2H, m), 6.77 (1H, d, *J =* 4.8 Hz), 6.50 (1H, s). * A proton from OH was not observed. MS Calcd.: 457.43; MS Found: 458.1 (M+H⁺).

### (152) A-152 : 4-((4-((4-Phenyl-2-(trifluoromethyl)thiazol-5-yl)oxy)pyridin-2-yl)amino)benzoic acid

¹H-NMR (400 MHz, DMSO-*d*₆): *δ* 12.4 (1H, brs), 9.52 (1H, s), 8.24 (1H, d, *J =* 6.0 Hz), 7.87-7.82 (4H, m), 7.74 (2H, d, *J =* 8.4 Hz), 7.49 (2H, t, *J =* 7.4 Hz), 7.42 (1H, t, *J =* 7.4 Hz), 6.84 (1H, dd, *J =* 6.0, 2.4 Hz), 6.57 (1H, d, *J =* 2.0 Hz). MS Calcd.: 457.43; MS Found: 458.1 (M+H⁺).

### (153) A-153 : 2-(3-((4-((2-(Dimethylamino)-4-phenylthiazol-5-yl)oxy)pyridin-2-vl)amino)phenyl)propan-2-ol

¹H-NMR (400 MHz, CDCl₃): *δ* 8.07 (1H, d, *J =* 6.0 Hz), 7.83-7.80 (2H, m), 7.52-7.27 (3H, m), 7.25-7.21 (2H, m), 7.15-7.09 (2H, m), 6.72 (1H, brs), 6.58 (1H, d, *J =* 2.4 Hz), 6.56-6.54 (1H, m), 3.10 (6H, s), 1.55 (6H, s). *A proton from NH was not observed. MS Calcd.: 446.56; MS Found: 447.2 (M+H⁺).

### (154) A-154 : 3-((4-((2-(Dimethylamino)-4-phenylthiazol-5-yl)oxy)pyridin-2-yl)amino)benzenesulfonamide

¹H-NMR (400 MHz, DMSO-d₆): *δ* 9.43 (1H, s), 8.20 (1H, t, *J =* 2.0 Hz), 8.14 (1H, d, *J =* 6.0 Hz), 7.81-7.77 (3H, m), 7.43-7.35 (3H, m), 7.32-7.25 (4H, m), 6.67 (1H, dd, *J =* 5.6 Hz, 2.0 Hz), 6.52 (1H, d, *J =* 2.4 Hz), 3.08 (6H, s). MS Calcd.: 467.56; MS Found: 468.2 (M+H⁺).

### (155) A-155 : 3-((4-((2-(Dimethylamino)-4-phenylthiazol-5-yl)oxy)pyridin-2-yl)amino)benzamide

¹H-NMR (400 MHz, DMSO-*d*₆): *δ* 9.22 (1H, s), 8.11 (1H, d, *J =* 6.0 Hz), 8.02 (1H, s), 7.83 (1H, d, *J* =9.6 Hz), 7,78 (3H, d, *J =* 7.2 Hz), 7.38-7.34 (3H, m), 7.30-7.24 (3H, m), 6.64 (1H, dd, *J =* 2.0 Hz, 5.0 Hz), 6.51 (1H, dd, *J* =2.4 Hz), 3.08 (6H, s). MS Calcd.: 431.51; MS Found: 432.2 (M+H⁺).

### (156) A-156 : 4-((4-((2-(Dimethylamino)-4-phenylthiazol-5-yl)oxy)pyridin-2-yl)amino)benzenesulfonamide

¹H-NMR (400 MHz, DMSO-*d*₆): *δ* 9.52 (1H, s), 8.17 (1H, d, *J =* 5.6 Hz), 7.80-7.77 (4H, m), 7.66 (2H, d, *J =* 9.2 Hz), 7.37 (2H, t, *J =* 7.2 Hz), 7.27 (1H, t, *J =* 7.2 Hz), 7.19 (2H, s), 6.73 (1H, dd, *J* = 2.0 Hz, 5.8 Hz), 6.56 (1H, d, *J =* 1.6 Hz), 3.08 (6H, s). MS Calcd.: 467.56; MS Found: 468.2 (M+H⁺).

### (157) A-157 : 4-((4-((2-(Dimethylamino)-4-phenylthiazol-5-yl)oxy)pyridin-2-yl)amino)benzamide

¹H-NMR (400 MHz, DMSO-*d*₆): *δ* 9.38 (1H, s), 8.16 (1H, d, *J =* 6.0 Hz), 7.79-7.75 (4H, m), 7.68 (2H, d, *J =* 8.4 Hz), 7.37 (2H, t, *J =* 8.0 Hz), 7.28-7.25 (1H ,m), 7.11 (1H, m), 6.70 (1H, dd, *J =* 2.0 Hz, 5.8 Hz), 6.54-6.50 (2H, m), 3.08 (6H, s). MS Calcd.: 431.51; MS Found: 432.2 (M+H⁺).

### (158) A-158 : 2-(4-((4-((2-(Dimethylamino)-4-phenylthiazol-5-yl)oxy)pyridin-2-yl)amino)pyridin-2-yl)propan-2-ol

¹H-NMR (400 MHz, DMSO-*d*₆): *δ* 9.51 (1H, s), 8.17 (2H, d, *J =* 5.6 Hz), 7.75 (2H, d, *J =* 7.2 Hz), 7.68 (1H, d, *J =* 1.2 Hz), 7.63-7.61 (1H, m), 7.36-7.32 (2H, m), 7.26-7.22 (1H, m), 6.72 (1H, dd, *J =* 2.4 Hz, 5.8 Hz), 6.58 (1H, d, *J =* 2.0 Hz), 5.06 (1H, s), 3.06 (6H, s), 1.36 (6H, s). MS Calcd.: 447.55; MS Found: 448.2 (M+H⁺).

### (159) A-159 : 2-(5-((4-((2-(Dimethylamino)-4-phenylthiazol-5-yl)oxy)pyridin-2-yl)amino)pyridin-2-yl)propan-2-ol

¹H-NMR (400 MHz, DMSO-*d*₆): *δ* 9.18 (1H, s), 8.57 (1H, d, *J =* 2.8 Hz), 8.08-8.05 (2H, m), 7.76 (2H, d, *J =* 6.8 Hz), 7.48 (1H, d, *J =* 8.8 Hz), 7.35 (2H, t, *J =* 7.8 Hz), 7.25 (1H, t, *J =* 7.4 Hz), 6.63 (1H, dd, *J =* 5.4, 2.2 Hz), 6.46 (1H, d, *J =* 2.0 Hz), 5.04 (1H, s), 3.06 (6H, s), 1.38 (6H, s). MS Calcd.: 447.55; MS Found: 448.2 (M+H⁺).

### (160) A-160 : 6-((4-((2-(Dimethylamino)-4-phenylthiazol-5-yl)oxy)pyridin-2-yl)amino)picolinamide

¹H-NMR (400 MHz, DMSO-*d*₆): *δ* 9.84 (1H, s), 8.16 (1H, d, *J =* 5.6 Hz), 8.00 (1H, d, *J* = 8.8 Hz), 7.83-7.76 (3H, m), 7.56 (1H, s), 7.50-7.47 (2H, m), 7.34 (2H, t, *J =* 7.6 Hz), 7.27-7.25 (2H, m), 6.71 (1H, dd, *J* = 6.0, 2.4Hz), 3.06 (6H, s). MS Calcd.: 432.50; MS Found: 433.2 (M+H⁺).

### (161) A-161 : 6-((4-((2-(Dimethylamino)-4-phenylthiazol-5-yl)oxy)pyridin-2-yl)amino)-N-methylpicolinamide

¹H-NMR (400 MHz, DMSO-*d*₆): *δ* 9.83 (1H, s), 8.17 (1H, d, *J =* 5.6), 8.02-7.95 (2H, m), 7.82-7.49 (3H, m), 7.47 (1H, d, *J =* 7.2 Hz), 7.35 (2H, t, *J =* 7.6 Hz), 7.26-7.22 (2H, m), 6.72 (1H, d, *J =* 5.6 Hz), 3.05 (6H, s), 2.77 (3H, d, *J =* 4.8 Hz). MS Calcd.: 446.52; MS Found: 447.2 (M+H⁺).

### (162) A-162 : Methyl 6-((4-((2-(dimethylamino)-4-phenylthiazol-5-yl)oxy)pyridin-2-yl)amino)picolinate

¹H-(400 MHz, DMSO-*d*₆): *δ* 10.1 (1H, s), 8.15 (1H, d, *J =* 5.2 Hz), 7.92 (1H, d, *J* = 8.4 Hz), 7.83-7.75 (4H, m), 7.54 (1H, d, *J =* 7.2 Hz), 7.34 (2H, t, *J =* 7.6 Hz), 7.24 (1H, t, *J =* 8.0 Hz), 6.68 (1H, dd, *J =* 5.8, 2.2 Hz), 3.80 (3H, s), 3.06 (6H, s). MS Calcd.: 447.51; MS Found: 448.2 (M+H⁺).

### (163) A-163 : 6-((4-((2-(Dimethylamino)-4-phenylthiazol-5-yl)oxy)pyridin-2-yl)amino)nicotinamide

¹H-NMR (400 MHz, DMSO-*d*₆): *δ* 10.1 (1H, s), 8.66 (1H, d, *J =* 2.4 Hz), 8.17 (1H, d, *J* = 6.0 Hz), 8.06 (1H, dd, *J =* 8.8, 2.0 Hz), 7.89 (1H, s), 7.75 (3H, d, *J* = 8.8 Hz), 7.58 (1H, d, *J =* 2.0 Hz), 7.34 (2H, t, *J =* 7.6 Hz), 7.29 (1H, s), 7.24 (1H, t, *J =* 7.6 Hz), 6.70 (1H, dd, *J* = 6.0, 2.4 Hz), 3.06 (6H, s). MS Calcd.: 432.50; MS Found: 433.2 (M+H⁺).

### (164) A-164 : 4-((4-((2-(Dimethylamino)-4-phenylthiazol-5-yl)oxy)pyridin-2-yl)amino)-N-methylbenzamide

¹H-NMR (400 MHz, DMSO-*d*₆): *δ* 9.35 (1H, s), 8.17-8.13 (2H, m), 7.76 (2H, d, *J* = 7.2 Hz), 7.68 (4H, q, *J =* 7.9 Hz), 7.35 (2H, t, *J =* 7.8 Hz), 7.25 (1H, t, *J =* 7.6 Hz), 6.68 (1H, dd, *J =* 5.6, 2.4 Hz), 6.52 (1H, d, *J =* 2.0 Hz), 3.06 (6H, s), 2.73 (3H, d, *J =* 4.4 Hz). MS Calcd.: 445.54; MS Found: 446.2 (M+H⁺).

### (165) A-165 : 6-((4-((2-(Dimethylamino)-4-phenylthiazol-5-yl)oxy)pyridin-2-yl)amino)nicotinic acid

¹H-NMR (400 MHz, DMSO-*d*₆): *δ* 8.71 (1H, s), 8.23 (1H, d, *J =* 6.0 Hz), 8.13 (1H, d, *J =* 8.0 Hz), 7.75 (2H, d, *J =* 7.6 Hz), 7.66 (1H, s), 7.48 (1H, s), 7.35 (2H, t, *J =* 7.6 Hz), 7.25 (1H, t, *J* = 7.4 Hz), 6.85 (1H, s), 3.06 (6H, s). * Two protons from NH and OH were not observed. MS Calcd.: 433.48; MS Found: 434.1 (M+H⁺).

### (166) A-166 : 6-((4-((2-(Dimethylamino)-4-phenylthiazol-5-yl)oxy)pyridin-2-yl)amino)-N-methylnicotinamide

¹H-NMR (400 MHz, DMSO-*d*₆): *δ* 10.1 (1H, s), 8.62 (1H, d, *J =* 2.4 Hz), 8.35 (1H, d, *J =* 4.8 Hz), 8.17 (1H, d, *J =* 5.6 Hz), 8.02 (1H, d, *J =* 8.4 Hz), 7.76 (3H, d, *J =* 8.4 Hz), 7.56 (1H, s), 7.34 (2H, t, *J =* 7.8 Hz), 7.24 (1H, t, *J =* 7.2 Hz), 6.70 (1H, dd, *J =* 6.0, 2.4 Hz), 3.06 (6H, s), 2.75 (3H, d, *J =* 4.0 Hz). MS Calcd.: 446.52; MS Found: 447.2 (M+H⁺).

### (167) A-167 : 3-((4-((2-(Dimethylamino)-4-phenylthiazol-5-yl)oxy)pyridin-2-yl)amino)benzoic acid

¹H-NMR (400 MHz, DMSO-*d₆*): *δ* 9.38 (1H, s), 8.23 (1H, s), 8.13 (1H, d, *J =* 5.6 Hz), 7.86 (1H, d, *J =* 8.0 Hz), 7.78 (2H, d, *J =* 8.4 Hz), 7.47 (1H, d, *J =* 7.2 Hz), 7.39-7.33 (3H, m), 7.29-7.27 (1H, m), 6.68 (1H, dd, *J =* 2.4 Hz, 5.6 Hz), 6.52 (1H, d, *J =* 2.0 Hz), 3.08 (6H, s). *A proton from OH was not observed. MS Calcd.: 432.49; MS Found: 433.2 (M+H⁺).

### (168) A-168 : 4-((4-((2-(Dimethylamino)-4-phenylthiazol-5-yl)oxy)pyridin-2-yl)amino)benzoic acid

¹H-NMR (400 MHz, DMSO-*d*₆): *δ* 9.49 (1H, s), 8.16 (1H, d, *J =* 6.0 Hz), 7.79 (4H, t, *J =* 7.6 Hz), 7.69 (2H, d, *J =* 8.4 Hz), 7.37 (2H, t, *J =* 7.6 Hz), 7.27 (1H, t, *J =* 7.2 Hz), 6.71 (1H, d, *J =* 3.2 Hz), 6.56 (1H, d, *J =* 2.0 Hz), 3.08 (6H, s). * A proton from NH was not observed. MS Calcd.: 432.49; MS Found: 433.2 (M+H⁺).

### (169) A-169 : 6-((4-((2-(Dimethylamino)-4-phenylthiazol-5-yl)oxy)pyridin-2-yl)amino)picolinic acid

¹H-NMR (400 MHz, DMSO-*d*₆): *δ* 10.1 (1H, s), 8.14 (1H, d, *J =* 5.6 Hz), 7.89 (1H, d, *J =* 8.8 Hz), 7.82-7.74 (4H, m), 7.53 (1H, d, *J =* 6.8 Hz), 7.34 (2H, t, *J =* 7.6 Hz), 7.23 (1H, t, *J =* 8.0 Hz), 6.64 (1H, m), 3.05 (6H, s). * A proton from OH was not observed. MS Calcd.: 433.48; MS Found: 434.1 (M+H⁺).

### (170) A-170 : Methyl 3-((4-((2-bromo-4-phenylthiazol-5-yl)oxy)pyridin-2-yl)amino)benzoate

¹H-NMR (400 MHz, DMSO-*d*₆): *δ* 9.38 (1H, s), 8.26 (1H, s), 8.18 (1H, d, *J =* 5.6 Hz), 7.92 (1H, d, *J =* 8.0 Hz), 7.79 (2H, d, *J =* 8.4 Hz), 7.49-7.35 (5H, m), 6.74 (1H, dd, *J =* 5.8, 2.2 Hz), 6.49 (1H, d, *J =* 2.4 Hz), 3.84 (3H, s). MS Calcd.: 482.35; MS Found: 484.0 (M+H⁺).

### (171) A-171 : 3-((4-((2-Bromo-4-phenylthiazol-5-yl)oxy)pyridin-2-yl)amino)benzoic acid

¹H-NMR (400 MHz, CDCl₃): *δ* 9.85 (1H, s), 8.05-8.01 (2H, m), 7.85 (1H, d, *J =* 7.6 Hz), 7.78 (2H, d, *J =* 8.0 Hz), 7.52-7.29 (5H, m), 6.69 (1H, d, *J =* 2.0 Hz), 6.58 (1H, dd, *J =* 6.6, 2.2 Hz). * A proton from OH was not observed. MS Calcd.: 468.32; MS Found: 469.0 (M+H⁺).

### (172) A-172 : 3-((4-((2-Bromo-4-phenylthiazol-5-yl)oxy)pyridin-2-yl)amino)benzamide

¹H-NMR (400 MHz, MeOD-*d*₄): *δ*8.15(1H,d,*J* = 6.0 Hz), 7.89 (1H, d, *J =* 8.0 Hz), 7.74 (2H, d, *J =* 7.2 Hz), 7.61 (1H, d, *J =* 2.0 Hz), 7.50 (1H, t, *J =* 8.0 Hz), 7.44 (1H, s), 7.30 (2H, t, *J =* 7.2 Hz), 7.28-7.24 (1H, m), 7.12 (1H, d, *J =* 8.0 Hz), 6.98 (1H, dd, *J =* 5.8, 2.2 Hz). * Three protons from NH and NH₂ were not observed. MS Calcd.:467.34; MS Found: 468.0 (M+H⁺).

### (173) A-173 : 6-((4-((2-Cyclopentyl-4-phenylthiazol-5-yl)oxy)pyridin-2-yl)amino)nicotinamide

¹H-NMR (400 MHz, DMSO-*d*₆): *δ* 10.1 (1H, s), 8.64 (1H, d, *J =* 2.0 Hz), 8.19 (1H, d, *J =* 5.6 Hz), 8.60 (1H, dd, *J =* 9.0, 2.2 Hz), 7.88 (1H, s), 7.81 (2H, d, *J =* 7.2 Hz), 7.69 (1H, d, *J =* 8.8 Hz), 7.61 (1H, d, *J =* 2.0 Hz), 7.39 (2H, t, *J =* 7.4 Hz), 7.29 (2H, t, *J =* 7.4 Hz), 6.72 (1H, dd, *J =* 5.6, 2.4 Hz), 3.45 (1H, quint, *J* = 7.8 Hz), 2.20-2.09 (2H, m), 1.87-1.74 (4H, m), 1.71-1.65 (2H, m). MS Calcd.: 457.55; MS Found: 458.2 (M+H⁺).

### (174) A-174 : 6-((4-((2-Cyclopentyl-4-phenylthiazol-5-yl)oxy)pyridin-2-yl)amino)nicotinic acid

¹H-NMR (400 MHz, DMSO-*d*₆): *δ* 10.70 (1H, brs), 8.69 (1H, s), 8.25 (1H, d, *J =* 5.6 Hz), 8.13 (1H, d, *J* = 8.0 Hz), 7.80 (2H, d, *J =* 7.6 Hz), 7.61 (1H, d, *J =* 7.6 Hz), 7.51 (1H, brs), 7.40 (2H, t, *J =* 7.6 Hz), 7.31 (1H, t, *J =* 7.2 Hz), 6.87 (1H, s), 3.37-3.44 (1H, t, *J =* 8.0 Hz), 2.15-2.13 (2H, m), 1.86-1.85 (2H, m), 1.86-1.83 (2H, m), 1.82-1.75 (2H, m), 1.68 -1.67 (2H, m). * A proton from OH was not observed. MS Calcd.: 458.53; MS Found: 459.2 (M+H⁺).

### (175) A-175 : 4-((4-((2-Cyclopentyl-4-phenylthiazol-5-yl)oxy)pyridin-2-yl)amino)benzoic acid

¹H-NMR (400 MHz, DMSO-*d*₆): *δ* 9.52 (1H, s), 8.18 (1H, d, *J =* 6.0 Hz), 7.81-7.79 (4H, m), 7.22 (2H, d, *J =* 8.8 Hz), 7.41 (2H, t, *J =* 8.0 Hz), 7.31 (1H, t, *J =* 7.2 Hz), 6.73 (1H, dd, *J =* 5.6, 2.0 Hz), 6.50 (1H, d, *J =* 2.0 Hz), 3.46 (1H, t, *J =* 7.6 Hz), 2.14-2.12 (2H, m), 1.85-1.76 (4H, m), 1.68-1.66 (2H, m). * A proton from OH were not observed. MS Calcd.: 457.54; MS Found: 458.2 (M+H⁺).

### (176) A-176 : 6-((4-((2-Cyclopentyl-4-phenylthiazol-5-yl)oxy)pyridin-2-yl)amino)-N-methylnicotinamide

¹H-NMR (400 MHz, DMSO-*d*₆): *δ* 10.12 ( 1H, s), 8.59 (1H, s), 8.34 (1H, d, *J =* 4.0 Hz), 8.19 (1H, d, *J* = 5.6 Hz), 8.03 (1H, d, *J* = 10.8 Hz), 7.81 (2H, d, *J* = 7.6 Hz), 7.71 (1H, d, *J =* 9.6 Hz), 7.59 (1H, s), 7.40 (2H, t, *J =* 8.0 Hz), 7.30 (1H, t, *J =* 7.2 Hz), 6.73 (1H, dd, *J =* 5.6, 2.4 Hz), 3.44 (1H, t, *J =* 8.0 Hz), 2.75 (3H, d, *J =* 4.8 Hz), 2.15-2.13 (2H, m), 1.84-1.76 (4H, m), 1.66 (2H, m). MS Calcd.: 471.17; MS Found: 472.2 (M+H⁺).

### (177) A-177 : 6-((4-((2-Cyclohexyl-4-phenylthiazol-5-yl)oxy)pyridin-2-yl)amino)nicotinamide

¹H-NMR (400 MHz, CDCl₃): *δ* 8.67 (1H, d, *J =* 2.0 Hz), 8.15 (1H, d, *J =* 6.0 Hz), 8.08 (1H, dd, *J =* 8.8, 2.4 Hz), 7.83 (2H, d, *J =* 7.6 Hz), 7.60 (1H, s), 7.56 (1H, d, *J =* 8.8 Hz), 7.36 (2H, t, *J =* 7.2 Hz), 7.28 (1H, t, *J =* 7.2 Hz), 6.68 (1H, dd, *J=* 5.2, 2.0 Hz), 3.03-2.97 (1H, m), 2.21-2.20 (2H, m), 1.91-1.88 (2H, m), 1.78-1.75 (1H, m), 1.61-1.32 (5H, m). * Three protons from NH and NH₂ were not observed. MS Calcd.: 471.57; MS Found: 472.2 (M+H⁺).

### (178) A-178 : 6-((4-((2-cyclohexyl-4-phenylthiazol-5-yl)oxy)pyridin-2-yl)amino)nicotinic acid

¹H-NMR (400 MHz, DMSO-*d*₆): *δ* 11.12 (1H, brs), 8.73 (1H, s), 8.30 (1H, d, *J =* 6.0 Hz), 8.20 (1H, d, *J =* 8.4 Hz), 7.80 (2H, d, *J =* 7.6 Hz), 7.50 (1H, d, *J =* 8.4 Hz), 7.41-7.37 (3H, m), 7.32 (1H, t, *J =* 7.6 Hz), 6.97 (1H, s), 3.03 (1H, m), 2.12 (2H, m), 1.81-1.78 (2H, m), 1.69-1.66 (1H, m), 1.55-1.45 (2H, m), 1.42-1.36 (2H, m), 1.27-1.21 (1H, m).* A proton from OH was not observed. MS Calcd.: 472.56; MS Found: 473.2 (M+H⁺).

### (179) A-179 : 4-((4-((2-Cyclohexyl-4-phenylthiazol-5-yl)oxy)pyridin-2-yl)amino)benzoic acid

¹H-NMR (400 MHz, DMSO-*d*₆): *δ* 9.51 (1H, s), 8.18 (1H, d, , *J =* 5.6 Hz), 7.81-7.78 (4H, m), 7.72 (2H, d, *J =* 8.8 Hz), 7.41 (2H, t, *J =* 8.0 Hz), 7.31 (1H, t, *J =* 7.2 Hz), 6.71 (1H, d, *J =* 4.4 Hz), 6.48 (1H, s), 2.97-2.91 (1H, m), 2.11-2.09 (2H, m), 1.77-1.75 (2H, m), 1.66-1.65 (1H, m), 2.53-1.38 (4H, m), 1.26-1.23 (1H, m). * A proton from OH was not observed. MS Calcd.: 471.57; MS Found: 472.2 (M+H⁺).

### (180) A-180 : 6-((4-((2-Cyclohexyl-4-phenylthiazol-5-yl)oxy)pyridin-2-yl)amino)-N-methylnicotinamide

¹H-NMR (400 MHz, DMSO-*d*₆): *δ* 10.13 (1H, s), 8.60 (1H, s), 8.33 (1H, s), 8.19 (1H, d, *J =* 5.6 Hz), 8.03 (1H, d, *J =* 8.8 Hz), 7.81 (2H, d, *J =* 8.0 Hz), 7.69 (1H, d, *J =* 9.6 Hz), 7.60 (1H, s), 7.40 (2H, t, *J =* 7.6 Hz), 7.30 (1H, t, *J =* 7.2 Hz), 6.72 (1H, d, *J =* 5.6 Hz), 2.98-2.91 (1H, m), 2.75 (3H, d, *J =* 4.4 Hz), 2.12-2.08 (2H, m), 1.80-1.77 (2H, m), 1.54-1.51 (1H, m), 1.54-1.51 (2H, m), 1.42-1.39 (2H, m), 1.27-1.20 (1H, m). MS Calcd.: 485.60; MS Found: 486.3 (M+H⁺).

### (181) A-181 : 3-((4-((2-Ethyl-4-phenylthiazol-5-yl)oxy)pyridin-2-yl)amino)benzenesulfonamide

¹H-NMR (400 MHz, DMSO-*d*₆): *δ* 9.44 (1H, s), 8.18-8.16 (2H, m), 7.84-7.79 (3H, m), 7.42 (3H, t, *J =* 7.4 Hz), 7.33 (2H, d, *J =* 7.2 Hz), 7.19 (2H, s), 6.70 (1H, dd, *J =* 6.0, 2.4 Hz), 6.47 (1H, d, *J =* 2.8 Hz), 3.03 (2H, q, *J =* 7.6 Hz), 1.36 (3H, t, *J =* 7.6 Hz). MS Calcd.: 452.55; MS Found: 453.1 (M+H⁺).

### (182) A-182 : 2-(3-((4-((2-Ethyl-4-phenylthiazol-5-yl)oxy)pyridin-2-vl)amino)phenyl)propan-2-ol

¹H-NMR (400 MHz, DMSO-*d*₆): *δ* 9.06 (1H, s), 8.10 (1H, d, *J =* 6.0 Hz), 7.83 (2H, d, *J =* 8.0 Hz), 7.61 (1H, d, *J =* 9.2 Hz), 7.50 (1H, s), 7.42 (2H, t, *J =* 7.6 Hz), 7.32 (1H, t, *J =* 7.2 Hz), 7.15 (1H, t, *J =* 7.8 Hz), 6.98 (1H, d, *J =* 7.6 Hz), 6.60 (1H, dd, *J =* 5.4, 1.8 Hz), 6.44 (1H, d, *J =* 2.0 Hz), 4.92 (1H, s), 3.02 (2H, q, *J =* 7.5 Hz), 1.38 (6H, s), 1.35 (3H, t, *J =* 7.6 Hz). MS Calcd.: 431.55; MS Found: 432.2 (M+H⁺).

### (183) A-183 : 3-((4-((2-Ethyl-4-phenylthiazol-5-yl)oxy)pyridin-2-yl)amino)benzamide

¹H-NMR (400 MHz, DMSO-*d*₆): *δ* 9.24 (1H, s), 8.14 (1H, d, *J =* 6.0 Hz), 8.02 (1H, s), 7.85-7.80 (4H, m), 7.42 (2H, t, *J =* 7.6 Hz), 7.36 (1H, t, *J =* 7.8 Hz), 7.32-7.27 (3H, m), 6.65 (1H, dd, *J =* 6.0, 1.6 Hz), 6.45 (1H, d, *J =* 2.0 Hz), 3.02 (2H, q, *J =* 7.5 Hz), 1.36 (3H, t, *J =* 7.4 Hz). MS Calcd.: 461.13; MS Found: 417.2 (M+H⁺).

### (184) A-184 : 4-((4-((2-Ethyl-4-phenylthiazol-5-yl)oxy)pyridin-2-yl)amino)benzenesulfonamide

¹H-NMR (400 MHz, DMSO-*d*₆): *δ* 9.53 (1H, s), 8.19 (1H, d, *J =* 6.0 Hz), 7.83 (2H, d, *J =* 7.2 Hz), 7.78 (2H, d, *J =* 8.8 Hz), 7.67 (2H, d, *J =* 8.8 Hz), 7.42 (2H, t, *J =* 7.6 Hz), 7.32 (1H, t, *J =* 8.0 Hz), 7.14 (2H, s), 6.75 (1H, dd, *J =* 5.8, 1.8 Hz), 6.50 (1H, d, *J* = 2.4 Hz), 3.03 (2H, q, *J =* 7.5 Hz), 1.36 (3H, t, *J =* 7.4 Hz). MS Calcd.: 452.55; MS Found: 453.1 (M+H⁺).

### (185) A-185 : 4-((4-((2-Ethyl-4-phenylthiazol-5-yl)oxy)pyridin-2-yl)amino)benzamide

¹H-NMR (400 MHz, DMSO-*d*₆): *δ* 9.39 (1H, s), 8.18 (1H, d, *J=* 5.6 Hz), 7.83 (2H, d, *J =* 7.2 Hz), 7.77 (3H, d, *J =* 8.8 Hz), 7.68 (2H, d, *J =* 8.8 Hz), 7.42 (2H, t, *J =* 7.6 Hz), 7.32 (1H, t, *J =* 7.8 Hz), 7.12 (1H, brs), 6.71 (1H, dd, *J =* 6.0, 2.4 Hz), 6.48 (1H, d, *J =* 2.0 Hz), 3.03 (2H, q, *J =* 7.6 Hz), 1.36 (3H, t, *J =* 7.4 Hz). MS Calcd.: 416.50; MS Found: 417.2 (M+H⁺).

### (186) A-186 : 2-(4-((4-((2-Ethyl-4-phenylthiazol-5-yl)oxy)pyridin-2-yl)amino)pyridin-2-yl)propan-2-ol

¹H-NMR (400 MHz, DMSO-*d*₆): *δ* 9.55 (1H, s), 8.21 (2H, t, *J =* 5.4 Hz), 7.83 (2H, d, *J =* 8.0 Hz), 7.69 (1H, d, *J =* 1.6 Hz), 7.66 (1H, dd, *J =* 5.6, 2.0 Hz), 7.42 (2H, t, *J =* 7.6 Hz), 7.32 (1H, t, *J =* 7.4 Hz), 6.76 (1H, dd, *J =* 7.0, 3.4 Hz), 6.55 (1H, d, *J =* 2.4 Hz), 5.10 (1H, s), 3.03 (2H, q, *J =* 7.6 Hz), 1.39 (6H, s), 1.36 (3H, t, *J =* 7.2 Hz). MS Calcd.: 432.54; MS Found: 433.2 (M+H⁺).

### (187) A-187 : 2-(5-((4-((2-Ethyl-4-phenylthiazol-5-yl)oxy)pyridin-2-yl)amino)pyridin-2-yl)propan-2-ol

¹H-NMR (400 MHz, DMSO-*d*₆): *δ* 9.21 (1H, s), 8.59 (1H, d, *J =* 2.4 Hz), 8.12 (1H, d, *J =* 6.0 Hz), 8.08 (1H, dd, *J =* 8.8, 2.4 Hz), 7.84 (2H, d, *J =* 8.0 Hz), 7.51 (1H, d, *J =* 9.2 Hz), 7.42 (2H, t, *J =* 7.6 Hz), 7.32 (1H, t, *J =* 7.6 Hz), 6.66 (1H, dd, *J =* 6.0, 2.4 Hz), 6.42 (1H, d, *J* = 2.4 Hz), 5.07 (1H, s), 3.03 (2H, q, *J =* 7.5 Hz), 1.40 (6H, s), 1.36 (3H, t, *J =* 7.6 Hz). MS Calcd.: 432.54; MS Found: 433.2 (M+H⁺).

### (188) A-188 : N-(3-((4-((2-Ethyl-4-phenylthiazol-5-yl)oxy)pyridin-2-yl)amino)phenyl)ethanesulfonamide

¹H-NMR (400 MHz, DMSO-*d*₆): *δ* 9.66 (1H, brs), 9.14 (1H, s), 8.08 (1H, d, *J =* 6.0 Hz), 7.80 (2H, d, *J =* 7.2 Hz), 7.37-7.45 (4H, m), 7.29 (1H, t, *J =* 7.4 Hz), 7.13 (1H, t, *J =* 8.2 Hz), 6.70 (1H, d, *J =* 7.6 Hz), 6.62 (1H, dd, *J =* 6.0 and 2.4 Hz), 6.42 (1H, s), 2.97-3.07 (4H, m), 1.33 (3H, t, *J =* 7.8 Hz), 1.16 (3H, t, *J =* 7.4 Hz). MS Calcd.: 480.13; MS Found: 481.1 (M+H⁺).

### (189) A-189 : 4-((4-((2-Ethyl-4-phenylthiazol-5-yl)oxy)pyridin-2-yl)amino)-N-methylbenzamide

¹H-NMR (400 MHz, DMSO-*d*₆): *δ* 9.35 (1H, s), 8.16 (2H, d, *J* = 5.6 Hz), 7.82 (2H, d, *J =* 7.6 Hz), 7.71 (4H, q, *J =* 8.8 Hz), 7.41 (2H, t, *J =* 8.0 Hz), 7.31 (1H, t, *J =* 7.6 Hz), 6.69 (1H, dd, *J =* 5.6, 2.4 Hz), 6.46 (1H, d, *J =* 2.4 Hz), 3.03 (2H, q, *J =* 7.6 Hz), 2.73 (3H, d, *J*= 5.6 Hz), 1.35 (3H, t, *J =* 7.6 Hz). MS Calcd.: 430.52; Found: 431.2 (M+H⁺).

### (190) A-190 : 6-((4-((2-Ethyl-4-phenylthiazol-5-yl)oxy)pyridin-2-yl)amino)nicotinamide

¹H-NMR (400 MHz, DMSO-*d*₆): *δ* 10.1 (1H, s), 8.65 (1H, d, *J =* 2.4 Hz), 8.19 (1H, d, *J =* 6.0 Hz), 8.06 (1H, d, *J =* 10.8 Hz), 7.88 (1H, s), 7.81 (2H, d, *J =* 7.6 Hz), 7.69 (1H, d, *J =* 8.4 Hz), 7.63 (1H, s), 7.39 (2H, t, *J =* 7.6 Hz), 7.29 (2H, t, *J =* 8.0 Hz), 6.72 (1H, d, *J =* 5.2 Hz), 3.00 (2H, q, *J =* 7.0 Hz), 1.34 (3H, t, *J =* 7.4 Hz). MS Calcd.: 432.50; Found: 433.2 (M+H⁺).

### (191) A-191 : 6-((4-((2-Ethyl-4-phenylthiazol-5-yl)oxy)pyridin-2-yl)amino)nicotinate

¹H-NMR (400 MHz, DMSO-*d*₆): *δ* 10.33 (1H, s), 8.69 (1H, s), 8.21 (1H, d, *J =* 6.0 Hz), 8.09-8.12 (1H, m), 7.76-7.81 (3H, m), 7.58 (1H, s), 7.62 (1H, s), 7.42 (1H, t, *J =* 5.0 Hz), 7.29 (1H, t, *J =* 7.2 Hz), 6.75-6.77 (1H, m), 3.81 (3H, s), 2.98-3.03 (2H, m), 1.34 (3H, t, *J* = 7.4 Hz). MS Calcd.: 432.13; MS Found: 433.1 (M+H⁺).

### (192) A-192 : 6-((4-((2-Ethyl-4-phenylthiazol-5-yl)oxy)pyridin-2-yl)amino)nicotinic acid

¹H-NMR (400 MHz, DMSO-*d*₆): *δ* 8.75 (1H, s), 8.30 (1H, d, *J =* 6.8 Hz), 8.21 (1H, d, *J =* 8.8 Hz), 7.80 (2H, d, *J=* 7.6 Hz), 7.49-7.51 (1H, m), 7.39 (2H, t, *J =* 7.8 Hz), 7.29-7.34 (2H, m), 7.00 (1H, brs), 3.00-3.05 (2H, m), 1.35 (3H, t, *J =* 7.6 Hz m). * Two protons from NH and OH were not observed. MS Calcd.: 418.11; MS Found: 419.1 (M+H⁺).

### (193) A-193 : 6-((4-((2-Ethyl-4-phenylthiazol-5-yl)oxy)pyridin-2-yl)amino)-N-methylnicotinamide

¹H-NMR (400 MHz, DMSO-*d*₆): *δ* 10.11 (1H, s), 8.60 (1H, s), 8.34 (1H, brs), 8.19 (1H, d, *J =* 6.0 Hz), 8.01-8.04 (1H, m), 7.81 (2H, d, *J =* 7.2 Hz), 7.70 (1H, d, *J =* 9.2 Hz), 7.60 (1H, s), 7.37-7.39 (2H, m), 7.29-7.30 (1H, m), 6.71-6.72 (1H, m), 2.98-3.04 (2H, m), 2.75 (3H, d, *J =* 4.4 Hz), 1.34 (3H, t, *J =* 7.4 Hz). MS Calcd.: 431.14; MS Found: 432.1 (M+H⁺).

### (194) A-194 : 3-((4-((2-Ethyl-4-phenylthiazol-5-yl)oxy)pyridin-2-yl)amino)benzoic acid

¹H-NMR (400 MHz, DMSO-*d*₆): *δ* 9.36 (1H, s), 8.23 (1H, s), 8.15 (1H, d, *J =* 6.0 Hz), 7.88-7.83 (3H, m), 7.48-7.40 (3H, m), 7.38-7.30 (2H, m), 6.69 (1H, dd, *J =* 5.8, 2.2 Hz), 6.45 (1H, d, *J =* 2.0 Hz), 3.03 (2H, q, *J =* 7.5 Hz), 1.36 (3H, t, *J =* 7.4 Hz). * A proton from OH was not observed. MS Calcd.: 417.48; MS Found: 418.2 (M+H⁺).

### (195) A-195 : 4-((4-((2-Ethyl-4-phenylthiazol-5-yl)oxy)pyridin-2-yl)amino)benzoic acid

¹H-NMR (400 MHz, DMSO-*d*₆): *δ* 9.54 (1H, s), 8.20 (1H, d, *J =* 6.0 Hz), 7.84-7.81 (4H, m), 7.74 (2H, d, *J =* 8.4 Hz), 7.42 (2H, t, *J =* 7.6 Hz), 7.32 (1H, t, *J =* 7.8 Hz), 6.75 (1H, dd, *J =* 6.0, 2.4 Hz), 6.52 (1H, d, *J =* 2.4 Hz), 3.03 (2H, q, *J =* 7.5 Hz), 1.36 (3H, t, *J =* 7.6 Hz). * A proton from OH was not observed. MS Calcd.: 417.48; MS Found: 418.1 (M+H⁺).

### (196) A-196 : 6-((4-((2-Ethyl-4-phenylthiazol-5-yl)oxy)pyridin-2-yl)amino)-N-isopropylnicotinamide

¹H-NMR (400 MHz, DMSO-*d*₆): *δ* 8.61 (1H, s), 8.15 (1H, d, *J =* 6.0 Hz), 8.03 (1H, d, *J =* 8.8 Hz), 7.83 (2H, d, *J =* 7.6 Hz), 7.62 (1H, s), 7.56 (1H, d, *J =* 8.8 Hz), 7.36 (2H, t, *J =* 7.6 Hz), 7.29 (1H, d, *J =* 6.8 Hz), 6.69 (1H, d, *J =* 5.2 Hz), 4.23-4.14 (1H, m), 3.05 (2H, q, *J =* 7.7 Hz), 1.43 (3H, t, *J =* 7.6 Hz), 1.24 (6H, d, *J =* 6.8 Hz). Two protons from NH and OH were not observed. MS Calcd.: 459.56; MS Found: 460.3 (M+H⁺).

### (197) A-197 : 6-((4-((2-Ethyl-4-phenylthiazol-5-yl)oxy)pyridin-2-yl)amino)pyridin-3-yl)(4-methylpiperazin-1-yl)methanone

¹H-NMR (400 MHz, DMSO-*d*₆): *δ* 10.1 (1H, s), 8.20-8.17 (2H, m), 7.81 (2H, d, *J* = 6.8 Hz), 7.68-7.64 (3H, m), 7.39-7.34 (2H, m), 7.30-7.23 (1H, m), 6.69 (1H, s), 3.48 (4H, s), 3.00 (2H, q, *J =* 8.0 Hz), 2.29 (4H, s), 2.06 (3H, s), 1.33 (3H, t, *J =* 7.0 Hz). MS Calcd.: 500.62; MS Found: 501.3 (M+H⁺).

### (198) A-198 : 3-Aminopyrrolidin-1-yl)(6-(4-(2-ethyl-4-phenylthiazol-5-yloxy) pyridin-2-ylamino)pyridin-3-yl)methanone

¹H-NMR (400 MHz, DMSO-*d*₆): *δ* 10.1 (1H, s), 8.34 (1H, s), 8.18 (1H, d, *J=* 6.0 Hz), 7.81 (3H, d, *J =* 7.6 Hz), 7.69 (1H, d, *J =* 8.4 Hz), 7.63 (1H, s), 7.39 (2H, t, *J =* 7.6 Hz), 7.29 (1H, t, *J =* 7.4 Hz), 6.70 (1H, d, *J =* 5.2 Hz), 3.70-3.52 (3H, m), 3.38-3.24 (2H, m), 3.00 (2H, q, *J* = 7.5 Hz), 1.98 (1H, brs), 1.88 (1H, brs), 1.33 (3H, t, *J* = 7.4 Hz). * Two protons from NH₂ were not observed. MS Calcd.:486.59; MS Found: 487.2 (M+H⁺).

### (199) A-199 : 6-((4-((2-Ethyl-4-phenylthiazol-5-yl)oxy)pyridin-2-yl)amino)-N-(2-(pyrrolidin-1-yl)ethyl)nicotinamide

¹H-NMR (400 MHz, MeOH-*d*₄): *δ* 8.64 (1H, s), 8.14 (1H, d, *J =* 6.0 Hz), 8.04 (1H, dd, *J =* 8.8, 2.0 Hz), 7.82 (2H, d, *J =* 7.6 Hz), 7.61 (1H, s), 7.57 (1H, d, *J =* 9.2 Hz), 7.35 (2H, t, *J* = 7.8 Hz), 7.27 (1H, t, *J =* 9.0 Hz), 6.67 (1H, dd, *J =* 5.6, 2.0 Hz), 3.54 (2H, t, *J =* 6.8 Hz), 3.04 (2H, q, *J =* 7.5 Hz), 2.74 (2H, t, *J =* 6.6 Hz), 2.65 (4H, s), 1.84 (4H, s), 1.42 (3H, t, *J =* 7.8 Hz). * Two protons from NH were not observed. MS Calcd.: 514.64; MS Found: 515.3 (M+H⁺).

### (200) A-200 : 6-((4-((2-Ethyl-4-phenylthiazol-5-yl)oxy)pyridin-2-yl)amino)pyridin-3-yl)(3-(methylamino)pyrrolidin-1-yl)methanone

¹H-NMR (400 MHz, MeOH-*d*₄): *δ* 8.39 (1H, d, *J =* 3.2 Hz), 8.13 (1H, d, *J =* 6.0 Hz), 7.82 (3H, d, *J =* 8.0 Hz), 7.61 (1H, s), 7.57 (1H, d, *J* = 8.8 Hz), 7.35 (2H, t, *J* = 7.8 Hz), 7.27 (1H, t, *J =* 7.4 Hz), 6.66 (1H, dd, *J =* 6.0, 2.0 Hz), 3.78-3.71 (2H, m), 3.70-3.57 (2H, m), 3.50-3.45 (1H, m), 3.40-3.33 (1H, m), 3.03 (2H, q, *J =* 7.5 Hz), 2.42 (1.5H, s), 2.32 (1.5H, s), 1.91-1.80 (1H, m), 1.42 (3H, t, *J =* 7.6 Hz). * A proton from NH was not observed. MS Calcd.: 500.62; MS Found: 501.2 (M+H⁺).

### (201) A-201 : 6-((4-((2-Ethyl-4-phenylthiazol-5-yl)oxy)pyridin-2-yl)amino)-N-(2-(4-methylpiperazin-1-yl)ethyl)nicotinamide

¹H-NMR (400 MHz, DMSO-*d*₆): *δ* 10.1 (1H, s), 8.60 (1H, d, *J =* 2.0 Hz), 8.34 (1H, t, *J =* 5.4 Hz), 8.19 (1H, d, *J* = 6.0 Hz), 8.02 (1H, dd, *J* = 8.6, 2.2 Hz), 7.81 (2H, d, *J* = 7.6 Hz), 7.70 (1H, d, *J =* 8.8 Hz), 7.61 (1H, d, *J =* 2.4 Hz), 7.41-7.37 (2H, m), 7.30-7.27 (1H, m), 6.72 (1H, dd, *J =* 5.6, 2.4 Hz), 3.34 (2H, s), 3.00 (2H, q, *J =* 7.5 Hz), 2.48-2.41 (3H, m), 2.39-2.11 (7H, m), 1.97 (3H, s), 1.33 (3H, t, *J =* 7.8 Hz). MS Calcd.: 543.68; MS Found: 544.3 (M+H⁺).

### (202) A-202 : 6-((4-((2-Ethyl-4-phenylthiazol-5-yl)oxy)pyridin-2-yl)amino)-N-(2-(4-i sopropylpiperazin-1 -yl)ethyl)nicotinamide

¹H-NMR (400 MHz, DMSO-*d*₆): *δ* 10.15 (1H, s), 8.61 (1H, s), 8.34 (1H, s), 8.19 (1H, d, *J =* 5.6 Hz), 8.03 (1H, d, *J =* 8.8 Hz), 7.81 (2H, d, *J =* 7.6 Hz), 7.70 (1H, d, *J =* 8.8 Hz), 7.61 (1H, s), 7.38 (2H, t, *J =* 7.4 Hz), 7.28 (1H, t, *J =* 7.2 Hz), 6.71 (1H, d, *J =* 5.2 Hz), 3.32 (2H, s), 3.00 (2H, q, *J =* 7.2 Hz), 2.54 (1H, t, *J =* 6.4 Hz), 2.48-2.38 (10H, m), 1.33 (3H, t, *J =* 7.4 Hz), 0.91 (6H, d, *J =* 6.0 Hz). MS Calcd.: 571.74; MS Found: 572.3 (M+H⁺).

### (203) A-203 : 6-((4-((2-Ethyl-4-phenylthiazol-5-yl)oxy)pyridin-2-yl)amino)-N-(2-(4-ethylpiperazin-1-yl)ethyl)nicotinamide

¹H-NMR (400 MHz, DMSO-d₆): *δ* 10.16 (1H, s), 8.60 (1H, d, *J* =1.6 Hz), 8.34 (1H, t, *J* =5.4 Hz), 8.19 (1H, d, *J* =5.6 Hz), 8.02 (1H, dd, *J* =8.4, 2.0 Hz), 7.81 (2H, d, *J* =7.6 Hz), 7.70 (1H, d, *J* =8.8 Hz), 7.61 (1H, d, *J* =2.0 Hz), 7.39 (2H, t, *J* =7.6 Hz), 7.29 (1H, d, *J* =6.8 Hz), 6.72 (1H, dd, *J* =5.4, 2.2 Hz), 3.34-3.31 (2H, m), 3.00 (2H, q, *J* =7.6 Hz), 2.48-2.39 (6H, m), 2.28-2.23 (6H, m), 1.34 (3H, t, *J* =7.6 Hz), 0.95 (3H, t, *J* =7.4 Hz). MS Calcd.: 557.71; MS Found: 558.3 (M+H⁺).

### (204) A-204 : 5-(3-Aminopyrrolidin-1-yl)-N-(4-((2-ethyl-4-phenylthiazol-5-yl)oxy)pyridin-2-yl)pyridin-2-amine

¹H-NMR (400 MHz, CDCl₃): *δ* 8.03(1H,d,*J* = 5.6 Hz), 7.87 (2H, d, *J =* 7.6 Hz), 7.59 (1H, s), 7.35 (2H, t, *J =* 7.6 Hz), 7.28-7.25 (3H, m), 6.88 (1H, d, *J =* 8.4 Hz), 6.47 (1H, d, *J =* 6.4 Hz), 3.80-3.73 (1H, m), 3.52-3.42 (2H, m), 3.29 (1H, q, *J =* 7.6 Hz), 3.01 (2H, q, *J =* 7.6 Hz), 2.29-2.20 (2H, m), 1.85-1.80 (1H, m), 1.42 (3H, t, *J =* 7.6 Hz). * Three protons from NH and NH₂ were not observed. MS Calcd.:458.58; MS Found:459.3(M+H+).

### (205) A-205 : N¹-(4-((2-Ethyl-4-phenylthiazol-5-yl)oxy)pyridin-2-yl)-N⁴-(2-(4-isopropylpiperazin-1-yl)ethyl)benzene-1,4-diamine

¹H-NMR (400 MHz, MeOH-*d*₄): *δ* 7.88 (1H, d, *J* =6.0 Hz), 7.78 (2H, d, *J* =8.0 Hz), 7.35 (2H, t, *J* =7.6 Hz), 7.28 (1H, d, *J* =7.2 Hz), 7.01 (2H, d, *J* =8.4 Hz), 6.60 (2H, d, *J* =8.8 Hz), 6.39-6.37 (1H, m), 6.17 (1H, s), 3.19 (2H, t, *J* =6.4 Hz), 2.97 (2H, dd, *J* =15.0, 7.8 Hz), 2.70-2.58 (11H, m), 1.37 (3H, t, *J* =7.4 Hz), 1.08 (6H, d, *J* =6.8 Hz). * Two protons from NH were not observed. MS Calcd.: 542.74; MS Found: 543.4 (M+H⁺).

### (206) A-206 : N¹-(4-((2-Ethyl-4-phenylthiazol-5-yl)oxy)pyridin-2-yl)-N⁴-(2-(4-methylpiperazin-1 -yl)ethyl)benzene-1,4-diamine

¹H-NMR (400 MHz, DMSO-*d*₆): *δ* 8.58 (1H, s), 7.97 (1H, d, *J* =6.0 Hz), 7.8 (2H, d, *J* =7.2 Hz), 7.39-7.37 (2H, m), 7.31-7.29 (1H, m), 7.16 (2H, d, *J* =8.0 Hz), 6.49-6.43 (3H, m), 6.22 (1H, s), 5.04 (1H, s), 3.04-2.97 (4H, m), 2.46-2.30 (10H, m), 2.12 (3H, s), 1.32 (3H, t, *J* =7.2 Hz), MS Calcd.: 514.68; MS Found: 515.3 (M+H⁺).

### (207) A-207 : N¹-(4-((2-Ethyl-4-phenylthiazol-5-yl)oxy)pyridin-2-yl)-N⁴-(2-(4-ethylpiperazin-1-yl)ethyl)benzene-1,4-diamine

¹H-NMR (400 MHz, MeOH-*d*₄): *δ*7.87(1H,d,*J* =6.0 Hz), 7.77 (2H, d, *J* =7.6 Hz), 7.34 (2H, t, *J* =7.6 Hz), 7.28 (1H, d, *J* =7.6 Hz), 7.01 (2H, d, *J* =8.8 Hz), 6.59 (2H, d, *J* =8.4 Hz), 6.37 (1H, dd, *J* =5.6, 2.4 Hz), 6.17 (1H, d, *J* =2.0 Hz), 3.18 (2H, t, *J* =6.4 Hz), 2.96 (2H, q, *J* =7.6 Hz), 2.60-2.57 (6H, m), 2.46-2.40 (6H, m), 1.36 (3H, t, *J* =7.6 Hz), 1.09 (3H, t, *J* =7.0 Hz). * Two protons from NH were not observed. MS Calcd.: 528.71; MS Found: 529.3 (M+H⁺).

### (208) A-208 : N¹-(4-((2-Ethyl-4-phenylthiazol-5-yl)oxy)pyridin-2-yl)-N⁴-(2-(pyrrolidin-1-yl)ethyl)benzene-1,4-diamine

¹H-NMR (400 MHz, DMSO-*d*₆): *δ* 8.58 (1H, s), 7.97 (1H, d, *J* =6.0 Hz), 7.80 (2H, d, *J* =7.6 Hz), 7.39 (2H, t, *J* =7.6 Hz), 7.29 (1H, t, *J* =7.4 Hz), 7.16 (2H, d, *J* =8.4 Hz), 6.48 (2H, d, *J* =8.8 Hz), 6.43 (1H, dd, *J* =6.0, 2.0 Hz), 6.22 (1H, d, *J* =2.0 Hz), 5.12 (1H, t, *J* =5.4 Hz), 3.05 (2H, m), 2.97 (2H, q, *J* =7.5 Hz), 2.56 (2H, t, *J* =6.8 Hz), 2.44 (4H, s), 1.66 (4H, s), 1.31 (3H, t, *J* =7.4 Hz). MS Calcd.: 485.64; MS Found: 486.3 (M+H⁺).

### (209) A-209 : N²-(4-((2-Ethyl-4-phenylthiazol-5-yl)oxy)pyridin-2-yl)-N⁵-(2-(4-methylpiperazin-1-yl)ethyl)pyridine-2,5-diamine

¹H-NMR (400 MHz, DMSO-*d*₆): *δ* 9.30 (1H, s), 8.04 (1H, d, *J=* 6.0 Hz), 7.80 (2H, d, *J=* 8.0 Hz), 7.56 (1H, s), 7.37-7.41 (4H, m), 7.28 (1H, t, *J =* 7.2 Hz), 6.99 (1H, d, *J =* 6.0 Hz), 6.50 (1H, d, *J=* 5.6 Hz), 5.15 (1H, t, *J=* 5.4 Hz), 3.02-3.06 (2H, m), 2.99 (2H, q, *J=* 6.4 Hz), 2.43-4.48 (2H, m), 2.00-2.42 (8H, m), 1.33 (3H, t, *J=* 7.6 Hz). MS Calcd.: 516.3; MS Found: 515.25 (M+H⁺).

### (210) A-210 : N²-(4-((2-Ethyl-4-phenylthiazol-5-yl)oxy)pyridin-2-yl)-N⁵-(2-(pyrrolidin-1-yl)ethyl)pyridine-2,5-diamine

¹H-NMR (400 MHz, DMSO-*d*₆): *δ*9.29(1H,s),8.04(1H,d,*J* = 6.0 Hz), 7.81 (2H, d, *J*= 7.2 Hz), 7.56 (1H, d, *J=* 2.4 Hz), 7.37-7.39 (4H, m), 7.28-7.30 (1H, m), 6.98 (1H, dd, *J =* 8.6 and 2.6 Hz), 6.50 (1H, dd, *J =* 5.6 and 2.4 Hz), 5.20-5.25 (1H, m), 3.06 (2H, q, *J =* 6.1 Hz), 2.99 (2H, q, *J=* 7.5 Hz), 2.56 (2H, t, *J=* 6.6 Hz), 2.44-2.48 (4H, m), 1.60-1.70 (4H, m), 1.33 (3H, t, *J=* 7.4 Hz). MS Calcd.: 487.3; MS Found: 486.2 (M+H⁺).

### (211) A-211 : N²-(4-((2-Ethyl-4-phenylthiazol-5-yl)oxy)pyridin-2-yl)-N⁵-(2-(4-ethylpiperazin-1-yl)ethyl)pyridine-2,5-diamine

¹H-NMR (400 MHz, DMSO-*d*₆): *δ* 9.29 (1H, s), 8.04 (1H, d, *J=* 6.0 Hz), 7.81 (2H, d, *J=* 7.6 Hz), 7.56 (1H, d, *J=* 3.2 Hz), 7.37-7.41 (4H, m), 7.27-7.30 (1H, m), 6.98-7.00 (1H, m), 6.49-6.51 (1H, m), 5.10-5.20 (1H, m), 3.02-3.06 (2H, m), 2.99 (2H, q, *J =* 7.3 Hz), 2.38-2.48 (10H, m), 2.27 (2H, q, *J=* 7.3 Hz), 1.33 (3H, t, *J=* 7.4 Hz), 0.95 (3H, t, *J=* 7.2 Hz). MS Calcd.: 529.2; MS Found: 530.3 (M+H⁺).

### (212) A-212 : N²-(4-((2-Ethyl-4-phenylthiazol-5-yl)oxy)pyridin-2-yl)-N⁵-(2-(4-isopropylpiperazin-1-yl)ethyl)pyridine-2,5-diamine

¹H-NMR (400 MHz, DMSO-*d*₆): *δ* 9.29 (1H, s), 8.04 (1H, d, *J=* 6.0 Hz), 7.80 (2H, d, *J=* 7.6 Hz), 7.56 (1H, d, *J=* 2.4 Hz), 7.37-7.41 (4H, m), 7.27-7.30 (1H, m), 6.99 (1H, d, *J=* 8.8 Hz), 6.50 (1H, d, *J =* 5.2 Hz), 5.10-5.15 (1H, m), 3.02-3.06 (2H, m), 2.99 (2H, q, *J =* 7.3 Hz), 2.30-2.49 (10H, m), 1.33 (3H, t, *J=* 7.6 Hz), 0.93 (6H, d, *J=* 6.8 Hz). MS Calcd.: 543.7; MS Found: 544.3 (M+H⁺).

### (213) A-213 : N-(5-(1H-Pyrazol-4-yl)pyridin-2-yl)-4-((2-ethyl-4-phenylthiazol-5-yl)oxy)pyridin-2-amine

¹H-NMR (400 MHz, DMSO-*d*₆): *δ* 12.9 (1H, s), 9.80 (1H, s), 8.44 (1H, s), 8.14 (2H, d, *J=* 6.0 Hz), 7.88-7.81 (4H, m), 7.67 (1H, d, *J=* 8.8 Hz), 7.58 (1H, s), 7.39 (2H, t, *J=* 7.8 Hz), 7.29 (1H, t, *J=* 7.4 Hz), 6.63 (1H, dd, *J=* 5.6, 1.6 Hz), 3.00 (2H, q, *J=* 7.6 Hz), 1.34 (3H, t, *J=* 7.6 Hz). MS Calcd.: 440.52; MS Found: 441.2 (M+H⁺).

### (214) A-214 : N-(4-(3-Aminopyrrolidin-1-yl)phenyl)-4-((2-ethyl-4-phenylthiazol-5-yl)oxy)pyridin-2-amine

¹H-NMR (400 MHz, CDCl₃): *δ* 7.96 (1H, d, *J=* 6.0 Hz), 7.81 (2H, d, *J=* 7.2 Hz), 7.34 (2H, t, *J=* 7.6 Hz), 7.28-7.24 (1H, m), 7.02 (2H, d, *J=* 8.4 Hz), 6.75 (1H, brs), 6.47 (2H, d, *J* = 8.4 Hz), 6.34 (1H, d, *J=* 6.0 Hz), 6.26 (1H, s), 3.79 (1H, brs), 3.47-3.45 (2H, m), 3.20-3.28 (, 2H, m), 2.96 (2H, q, *J=* 7.6 Hz), 2.25-2.22 (1H, m), 2.04-1.90 (1H, m), 1.38 (3H, t, *J =* 8.0 Hz). * Two protons from NH₂ were not observed. MS Calcd.: 457.19; MS Found: 458.3 (M+H⁺).

### (215) A-215 : 4-((2-Ethyl-4-phenylthiazol-5-yl)oxy)-N-(4-(3-(methylamino)pyrrolidin-1-yl)phenyl)pyridin-2-amine

¹H-NMR (400 MHz, DMSO-*d*₆): *δ* 8.73 (1H, s), 7.99 (1H, d, *J* =6.0 Hz), 7.81 (2H, d, *J* =7.6 Hz), 7.41-7.27 (6H, m), 6.50 (2H, d, *J* =8.8 Hz), 6.46 (1H, dd, *J* =5.6, 2.0 Hz), 6.26 (1H, d, *J* =2.0 Hz), 3.74-3.71 (1H, m), 3.42-3.33 (2H, m), 3.28-3.25 (1H, m), 3.19-3.15 (1H, m), 3.00 (2H, q, *J* =7.6 Hz), 2.55 (3H, s), 2.29-2.24 (1H, m), 2.05-2.00 (1H, m), 1.32 (3H, t, *J* =7.4 Hz). MS Calcd.: 471.62; MS Found: 472.3 (M+H⁺).

### (216) A-216 : Methyl 6-((4-((2-Isopropyl-4-phenylthiazol-5-yl)oxy)pyridin-2-yl)amino)picolinate

¹H-NMR (400 MHz, DMSO-*d*₆): *δ* 10.15 (1H, s), 8.18 (1H, d, *J =* 6.0 Hz), 7.93 (1H, d, *J=* 8.8 Hz), 7.83-7.79 (3H, m), 7.75 (1H, d, *J =* 2.0 Hz), 7.54 (1H, d, *J =* 6.8 Hz), 7.41 (2H, t, *J =* 7.6 Hz), 7.30 (1H, t, *J =* 7.6 Hz), 6.71 (1H, dd, *J =* 5.6, 2.0 Hz), 3.78 (3H, s), 3.28-3.24 (1H, m), 1.36 (6H, d, *J =* 6.4 Hz). MS Calcd.: 446.52; MS Found: 447.2 (M+H⁺).

### (217) A-217 : 6-((4-((2-Isopropyl-4-phenylthiazol-5-yl)oxy)pyridin-2-yl)amino)picolinic acid

¹H-NMR (400 MHz, DMSO-*d*₆): *δ* 10.31 (1H, brs), 8.17 (1H, d, *J* = 6.0 Hz), 7.83-7.76 (4H, m), 7.52 (1H, d, *J =* 7.6 Hz), 7.41 (2H, t, *J =* 7.6 Hz), 7.30 (2H, t, *J =* 7.2 Hz), 6.62 (1H, s), 3.28-3.23 (1H, m), 1.37 (6H, d, *J =* 7.2 Hz) * A proton from OH was not observed. MS Calcd.: 432.49; MS Found: 433.2 (M+H⁺).

### (218) A-218 : 3-((4-((2-Isopropyl-4-phenylthiazol-5-yl)oxy)pyridin-2-yl)amino)benzenesulfonamide

¹H-NMR (400 MHz, DMSO-*d*₆): *δ* 9.44 (1H, s), 8.16 (2H, d, *J =* 6.0 Hz), 7.85-7.80 (3H, m), 7.44-7.39 (3H, m), 7.33-7.29 (4H, m), 6.69 (1H, dd, *J =* 2.4 Hz, 5.8 Hz), 6.46 (1H, d, *J=* 2.4 Hz), 3.29-3.26 (1H, m), 1.39 (6H, d, *J =* 7.2 Hz). MS Calcd.: 466.58; MS Found: 467.1 (M+H⁺).

### (219) A-219 : 2-(3-((4-((2-Isopropyl-4-phenylthiazol-5-yl)oxy)pyridin-2-yl)amino)phenyl)propan-2-ol

¹H-NMR (400 MHz, DMSO-*d*₆): *δ* 9.05 (1H, s), 8.10 (1H, d, *J =* 6.0 Hz), 7.83 (2H, d, *J =* 7.6 Hz), 7.61 (1H, d, *J =* 8.0 Hz), 7.49 (1H, s), 7.41 (2H, t, *J =* 7.6 Hz), 7.31 (1H, t, *J =* 7.2 Hz), 7.15 (1H, t, *J =* 7.6 Hz), 6.97 (1H, d, *J =* 8.0 Hz), 6.59 (1H, dd, *J =* 2.0 Hz, 5.8 Hz), 6.43 (1H, d, *J=* 2.4 Hz), 4.91 (1H, s), 3.32-3.25 (1H, m), 1.39-1.38 (12H, m). MS Calcd.: 445.58; MS Found: 446.2 (M+H⁺).

### (220) A-220 : 3-((4-((2-Isopropyl-4-phenylthiazol-5-yl)oxy)pyridin-2-yl)amino)benzamide

¹H-NMR (400 MHz, DMSO-*d*₆): *δ* 9.23 (1H, s), 8.13 (1H, d, *J=* 6.0 Hz), 7.96 (1H, s), 7.84-7.80 (4H, m), 7.42 (2H, t, *J =* 7.2 Hz), 7.35 (1H, t, *J* =8.0 Hz), 7.29 (3H, t, *J =* 8.0 Hz), 6.64 (1H, dd, *J =* 2.4 Hz, 5.6 Hz), 6.44 (1H, d, *J =* 2.0 Hz), 3.30-3.25 (1H, m), 1.39 (6H, d, *J =* 6.8 Hz). MS Calcd.: 430.52; MS Found: 431.2 (M+H⁺).

### (221) A-221 : 4-((4-((2-Isopropyl-4-phenylthiazol-5-yl)oxy)pyridin-2-yl)amino)benzenesulfonamide

¹H-NMR (400 MHz, DMSO-*d*₆): *δ* 9.50 (1H, s), 8.17 (1H , d, *J=* 6.0 Hz), 7.81 (2H, d, *J =* 8.0 Hz), 7.75 (2H, d, *J =* 8.4 Hz), 7.65 (2H, d, *J =* 9.2 Hz), 7.40 (2H, t, *J =* 7.6 Hz), 7.30 (1H, t, *J =* 7.2 Hz), 7.11 (2H, s), 6.71 (1H, dd, *J =* 2.0 Hz, 5.8 Hz), 6.47 (1H, d, *J =* 4.0 Hz), 3.29-3.25 (1H, m), 1.37 (6H, d, *J=* 7.2 Hz). MS Calcd.: 466.58; MS Found: 467.2 (M+H⁺).

### (222) A-222 : 4-((4-((2-Isopropyl-4-phenylthiazol-5-yl)oxy)pyridin-2-yl)amino)benzamide

¹H-NMR (400 MHz, DMSO-*d*₆): *δ* 9.39 (1H, s), 8.18 (1H, d, *J=* 6.0 Hz), 7.83 (2H, d, *J =* 7.2 Hz), 7.76 (3H, d, *J =* 9.2 Hz), 7.67 (2H, d, *J =* 9.2 Hz), 7.42 (2H, t, *J* = 7.2 Hz), 7.32 (1H, t, *J =* 7.6 Hz), 7.11 (1H, s), 6.70 (1H, dd, *J =* 2.4 Hz, 6.0 Hz), 6.48 (1H, d, *J =* 2.0 Hz), 3.32-3.26 (1H, m), 1.39 (6H, d, *J* = 7.2 Hz). MS Calcd.: 430.52; MS Found: 431.2 (M+H⁺).

### (223) A-223 : 2-(4-((4-((2-Isopropyl-4-phenylthiazol-5-yl)oxy)pyridin-2-yl)amino)pyridin-2-yl)propan-2-ol

¹H-NMR (400 MHz, DMSO-*d*₆): *δ* 9.55 (1H, s), 8.21 (2H, t, *J* =5.6 Hz), 7.83 (2H, d, *J =* 7.2 Hz), 7.68-7.65 (2H, m), 7.42 (2H, t, *J =* 7.2 Hz), 7.32 (1H, t, *J =* 7.2 Hz), 6.75 (1H, dd, *J =* 6.0 Hz), 6.54 (1H, d, *J =* 2.4 Hz), 5.09 (1H, s), 3.32-3.28 (1H, m), 1.39 (12H, d, *J =* 9.2 Hz). MS Calcd.: 446.56; MS Found: 447.2 (M+H⁺).

### (224) A-224 : 2-(5-((4-((2-Isopropyl-4-phenylthiazol-5-yl)oxy)pyridin-2-yl)amino)pyridin-2-yl)propan-2-ol

¹H-NMR (400 MHz, DMSO-*d*₆): *δ* 9.19 (1H, s), 8.56 (1H, d, *J=* 2.4 Hz), 8.09 (1H, d, *J =* 6.0 Hz), 8.04 (1H, dd, *J =* 2.4 Hz, 9.0 Hz), 7.81 (2H, d, *J=* 6.8 Hz), 7.48 (1H, d, *J =* 8.8 Hz), 7.40 (2H, t, *J =* 8.0 Hz), 7.30 (1H, t, *J =* 7.2 Hz), 6.64 (1H, dd, *J =* 2.4 Hz, 5.6 Hz), 6.39 (1H, d, *J =* 2.4 Hz), 5.04 (1H, s), 3.29-3.27 (1H, m), 1.37 (12H, d, *J =* 6.8 Hz). MS Calcd.: 446.56; MS Found: 447.3 (M+H⁺).

### (225) A-225 : 6-((4-((2-Isopropyl-4-phenylthiazol-5-yl)oxy)pyridin-2-yl)amino)-N-methylpicolinamide

¹H-NMR (400 MHz, DMSO-*d*₆): *δ* 9.87 (1H, s), 8.19 (1H, d, *J=* 6.0 Hz), 8.02 (1H, s), 7.93 (1H, d, *J=* 8.4 Hz), 7.83-7.78 (3H, m), 7.47 (1H, d, *J=* 6.8 Hz), 7.41 (2H, t, *J=* 7.6 Hz), 7.31-7.28 (2H, m), 6.74 (1H, d, *J=* 3.6 Hz), 3.25-3.23 (1H, m), 2.76 (3H, d, *J=* 4.4 Hz), 1.35 (6H, d, *J =* 6.8 Hz). MS Calcd.: 445.54; MS Found: 446.2 (M+H⁺).

### (226) A-226 : 6-((4-((2-Isopropyl-4-phenylthiazol-5-yl)oxy)pyri yl)amino)picolinamide

¹H-NMR (400 MHz, DMSO-*d*₆): *δ* 9.87 (1H, s), 8.19 (1H, d, *J=* 6.0 Hz), 7.96 (1H, d, *J=* 8.4 Hz), 7.83-7.79 (3H, m), 7.59 (1H, s), 7.50-7.44 (2H, m), 7.40 (2H, t, *J=* 7.6 Hz), 7.30-7.24 (2H, m), 6.72 (1H, dd, *J =* 5.6, 2.4 Hz), 3.27-3.26 (1H, m), 1.37 (6H, d, *J =* 6.4 Hz). MS Calcd.: 431.51; MS Found: 432.2 (M+H⁺).

### (227) A-227 : 6-((4-((2-Isopropyl-4-phenylthiazol-5-yl)oxy)pyridin-2-yl)amino)nicotinamide

¹H-NMR (400 MHz, DMSO-*d*₆): *δ* 10.14 (1H, s), 8.64 (1H, s), 8.20 (1H, d, *J =* 4.2 Hz), 8.06 (1H, d, *J=* 8.4 Hz), 7.88 (1H, s), 7.82 (2H, d, *J=* 6.8 Hz), 7.69 (1H, d, *J=* 8.8 Hz), 7.61 (1H, s), 7.41 (2H, t, *J =* 7.2 Hz), 7.30 (2H, t, *J =* 7.6 Hz), 6.72 (1H, d, *J =* 4.2 Hz), 3.28-3.26 (1H, m), 1.38 (6H, d, *J=* 6.8 Hz). MS Calcd.: 431.51; MS Found: 432.2 (M+H⁺).

### (228) A-228 : 6-((4-((2-Isopropyl-4-phenylthiazol-5-yl)oxy)pyridin-2-yl)amino)-N-methylnicotinamide

¹H-NMR (400 MHz, DMSO-*d*₆): *δ* 10.12 (1H, s), 8.59 (1H, s), 8.34 (1H, brs), 8.20 (1H, d, *J=* 6.4 Hz), 8.01 (1H, m), 7.82 (2H, d, *J=* 8.0 Hz), 7.70 (1H, d, *J=* 9.2 Hz), 7.60 (1H, s), 7.39-7.37 (2H, m), 7.30 (1H, d, *J=* 7.2 Hz), 6.72-6.70 (1H, m), 2.75 (3H, d, *J=* 4.4 Hz), 1.38 (6H, d, *J=* 7.2 Hz). MS Calcd.: 445.54; MS Found: 446.2 (M+H⁺).

### (229) A-229 : 4-((4-((2-Isopropyl-4-phenylthiazol-5-yl)oxy)pyridin-2-yl)amino)-N-methylbenzamide

¹H-NMR (400 MHz, DMSO-*d*₆): *δ* 9.35 (1H, s), 8.17 (2H, t, *J=* 4.2 Hz), 7.82 (2H, d, *J =* 7.6 Hz), 7.22 (4H, q, *J =* 8.8 Hz), 7.81 (2H, t, *J =* 7.2 Hz), 7.31 (1H, t, *J =* 8.0 Hz), 6.68 (1H, dd, *J =* 5.6, 2.4 Hz), 6.46 (1H, d, *J =* 2.0 Hz), 3.27-3.25 (1H, m), 2.73 (3H, d, *J =* 4.4 Hz), 1.38 (6H, d, *J =* 7.2 Hz). MS Calcd.: 444.16; MS Found: 445.2 (M+H⁺).

### (230) A-230 : 3-((4-((2-Isopropyl-4-phenylthiazol-5-yl)oxy)pyridin-2-yl)amino)benzoic acid

¹H-NMR (400 MHz, DMSO-*d*₆): *δ*12.85 (1H, s), 9.31 (1H, s), 8.22 (1H, s), 8.15 (1H, d, *J=* 6.0 Hz), 7.88-7.83 (3H, m), 7.46-7.40 (2H, m), 7.36-7.30 (3H, m), 6.66 (1H, dd, *J=* 2.0 Hz, 5.8 Hz), 6.44 (1H, d, *J=* 2.0 Hz), 3.32-3.25 (1H, m), 1.39 (6H, d, *J=* 7.2 Hz). MS Calcd.: 431.51; MS Found: 432.2 (M+H⁺).

### (231) A-231 : 4-((4-((2-Isopropyl-4-phenylthiazol-5-yl)oxy)pyridin-2-yl)amino)benzoic acid

¹H-NMR (400 MHz, DMSO-*d*₆): *δ* 12.43 (1H, s), 9.52 (1H, s), 8.20 (1H, d, *J =* 6.0 Hz), 7.84-7.81 (4H, m), 7.73 (2H, d, *J =* 8.4 Hz), 7.42 (2H, t, *J =* 7.2 Hz), 7.34-7.30 (1H, m), 6.73 (1H, dd, *J =* 2.0 Hz, 5.6 Hz), 6.51 (1H, d, *J =* 2.0 Hz), 3.32-3.26 (1H, m), 1.39 (6H, d, *J* = 7.2 Hz). MS Calcd.: 431.51; MS Found: 432.2 (M+H⁺).

### (232) A-232 : 6-((4-((2-Isopropyl-4-phenylthiazol-5-yl)oxy)pyridin-2-yl)amino)nicotinic acid

¹H-NMR (400 MHz, DMSO-*d*₆): *δ*12.79 (1H, brs), 10.29 (1H, s), 8.65 (1H, s), 8.21 (1H, d, *J=* 6.0 Hz), 8.08 (1H, d, *J=* 8.8 Hz), 7.81 (2H, d, *J=* 7.2 Hz), 7.71 (1H, d, *J=* 8.8 Hz), 7.66 (1H, s), 7.40 (2H, t, *J =* 7.6 Hz), 7.30 (1H, t, *J =* 7.6 Hz), 6.76 (1H, d, *J =* 6.0 Hz), 3.26-3.25 (1H, m), 1.38 (6H, d, *J=* 7.2 Hz). MS Calcd.: 432.49; MS Found: 433.2 (M+H⁺).

### (233) A-233 : 3-((4-((2-(Methylamino)-4-(naphthalen-2-yl)thiazol-5-yl)oxy)pyridin-2-yl)amino)benzenesulfonamide

¹H-NMR (400 MHz, DMSO-*d*₆): *δ* 9.43 (1H, s), 8.30 (1H, s), 8.18 (1H, s), 8.14 (1H, d, *J=* 5.6 Hz), 7.91-7.83 (4H, m), 7.80 (1H, d, *J=* 8.0 Hz), 7.74 (1H, d, *J=* 5.2 Hz), 7.49-7.47 (2H, m), 7.39 (1H, t, *J =* 7.6 Hz), 7.30 (1H, d, *J =* 6.4 Hz), 7.26 (2H, s), 6.75 (1H, dd, *J =* 2.4 Hz, 5.6 Hz), 6.57 (1H, d, *J =* 2.0 Hz), 2.94 (3H, d, *J =* 4.4 Hz). MS Calcd.: 503.6; MS Found: 504.2 (M+H⁺).

### (234) A-234 : 3-((4-((2-Amino-4-(naphthalen-2-yl)thiazol-5-yl)oxy)pyridin-2-yl)amino)benzenesulfonamide

¹H-NMR (400 MHz, DMSO-d₆): *δ* 9.47 (1H, s), 8.26 (1H, s), 8.17-8.13 (2H, m), 7.85-7.79 (4H, m), 7.49-7.47 (2H, m), 7.42 (1H, t, *J=* 8.0 Hz), 7.38-7.24 (4H, m), 6.75 (1H, dd, *J=* 2.0 Hz, 5.8 Hz), 6.58 (1H, s). * Two protons from NH₂ were not observed. MS Calcd.: 489.57; MS Found: 490.1 (M+H⁺).

### (235) A-235 : 3-((4-((2-(Dimethylamino)-4-(naphthalen-2-yl)thiazol-5-yl)oxy)pyridin-2-yl)amino)benzenesulfonamide

¹H-NMR (400 MHz, DMSO-d₆): *δ* 9.43 (1H, s), 8.33 (1H, s), 8.18 (1H, s), 8.15 (1H, d, *J=* 6.0 Hz), 7.93-7.84 (4H, m), 7.78 (1H, d, *J=* 8.0 Hz), 7.50-7.48 (2H, m), 7.39 (1H, t, *J=* 8.0 Hz), 7.31-7.27 (3H, m), 6.75 (1H, dd, *J =* 2.4 Hz, 6.0 Hz) 6.56 (1H, d, *J=* 2.0 Hz), 3.12 (6H, s). MS Calcd.: 517.62; MS Found: 518.2 (M+H⁺).

### (236) A-236 : 3-((6-((2-Amino-4-phenylthiazol-5-yl)oxy)pyrimidin-4-yl)amino)benzenesulfonamide

¹H-NMR (400 MHz, MeOD-*d*₄): *δ* 7.98-7.97 (2H, m), 7.61 (1H, d, *J =* 7.6 Hz), 7.54 (1H, d, *J=* 8.0 Hz), 7.49-7.42 (3H, m), 7.37-7.30 (3H, m), 7.22-7.18 (1H, m), 7.13 (1H, d, *J=* 8.0 Hz), 6.83 (1H, d, *J =* 8.0 Hz), 5.77 (1H, s). * Two protons from NH₂ were not observed. MS Calcd.: 440.50; MS Found: 441.1 (M+H⁺).

### (237) A-237 : 3-((6-((2-Methyl-4-phenylthiazol-5-yl)oxy)pyrimidin-4-yl)amino)benzenesulfonamide

¹H-NMR (400 MHz, DMSO-*d*₆): *δ* 9.75 (1H, s), 8.26 (1H, s), 7.83 (1H, s), 7.59-7.57 (1H, m), 7.57-7.48 (4H, m), 7.47-7.32 (5H, m), 5.67 (1H, s), 2.72 (3H, s). MS Calcd.: 439.51; MS Found: 440.1 (M+H⁺).

### (238) A-238 : 3-((4-((2-Methyl-4-phenylthiazol-5-yl)oxy)pyrimidin-2-yl)amino)benzenesulfonamide

¹H-NMR (400 MHz, DMSO-*d*₆): *δ* 10.0 (1H, s), 8.47 (1H, d, *J=* 6.0 Hz), 8.11 (1H, s), 7.82 (3H, d, *J=* 8.4 Hz), 7.39 (4H, t, *J=* 7.4 Hz), 7.29 (3H, s), 6.75 (1H, d, *J=* 4.8 Hz), 2.67 (3H, s). MS Calcd.: 439.51; MS Found: 440.1 (M+H⁺).

### (239) A-239 : 3-((6-((2-Amino-4-phenylthiazol-5-yl)oxy)pyrimidin-4-yl)amino)benzenesulfonamide

¹H-NMR (400 MHz, DMSO-*d*₆): *δ* 10.1 (1H, s), 8.43 (1H, d, *J=* 5.2 Hz), 8.09 (1H, s), 7.97 (1H, s), 7.68 (1H, d, *J=* 8.0 Hz), 7.38 (1H, d, *J=* 4.8 Hz), 7.33 (2H, d, *J=* 7.2 Hz), 7.25-7.20 (1H, m), 6.99 (2H, s), 6.65 (1H, d, *J =* 5.2 Hz), 6.55 (1H, s). * Three protons from NH and NH₂ were not observed. MS Calcd.: 440.50; MS Found: 441.1 (M+H⁺).

### (240) A-240 : 3-((6-((2-Amino-4-phenylthiazol-5-yl)oxy)pyrimidin-4-yl)amino)benzenesulfonamide

¹H-NMR (400 MHz, DMSO-*d*₆): *δ* 10.06 (1H, s), 8.48 (1H, d, *J =* 5.6 Hz), 8.08 (1H, brs), 7.83 (3H, d, *J=* 8.0 Hz), 7.42-7.35 (4H, m), 7.31-7.28 (3H, m), 6.75 (1H, d, *J=* 5.6 Hz), 3.00 (2H, q, *J* = 7.3 Hz), 1.33 (3H, t, *J =* 7.6 Hz). MS Calcd.: 453.09; MS Found: 454.1 (M+H⁺).

### (241) A-241 : 6-((4-((2-Ethyl-4-phenylthiazol-5-yl)oxy)pyrimidin-2-yl)amino)picolinamide

¹H-NMR (400 MHz, DMSO-*d*₆): *δ* 9.95 (1H, s), 8.54 (1H, d, *J=* 6.0 Hz), 8.02 (1H, d, *J=* 8.4 Hz), 7.83-7.77 (4H, m), 7.65 (1H, s), 7.58 (1H, d, *J=* 7.2 Hz), 7.39 (2H, t, *J=* 7.6 Hz), 7.31-7.29 (1H, m), 6.86 (1H, d, *J=* 5.6 Hz), 3.01 (2H, q, *J=* 7.6 Hz), 1.34 (3H, t, *J=* 7.8 Hz). MS Calcd.: 418.12; MS Found: 419.2 (M+H⁺).

### (242) A-242 : 2-((4-((2-Ethyl-4-phenylthiazol-5-yl)oxy)pyrimidin-2-yl)amino)isonicotinamide

¹H-NMR (400 MHz, DMSO-*d*₆): *δ* 10.23 (1H, s), 8.53 (1H, d, *J =* 5.6 Hz), 8.36 (1H, d, *J =* 5.2 Hz), 8.31 (1H, s), 8.08 (1H, s), 7.85 (2H, d, *J =* 7.2 Hz), 7.65 (1H, s), 7.41 (2H, t, *J* = 7.6 Hz), 7.35-7.30 (2H, m), 6.81 (1H, d, *J=* 5.2 Hz), 2.96 (2H, q, *J=* 7.6 Hz), 1.30 (3H, t, *J* = 7.6 Hz). MS Calcd.: 418.12; MS Found: 419.2 (M+H⁺).

### (243) A-243 : 3-((4-((2-Ethyl-4-phenylthiazol-5-yl)oxy)pyrimidin-2-yl)amino)benzamide

¹H-NMR (400 MHz, DMSO-*d*₆): *δ* 9.86 (1H, s), 8.45 (1H, d, *J=* 5.6 Hz), 7.99 (1H, s), 7.83 (3H, d, *J=* 7.6 Hz), 7.71 (1H, d, *J=* 9.6 Hz), 7.42-7.38 (3H, m), 7.29-7.22 (3H, m), 6.70 (1H, d, *J=* 5.6 Hz), 2.98 (2H, q, *J=* 7.5 Hz), 1.32 (3H, t, *J=* 7.4 Hz). MS Calcd.: 417.13; MS Found: 418.2 (M+H⁺).

### (244) A-244 : 6-((4-((2-Ethyl-4-phenylthiazol-5-yl)oxy)pyrimidin-2-yl)amino)nicotinic acid

¹H-NMR (400 MHz, DMSO-*d*₆): *δ* 10.9 (1H, brs), 8.77 (1H, s), 8.58 (1H, d, *J =* 6.0 Hz), 8.09 (1H, d, *J=* 8.8 Hz), 7.90 (1H, d, *J=* 8.8 Hz), 7.82 (2H, d, *J=* 7.2 Hz), 7.39 (3H, t, *J* = 7.6 Hz), 7.30-7.27 (1H, m), 6.94 (1H, d, *J =* 5.6 Hz), 3.01 (2H, q, *J =* 7.5 Hz), 1.34 (3H, t, *J* = 7.4 Hz). MS Calcd.: 419.11; MS Found: 420.1 (M+H⁺).

### (245) A-245 : 6-((4-((2-Ethyl-4-phenylthiazol-5-yl)oxy)pyrimidin-2-yl)amino)nicotinamide

¹H-NMR (400 MHz, DMSO-*d*₆): *δ* 10.44 (1H, s), 8.73 (1H, s), 8.54 (1H, d, *J =* 5.6 Hz), 8.02-8.01 (2H, m), 7.90-7.87 (1H, m), 7.83 (2H, d, *J=* 7.6 Hz), 7.41-7.37 (3H, m), 7.31-7.27 (1H, m), 6.86 (1H, d, *J=* 6.0 Hz), 3.02 (2H, q, *J=* 7.6 Hz), 1.34 (3H, t, *J=* 7.6 Hz). MS Calcd.: 418.12; MS Found: 419.2 (M+H⁺).

### (246) A-246 : 4-((4-((2-Ethyl-4-phenylthiazol-5-yl)oxy)pyrimidin-2-yl)amino)benzoic acid

¹H-NMR (400 MHz, DMSO-*d*₆): *δ* 12.57 (1H, s), 10.16 (1H, s), 8.50 (1H, d, *J =* 5.2 Hz), 7.81 (2H, d, *J =* 7.6 Hz), 7.75 (2H, d, *J =* 8.8 Hz), 7.64 (2H, d, *J =* 8.0 Hz), 7.39 (2H, t, *J* = 7.4 Hz), 7.30-7.27 (1H, m), 6.79 (1H, d, *J=* 5.6 Hz), 3.01 (2H, q, J = 7.3 Hz), 1.35 (3H, t, *J* = 7.4 Hz). MS Calcd.: 418.11; MS Found: 419.2 (M+H⁺).

### (247) A-247 : 3-((4-((2-Amino-4-(6-methylpyridin-2-yl)thiazol-5-yl)oxy)pyridin-2-yl)amino)benzenesulfonamide

¹H-NMR (400 MHz, DMSO-*d*₆): *δ* 9.35 (1H, s), 8.22 (1H, s), 8.09 (1H, d, *J=* 5.6 Hz), 7.80 (1H, d, *J =* 8.4 Hz), 7.65 (1H, t, *J =* 7.6 Hz), 7.54 (1H, d, *J =* 8.0 Hz), 7.39 (1H, t, *J =* 7.8 Hz), 7.30 (1H, d, *J=* 7.6 Hz), 7.27 (2H, s), 7.16 (2H, s), 7.03 (1H, d, *J=* 7.6 Hz), 6.60 (1H, dd, *J=* 5.6, 2.4 Hz), 6.47 (1H, d, *J=* 2.0 Hz), 2.20 (3H, s). MS Calcd.: 454.53; MS Found: 455.1 (M+H⁺).

### (248) A-248 : 4-((4-((2-Amino-4-(6-methylpyridin-2-yl)thiazol-5-yl)oxy)pyridin-2-yl)amino)benzoic acid

¹H-NMR (400 MHz, DMSO-*d*₆): *δ* 9.50 (1H, s), 8.14 (1H, d, *J=* 6.0 Hz), 7.81 (2H, d, *J =* 9.2 Hz), 7.72 (3H, d, *J =* 8.8 Hz), 7.59 (1H, d, *J =* 7.6 Hz), 7.31 (2H, s), 7.13 (1H, d, *J =* 7.2 Hz), 6.68 (1H, d, *J=* 6.4 Hz), 6.55 (1H, d, *J=* 2.0 Hz), 2.28 (3H, s). * Two protons from OH and TFA were not observed. MS Calcd.: 419.46; MS Found: 420.1(M+H⁺).

### (249) A-249 : 3-((4-((2-Amino-4-(6-methylpyridin-2-yl)thiazol-5-yl)oxy)pyridin-2-yl)amino)benzoic acid

¹H-NMR (400 MHz, DMSO-*d*₆): *δ* 9.38 (1H, s), 8.21 (1H, s), 8.10 (1H, d, *J=* 6.0 Hz), 7.85 (1H, d, *J=* 8.4 Hz), 7.76 (1H, t, *J=* 7.6 Hz), 7.59 (1H, d, *J=* 8.4 Hz), 7.48 (1H, d, *J=* 6.8 Hz), 7.36 (1H, t, *J =* 8.0 Hz), 7.34 (2H, s), 7.14 (1H, d, *J =* 7.6 Hz), 6.65 (1H, d, *J =* 3.2 Hz), 6.50 (1H, d, *J=* 2.0 Hz), 2.30 (3H, s). * Two protons from OH and TFA were not observed. MS Calcd.: 419.46; MS Found: 420.1 (M+H⁺).

### (250) A-250 : 3-((4-((2-Ethyl-4-(6-methylpyridin-2-yl)thiazol-5-yl)oxy)pyridin-2-yl)amino)benzenesulfonamide

¹H-NMR (400 MHz, DMSO-*d*₆): *δ* 9.33 (1H, s), 8.18 (1H, s), 8.09 (1H, d, *J=* 6.0 Hz), 7.77 (1H, d, *J=* 8.0 Hz), 7.71-7.69 (2H, m), 7.39 (1H, t, *J=* 8.0 Hz), 7.28 (1H, d, *J=* 7.6 Hz), 7.27 (2H, s), 7.09 (1H, d , *J=* 6.8 Hz), 6.60 (1H, dd, *J=* 5.6, 4.0 Hz), 6.36 (1H, d, *J=* 2.0 Hz), 3.04 (2H, q, *J=* 8.0 Hz), 2.20 (3H, s), 1.36 (3H, t, *J=* 7.2 Hz). MS Calcd.: 467.56; MS Found: 468.1 (M+H⁺).

### (251) A-251 : 6-((4-((2-Ethyl-4-(6-methylpyridin-2-yl)thiazol-5-yl)oxy)pyridin-2-yl)amino)nicotinamide

¹H-NMR (400 MHz, DMSO-*d*₆): *δ* 10.0 (1H, s), 8.63 (1H, d, *J=* 2.4 Hz), 8.13 (1H, d, *J=* 6.0 Hz), 8.05 (1H, dd, *J =* 9.0, 2.2 Hz), 7.87 (1H, s), 7.72-7.66 (3H, m), 7.51 (1H, d, *J =* 2.4 Hz), 7.26 (1H, s), 7.07 (1H, d, *J =* 6.4 Hz), 6.61 (1H, dd, *J =* 6.0, 2.4 Hz), 3.02 (2H, q, *J =* 7.5 Hz), 2.19 (3H, s), 1.35 (3H, t, *J=* 7.4 Hz). MS Calcd.: 432.50; MS Found: 433.2 (M+H⁺).

### (252) A-252 : 6-((4-((2-Ethyl-4-(6-methylpyridin-2-yl)thiazol-5-yl)oxy)pyridin-2-yl)amino)nicotinic acid

¹H-NMR (400 MHz, DMSO-*d*₆): *δ* 12.5 (1H, s), 8.84 (1H, d, *J=* 2.4 Hz), 8.39 (1H, d, *J =* 7.6 Hz), 8.30 (1H, dd, *J =* 8.6, 2.2 Hz), 7.89-7.78 (2H, m), 7.32 (1H, d, *J =* 8.4 Hz), 7.18-7.15 (3H, m), 3.05 (2H, q, *J =* 7.6 Hz), 2.19 (3H, s), 1.36 (3H, t, *J =* 7.6 Hz). * A proton from OH was not observed. MS Calcd.: 433.48; MS Found: 434.2 (M+H⁺).

### (253) A-253 : 6-((4-((2-Ethyl-4-(6-methylpyridin-2-yl)thiazol-5-yl)oxy)pyridin-2-yl)amino)-N-methylnicotinamide

¹H-NMR (400 MHz, DMSO-*d*₆): *δ* 10.0 (1H, s), 8.59 (1H, s), 8.32 (1H, s), 8.13 (1H, d, *J=* 5.2 Hz), 8.02 (1H, d, *J =* 9.6 Hz), 7.70-7.65 (3H, m), 7.48 (1H, s), 7.07 (1H, d, *J =* 7.2 Hz), 6.63 (1H, s), 3.02 (2H, q, *J =* 7.6 Hz), 2.75 (3H, d, *J =* 4.4 Hz), 2.19 (3H, s), 1.35 (3H, t, *J=* 7.6 Hz). MS Calcd.: 446.52; MS Found: 447.2 (M+H⁺).

### (254) A-254 : 6-((4-((2-Ethyl-4-(6-methylpyridin-2-yl)thiazol-5-yl)oxy)pyridin-2-yl)amino)picolinic acid

¹H-NMR (400 MHz, DMSO-*d*₆): *δ* 9.97 (1H, s), 8.10 (1H, d, *J=* 6.0 Hz), 7.86 (1H, d, *J=* 8.0 Hz), 7.78 (1H, t, *J =* 7.8 Hz), 7.71-7.65 (3H, m), 7.50 (1H, d, *J=* 6.8 Hz), 7.06 (1H, d, *J =* 5.6 Hz), 6.56 (1H, dd, *J =* 5.6, 2.4 Hz), 3.01 (2H, q, *J =* 7.6 Hz), 2.19 (3H, s), 1.34 (3H, t, *J =* 7.6 Hz). * A proton from OH was not observed. MS Calcd.: 433.48; MS Found: 434.1 (M+H⁺).

### (255) A-255 : 6-((4-((2-Ethyl-4-(6-methylpyridin-2-yl)thiazol-5-yl)oxy)pyridin-2-yl)amino)picolinamide

¹H-NMR (400 MHz, DMSO-*d*₆): *δ* 9.77 (1H, s), 8.13 (1H, d, *J=* 6.0 Hz), 7.93 (1H, d, *J=* 8.4 Hz), 7.80 (1H, t, *J=* 7.6 Hz), 7.70-7.57 (2H, m), 7.60 (1H, s), 7.47 (1H, d, *J=* 7.2 Hz), 7.38 (1H, s), 7.15 (1H, d, *J =* 2.4 Hz), 7.07 (1H, d, *J =* 7.2 Hz), 6.64 (1H, d, *J =* 5.6 Hz), 3.02 (2H, q, *J=* 7.6 Hz), 2.18 (3H, s), 1.35 (3H, t, *J =* 7.6 Hz). MS Calcd.: 432.50; MS Found: 433.2 (M+H⁺).

### (256) A-256 : 6-((4-((2-Ethyl-4-(6-methylpyridin-2-yl)thiazol-5-yl)oxy)pyridin-2-yl)amino)-N-methylpicolinamide

¹H-NMR (400 MHz, DMSO-*d*₆): *δ* 9.75 (1H, s), 8.13 (1H, d, *J=* 5.6 Hz), 8.00 (1H, s), 7.94 (1H, d, *J=* 6.8 Hz), 7.79 (1H, t, *J =* 8.0 Hz), 7.70-7.67 (2H, m), 7.45 (1H, d, *J =* 7.2 Hz), 7.12 (1H, s), 7.08 (1H, d, *J =* 8.8 Hz), 6.66 (1H, s), 3.00 (2H, q, *J =* 7.5 Hz), 2.78 (3H, d, *J* = 5.2 Hz), 2.19 (3H, s), 1.33 (3H, t, *J =* 7.4 Hz). 446.52; MS Found: 447.2 (M+H⁺).

### (257) A-257 : 3-((4-((2-Ethyl-4-(6-methylpyridin-2-yl)thiazol-5-yl)oxy)pyridin-2-yl)amino)benzoic acid

¹H-NMR (400 MHz, DMSO-*d*₆): *δ* 9.18 (1H, s), 8.21 (1H, s), 8.08 (1H, d, *J =* 6.0 Hz), 7.83 (1H, d, *J=* 8.0 Hz), 7.72-7.68 (2H, m), 7.41 (1H, d, *J =* 7.2 Hz), 7.30 (1H, t, *J =* 7.8 Hz), 7.08 (1H, d, *J =* 7.6 Hz), 6.56 (1H, d, *J =* 6.0 Hz), 6.34 (1H, d, *J =* 1.6 Hz), 3.01 (2H, q, *J =* 7.6 Hz), 2.22 (3H, s), 1.34 (3H, t, *J=* 7.4 Hz). * A proton from OH was not observed. MS Calcd.: 432.49; MS Found: 433.2 (M+H⁺).

### (258) A-258 : 3-((4-((2-Ethyl-4-(6-methylpyridin-2-yl)thiazol-5-yl)oxy)pyridin-2-yl)amino)benzamide

¹H-NMR (400 MHz, DMSO-*d*₆): *δ* 9.10 (1H, s), 8.07 (1H, d, *J=* 5.6 Hz), 7.99 (1H, s), 7.80-7.78 (2H, m), 7.70-7.69 (2H, m), 7.32 (1H, d, *J=* 8.4 Hz), 7.26-7.23 (2H, m), 7.09 (1H, dd, *J =* 6.0, 2.4 Hz), 6.55 (1H, dd, *J=* 5.6, 3.2 Hz), 6.35 (1H, d, *J =* 2.0 Hz), 3.03 (2H, q, *J =* 8.0 Hz), 2.22 (3H, s), 1.36 (3H, t, *J=* 7.2 Hz). MS Calcd.: 431.52; MS Found: 432.2 (M+H⁺).

### (259) A-259 : 3-((4-((2-Ethyl-4-(6-methylpyridin-2-yl)thiazol-5-yl)oxy)pyridin-2-yl)amino)-N-methylbenzamide

¹H-NMR (400 MHz, DMSO-*d*₆): *δ* 9.12 (1H, s), 8.27 (1H, d, *J=* 4.4 Hz), 8.07 (1H, d, *J =* 5.6 Hz), 7.97 (1H, s), 7.77 (1H, s), 7.70-7.69 (2H, m), 7.26 (2H, d, *J =* 5.6 Hz), 7.08 (1H, dd, *J =* 8.8, 2.4 Hz), 6.55 (1H, dd, *J=* 5.2, 3.2 Hz), 6.35 (1H, d, *J =* 2.4 Hz), 3.03 (2H, q, *J =* 7.6 Hz), 2.73 (3H, d, *J=* 4.4 Hz), 2.22 (3H, s), 1.36 (3H, t, *J=* 7.2 Hz). MS Calcd.: 445.16; MS Found: 446.2 (M+H⁺).

### (260) A-260 : 5-((4-((2-Ethyl-4-(6-methylpyridin-2-yl)thiazol-5-yl)oxy)pyridin-2-yl)amino)nicotinic acid

¹H-NMR (400 MHz, DMSO-*d*₆): *δ* 9.42 (1H, s), 8.84 (1H, d, *J=* 2.8 Hz), 8.73 (1H, s), 8.54 (1H, s), 8.14 (1H, d, *J=* 6.0 Hz), 7.72-7.67 (2H, m), 7.09 (1H, d, *J=* 6.8 Hz), 6.64 (1H, dd, *J=* 6.0, 1.8 Hz), 6.37 (1H, d, *J=* 1.6 Hz), 3.09 (2H, q, *J=* 7.7 Hz), 2.20 (3H, s), 1.35 (3H, t, *J =* 7.6 Hz). * A proton from OH was not observed. MS Calcd.: 433.48; MS Found: 434.2 (M+H⁺).

### (261) A-261 : 5-((4-((2-Ethyl-4-(6-methylpyridin-2-yl)thiazol-5-yl)oxy)pyridin-2-yl)amino)nicotinamide

¹H-NMR (400 MHz, DMSO-*d*₆): *δ* 9.32 (1H, s), 8.79 (1H, d, *J=* 2.4 Hz), 8.53 (1H, s), 8.49 (1H, s), 8.12 (1H, d, *J* = 4.2 Hz), 8.03 (1H, s), 7.73-7.67 (2H, m), 7.48 (1H, s), 7.09 (1H, d, *J =* 8.0 Hz), 6.63 (1H, dd, *J =* 6.0, 3.6 Hz), 6.63 (1H, dd, *J =* 6.0, 4.6 Hz), 6.37 (1H, d, *J =* 2.0 Hz), 3.04 (2H, q, *J =* 7.6 Hz), 2.30 (3H, s), 1.36 (3H, t, *J =* 7.2 Hz). MS Calcd.: 432.50; MS Found: 433.2 (M+H⁺).

### (262) A-262 : 5-((4-((2-Ethyl-4-(6-methylpyridin-2-yl)thiazol-5-yl)oxy)pyridin-2-yl)amino)-N-methylnicotinamide

¹H-NMR (400 MHz, DMSO-*d*₆): *δ* 9.34 (1H, s), 8.79 (1H, d, *J =* 2.8 Hz), 8.52-8.49 (2H, m), 8.44 (1H, s), 8.12 (1H, d, *J =* 5.6 Hz), 7.65-7.58 (2H, m), 7.09 (1H, d, *J =* 7.2 Hz), 6.63 (1H, dd, *J =* 5.6, 1.8 Hz), 6.37 (1H, d, *J=* 1.6 Hz), 3.02 (2H, q, *J=* 7.5 Hz), 2.76 (3H, d, *J =* 4.4 Hz), 2.21 (3H, s), 1.35 (3H, t, *J =* 7.4 Hz). MS Calcd.: 446.52; MS Found: 447.2 (M+H⁺).

### (263) A-263 : 4-((4-((2-Ethyl-4-(6-methylpyridin-2-yl)thiazol-5-yl)oxy)pyridin-2-yl)amino)picolinic acid

¹H-NMR (400 MHz, DMSO-*d*₆): *δ* 10.2 (1H, s), 8.29-8.25 (3H, m), 7.93 (1H, s), 7.75-7.68 (2H, m), 7.08 (1H, d, *J=* 7.2 Hz), 6.81 (1H, d, *J=* 5.2 Hz), 6.523 (1H, s), 3.02 (2H, q, *J* = 7.5 Hz), 2.16 (3H, s), 1.35 (3H, t, *J =* 7.4 Hz). * A proton from OH was not observed. MS Calcd.: 433.48; MS Found: 434.2 (M+H⁺).

### (264) A-264 : 4-((4-((2-Ethyl-4-(6-methylpyridin-2-yl)thiazol-5-yl)oxy)pyridin-2-yl)amino)picolinamide

¹H-NMR (400 MHz, DMSO-*d*₆): *δ* 9.69 (1H, s), 8.29 (1H, d, *J=* 5.6 Hz), 8.22-8.19 (2H, m), 7.97 (1H, s), 7.80 (1H, d, *J=* 4.0 Hz), 7.74-7.67 (2H, m), 7.51 (1H, s), 7.07 (1H, d, *J* = 7.2 Hz), 6.71 (1H, d, *J =* 4.0 Hz), 6.44 (1H, s), 3.01 (2H, q, *J =* 7.6 Hz), 2.18 (3H, s), 1.34 (3H, t, *J=* 7.4 Hz). MS Calcd.: 432.50; MS Found: 433.2 (M+H⁺).

### (265) A-265 : 4-((4-((2-Ethyl-4-(6-methylpyridin-2-yl)thiazol-5-yl)oxy)pyridin-2-yl)amino)-N-methylpicolinamide

¹H-NMR (400 MHz, DMSO-*d*₆): *δ* 9.72 (1H, s), 8.64 (1H, s), 8.30 (1H, d, *J=* 5.6 Hz), 8.20 (1H, d, *J =* 5.6 Hz), 8.16 (1H, s), 7.86 (1H, d, *J =* 4.0 Hz), 7.74-7.68 (2H, m), 7.08 (1H, d, *J =* 7.2 Hz), 6.71 (1H, d, *J =* 5.6 Hz), 6.45 (1H, d, *J =* 1.2 Hz), 3.02 (2H, q, *J =* 7.5 Hz), 2.77 (3H, d, *J =* 4.8 Hz), 2.18 (3H, s), 1.35 (3H, t, *J =* 7.6 Hz). MS Calcd.: 446.52; MS Found: 447.2 (M+H⁺).

### (266) A-266 : 2-((4-((2-Ethyl-4-(6-methylpyridin-2-yl)thiazol-5-yl)oxy)pyridin-2-yl)amino)isonicotinic acid

¹H-NMR (400 MHz, DMSO-*d*₆): *δ* 8.35 (1H, d, *J =* 5.6 Hz), 8.25 (1H, d, *J =* 6.4 Hz), 7.78 (1H, d, *J=* 7.6 Hz), 7.71-7.66 (2H, m), 7.53 (1H, d, *J=* 5.6 Hz), 7.09 (1H, d, *J=* 8.0 Hz), 6.97-6.93 (2H, m), 3.07 (2H, q, *J =* 7.5 Hz), 2.29 (3H, s), 1.44 (3H, t, *J =* 7.4 Hz). * Two protons from NH and OH were not observed. MS Calcd.: 433.48; MS Found: 434.2 (M+H⁺).

### (267) A-267 : 2-((4-((2-Ethyl-4-(6-methylpyridin-2-yl)thiazol-5-yl)oxy)pyridin-2-yl)amino)isonicotinamide

¹H-NMR (400 MHz, DMSO-*d*₆): *δ* 9.82 (1H, s), 8.21 (1H, d, *J=* 5.2 Hz), 8.11 (1H, d, *J =* 5.6 Hz), 8.05-8.02 (2H, m), 7.69-7.67 (2H, m), 7.58 (1H, s), 7.49 (1H, s), 7.17 (1H, d,*J* = 4.0 Hz), 7.07 (1H, d, *J =* 6.8 Hz), 6.57 (1H, d, *J =* 4.4 Hz), 3.04 (2H, q, *J =* 7.6 Hz), 2.19 (3H, s), 1.36 (3H, t, *J=* 7.6 Hz). MS Calcd.: 432.50; MS Found: 433.2 (M+H⁺).

### (268) A-268 : 2-((4-((2-Ethyl-4-(6-methylpyridin-2-yl)thiazol-5-yl)oxy)pyridin-2-yl)amino)-N-methylisonicotinamide

¹H-NMR (400 MHz, DMSO-*d*₆): *δ* 8.23 (1H, d, *J=* 5.2 Hz), 8.09 (1H, d, *J=* 6.0 Hz), 7.86 (1H, s), 7.67-7.66 (2H, m), 7.44 (1H, s), 7.14 (1H, d, *J* = 5.6 Hz), 7.10 (1H, t, *J* = 4.0 Hz), 6.63 (1H, dd, *J* = 6.0, 2.0 Hz), 3.07 (2H, q, *J* = 7.8 Hz), 2.91 (3H, s), 2.38 (3H, s), 1.44 (3H, t, *J=* 7.6 Hz). * Two protons from NH were not observed. MS Calcd.: 446.52; MS Found: 447.2 (M+H⁺).

### (269) A-269 : N-(2-(Dimethylamino)ethyl)-6-((4-((2-ethyl-4-(6-methylpyridin-2-yl)thiazol-5-yl)oxy)pyridin-2-yl)amino)nicotinamide

¹H-NMR (400 MHz, DMSO-*d*₆): *δ* 10.2 (1H, s), 9.25 (1H, s), 8.63 (1H, s), 8.58-8.57 (1H, m), 8.14 (1H, d, *J =* 6.0 Hz), 8.04 (1H, d, *J =* 11.2 Hz), 7.78 (1H, d, *J =* 8.4 Hz), 7.74-7.67 (1H, m), 7.58 (1H, s), 7.14 (1H, d, *J=* 17.6 Hz), 6.63 (1H, d, *J=* 3.2 Hz), 3.61-3.51 (2H, m), 3.26-3.19 (2H, m), 3.02 (2H, q, *J =* 7.6 Hz), 2.82 (6H, s), 2.19 (3H, s), 1.35 (3H, t, *J =* 7.4 Hz). MS Calcd.: 503.62; MS Found: 504.3 (M+H⁺).

### (270) A-270 : N-(Cyclopropylmethyl)-6-((4-((2-ethyl-4-(6-methylpyridin-2-yl)thiazol-5-yl)oxy)pyridin-2-yl)amino)nicotinamide

¹H-NMR (400 MHz, DMSO-*d*₆): *δ* 10.2 (1H, s), 8.68 (1H, d, *J=* 2.0 Hz), 8.15 (1H, d, *J=* 5.2 Hz), 8.09 (1H, d, *J=* 9.6 Hz), 7.77 (1H, d, *J=* 8.8 Hz), 7.73-7.66 (2H, m), 7.54 (1H, s), 7.06 (1H, d, *J =* 6.8 Hz), 6.66 (1H, d, *J =* 6.0 Hz), 4.07 (2H, d, *J =* 7.2 Hz), 3.01 (2H, q, *J =* 7.5 Hz), 1.33 (3H, t, *J =* 7.6 Hz), 0.77-0.89 (1H, m), 0.56-0.52 (2H, m), 0.35-0.31 (2H, m). MS Calcd.: 487.57; MS Found: 488.2 (M+H⁺).

### (271) A-271 : 2-(Dimethylamino)ethyl 6-((4-((2-ethyl-4-(6-methylpyridin-2-yl)thiazol-5-yl)oxy)pyridin-2-yl)amino)nicotinate

¹H-NMR (400 MHz, DMSO-*d*₆): *δ* 8.78 (1H, d, *J* = 2.0 Hz), 8.16 (1H, dd, *J* = 8.4, 2.4 Hz), 8.12 (1H, d, *J =* 6.0 Hz), 7.69-7.64 (2H, m), 7.59 (1H, d, *J =* 2.0 Hz), 7.56 (1H, d, *J =* 8.8 Hz), 7.10 (1H, dd, *J =* 8.4, 2.0 Hz), 6.67 (1H, dd, *J =* 5.8, 1.8 Hz), 4.44 (2H, t, *J =* 5.6 Hz), 3.07 (2H, q, *J=* 7.6 Hz), 2.81 (2H, t, *J=* 5.6 Hz), 2.39 (6H, s), 2.37 (3H, s), 1.44 (3H, t, *J=* 7.6 Hz). * A proton from NH was not observed. MS Calcd.: 504.60; MS Found: 505.3 (M+H⁺).

### (272) A-272 : N-(3-((4-((2-ethyl-4-(6-methylpyridin-2-yl)thiazol-5-yl)oxy)pyridin-2-yl)amino)phenyl)methanesulfonamide

¹H-NMR (400 MHz, DMSO-*d*₆): *δ* 9.63 (1H, s), 9.06 (1H, s), 8.04 (1H, d, *J=* 6.0 Hz), 7.70-7.69 (2H, m), 7.43 (2H, s), 7.14-7.09 (2H, m), 6.68 (1H, d, *J=* 6.8 Hz), 6.54 (1H, d, *J =* 3.6 Hz), 6.33 (1H, s), 3.03 (2H ,q, *J* = 8.0 Hz), 2.89 (3H, s), 2.30 (3H, s), 1.35 (3H, t, *J=* 7.2 Hz). MS Calcd.: 481.59; MS Found: 482.2 (M+H⁺).

### (273) A-273 : 2-(Dimethylamino)ethyl 3-(6-((4-((2-ethyl-4-(6-methylpyridin-2-yl)thiazol-5-yl)oxy)pyridin-2-yl)amino)nicotinamido)-5-fluorobenzoate

¹H-NMR (400 MHz, DMSO-*d*₆): *δ* 8.76 (1H, s), 8.17-8.13 (3H, m), 7.95 (1H, d, *J* = 10.4 Hz), 7.70-7.62 (2H, m), 7.61 (2H, d, *J=* 8.4 Hz), 7.54 (1H, d, *J=* 8.8 Hz), 7.11 (1H, d, *J* = 6.0 Hz), 6.67 (1H, d, *J=* 4.0 Hz), 4.49 (2H, t, *J=* 5.4 Hz), 3.08 (2H, q, *J=* 7.6 Hz), 2.70-2.60 (2H, m), 2.42 (3H, s), 2.37 (6H, d, *J =* 8.4 Hz), 1.45 (3H, t, *J =* 7.4 Hz). * Two protons from NH were not observed. MS Calcd.: 641.72; MS Found: 642.3 (M+H⁺).

### (274) A-274 : 3-((4-((2-Cyclopropyl-4-(6-methylpyridin-2-yl)thiazol-5-yl)oxy)pyridin-2-yl)amino)benzamide

¹H-NMR (400 MHz, DMSO-*d*₆): *δ* 9.12 (1H, s), 8.06 (1H , d, *J=* 6.0 Hz), 8.00 (1H, s), 7.83-7.78 (2H, m), 7.71-7.66 (2H, m), 7.32 (1H, d, *J=* 8.0 Hz), 7.27-7.23 (2H, m), 7.07 (1 H, dd, *J =* 6.4, 2.0 Hz), 6.85 (1H, dd, *J =* 5.6, 2.0 Hz), 6.34 (1H, d, *J =* 2.0 Hz), 2.45-2.40 (1 H, m), 2.21 (3 H, s), 1.66-1.13 (2 H, m), 1.07-1.03 (2 H, m). MS Calcd.: 443.53; MS Found: 444.28 (M+H⁺).

### (275) A-275 : 6-((4-((2-Ethyl-4-(6-methylpyridin-2-yl)thiazol-5-yl)oxy)pyridin-2-yl)amino)nicotinamide

¹H-NMR (400 MHz, DMSO-*d*₆): *δ* 10.01 (1H, s), 8.64 (1H, d, *J=* 1.6 Hz), 8.12 (1H, d, *J=* 5.6 Hz), 8.05 (1H, dd, *J =* 9.2, 2.4 Hz), 7.88 (1H, s), 7.75 (1H, d, *J =* 9.2 Hz), 7.68-7.67 (2H, m), 7.46 (1H, d, *J=* 1.6 Hz), 7.28 (1H, s), 7.05 (1H, t, *J=* 4.0 Hz), 6.61 (1H, dd, *J=* 5.6, 2.4 Hz), 2.44-2.40 (1H, m), 2.17 (3H, s), 1.17-1.13 (2H, m), 1.07-1.04 (2H, m). MS Calcd.: 444.51; MS Found: 445.27 (M+H⁺).

### (276) A-276 : 2-((4-((2-Cyclopropyl-4-(6-methylpyridin-2-yl)thiazol-5-yl)oxy)pyridin-2-yl)amino)isonicotinamide

¹H-NMR (400 MHz, DMSO-*d*₆): *δ* 9.81 (s, 1H), 8.21 (d, *J =* 4.8 Hz, 1H), 8.10 (d, *J* = 6.0 Hz, 1H), 8.05 (s, 1H), 7.44 (d, *J =* 2.4 Hz, 1H), 7.18 (d, *J =* 5.2 Hz, 1H), 7.06 (t, *J =* 4.4 Hz, 1H), 6.57 (dd, *J =* 6.0, 2.4 Hz, 1H), 2.44-2.30 (m, 2H), 2.18 (s, 3H), 1.21-1.12 (m, 2H), 1.09-1.03 (m, 2H). MS Calcd.: 444.51; MS Found: 445.27 (M+H⁺).

### (277) A-277 : 3-((4-((2-Cyclopropyl-4-(6-methylpyridin-2-yl)thiazol-5-yl)oxy)pyridin-2-yl)amino)benzenesulfonamide

¹H-NMR (400 MHz, DMSO-*d*₆): *δ* 9.33 (1H, s), 8.19 (1H, s), 8.09 (1H, d, *J =* 6.0 Hz), 7.76 (1H, d, *J=* 7.6 Hz), 7.70-7.68 (2H, m), 7.34 (1H, t, *J=* 8.0 Hz), 7.29-7.27 (3H, m), 7.09-7.07 (1H, m), 6.65 (1H, dd, *J=* 5.6, 2.4 Hz), 6.36 (1H, d, *J=* 2.0 Hz), 2.45-2.40 (1H, m), 2.20 (3H, s), 1.18-1.13 (2H, m), 1.08-1.04 (2H, m). MS Calcd.: 479.57; MS Found: 480.29 (M+H⁺).

### (278) A-278 : 6-((4-((2-Cyclopropyl-4-(6-methylpyridin-2-yl)thiazol-5-yl)oxy)pyridin-2-yl)amino)picolinamide

¹H-NMR (400 MHz, DMSO-*d*₆): *δ* 9.76 (1H, s), 8.12 (1H, d, *J =* 5.6 Hz), 7.79 (1H, t, *J=* 7.6 Hz), 7.68-7.65 (2H, m), 7.59 (1H, br s), 7.47 (1H, d, *J=* 7.2 Hz), 7.41 (1H, br s), 7.10 (1H, d, *J =* 2.4 Hz), 7.05 (1H, dd, *J =* 6.0, 2.4 Hz), 6.63 (1H, dd, *J =* 5.6, 2.4 Hz), 2.47-2.41 (1H, m), 2.17 (3H, s), 1.16-1.12 (2H, m), 1.10-1.05 (2H, m). MS Calcd.: 444.51; MS Found: 445.32 (M+H⁺).

### (279) A-279 : 6-((4-((2-Cyclopropyl-4-(6-methylpyridin-2-yl)thiazol-5-yl)oxy)pyridin-2-yl)amino)-N-methylpicolinamide

¹H-NMR (400 MHz, DMSO-*d*₆): *δ* 9.75 (1H, s), 8.13 (1H, d, *J=* 5.2 Hz), 8.01-7.95 (2H, m), 7.79 (1H, t, *J=* 7.2 Hz), 7.68-7.65 (2H, m), 7.45 (1H, d, *J=* 7.6 Hz), 7.09 (1H, d, *J=* 4.4 Hz), 6.65 (1H, dd, *J =* 5.6, 2.4 Hz), 2.78 (3 H, d, *J =* 5.2 Hz), 2.44-2.38 (1H, m), 2.18 (3H, s), 1.66-1.12 (2H, m), 1.05-1.01 (2H, m). MS Calcd.: 458.54; MS Found: 459.31 (M+H⁺).

### (280) A-280 : 2-((4-((2-Cyclopropyl-4-(6-methylpyridin-2-yl)thiazol-5-yl)oxy)pyridin-2-yl)amino)-N-methylisonicotinamide

¹H-NMR (400 MHz, DMSO-*d*₆): *δ* 9.84 (1H, s), 8.56 (1H, d, *J=* 4.8 Hz), 8.22 (1H, d, *J=* 5.2 Hz), 8.11 (1H, d, *J=* 6.1 Hz), 8.04 (1H, s), 7.63 (2H, d, *J=* 4.8 Hz), 7.44 (1H, d, *J =* 2.4 Hz), 7.14 (1H, d, *J =* 1.6 Hz), 7.06 (1H, t, *J =* 4.4 Hz), 6.57 (1H, dd, *J =* 6.0, 2.4 Hz), 2.75 (3H, d, *J =* 4.2 Hz), 2.46-2.31 (1H, m), 2.07 (3H, s), 1.21-1.27 (2 H, m), 1.07-1.03 (2H, m). MS Calcd.: 458.54; MS Found: 459.37 (M+H⁺).

### (281) A-281 : 3-((4-((2-Cyclopropyl-4-(6-methylpyridin-2-yl)thiazol-5-yl)oxy)pyridin-2-yl)amino)-N-methylbenzamide

¹H-NMR (400 MHz, DMSO-*d*₆): *δ* 9.14 (1H, s), 8.30 (1H, d, *J=* 4.9 Hz), 8.06 (1H, d, *J =* 5.7 Hz), 7.98 (1H, s), 7.78 (1H, dd, *J =* 6.7, 3.0 Hz), 7.73-7.63 (2H, m), 7.25 (2H, d, *J =* 4.7 Hz), 7.07 (1H, dd, *J =* 5.6, 2.7 Hz), 6.55 (1 H, dd, *J =* 5.6, 1.8 Hz), 6.33 (1H, d, *J =* 2.0 Hz), 2.73 (3H, d, *J =* 4.4 Hz), 2.42 (1H, dd, *J=* 9.1, 4.6 Hz), 2.21 (3 H, s), 1.19-1.11 (2H, m), 1.07-1.00 (2H, m). MS Calcd.: 457.55; MS Found: 458.24 (M+H⁺).

### (282) A-282 : 6-((4-((2-Cyclopropyl-4-(6-methylpyridin-2-yl)thiazol-5-yl)oxy)pyridin-2-yl)amino)-N-methylnicotinamide

¹H-NMR (400 MHz, DMSO-*d*₆): *δ* 10.01 (1H, s), 8.59 (1H, d, *J =* 2.2 Hz), 8.35 (1H, d, *J=* 4.3 Hz), 8.12 (1H, d, *J =* 5.7 Hz), 8.01 (1H, dd, *J =* 9.1, 2.4 Hz), 7.76 (1H, d, *J =* 8.6 Hz), 7.68 (2H, dd, *J=* 6.3, 2.3 Hz), 7.44 (1H, d, *J=* 1.9 Hz), 7.09-7.02 (1H, m), 6.65-6.57 (1H, m), 2.74 (2H, d, *J* = 4.4 Hz), 2.41 (1H, dd, *J* = 13.2, 1.0 Hz), 2.17 (2H, s), 1.19-1.11 (2H, m), 1.06-1.03 (2H, m). MS Calcd.: 458.54; MS Found: 459.25 (M+H⁺).

### (283) A-283 : 3-((4-((2-(Dimethylamino)-4-(6-methylpyridin-2-yl)thiazol-5-yl)oxy)pyridin-2-yl)amino)benzenesulfonamide

¹H-NMR (400 MHz, DMSO-d₆): *δ* 9.32 (1H, s), 8.22 (1H, s), 8.09 (1H, d, *J=* 5.6 Hz), 7.78 (1H, d, *J=* 8.4 Hz), 7.70-7.65 (2H, m), 7.39 (1H, t, *J=* 8.0 Hz), 7.30-7.26 (3H, m), 7.07-7.04 (1H, m), 6.61 (1H, dd, *J =* 2.0 Hz, 5.8 Hz), 6.45 (1H, d, *J =* 2.0 Hz), 3.08 (6H, s), 2.08 (3H, s). MS Calcd.: 482.58; MS Found: 483.2 (M+H⁺).

### (284) A-284 : 6-((4-((2-(Dimethylamino)-4-(6-methylpyridin-2-yl)thiazol-5-yl)oxy)pyridin-2-yl)amino)nicotinamide

¹H-NMR (400 MHz, DMSO-*d*₆): *δ* 9.98 (1H, s), 8.64 (1H, d, *J=* 2.0 Hz), 8.11 (1H, d, *J=* 6.0 Hz), 8.05 (1H, dd, *J =* 8.8, 2.4 Hz), 7.87 (1H, s), 7.76 (1H, d, *J=* 8.8 Hz), 7.65 (2H, d, *J =* 4.4 Hz), 7.48 (1H, d, *J =* 2.0 Hz), 7.27 (1H, s), 7.01 (1H, t, *J =* 4.2 Hz), 6.61 (1H, dd, *J =* 6.0, 2.4 Hz), 3.07 (6H, s), 2.16 (3H, s). MS Calcd.: 447.51; MS Found: 448.2 (M+H⁺).

### (285) A-285 : 6-((4-((2-(Dimethylamino)-4-(6-methylpyridin-2-yl)thiazol-5-yl)oxy)pyridin-2-yl)amino)nicotinic acid

¹H-NMR (400 MHz, DMSO-*d*₆): *δ* 8.79 (1H, s), 8.31 (1H, d, *J=* 6.8 Hz), 8.24 (1H, d, *J =* 8.8 Hz), 7.72 (2H, s), 7.33 (1H, s), 7.14-7.03 (3H, m), 3.09 (6H, s), 2.16 (3H, s). * Two protons from NH and OH were not observed. MS Calcd.: 448.50; MS Found: 449.2 (M+H⁺).

### (286) A-286 : 6-((4-((2-(Dimethylamino)-4-(6-methylpyridin-2-yl)thiazol-5-yl)oxy)pyridin-2-yl)amino)-N-methylnicotinamide

¹H-NMR (400 MHz, DMSO-*d*₆): *δ* 9.96 (1H, s), 8.60 (1H, s), 8.32 (1H, d, *J =* 4.0 Hz), 8.11 (1H, d, *J* = 5.6 Hz), 8.01 (1H, dd, *J* = 9.2, 2.4 Hz), 7.78 (1H, d, *J* = 8.8 Hz), 7.65 (2H, d, *J =* 4.8 Hz), 7.46 (1H, d, *J =* 1.6 Hz), 7.01 (1H, t, *J =* 4.6 Hz), 6.61 (1H, dd, *J =* 5.8, 2.2 Hz), 3.07 (6H, s), 2.75 (3H, d, *J =* 4.0 Hz), 2.16 (3H, s). MS Calcd.: 461.54; MS Found: 462.2 (M+H⁺).

### (287) A-287 3-((4-((2-Isopropyl-4-(6-methylpyridin-2-yl)thiazol-5-yl)oxy)pyridin-2-yl)amino)benzenesulfonamide

¹H-NMR (400 MHz, DMSO-*d*₆): *δ* 9.33 (1H, s), 8.18 (1H, s), 8.09 (1H, d, *J =* 6.0 Hz), 7.78-7.68 (3H, m), 7.38 (1H, t, *J=* 7.8 Hz), 7.28 (1H, d, *J=* 7.8 Hz), 7.26 (2H, s), 7.08 (1H, d, *J =* 6.0 Hz), 6.59 (1H, dd, *J =* 5.6, 2.4 Hz), 6.36 (1H, d, *J =* 2.4 Hz), 3.31-3.27 (1H, m), 2.21 (3H, s), 1.38 (6H, d, *J=* 7.2 Hz). MS Calcd.: 481.59; MS Found: 482.2 (M+H⁺).

### (288) A-288 :6-((4-((2-Isopropyl-4-(6-methylpyridin-2-yl)thiazol-5-yl)oxy)pyridin-2-yl)amino)nicotinamide

¹H-NMR (400 MHz, DMSO-*d*₆): *δ* 10.01 (1H, s), 8.62 (1H, s), 8.13(1H, d, *J* = 6.4 Hz), 8.05 (1H, d, *J=* 9.2 Hz), 7.86 (1H, s), 7.71-7.68 (3H, m), 7.50 (1H, s), 7.25 (1H, s), 7.07 (1H, d, *J=* 6.4 Hz), 6.62 (1H, d, *J=* 6.4 Hz), 3.32-3.25 (1H, m), 2.06 (3H, s), 1.39 (6H, d, *J=* 7.2 Hz). MS Calcd.: 446.52; MS Found: 447.2 (M+H⁺).

### (289) A-289 : 3-((4-((4-(6-Methylpyridin-2-yl)-2-(trifluoromethyl)thiazol-5-yl)oxy)pyridin-2-yl)amino)benzenesulfonamide

¹H-NMR (400 MHz, DMSO-*d*₆): *δ* 9.34 (1H, s), 8.17 (1H, s), 8.13 (1H, d, *J =* 6.0 Hz), 7.79-7.76 (3H, m), 7.39 (1H, t, *J=* 8.0 Hz), 7.30 (1H, d, *J=* 9.2 Hz), 7.27 (2H, s), 7.19 (1H, dd, *J* = 6.4, 2.8 Hz), 6.69 (1H, dd, *J=* 6.0, 2.4 Hz), 6.41 (1H, d, *J=* 2.0 Hz), 2.25 (3H, s). MS Calcd.: 507.51; MS Found: 508.1 (M+H⁺).

### (290) A-290 : 6-((4-((2-Isopropyl-4-(6-methylpyridin-2-yl)thiazol-5-yl)oxy)pyridin-2-yl)amino)nicotinamide

¹H NMR (400 MHz, CDCl₃): δ 8.63 (s, 1H), 8.17 (d, *J* = 5.8 Hz, 1H), 8.05 (d, *J* = 8.6 Hz, 1H), 7.82 (d, *J =* 8.4 Hz, 2H), 7.62 (t, *J =* 7.7 Hz, 1H), 7.54 (s, 1H), 7.46 (d, *J =* 8.8 Hz, 1H), 7.04 (d, *J=* 7.7 Hz, 1H), 6.62 (d, *J=* 5.8 Hz, 1H). MS Calcd.: 472.45; MS Found: 473.27 (M+H⁺).

### (291) A-291 : 3-((4-((4-(6-Methylpyridin-2-yl)-2-(trifluoromethyl)thiazol-5-yl)oxy)pyridin-2-yl)amino)benzamide

¹H-NMR (400 MHz, DMSO-*d*₆): *δ* 9.14 (s, 1H), 8.10 (d, *J* = 6.0 Hz, 1H), 7.99 (br s, 1H), 7.83 (br s, 1H), 7.80-7.74 (m, 3H), 7.33 (d, *J=* 7.2 Hz, 1H), 7.28-7.24 (m, 2H), 7.19 (dd, *J =* 6.4, 2.0 Hz, 1H), 6.64 (dd, *J =* 6.0, 2.0 Hz, 1H), 6.39 (d, *J =* 2.0 Hz, 1H), 2.26 (s, 3H). MS Calcd.: 471.46; MS Found: 472.28 (M+H⁺).

### (292) A-292 : 3-((4-((2-(Methylamino)-4-(6-methylpyridin-2-yl)thiazol-5-yl)oxy)pyridin-2-yl)amino)benzenesulfonamide

¹H-NMR (400 MHz, DMSO-*d*₆): *δ* 9.33 (1H, s), 8.22 (1H, s), 8.09 (1H, d, *J=* 5.6 Hz), 7.79 (1H, d, *J=* 8.0 Hz), 7.69-7.67 (2H, m), 7.65-7.62 (1H, m), 7.39 (1H, t, *J=* 8.0 Hz), 7.30 (1H, d, *J =* 6.0 Hz), 7.27 (2H, s), 7.04 (1H, d, *J =* 7.2 Hz), 6.61 (1H, dd, *J =* 5.6, 2.4 Hz), 6.46 (1H, d, *J =* 2.4 Hz), 2.89 (3H, d, *J =* 4.8 Hz), 2.20 (3H, s). MS Calcd.: 468.55; MS Found: 469.2 (M+H⁺).

### (293) A-293 : 6-((4-((2-Cyclopropyl-4-phenyloxazol-5-yl)oxy)pyridin-2-yl)amino)nicotinic acid

¹H-NMR (400 MHz, DMSO-*d*₆): *δ* 10.4 (1H, brs), 8.68 (1H, s), 8.25 (1H, d, *J =* 5.2 Hz), 8.10 (1H, d, *J=* 9.2 Hz), 7.71 (1H, d, *J=* 8.8 Hz), 7.63-7.61 (3H, m), 7.38 (2H, t, *J=* 7.6 Hz), 7.26 (1H, t, *J =* 7.6 Hz), 6.80 (1H, d, *J =* 5.6 Hz), 2.17-2.13 (1H, m), 1.08-0.99 (4H, m). * A proton from NH was not observed. MS Calcd.: 414.41; MS Found: 415.2 (M+H⁺).

### (294) A-294 : 6-((4-((2-Cyclopropyl-4-phenyloxazol-5-yl)oxy)pyridin-2-yl)amino)nicotinamide

¹H-NMR (400 MHz, DMSO-*d*₆): *δ* 10.2 (1H, s), 8.66 (1H, s), 8.23 (1H, d, *J =* 5.6 Hz), 8.08 (1H, d, *J=* 8.4 Hz), 7.92 (1H, s), 7.70 (1H, d, *J=* 8.8 Hz), 7.63-7.58 (3H, m), 7.38 (2H, t, *J=* 7.4 Hz), 7.31 (1H, s), 7.26 (1H, t, *J=* 7.6 Hz), 6.74 (1H, d, *J=* 6.0 Hz), 2.19-2.10 (1H, m), 1.10-1.00 (4H, m). MS Calcd.: 446.52; MS Found: 414.2 (M+H⁺).

### (295) A-295 : 4-((4-((2-Cyclopropyl-4-phenyloxazol-5-yl)oxy)pyridin-2-yl)amino)benzoic acid

¹H-NMR (400 MHz, DMSO-*d*₆): *δ* 9.54 (1H, s), 8.21 (1H, d, *J=* 6.0 Hz), 7.81 (2H, d, *J =* 8.4 Hz), 7.72 (2H, d, *J =* 8.4 Hz), 7.61 (2H, d, *J =* 7.6 Hz), 7.39 (2H, t, *J =* 7.6 Hz), 7.25 (1H, t, *J =* 7.2 Hz), 6.73 (1H, d, *J=* 6.0 Hz), 6.46 (1H, s), 2.18-2.11 (1H, m), 1.08-1.01 (4H, m). * A proton from OH was not observed. MS Calcd.: 413.43; MS Found: 414.2 (M+H⁺).

### (296) A-296 : 3-((4-((2-Cyclopropyl-4-phenyloxazol-5-yl)oxy)pyridin-2-yl)amino)benzamide

¹H-NMR (400 MHz, DMSO-*d*₆): *δ* 9.28 (s, 1H), 8.17 (d, *J =* 5.8 Hz, 1H), 8.02 (s, 1H), 788-7.83 (m, 2H), 7.64 (d, *J=* 7.4 Hz, 2H), 7.44-7.37 (m, 3H), 7.32-7.28 (m, 3H), 6.66 (d, *J=* 5.8, 2.0 Hz, 1H), 6.41 (d, *J=* 2.0 Hz, 1H), 2.19-2.15 (m, 1H), 1.10-1.03 (m, 4H). MS Calcd.: 412.45; MS Found: 413.30 (M+H⁺).

### (297) A-297 : 3-((4-((2-Cyclopropyl-4-phenyloxazol-5-yl)oxy)pyridin-2-yl)amino)-N-methylbenzamide

¹H-NMR (400 MHz, DMSO-*d*₆): *δ* 9.28 (s, 1H), 8.32 (d, *J =* 5.5 Hz, 1H), 8.15 (d, *J* = 5.8 Hz, 1H), 7.97 (s, 1H), 7.86-7.76 (m, 1H), 7.61 (d, *J=* 7.4 Hz, 2H), 7.39 (t, *J=* 7.5 Hz, 2H), 7.28 (d, *J=* 10.6 Hz, 3H), 6.64 (dd, *J=* 5.8, 2.2 Hz, 1H), 6.38 (d, *J=* 2.3 Hz, 1H), 2.73 (d, *J=* 4.4 Hz, 3H), 2.14 (s, 1H), 1.11-0.96 (m, 4H). MS Calcd.: 426.48; MS Found: 427.29 (M+H⁺).

### (298) A-298 : 3-((4-((2-Cyclopropyl-4-phenyloxazol-5-yl)oxy)pyridin-2-yl)amino)benzenesulfonamide

¹H-NMR (400 MHz, DMSO-*d*₆): *δ* 9.47 (s, 1H), 8.21-8.13 (m, 2H), 7.80 (d, *J =* 7.5 Hz, 1H), 7.62 (d, *J =* 8.0 Hz, 2H), 7.43-7.37 (m, 3H), 7.32-7.30 (m, 4H), 6.69 (d, *J =* 4.4 Hz, 1H), 6.40 (s, 1H), 2.17-2.11 (m, 1H), 1.13-0.95 (m, 4H). MS Calcd.: 448.50; MS Found: 449.23 (M+H⁺).

### (299) A-299 : 6-((4-((2-Cyclopropyl-4-phenyloxazol-5-yl)oxy)pyridin-2-yl)amino)picolinamide

¹H-NMR (400 MHz, DMSO-*d*₆): *δ* 10.19 (s, 1H), 8.66 (d, *J =* 2.1 Hz, 1H), 8.23 (d, *J* = 5.7 Hz, 1H), 8.07 (dd, *J =* 8.8, 2.3 Hz, 1H), 7.91 (s, 1H), 7.70 (d, *J =* 8.8 Hz, 1H), 7.62 (d, *J* = 7.6 Hz, 2H), 7.58 (d, *J =* 2.2 Hz, 1H), 7.39 (t, *J =* 7.7 Hz, 2H), 7.32 (s, 1H), 7.27 (t, *J =* 7.3 Hz, 1H), 6.75 (dd, *J =* 5.8, 2.4 Hz, 1H), 2.20-2.09 (m, 1H), 1.13-0.97 (m, 4H). MS Calcd.: 413.15; MS Found: 414.31 (M+H⁺).

### (300) A-300 : 3-((4-((2-Cyclopropyl-4-phenyloxazol-5-yl)oxy)pyridin-2-yl)amino)benzoic acid

¹H-NMR (400 MHz, DMSO-*d*₆): *δ* 12.85 (s, 1H), 9.34 (s, 1H), 8.22 (s, 1H), 8.18 (d, *J* = 5.7 Hz, 1H), 7.87 (d, *J =* 8.9 Hz, 1H), 7.62 (d, *J =* 7.7 Hz, 2H), 7.45 (d, *J =* 7.9 Hz, 1H), 7.40 (t, *J =* 7.6 Hz, 2H), 7.34 (t, *J =* 7.9 Hz, 1H), 7.27 (t, *J =* 7.3 Hz, 1H), 6.67 (d, *J =* 5.5 Hz, 1H), 6.38 (s, 1H), 2.15 (d, *J =* 4.9 Hz, 1H), 1.12-0.97 (m, 4H). MS Calcd.: 413.14; MS Found: 414.25 (M+H⁺).

### (301) A-301 : 6-((4-((2-Cyclopropyl-4-phenyloxazol-5-yl)oxy)pyridin-2-yl)amino)picolinic acid

¹H-NMR (400 MHz, DMSO-*d*₆): *δ* 13.05 (br s, 1H), 10.99 (br s, 1H), 8.73 (d, *J=* 2.1 Hz, 1H), 8.32 (d, *J =* 6.1 Hz, 1H), 8.18 (dd, *J* = 8.7, 2.0 Hz, 1H), 7.67 - 7.56 (m, 3H), 7.40 (dd, *J =* 15.6, 8.1 Hz, 3H), 7.28 (t, *J =* 7.4 Hz, 1H), 6.95 (d, *J =* 4.5 Hz, 1H), 2.16 (m, 1H), 1.14 - 0.99 (m, 4H). MS Calcd.: 414.42; MS Found: 415.26 (M+H⁺).

### (302) A-302 : 6-((4-((2-Cyclopropyl-4-phenyloxazol-5-yl)oxy)pyridin-2-yl)amino)-N-methylpicolinamide

¹H-NMR (400 MHz, DMSO-*d*₆): *δ* 11.04 (s, 1H), 8.70 (s, 1H), 8.55 (d, *J =* 4.9 Hz, 1H), 8.34 (d, *J=* 6.4 Hz, 1H), 8.20 (d, *J=* 8.7 Hz, 1H), 7.63 (d, *J=* 7.8 Hz, 2H), 7.53-7.35 (m, 3H), 7.29 (t, *J=* 7.4 Hz, 1H), 7.20 (s, 1H), 7.03 (s, 1H), 2.77 (d, *J =* 4.4 Hz, 3H), 2.20-2.13 (m, 1H), 1.13-0.99 (m, 4H). MS Calcd.: 427.46; MS Found: 428.24 (M+H⁺).

### (303) A-303 : 3-((4-((2-Methyl-4-(6-methylpyridin-2-yl)thiazol-5-yl)oxy)pyridin-2-yl)amino)benzenesulfonamide

¹H-NMR (400 MHz, DMSO-*d*₆): *δ* 9.47 (1H, s), 8.18 (1H, s), 8.09 (1H, d, *J =* 6.0 Hz), 7.76 (1H, d, *J=* 8.0 Hz), 7.70-7.67 (2H, m), 7.37 (1H, t, *J =* 8.0 Hz), 7.29-7.27 (3H, m), 7.09-7.05 (1H, m), 6.59 (1H, dd, *J=* 7.6, 2.0 Hz), 6.36 (1H, d, *J=* 2.0 Hz), 2.69 (3H, s), 2.21 (3H, s). MS Calcd.: 453.54; MS Found: 454.1 (M+H⁺).

### (304) A-304 : 2-(3-((4-((2-Methyl-4-phenylthiazol-5-yl)oxy)pyridin-2-yl)amino)phenyl)acetonitrile

¹H-NMR (400 MHz, DMSO-*d*₆): *δ* 9.10 (1H, s), 8.06 (1H, d, *J=* 6.0 Hz), 7.70-7.69 (2H, m), 7.61 (1H, s), 7.55 (1H, d, *J =* 8.4 Hz), 7.20 (1H, t, *J =* 7.8 Hz), 7.08 (1H, t, *J =* 4.6 Hz), 6.79 (1H, d, *J =* 8.0 Hz), 6.55 (1H, dd, *J =* 5.8, 2.2 Hz), 6.34 (1H, d, *J =* 2.4 Hz), 3.96 (2H, s), 2.68 (3H, s), 2.21 (3H, s). MS Calcd.: 413.49; MS Found: 414.2 (M+H⁺).

### (305) A-305 : N-(3-((1H-Tetrazol-5-yl)methyl)phenyl)-4-((2-methyl-4-(6-methylpyridin-2-yl)thiazol-5-yl)oxy)pyridin-2-amine

¹H-NMR (400 MHz, DMSO-*d*₆): *δ* 8.99 (1H, s), 8.03 (1H, d, *J=* 5.6 Hz), 7.69 (2H, d, *J =* 4.4 Hz), 7.55 (1H, d, *J =* 7.6 Hz), 7.37 (1H, s), 7.15 (1H, t, *J =* 7.8 Hz), 7.08 (1H, t, *J =* 4.4 Hz), 6.72 (1H, d, *J =* 7.6 Hz), 6.53 (1H, d, *J =* 5.6 Hz), 6.31 (1H, d, *J =* 2.0 Hz), 4.18 (2H, s), 2.68 (3H, s), 2.21 (3H, s). * A proton from NH was not observed. MS Calcd.: 456.52; MS Found: 457.2 (M+H⁺).

### (306) A-306 : 3-(3-((4-((2-Methyl-4-(6-methylpyridin-2-yl)thiazol-5-yl)oxy)pyridin-2-yl)amino)phenyl)propanenitrile

¹H-NMR (400 MHz, DMSO-*d*₆): *δ* 8.98 (1H, s), 8.05 (1H, d, *J=* 6.0 Hz), 7.69 (2H, d, *J=* 4.8 Hz), 7.49 (1H, d, *J=* 8.0 Hz), 7.41 (1H, s), 7.15 (1H, t, *J=* 8.0 Hz), 7.08 (1H, t, *J=* 4.4 Hz), 6.77 (1H, d, *J =* 6.8 Hz), 6.53 (1H, dd, *J =* 6.0, 2.0 Hz), 6.34 (1H, d, *J =* 2.4 Hz), 2.78-2.73 (4H, m), 2.68 (3H, s), 2.22 (3H, s). MS Calcd.: 427.52; MS Found: 428.2 (M+H⁺).

### (307) A-307 : N-(3-(2-(2H-tetrazol-5-yl)ethyl)phenyl)-4-((2-methyl-4-(6-methylpyridin-2-yl)thiazol-5-yl)oxy)pyridin-2-amine

¹H-NMR (400 MHz, DMSO-*d*₆): *δ* 8.92 (1H, s), 8.06 (1H, d, *J=* 5.6 Hz), 7.69 (2H, d, *J =* 4.0 Hz), 7.43-7.39 (2H, m), 7.20-7.06 (2H, m), 6.71 (1H, d, *J=* 7.6 Hz), 6.52 (1H, d, *J =* 6.4 Hz), 6.33 (1H, s), 2.92-2.88 (2H, m), 2.87-2.80 (2H, m), 2.65 (3H, s), 2.23 (3H, s). * A proton from NH was not observed. MS Calcd.: 470.55; MS Found: 471.2 (M+H⁺).

### (308) A-308 : Methyl 2-methyl-2-(3-((4-((2-methyl-4-(6-methylpyridin-2-yl)thiazol-5-yl)oxy)pyridin-2-yl)amino)phenyl)propanoate

¹H-NMR (400 MHz, CDCl₃): *δ* 9.00 (1H, s), 8.06 (1H, d, *J =* 6.0 Hz), 7.12 (2H, d, *J* =4.4 Hz), 7.63 (1H, d, *J =* 8.0 Hz), 7.40 (1H, s), 7.17 (1H, t, *J =* 7.6 Hz), 7.10 (1H, t, *J =* 4.8 Hz), 6.80 (1H, d, *J =* 8.4 Hz), 6.55-6.54 (1H, m), 6.33 (1H, d, *J* =1.6 Hz), 3.58 (3H, s), 2.70 (3H, s), 2.24 (3H, s), 1.45 (6H, s). MS Calcd.: 474.57; MS Found: 475.2 (M+H⁺).

### (309) A-309 : 2-Methyl-2-(3-((4-((2-methyl-4-(6-methylpyridin-2-yl)thiazol-5-yl)oxy)pyridin-2-yl)amino)phenyl)propanoic acid

¹H-NMR (400 MHz, DMSO-d₆): *δ* 8.06 (1H, d, *J =* 6.8 Hz), 7.80-7.78 (2H, m), 7.41 (1H, brs), 7.31-7.25 (2H, m), 7.19 (1H, d, *J=* 6.8 Hz), 7.04 (1H, brs), 6.73 (1H, brs), 6.45 (1H, s), 2.71 (3H, s), 2.28 (3H, s), 1.43 (6H, s). * Two protons from OH, NH were not observed. MS Calcd.: 460.55; MS Found: 461.2 (M+H⁺).

### (310) A-310 : 4-((4-((2-(Methylamino)-4-phenylthiazol-5-yl)oxy)pyridin-2-yl)amino)benzamide

¹H-NMR (400 MHz, DMSO-*d*₆): *δ* 9.39 (1H, s), 8.15 (1H, d, *J =* 5.6 Hz), 7.77-7.74 (4H, m), 7.69-7.67 (3H, m), 7.35 (2H, t, *J=* 7.6 Hz), 7.25 (1H, t, *J=* 7.2 Hz), 7.10 (1H, s), 6.69 (1H, dd, *J =* 2.0 Hz, 5.6 Hz), 6.55 (1H, d, *J =* 2.4 Hz), 2.89 (3H, d, *J =* 4.8 Hz). * A proton from NH was not observed. MS Calcd.: 417.48; MS Found: 418.2 (M+H⁺).

### (311) A-311 : N-(3-((4-((2-Ethyl-4-phenylthiazol-5-yl)oxy)pyridin-2-yl)amino)phenyl)-1,1,1-trifluoromethanesulfonamide

¹H-NMR (400 MHz, DMSO-*d*₆): *δ* 9.23 (1H, brs), 8.11 (1H, d, *J=* 5.6 Hz), 7.81 (2H, d, *J=* 7.2 Hz), 7.59 (1H, brs), 7.51 (1H, brs), 7.40 (2H, t, *J=* 7.6 Hz), 7.29-7.31 (1H, m), 7.19-7.21 (1H, m), 6.72 (1H, d, *J =* 9.2 Hz), 6.64 (1H, d, *J =* 3.2 Hz), 6.43 (1H, s), 3.00 (2H, t, *J =* 7.5 Hz), 1.33 (3H, t, *J =* 7.6 Hz). *A proton from NH was not observed. MS Calcd.: 520.55; MS Found: 521.1 (M+H⁺).

### (312) A-312 : 4-((2-Ethyl-4-phenylthiazol-5-yl)oxy)-N-(5-(3-(methylamino)pyrrolidin-1-yl)pyridin-2-yl)pyridin-2-amine

¹H-NMR (400 MHz, CDCl₃): *δ* 8.04 (1H, d, *J=* 5.2 Hz), 7.87 (2H, d, *J=* 8.4 Hz), 7.58 (1H, s), 7.35 (2H, t, *J=* 7.2 Hz), 7.26-7.20 (3H, m), 6.88 (1H, d, *J=* 10.4 Hz), 6.48 (1H, dd, *J* = 5.6 Hz), 3.46-3.41 (3H, m), 3.27-3.18 (2H, m), 3.01 (2H, q, *J =* 7.2 Hz), 2.52 (3H, s), 2.24-1.94 (2H, m), 1.42 (3H, t, *J=* 7.6 Hz). * Two protons from NH were not observed. MS Calcd.: 472.61; MS Found: 473.3 (M+H⁺).

### [Synthetic Example 2] Method for Synthesizing Novel Thiazole Derivative Compounds

### 1. Synthesis of intermediate 1-6 and 1-6'

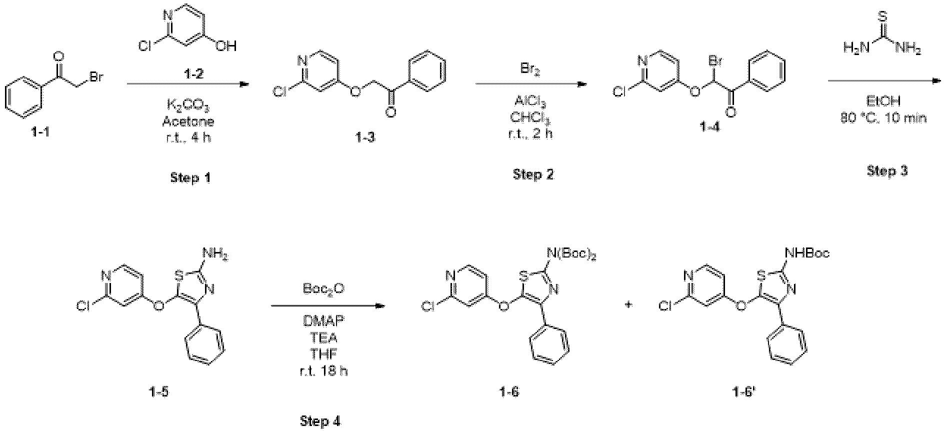

### (1) Preparation of 2-((2-chloropyridin-4-yl)oxy)-1-phenylethanone (1-3)

To a solution of 2-bromo-1-phenylethanone (**1-1**) (9.22 g, 46.3 mmol) in acetone (129 mL) was added 2-chloropyridin-4-ol (**1-2**) (5.00 g, 38.6 mmol) and K₂CO₃ (8.00 g, 57.9 mmol) at room temperature. After stirred at room temperature for 4 hours, the reaction mixture was poured into water and extracted with EtOAc. The organic layer was dried over Na₂SO₄, filtered and concentrated in *vacuo.* The residue was purified by column chromatography on SiO₂ (Hexane:EtOAc = 2:1) to give the **1-3** (6.00 g, 63%) as a yellow solid.

¹H-NMR (400 MHz, CDCl₃): *δ* 8.21 (1H, d, *J* = 5.2 Hz), 7.98 (2H, d, *J* = 7.2 Hz), 7.67 (1H, t, *J* = 7.6 Hz), 7.54 (2H, t, *J* = 7.0 Hz), 6.85 (1H, d, *J* = 2.4 Hz), 6.79 (1H, dd, *J* = 6.0, 2.4 Hz), 5.38 (2H, s).

### (2) Preparation of 2-bromo-2-((2-chloropyridin-4-yl)oxy)-1-phenylethan-1-one (1-4)

To a solution of **1-3** (6.00 g, 24.2 mmol) in CHCl₃ (242 mL) was added slowly AlCl₃ (1.29 g, 9.69 mmol) and Br₂ (1.87 mL, 36.3 mmol) at room temperature. The mixture was stirred at room temperature for 2 hours. The reaction mixture was quenched with sat. NaHCO₃ and extracted with EtOAc. The organic layer was dried over Na₂SO₄, filtered and concentrated in *vacuo.* The residue was purified by column chromatography on SiO₂ (Hexane:EtOAc = 4:1) to give the **1-4** (5.74 g, 73%) as a yellow solid.

¹H-NMR (400 MHz, CDCl₃): *δ* 8.38 (1H, t, *J* = 5.4 Hz), 8.16 (2H, t, *J* = 7.2 Hz), 7.68 (1H, t, *J* = 7.4 Hz), 7.55 (2H, t, *J* = 7.6 Hz), 7.21 (1H, dd, *J* = 14.8, 2.0 Hz), 7.13 (1H, dd, *J*= 5.4, 1.8 Hz), 7.08 (1H, s).

### (3) Preparation of 5-((2-chloropyridin-4-yl)oxy)-4-phenylthiazol-2-amine (1-5)

A mixture of **1-4** (4.70 g, 14.4 mmol) and thiourea (2.19 g, 28.8 mmol) in EtOH (144 mL) was heated at 80 °C for 10 min. After cooled to room temperature, the reaction mixture was concentrated in *vacuo.* The residue was purified by column chromatography on NH-SiO₂ (DCM:MeOH = 100:1) to give the **1-5** (2.72 g, 62%) as a pale yellow solid.

¹H-NMR (400 MHz, DMSO-*d*₆): *δ* 7.55(1H, d, *J=* 8.31 Hz), 7.65 (2H, d, *J=* 8.0 Hz), 7.33 (2H, t, *J=* 4.2 Hz), 7.26 (1H, d, *J=* 2.4 Hz), 7.24-7.21 (1H, m), 7.20 (2H, s), 7.16 (1H, dd, *J* = 5.8, 2.2 Hz).

### (4) Preparation of tert-butyl (5-((2-chloropyridin-4-yl)oxy)-4-phenylthiazol-2-yl)carbamate (1-6'), (1-6)

To a solution of **1-5** (2.72 g, 8.95 mmol) in THF (90 mL) was added Boc₂O (5.86 g, 26.9 mmol), TEA (3.74 mL, 26.9 mmol) and DMAP (438 mg, 3.58 mmol) at room temperature. The mixture was stirred at room temperature for 18 hours. The reaction mixture was poured into water and extracted with EtOAc. The organic layer was dried over Na₂SO₄, filtered and concentrated in *vacuo.* The residue was purified by column chromatography on SiO₂ (Hexane:EtOAc = 7:1 to 4:1) to give the **1-6** ( 1.50 g, 33%) and **1-6'**(1.10 g, 30 %) as a pale yellow solid.

¹H-NMR (400 MHz, DMSO-*d*₆): *δ* 8.34-8.31 (1H, m), 7.71-7.69 (2H, m), 7.40-7.38 (2H, m), 7.34-7.32 (1H, m), 7.32-7.25 (1H, m), 7.22-7.19 (1H, m), 1.51 (18H, s).

### 2. Synthesis of A-1

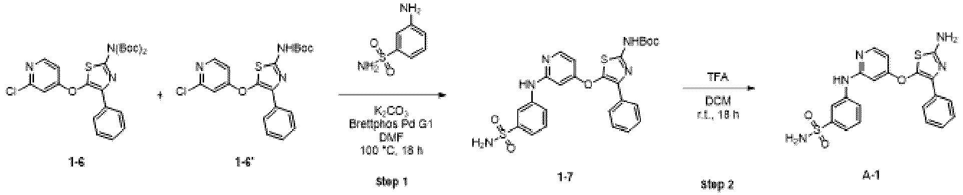

### (1) Preparation of tert-butyl (4-phenyl-5-((2-((3-sulfamoylphenyl)amino)pyridin-4-yl)oxy)thiazol-2-yl)carbamate (1-7)

To a solution of compound **1-6** & **1-6'** mixture (200 mg, 0.50 mmol) in DMF (5.0 mL) was added 3-aminobenzenesulfonamide (128 mg, 0.74 mmol) and K₂CO₃ (205 mg, 1.49 mmol) at room temperature. The mixture was degassed by purging and refilled with argon several times. After addition of BrettPhos Pd G1, methyl t-butyl ether adducts (185 mg, 0.20 mmol), the reaction mixture was heated at 100 °C for 4 hours. After being cooled to room temperature, the reaction mixture was quenched with water and extracted with EtOAc. The organic layers were dried over Na₂SO₄, filtered, and concentrated in *vacuo.* The residue was purified by column chromatography on SiO₂ (Hexane:EtOAc = 3:2) to afford the **1-7** (140 mg, 52%) as a brown solid.

¹H-NMR (400 MHz, CDCl₃): *δ* 9.80 (1H, brs), 8.14 (1H, d, *J=* 6.0 Hz), 7.76-7.72 (3H, m), 7.72-7.42 (1H, m), 7.34-7.21 (5H, m), 6.63 (1H, dd, *J* = 2.0 Hz, 6.0 Hz), 6.51 (1H, d, *J*= 2.0 Hz), 5.18 (2H, brs), 1.47 (9H, s). * A proton from NH was not observed.

### (2) Preparation ofA-1

To a solution of **1-7** (140 mg, 0.26 mmol) in DCM (3 mL) was added TFA (0.2 mL, 2.60 mmol) and stirred at room temperature for 18 hours. The reaction mixture was quenched with saturated aq. NaHCO₃ solution and extracted with EtOAc. The organic layers were dried over Na₂SO₄, filtered, and concentrated in *vacuo.* The residue was purified by column chromatography on SiO₂ (Hexane:EtOAc = 3:2 to 1:1) to afford the **A-1** (52 mg, 45%) as a white solid.

The following **A-2** to **A-38** were synthesized using the procedure described for the synthesis of **A-1.**

### 3. Synthesis of A-39

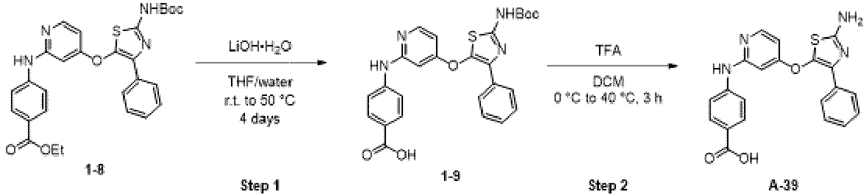

### (1) Preparation of 4-((4-((2-((tert-butoxycarbonyl)amino)-4-phenylthiazol-5-yl)oxy)pyridin-2-yl)amino)benzoic acid (1-9)

To a solution of the 1-8 (270 mg, 0.51 mmol) in THF (5.0 mL) and water (5.0 mL) was added LIOH·H₂O (42.0 mg, 1.01 mmol) at room temperature. After stirred at 50 °C for 4 days, the reaction mixture was cooled to room temperature and concentrated in *vacuo.* The residue was acidified with 2 N aq. HCl solution. A precipitated solid was collected by filtration to afford the **1-9** (185 mg, 72%) as a brown solid.

¹H-NMR (400 MHz, DMSO-*d*₆): *δ* 11.8 (1H, s), 9.66 (1H, s), 8.18 (1H, d, *J=* 6.0 Hz), 7.83 (2H, d, *J=* 7.2 Hz), 7.76 (2H, d, *J=* 8.0 Hz), 7.72 (2H, d, *J=* 8.8 Hz), 7.40 (2H, t, *J=* 7.0 Hz), 7.32-7.28 (1H, m), 6.79 (1H, dd, *J=* 6.0, 2.0 Hz), 6.59 (1H, d, *J* = 2.4 Hz), 1.50 (9H, s). * A proton from NH was not observed.

### (2) Preparation ofA-39

To a solution of the **1-9** (270 mg, 0.51 mmol) in DCM (3.7 mL) was added TFA (0.57 mL, 7.33 mmol) at 0 °C. After stirred at 40 °C for 3 hours, the reaction mixture was cooled to room temperature, quenched with sat. NaHCO₃ solution and extracted with EtOAc. The organic layer was dried over Na₂SO₄, filtered and concentrated in *vacuo.* The residue was triturated with DCM. A precipitated solid was collected by filtration to afford the **A-39** (37.4 mg, 25%) as a beige solid.

The following **A-40** was synthesized using the procedure described for the synthesis of **A-39.**

### 4. Synthesis of A-41

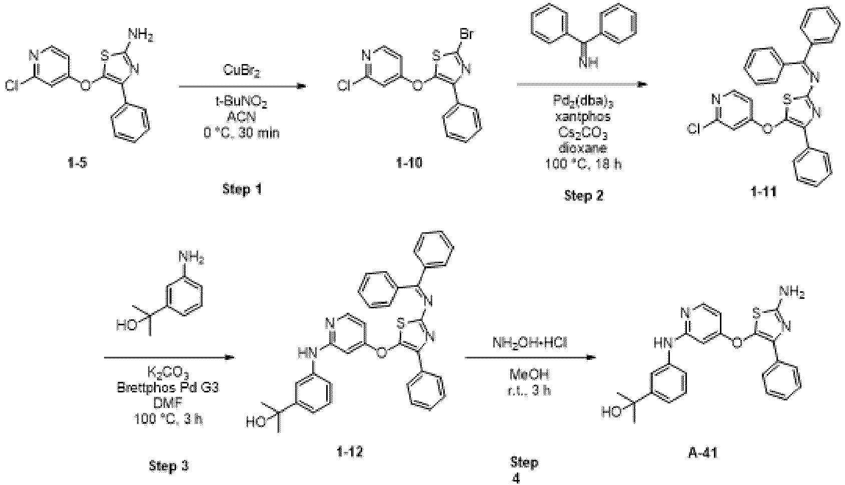

### (1) Preparation of 2-bromo-5-((2-chloropyridin-4-yl)oxy)-4-phenylthiazole (1-10)

To a solution of **1-5** (460 mg, 1.51 mmol) was added copper (II) bromide (338 mg, 1.51 mmol) and *tert*-butyl nitrite (0.27 mL, 2.27 mmol) at 0 °C. The mixture was stirred at 0 °C for 30 min. The reaction mixture was quenched with ammonia water and extracted with EtOAc. The organic layer was dried over Na₂SO₄, filtered and concentrated in *vacuo.* The residue was purified by column chromatography on SiO₂ (Hexane:EtOAc = 3:1) to give the **1-10** (358 mg, 64%) as a yellow solid.

### (2) Preparation of N-(5-((2-chloropyridin-4-yl)oxy)-4-phenylthiazol-2-yl)-1,1-diphenylmethanimine (1-11)

To a solution of **1-10** (100 mg, 0.27 mmol) in dioxane (2.7 mL) were added diphenylmethanimine (0.07 mL, 0.41 mmol), Xantphos (16.0 mg, 0.03 mmol), and Cs₂CO₃ (266 mg, 0.82 mmol) at room temperature. The mixture was degassed by purging and re-filled with Ar in several times. After addition of Pd₂(dba)₃ (16.0 mg, 0.03 mmol), the reaction mixture was heated at 100 °C for 18 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane: EtOAc = 5:1) to give the *N*-(5-((2-chloropyridin-4-yl)oxy)-4-phenylthiazol-2-yl)-1,1-diphenylmethanimine (**1-11**) (62.0 mg, 49%) as a yellow solid.

¹H-NMR (400 MHz, DMSO-*d*₆): *δ* 8.33 (1H, d, *J* = 6.0 Hz), 7.79 (2H, d, *J* = 7.6 Hz), 7.75-7.70 (4H, m), 7.67-7.63 (4H, m), 7.42-7.38 (4H, m), 7.33-7.30 (1H, m), 7.16 (1H, d, *J=* 2.4 Hz), 7.07 (1H, dd, *J* = 6.0, 2.4 Hz).

### (3) Preparation of 2-(3-((4-((2-((diphenylmethylene)amino)-4-phenylthiazol-5-yl)oxy)pyridin-2-yl)amino)phenyl)propan-2-ol (1-12)

To a solution of **1-11** (62.0 mg, 0.13 mmol) in DMF (2.7 mL) were added 2-(3-aminophenyl)propan-2-ol (30.0 mg, 0.20 mmol) and K₂CO₃ (55.0 mg, 0.40 mmol) at room temperature. The mixture was degassed by purging and re-filled with Ar in several times. After addition of BrettPhos Pd G3 (24.0 mg, 0.03 mmol), the reaction mixture was heated at 100 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc =1:1) to give the **1-12** (15.4 mg, 20%) as a yellow solid.

¹H-NMR (400 MHz, DMSO-*d*₆): *δ* 9.04 (1H, s), 8.06 (1H, d, *J=* 6.0 Hz), 7.79-7.74 (4H, m), 7.64-7.59 (1H, m), 7.59-7.55 (6H, m), 7.53 (1H, s), 7.53-7.42 (4H, m), 7.33-7.29 (1H, m), 7.16 (1H, t, *J=* 7.8 Hz), 6.98 (1H, d, *J=* 8.4 Hz), 6.45 (1H, dd, *J=* 6.2, 2.2 Hz), 6.37 (1H, d, *J* = 2.0 Hz), 4.93 (1H, s), 1.38 (6H, s).

### (4) Preparation ofA-41

To a solution of **1-12** (15.0 mg, 0.03 mmol) in MeOH (0.50 mL) were added NH₂OH.HCl (3.58 mg, 0.05 mmol) at room temperature. After stirred at room temperature for 3 hours, the reaction mixture was concentrated in *vacuo.* The residue was purified by column chromatography on NH-SiO₂ (DCM:MeOH = 100:1 to 50:1) to give the **A-41** (2.20 mg, 20%) as a white solid.

The following **A-42** and **A-43** were synthesized using the procedure described for the synthesis of **A-41.**

### 5. Synthesis of A-44

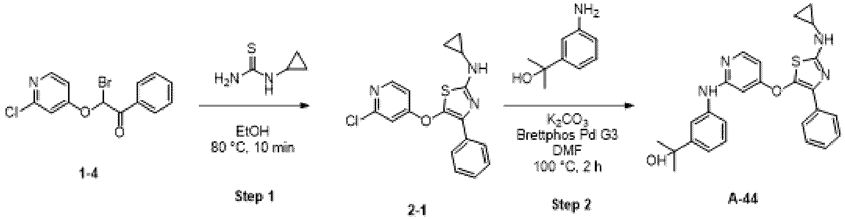

### (1) Preparation of 5-((2-chloropyridin-4-yl)oxy)-N-cyclopropyl-4-phenylthiazol-2-amine (2-1)

A mixture of 2-bromo-2-((2-chloropyridin-4-yl)oxy)-1-phenylethan-1-one (**1-4**) (200 mg, 0.61 mmol) and 1-cyclopropylthiourea (142 mg, 1.23 mmol) in EtOH (6.1mL) was heated at 80 °C for 10 min. After cooled to room temperature, the reaction mixture was concentrated in *vacuo.* The residue was purified by column chromatography on SiO₂ (Hexane:EtOAc = 2:1) to give the **2-1** (74.0 mg, 35%) as a yellow solid.

¹H-NMR (400 MHz, DMSO-*d*₆): *δ* 8.34 (1H, d, *J=* 5.2 Hz), 8.24 (1H, d, *J=* 1.6 Hz), 7.69 (2H, d, *J=* 7.6 Hz), 7.36 (2H, t, *J=* 7.6 Hz), 7.31 (1H, d, *J=* 2.0 Hz), 7.27 (1H, d, *J=* 7.6 Hz), 7.20 (1H, dd, *J=* 5.8, 2.2 Hz), 2.60-2.54 (1H, m), 0.76-0.71 (2H, m), 0.59-0.57 (2H, m).

### (2) Preparation ofA-44

To a solution of **2-1** (70.0 mg, 0.20 mmol) in DMF (4.1 mL) was added 2-(3-aminophenyl)propan-2-ol (46.0 mg, 0.31 mmol) and K₂CO₃ (84.0 mg, 0.61 mmol) at room temperature. The mixture was degassed by purging and re-filled with Ar in several times. After addition of BrettPhos Pd G3 (37.0 mg, 0.04 mmol), the reaction mixture was heated at 100 °C for 2 hours. After cooled to room temperature, the reaction mixture was quenched with water and extracted with EtOAc. The organic layer was dried over Na₂SO₄, filtered and concentrated in *vacuo.* The residue was purified by column chromatography on NH-SiO₂ (only DCM) to give the **A-44** (19.6 mg, 21%) as an ivory solid.

The following **A-45** to **A-47,** were synthesized using the procedure described for the synthesis of **A-44.**

### 6. Synthesis of A-48

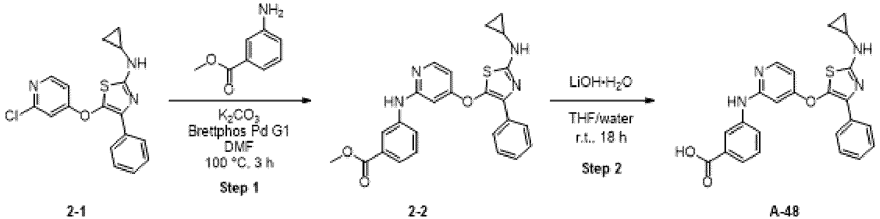

### (1) Preparation of methyl methyl 3-((4-((2-(cyclopropylamino)-4-phenylthiazol-5-yl)oxy)pyridin-2-yl)amino)benzoate (2-2)

To a solution of **2-1** (200 mg, 0.58 mmol) in DMF (12 mL) was added methyl 3-aminobenzoate (132 mg, 0.87 mmol) and K₂CO₃ (241 mg, 1.75 mmol) at room temperature. The mixture was degassed by purging and re-filled with Ar in several times. After addition of BrettPhos Pd G1 (207 mg, 0.23 mmol), the reaction mixture was heated at 100 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 4:1) to give the **2-2** (103 mg, 39%) as a yellow solid.

¹H-NMR (400 MHz, CDCl₃): *δ* 8.12 (1H, d, *J=* 5.6 Hz), 7.94 (1H, t, *J=* 1.8 Hz), 7.75 (2H, d, *J=* 8.0 Hz), 7.69 (1H, d, *J=* 7.2 Hz), 7.55 (1H, dd, *J=* 8.0, 2.0 Hz), 7.37-7.32 (3H, m), 7.26-7.24 (1H, m), 6.71 (1H, s), 6.59 (1H, dd, *J =* 5.6, 2.0 Hz), 6.52 (1H, d, *J=* 2.4 Hz), 3.90 (3H, s), 2.62-2.57 (1H, m), 0.79-0.77 (2H, m), 0.72-0.68 (2H, m). * A proton from NH was not observed.

### (2) Preparation ofA-48

To a solution of the **2-2** (60.0 mg, 0.13 mmol) in THF (2.0 mL) and water (0.65 mL) was added LiOH·H₂O (55.0 mg, 1.31 mmol) at room temperature. After stirred at room temperature for 18 hours, the reaction mixture was concentrated in *vacuo.* The residue was acidified with 2 N aq. HCl solution. A precipitated solid was collected by filtration to afford the **A-48** (1.0 mg, 1.7%) as an ivory solid.

The following **A-49** was synthesized using the procedure described for the synthesis of **A-48.**

### 7. Synthesis of A-50

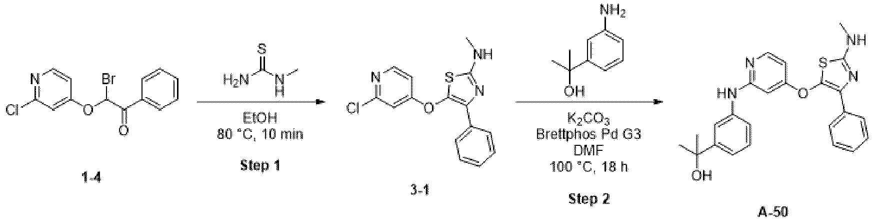

### (1) Preparation of 5-((2-chloropyridin-4-yl)oxy)-N-methyl-4-phenylthiazol-2-amine (3-1)

A mixture of **1-4** (300 mg, 0.92 mmol), and 1-methylthiourea (120 mg, 1.38 mmol) in EtOH (10 mL) was stirred at 80 °C for 10 min. After being cooled at room temperature, the solvent was removed in *vacuo.* The residue was dissolved with EtOAc and washed with water. The organic layer was dried over Na₂SO₄, filtered, and concentrated in *vacuo.* The residue was purified by column chromatography on SiO₂ (Hexane:EtOAc = 4:1) to afford the **3-1** (58.0 mg, 20 %) as a yellow oil.

¹H-NMR (400 MHz, CDCl₃): *δ* 8.24 (1H, d, *J* = 5.6 Hz), 7.74-7.71 (2H, m), 7.33-7.29 (2H, m), 7.26-7.24 (1H, m), 7.04 (1H, d, *J* = 2.4 Hz), 6.96 (1H, dd, *J* = 5.9 Hz, 2.0 Hz), 2.97 (3H, d, *J=* 4.8 Hz). * A proton from NH was not observed.

### (2) Preparation ofA-50

To a solution of **3-1** (57.0 mg, 0.18 mmol) in DMF (2.0 mL) was added 2-(3-aminophenyl)propan-2-ol (41.0 mg, 0.27 mmol) and K₂CO₃ (74.0 mg, 0.54 mmol) at room temperature. The mixture was degassed by purging and re-filled with argon several times. After addition of BrettPhos Pd G3 (30.0 mg, 0.04 mmol), the reaction mixture was heated at 100 °C for 18 hours. After being cooled to room temperature, the reaction mixture was quenched with water and extracted with EtOAc. The organic layers were dried over Na₂SO₄, filtered, and concentrated in *vacuo.* The residue was purified by column chromatography on SiO₂ (Hexane:EtOAc = 1:1) to afford the **A-50** (3.4 mg, 4%) as an ivory solid.

The following **A-51** to **A-55,** were synthesized using the procedure described for the synthesis of **A-50.**

### 8. Synthesis of A-56 and A-56 salt

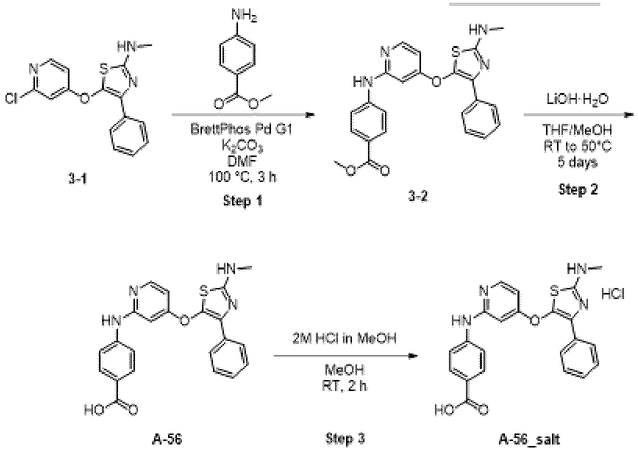

### (1) Preparation of methyl 4-((4-((2-(methylamino)-4-phenylthiazol-5-yl)oxy)pyridin-2-yl)amino)benzoate (3-2)

To a solution of **3-1** (800 mg, 2.52 mmol) in DMF (20.0 mL) was added methyl 4-aminobenzoate (570 mg, 3.78 mmol) and K₂CO₃ (1.04 g, 7.55 mmol) at room temperature. The mixture was degassed by purging and re-filled with argon several times. After addition of BrettPhos Pd G1 (580 mg, 0.63 mmol), the reaction mixture was heated at 100 °C for 3 hours. After being cooled to room temperature, the reaction mixture was quenched with water and extracted with EtOAc. The organic layers were dried over Na₂SO₄, filtered, and concentrated in *vacuo.* The residue was purified by column chromatography on MPLC (Hexane:EtOAc = 7:3) to afford the **3-2** (630 mg, 58%) as a brown oil.

¹H-NMR (400 MHz, CDCl₃): *δ* 8.15 (1H, d, *J* = 5.6 Hz), 7.96-7.93 (2H, m), 7.79-7.77 (2H, m), 7.38-7.32 (2H, m), 7.28-7.22 (2H, m), 6.94 (1H, s), 6.64 (1H, dd, *J=* 2.0 Hz, 5.6 Hz), 6.58 (1H, d, *J* = 2.4 Hz), 3.88 (3H, s), 2.99 (3H, d, *J* = 6.4 Hz). *Two protons from two NH were not observed.

### (2) Preparation of A-56

A mixture of **3-2** (630 mg, 1.46 mmol) in THF/H₂O (*v*/*v* = 1/1, 30.0 mL) was added LiOH·H₂O (1.22 g, 29.1 mmol) and stirred at room temperature to 50 °C for 5 days. After being cooled at room temperature, the reaction mixture was neutralized with aq. 1N HCl solution and extracted with EtOAc. The organic layer was dried over Na₂SO₄, filtered and concentrated in *vacuo.* The residue was purified by column chromatography on SiO₂ (Hexane:EtOAc = 2:3) and triturated with DCM to afford **A-56** (180 mg, 30%) as an ivory solid.

### (3) Preparation of A-56_Salt

To a solution **of A-56** (30.0 mg, 0.07 mmol) in MeOH (1.5 mL) was added HCl (2 M in MeOH, 0.08 mL, 0.15 mmol). The reaction mixture was stirred at room temperature for 2 hours and the solvent was removed in *vacuo.* The precipitated solid was filtered and washed with EtO₂ to afford **A-56**_Salt (17.0 mg, 52%) as a yellow solid.

¹H-NMR (400 MHz, DMSO-d₆): *δ* 9.70 (1H, s), 8.16 (1H, d, *J* =6.4 Hz), 7.83 (2H ,d, *J* =8.8 Hz), 7.75-7.70 (5H, m), 7.36 (2H, t, *J* = 7.6 Hz), 7.26 (1H, t, *J* = 7.6 Hz), 6.76 (1H, dd, *J* = 2.4 Hz, 6.2 Hz), 6.62 (1H, d, *J* = 2.4 Hz), 2.83 (3H, s). * A proton from NH was not observed. MS Calcd.: 418.47; MS Found: 419.1 (M+H⁺).

The following **A-57** was synthesized using the procedure described for the synthesis of A-56

### 9. Synthesis of A-58

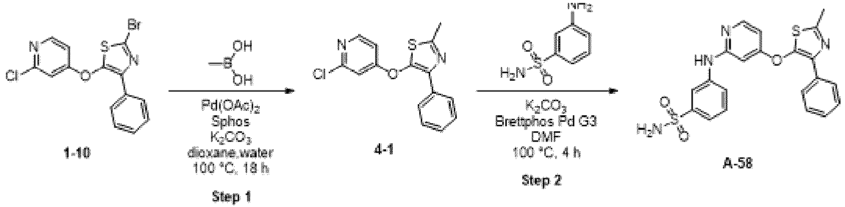

### (1) Preparation of 5-((2-chloropyridin-4-yl)oxy)-2-methyl-4-phenylthiazole (4-1)

To a solution of **1-10** (1.50 g, 4.08 mmol) in dioxane (37 mL) and water (3.7 mL) were added methylboronic acid (733 mg, 12.2 mmol), Sphos (837 mg, 2.04 mmol), and K₂CO₃ (1.69 mg, 12.2 mmol) at room temperature. The mixture was degassed by purging and re-filled with Ar in several times. After addition of Pd(OAc)₂ (229 mg, 1.02 mmol), the reaction mixture was heated at 100 °C for 18 hours. After cooled to room temperature, the reaction mixture was quenched with water and extracted with EtOAc. The organic layer was dried over Na₂SO₄, filtered, and concentrated in *vacuo.* The residue was purified by column chromatography on SiO2 (Hexane:EtOAc = 10:1 to 7:1) to give the **4-1** (403 mg, 33%) as a yellow solid.

¹H-NMR (400 MHz, DMSO-*d*₆): *δ* 8.35 (1H, d, *J* = 5.6 Hz), 7.79 (2H, d, *J* = 8.0 Hz), 7.41 (2H, t, *J =* 7.8 Hz), 7.35 (1H, d, *J* = 2.0 Hz), 7.32 (1H, t, *J* = 7.6 Hz), 7.21 (1H, dd, *J* = 5.4, 2.2 Hz), 2.69 (3H, s).

### (2) Preparation ofA-58

To a solution of **4-1** (18.0 mg, 0.06 mmol) in DMF (1.2 mL) were added 3-aminobenzenesulfonamide (15.0 mg, 0.09 mmol) and K₂CO₃ (25.0 mg, 0.18 mmol) at room temperature. The mixture was degassed by purging and re-filled with Ar in several times. After addition of BrettPhos Pd G3 (10.8 mg, 0.01 mmol), the reaction mixture was heated at 100 °C for 4 hours. After cooled to room temperature, the reaction mixture was quenched with water and extracted with EtOAc. The organic layer was dried over Na₂SO₄, filtered, and concentrated in *vacuo.* The residue was purified by column chromatography on NH-SiO₂ (Hexane:EtOAc = 1:1) to give the **A-58** (6.6 mg, 25%) as an ivory solid.

The following **A-59** to **A-108,** were synthesized using the procedure described for the step 2 from synthesis of **A-58.**

### 10. Synthesis of A-109

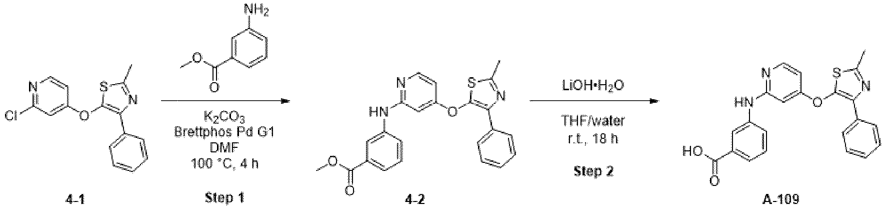

### (1) Preparation of methyl 3-((4-((2-methyl-4-phenylthiazol-5-yl)oxy)pyridin-2-yl)amino)benzoate (4-2)

To a solution of **4-1** (50.0 mg, 0.17 mmol) in DMF (3.3 mL) were added methyl 3-aminobenzoate (37.0 mg, 0.25 mmol), and K₂CO₃ (68.0 mg, 0.50 mmol) at room temperature. The mixture was degassed by purging and re-filled with Ar in several times. After addition of BrettPhos Pd G1 (44.0 mg, 0.05 mmol), the reaction mixture was heated at 100 °C for 4 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 3:1) to give the **4-2** (61.0 mg, 88%) as a yellow oil.

¹H-NMR (400 MHz, DMSO-d₆): *δ* 9.37 (1H, s), 8.26 (1H, s), 8.16 (1H, d, *J=* 6.0 Hz), 7.92 (1H, d, *J=* 8.0 Hz), 7.83 (2H, d, *J=* 7.6 Hz), 7.48-7.30 (5H, m), 6.69 (1H, d, *J=* 4.4 Hz), 6.45 (1H, d, *J=* 1.6 Hz), 3.84 (3H, s), 2.71 (3H, s).

### (2) Preparation ofA-109

To a solution of the 4-2 (61.0 mg, 0.15 mmol) in THF/water (v/v = 1/1, 3 mL) were added LiOH·H₂O (61.0 mg, 1.46 mmol) at room temperature. After stirred at room temperature for 18 hours, the reaction mixture was concentrated in *vacuo.* The residue was acidified with 2 N aq. HCl solution. A precipitated solid was collected by filtration to afford the **A-109** (21 mg, 36%) as an ivory solid.

The following **A-110** was synthesized using the procedure described for the synthesis of **A-109.**

### 11. Synthesis of A-111 and A-112

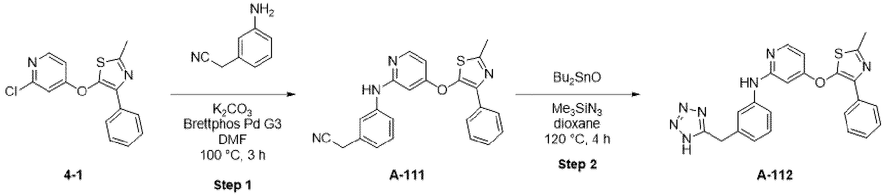

### (1) Preparation of A-111

To a solution of **4-1** (300 mg, 0.99 mmol) in DMF (9.9 mL) was added 2-(3-aminophenyl)acetonitrile (196 mg, 1.49 mmol), and K₂CO₃ (411 mg, 2.97 mmol) at room temperature. The mixture was degassed by purging and re-filled with Ar in several times. After addition of Brettphos Pd G3 (269 mg, 0.30 mmol), the reaction mixture was heated at 100 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 1:4) to give the **A-111** (343 mg, 87%) as a pale yellow solid.

### (2) Preparation ofA-112

To a solution of **A-111** (150 mg, 0.376 mmol) in dioxane (4.7 mL) was added Bu₂SnO (187 mg, 0.75 mmol), and Me₃SiN₃ (0.25 mL, 1.88 mmol) at room temperature. The mixture was heated at 120 °C for 4 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (only EtOAc) to give the **A-112** (23.5 mg, 14%) as an ivory solid.

The following **A-113** was synthesized using the procedure described for the synthesis of **A-111.**

The following **A-114** was synthesized using the procedure described for the synthesis of **A-112.**

### 12. Synthesis of A-115

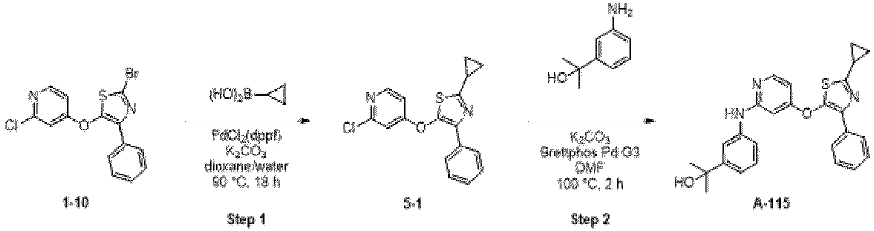

### (1) Preparation of 5-((2-chloropyridin-4-yl)oxy)-2-cyclopropyl-4-phenylthiazole (5-1)

To a solution of **1-10** (358 mg, 0.97 mmol) in dioxane (7.3 mL) and water (2.4 mL) was added cyclopropylboronic acid (335 mg, 3.90 mmol) and K₂CO₃ (404 mg, 2.92 mmol) at room temperature. The mixture was degassed by purging and re-filled with Ar in several time and then added PdCl₂(dppf) (143 mg, 0.20 mmol) at room temperature. The reaction mixture was heated at 90 °C for 18 hours. After cooled to room temperature, the reaction mixture was diluted with water and extracted with EtOAc. The organic layer was dried over Na₂SO₄, filtered and concenatrated in *vacuo.* The residue was purified by column chromatography on SiO₂ (Hexane:EtOAc = 1:1) to give the **5-1** (92.0 mg, 29%) as a white solid.

¹H-NMR (400 MHz, DMSO-*d*₆): *δ* 8.32 (1H, dd, *J* = 6.0 Hz), 7.73 (2H, d, *J* = 7.2 Hz), 7.39-7.33 (3H, m), 7.31-7.27 (1H, m), 7.17 (1H, dd, *J* = 5.8, 2.2 Hz), 2.42-2.37 (1H, m), 1.15-1.11 (2H, m), 1.09-1.03 (2H, m).

### (2) Preparation ofA-115

To a solution of **5-1** (20 mg, 0.06 mmol) in DMF (1.2 mL) was added 2-(3-aminophenyl)propan-2-ol (14.0 mg, 0.09 mmol) and K₂CO₃ (25.0 mg, 0.18 mmol) at room temperature. The mixture was degassed by purging and re-filled with Ar in several times. After addition of BrettPhos Pd G3 (11.0 mg, 0.01 mmol), the reaction mixture was heated at 100 °C for 2 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 2:1) to give the **A-115** (10.7 mg, 40%) as a yellow solid.

The following **A-116** to **A-122** were synthesized using the procedure described for the synthesis of **A-106.**

### 13. Synthesis of A-132 and A-133

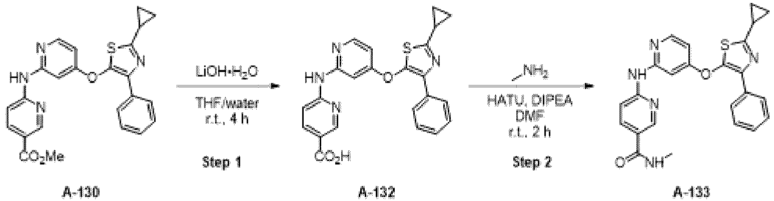

### (1) Preparation of A-132

A solution of **A-130** (50.0 mg, 0.11 mmol) in THF (0.94 mL) and water (0.19 mL) was added LiOH·H₂O (27.0 mg, 1.12 mmol) at 0 °C. The reaction mixture was stirred at room temperature for 4 hours. After cooling to 0 °C, the reaction mixture was acidified with aq. 1 N HCl and concentrated in *vacuo* to give the **A-132** (40.0 mg, 83%) as a white solid, which was used for the next step without further purification.

### (2) Preparation ofA-133

A mixture of **A-132** (40.0 mg, 0.09 mmol), HATU (53.0 mg, 0.14 mmol), DIPEA (0.049 mL, 0.28 mmol) and methanamine (2.0 M in THF, 0.07 mL, 0.14 mmol) in DMF (0.93 mL) was stirred at room temperature for 2 hours. The reaction mixture was dissolved with EtOAc, washed with water and brine. The separated organic layer was dried with Na₂SO₄, filtered off and concentrated in *vacuo.* The residue was purified by column chromatography on SiO₂ (DCM:MeOH = 10:1) to give the A-133 (35.0 mg, 85%) as a white solid.

The following **A-134** to **A-136,** were synthesized using the procedure described for the step 1 from synthesis of **A-132.**

### 14. Synthesis of A-138

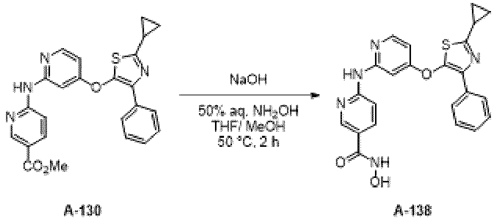

To a solution of **A-130** (50.0 mg, 0.11 mmol) in THF (1.0 mL) and MeOH (1.0 mL) was added NaOH (22.0 mg, 0.56 mmol) and aq. NH₂OH (50 wt%, 0.17 mL, 0.11 mmol) at room temperature. The mixture was heated at 50 °C for 2 hours. After cooled to room temperature, the reaction mixture was concentrated in *vacuo.* The residue was acidified with 2 N aq. HCl solution at 0 °C. The precipitated solid was collected by filtration to afford the **A-138** (8.9 mg, 18%) as a white solid.

The following **A-139** was synthesized using the procedure described for the synthesis of A-138.

### 15. Synthesis of A-140

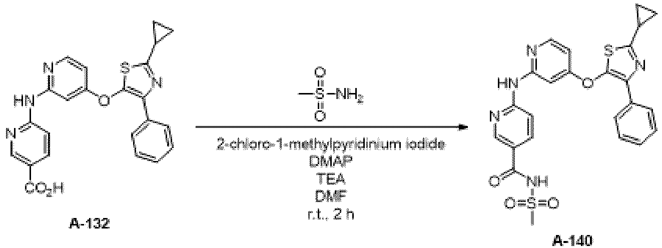

To a solution of **A-132** (35.0 mg, 0.08 mmol) in DMF (1.6 mL) was added methanesulfonamide (9.28 mg, 0.10 mmol), DMAP (10.9 mg, 0.09 mmol), 2-chloro-1-methylpyridinium iodide (31.0 mg, 0.12 mmol) and TEA (34.0 µL, 0.24 mmol) at room temperature. The mixture was stirred for 2 hours at room temperature. The reaction mixture was quenched with water and extracted with EtOAc. The organic layer was dried over Na₂SO₄, filtered, and concentrated in *vacuo.* The residue was triturated with DCM and Hexane and resulted solid was collected by filtration and dried under vacuum to afford the **A-140** (11.0 mg, 27%) as a pale red solid.

### 16, Synthesis of A-141

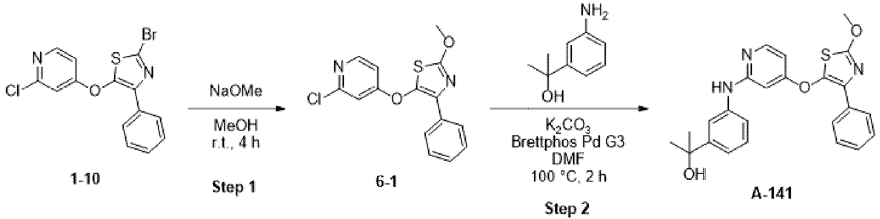

### (1) Preparation of 5-((2-chloropyridin-4-yl)oxy)-2-methoxy-4-phenylthiazole (6-1)

To a solution of 1-10 (60.0 mg, 0.16 mmol) in MeOH (1.6 mL) was added sodium methoxide (25 wt% in MeOH, 0.15mL, 0.65 mmol) at room temperature. After stirred for 4 hours, the reaction mixture was diluted with water and extracted with EtOAc. The organic layer was dried over Na₂SO₄, filtered and concentrated in *vacuo.* The residue was purified by column chromatography on SiO₂ (Hexane:EtAOc = 3:1) to give 5-((2-chloropyridin-4-yl)oxy)-2-methoxy-4-phenylthiazole (**6-1**) (13.0 mg, 25%) as a yellow solid.

MS Calcd.: 318.78; MS Found: 319.02 (M+H⁺).

### (2) Preparation ofA-141

To a solution of **6-1** (13 mg, 0.0410 mmol) in DMF (1.0 mL) was added 2-(3-aminophenyl)propan-2-ol (9.3 mg, 0.0610 mmol) and K₂CO₃ (17.0 mg, 0.122 mmol) at room temperature. The mixture was degassed by purging and re-filled with Ar in several times. After addition of Brettphos Pd G3 (7.4 mg, 8.16 µmol), the reaction mixture was heated at 100 °C for 2 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 3:1) to give the **A-141** (1.1 mg, 6%) as a yellow solid.

The following from **A-142** to **A-144,** were synthesized using the procedure described for the step 2 from synthesis of **A-141.**

### 17. Synthesis of A-145

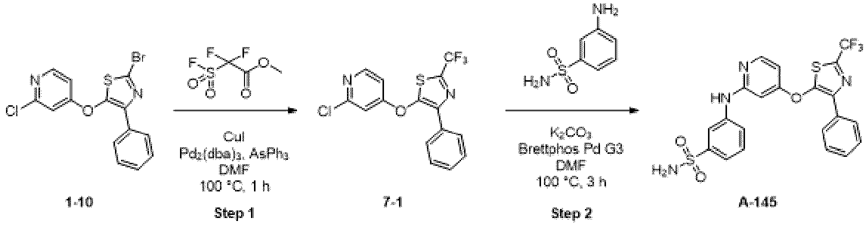

### (1) Preparation of 5-((2-chloropyridin-4-yl)oxy)-4-phenyl-2-(trifluoromethyl)thiazole (7-1)

To a solution of **1-10** (200 mg, 0.54 mmol) in DMF (11 mL) were added methyl 2,2-difluoro-2-(fluorosulfonyl)acetate (0.35 mL, 2.72 mmol), CuI (518 mg, 2.72 mmol), and AsPh₃ (67.0 mg, 0.22 mmol) at room temperature. The mixture was degassed by purging and re-filled with Ar in several times. After addition of Pd₂(dba)₃ (25.0 mg, 0.03 mmol), the reaction mixture was heated at 100 °C for 1 hour. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 20:1) to give **7-1** (95.0 mg, 49%) as a yellow solid.

### (2) Preparation ofA-145

To a solution of **7-1** (100 mg, 0.28 mmol) in DMF (2.8 mL) were added 3-aminobenzenesulfonamide (72.0 mg, 0.14 mmol) and K₂CO₃ (116 mg, 0.84 mmol) at room temperature. The mixture was degassed by purging and re-filled with Ar in several times. After addition of BrettPhos Pd G3 (47.0 mg, 0.06 mmol), the reaction mixture was heated at 100 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 1: 1) to give **A-145** (10.0 mg, 7%) as a white solid.

The following **A-146** to **A-148,** were synthesized using the procedure described for the step 2 from synthesis of A- **145.**

### 18. Synthesis of A-149 and A-150

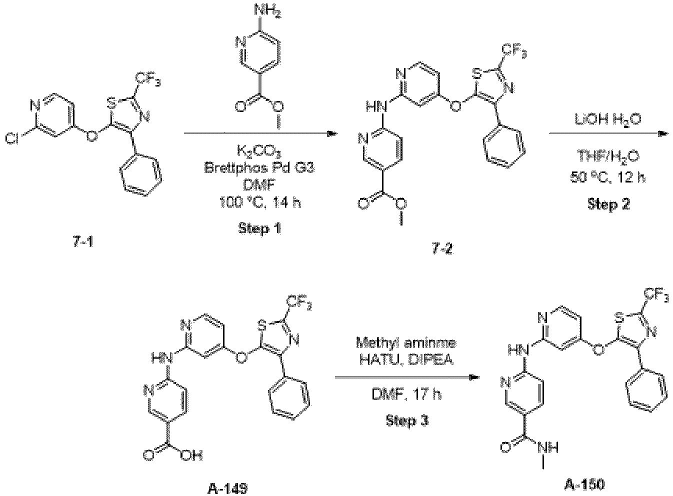

### (1) Preparation of methyl 6-((4-((4-phenyl-2-(trifluoromethyl)thiazol-5-yl)oxy)pyridin-2-yl)amino)nicotinate (7-2)

To a solution of **7-1** (184.3 mg, 0.52 mmol) in DMF (2.0 mL) was added methyl 6-aminonicotinate (86.0 mg, 0.57 mmol) and K₂CO₃ (214.0 mg, 1.55 mmol) at room temperature. The mixture was degassed by purging and re-filled with Ar in several times. After addition of BrettPhos Pd G3 (187.0 mg, 0.21 mmol), the reaction mixture was heated at 100 °C for 14 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc=1:1) to give **7-2** (127.6 mg, 52%) as a yellow crude solid.

MS Calcd: 472.44; MS Found: 473.14 (M+H⁺).

### (2) Preparation ofA-149

To a solution of **7-2** (127.6 mg, 0.27 mmol) in THF (2.0 mL) and water (2.0 mL) was added LiOH·H₂O (34.0 mg, 0.81 mmol) at room temperature. The mixture was heated at 50 °C for 12 hours. After cooled to room temperature, the reaction mixture was concentrated in *vacuo.* The residue was acidified with 1 N aq. HCl solution. A precipitated solid was collected by filtration to afford the **A-149** (66.6 mg, 54%) as a white solid

### (3) Preparation ofA-150

To a solution of **A-149** (30.0 mg, 0.07 mmol) in DMF (4.0 mL) was added HATU (37.3 mg, 0.11 mmol), DIPEA (0.02 mL, 0.11 mmol) and 2 M methyl amine in THF solution (0.05 mL, 0.11 mmol) at room temperature. The mixture was stirred for 17 hours at room temperature. The organic layer was dried over Na₂SO₄, filtered and concentrated in *vacuo.* The residue was purified by column chromatography on SiO₂ (DCM:MeOH = 10:1) to give the A-150 (3.1 mg, 10%) as a white soild.

The following **A-151** and **A-152** were synthesized using the procedure described for the synthesis of **A-149.**

### 19. Synthesis of A-153

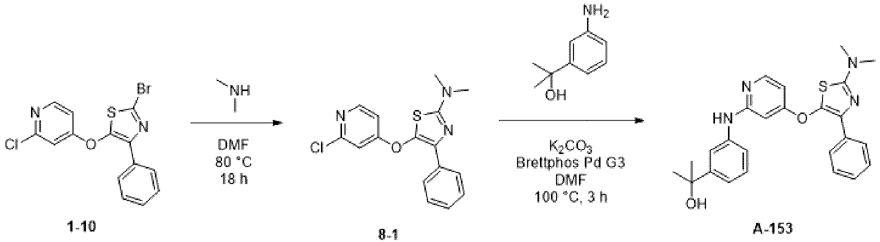

### (1) Preparation of 5-((2-chloropyridin-4-yl)oxy)-N,N-dimethyl-4-phenylthiazol-2-amine (8-1)

To a solution of compound **1-10** (100 mg, 0.27 mmol) in DMF (5.50 mL) was added dimethyl amine (2 M in THF, 0.20 mL, 0.41 mmol) and stirred at 80 °C for 18 hrs. The reaction mixture was washed with water and extracted with EtOAc. The combined organic layers were dried over Na₂SO₄, filtered, and concentrated in *vacuo.* The residue was purified by column chromatography on SiO2 (Hexane:EtOAc: = 3:2) to afford the **8-1** (32 mg, 36%) as a yellow oil.

¹H-NMR (400 MHz, CDCl₃): *δ* 8.23 (1H, d, *J=* 6.0 Hz), 7.81-7.78 (2H, m), 7.40-7.32 (3H, m), 7.03 (1H, m), 7.00-6.99 (1H, m), 3.12 (6H, s).

### (2) Preparation ofA-153

To a solution of compound **8-1** (30.0 mg, 0.09 mmol) in DMF (1.0 mL) was added 2-(3-aminophenyl)propan-2-ol (21.0 mg, 0.14 mmol) and K₂CO₃ (37.0 mg, 0.27 mmol) at room temperature. The mixture was degassed by purging and re-filled with argon several times. After addition of BrettPhos Pd G3 (15.0 mg, 0.02 mmol), the reaction mixture was heated at 100 °C for 3 hours. After being cooled to room temperature, the reaction mixture was quenched with water and extracted with EtOAc. The organic layers were dried over Na₂SO₄, filtered, and concentrated in *vacuo.* The residue was purified by column chromatography on SiO₂ (Hexane:EtOAc = 1:1) to afford the **A-153** (1.3 mg, 3%) as a white solid.

The following **A-154** to **A-164** were synthesized using the procedure described for the synthesis of **A-153**

### 20. Synthesis of A-165 and A-166

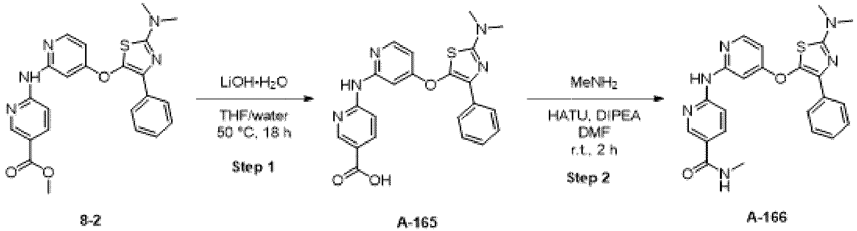

### (1) Preparation of A-165

To a solution of **8-2** (100 mg, 0.22 mmol) in THF (2.2 mL) and water (2.2 mL) was added LiOH·H₂O (94.0 mg, 2.24 mmol) at room temperature. The mixture was heated at 50 °C for 18 hours. After cooled to room temperature, the reaction mixture was concentrated in *vacuo.* The residue was acidified with 2 N aq. HCl solution. A precipitated solid was collected by filtration to afford the **A-165** (19.5 mg, 20%) as a yellow solid.

### (2) Preparation ofA-166

To a solution of **A-165** (60.0 mg, 0.14 mmol) in DMF (1.4 mL) was added HATU (79.0 mg, 0.21 mmol), MeNH₂ (2M in THF, 10.4 µL, 0.201 mmol) and DIPEA (73.0 µL, 0.42 mmol) at room temperature. The mixture was stirred for 2 hours at room temperature. The reaction mixture was quenched with water and extracted with EtOAc. The organic layer was dried over Na₂SO₄, filtered, and concentrated in *vacuo.* The residue was triturated with DCM and resulted solid was filtered to give the **A-166** (26.0 mg, 42%) as a beige solid.

The following **A-167** to **A-169** were synthesized using the procedure described for the synthesis of **A-165.**

### 21. Synthesis of A-170 and A-171

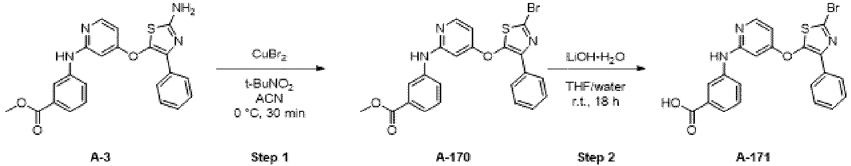

### (1) Preparation of A-170

To a solution of **A-3** (104 mg, 0.25 mmol) in ACN (2.5 mL) was added copper (II) bromide (56.0 mg, 0.25 mmol) and *tert-*butyl nitrite (38.0 mg, 0.37 mmol) at 0 °C. The mixture was stirred at 0 °C for 30 min. The reaction mixture was quenched with ammonia water and extracted with EtOAc. The organic layer was dried over Na₂SO₄, filtered and concentrated in *vacuo.* The residue was purified by column chromatography on SiO₂ (Hexane:EtOAc = 3:1) to give the **A-170** (47.8 mg, 40%) as a pale yellow solid.

### (2) Preparation ofA-171

To a solution of **A-170** (7.50 mg, 0.02 mmol) in THF (0.23 mL) and water (0.08 mL) was added lithium hydroxide hydrate (6.52 mg, 0.16 mmol) at room temperature. After stirred at room temperature for 18 hours, the reaction mixture was concentrated in *vacuo.* The residue was acidified with 2 N aq. HCl. A precipitated solid was collected by filtration to afford the **A-171** (1.50 mg, 21%) as a white solid.

### 22. Synthesis of A-172

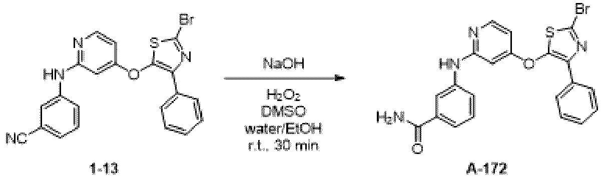

To a solution of **1-13** (15.0 mg, 0.03 mol) in water (0.24 mL) and EtOH (1.4 mL) was added NaOH (1.34 mg, 0.03 mmol), DMSO (3.55 µL, 0.05 mmol) and hydrogen peroxide (30 wt%, 3.92 µL, 0.05 mmol) at room temperature. After stirred at room temperature for 30 min, the reaction mixture was quenched with ammonia water and extracted with EtOAc. The organic layer was dried over Na₂SO₄, filtered and concentrated in *vacuo.* The residue was purified by column chromatography on NH-SiO₂ (DCM:EtOAc = 1:1) to give the **A-172** (11.0 mg, 71%) as a yellow solid.

### 23. Synthesis of A-173

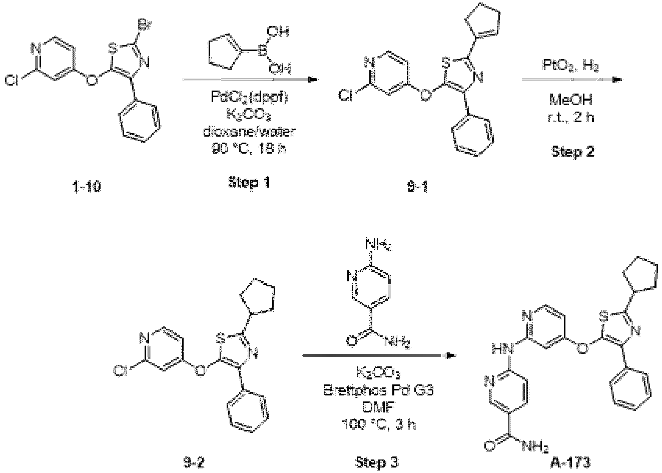

### (1) Preparation of 5-((2-chloropyridin-4-yl)oxy)-2-(cyclopent-1-en-1-yl)-4-phenylthiazole (9-1)

To a solution of **1-10** (300 mg, 0.82 mmol) in dioxane (6.1 mL) and water (2.0 mL) was added cyclopentenylboronic acid (100 mg, 0.90 mmol) and K₂CO₃ (189 mg, 1.37 mmol) at room temperature. The mixture was degassed by purging and re-filled with Ar in several times. After addition of PdCl₂(dppf) (119 mg, 0.16 mmol), the reaction mixture was heated at 90 °C for 18 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 10:1) to give the **9-1** (125 mg, 42%) as a brown oil.

¹H-NMR (400 MHz, DMSO-*d*₆): *δ* 8.33 (1H, d, *J* = 5.6 Hz), 7.79 (2H, d, *J* = 7.2 Hz), 7.42-7.39 (3H, m), 7.32 (1H, t, *J* = 7.4 Hz), 7.22 (1H, dd, *J* = 6.0, 2.4 Hz), 6.67 (1H, s), 2.78 (2H, t, *J=* 6.4 Hz), 2.54-2.51 (2H, m), 1.99 (2H, quint. *J* = 7.6 Hz)

### (2) Preparation of 5-((2-chloropyridin-4-yl)oxy)-2-cyclopentyl-4-phenylthiazole (9-2)

To a solution of **9-1** (125 mg, 0.35 mmol) in MeOH (3.5 mL) was added PtO₂ (96.0 mg, 0.42 mmol). The mixture was stirred for 2 hours at room temperature under an H₂ atmosphere (balloon). The reaction mixture was filtered through a Celite pad. The filtrate was concentrated in *vacuo* to afford the **9-2** (113 mg, 90%) as a yellow oil that used for the next step without further purification.

¹H-NMR (400 MHz, DMSO-*d*₆): *δ* 8.33 (1H, d, *J* = 5.6 Hz), 7.77 (2H, d, *J* = 7.2 Hz), 7.41-7.28 (4H, m), 7.17 (1H, dd, *J* = 6.0, 2.4 Hz), 3.43 (1H, quint, *J* = 8.2 Hz), 2.16-2.08 (2H, m), 1.85-1.73 (4H, m), 1.67-1.65 (2H, m).

### (3) Preparation ofA-173

To a solution of **9-2** (113 mg, 0.32 mmol) in DMF (6.3 mL) was added 6-aminonicotinamide (48.0 mg, 0.35 mmol) and K₂CO₃ (131 mg, 0.95 mmol) at room temperature. The mixture was degassed by purging and re-filled with Ar in several times. After addition of Brettphos Pd G3 (86.0 mg, 95.0 µmol), the reaction mixture was heated at 100 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (EtOAc only) to give the **A-173** (39.0 mg, 27%) as a white solid.

The following **A-174** to **A-176** were synthesized using the procedure described for the synthesis of **A-173.**

### 24. Synthesis of A-177

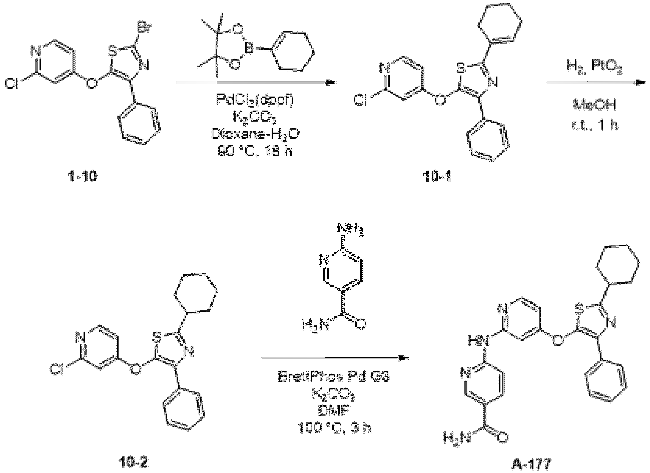

### (1) Preparation of 5-((2-chloropyridin-4-yl)oxy)-2-(cyclohex-1-en-1-yl)-4-phenylthiazole (10-1)

To a solution of **1-10** (1.0 g, 2.72 mmol), 2-cyclohexenyl-4,4,5,5-tetramethyl-1,3,2-dioxaborolane (0.7 mL, 3.26 mmol), K₂CO₃ (1.12 g, 8.16 mmol), and PdCl₂(dppf) (398 mg, 0.54mmol) was flushed with argon and the mixture was stirred at 90 °C for 18 hours. After being cooled to room temperature, the reaction mixture was quenched with water and extracted with EtOAc. The organic layers were dried over Na₂SO₄, filtered, and concentrated in *vacuo.* The residue was purified by column chromatography on SiO₂ (EtOAc:Hexane = 1:10) to afford the **10-1** (728 mg, 72%) as a yellow solid.

¹H-NMR (400 MHz, CDCl₃): *δ* 8.27 (1H, d, *J=* 6.0 Hz), 7.85 (2H, d, *J=* 7.2 Hz), 7.37 (2H, t, *J* = 6.8 Hz), 7.30 (1H, d, *J* = 7.2 Hz), 7.02 (1H, d, *J* = 2.4 Hz), 6.95 (1H, dd, *J* = 6.0, 2.4 Hz), 6.63 (1H, s), 2.58 (2H, m), 2.27-2.26 (2H, m), 1.81-1.78 (2H, m), 1.73-1.69 (2H, m).

### (2) Preparation of 5-((2-chloropyridin-4-yl)oxy)-2-cyclohexyl-4-phenylthiazole (10-2)

A mixture of **10-1** (710 mg, 1.92 mmol) in MeOH (19 mL) was added PtO₂ (524 mg, 2.3 mmol) and sitrred at room temperature under a H₂ atmosphere supplied from a latex balloon for 1 hour. The mixture was filtered through Celite pad and washed with MeOH. The filtrate was concentrated in *vacuo* to afford the **10-2** (210 mg, 30%) as a white solid.

¹H-NMR (400 MHz, CDCl₃): *δ* 8.27 (1H, d, *J=* 6.0 Hz), 7.82 (2H, 7.2 Hz), 7.37 (2H, t, *J* = 6.8 Hz), 7.27 (1H, d, *J* = 8.8 Hz), 7.00 (1H, d, *J* = 2.0 Hz), 6.93 (1H, dd, *J* = 6.0, 2.4 Hz), 2.96 (1H, m), 2.21-2.18 (2H, m), 2.09 (1H, s), 1.89-1.86 (2H, m), 1.77-1.74 (1H, m), 1.48-1.45 (2H, m), 0.82-0.80 (2H, m).

### (3) Preparation ofA-177

To a solution of **10-2** (50.0 mg, 0.13 mmol) in DMF (1.3 mL) was added 6-aminonicotinamide (22 mg, 0.16 mmol) and K₂CO₃ (55 mg, 0.4 mmol) at room temperature. The mixture was degassed by purging and refilled with argon in several times. After addition of BrettPhos Pd G3 (36.0 mg, 0.04 mmol), the reaction mixture was heated at 100 °C for 3 hours. After being cooled to room temperature, the reaction mixture was washed with water and extracted with EtOAc. The organic layer was dried over Na₂SO₄, filtered and concentrated in *vacuo.* The residue was purified by column chromatography on SiO₂ (DCM:MeOH = 9:1) to afford the **A-177** (1.8 mg, 2%) as a pink solid.

The following **A-178** to **A-180** were synthesized using the procedure described for the synthesis of **A-177**

### 25. Synthesis of A-181

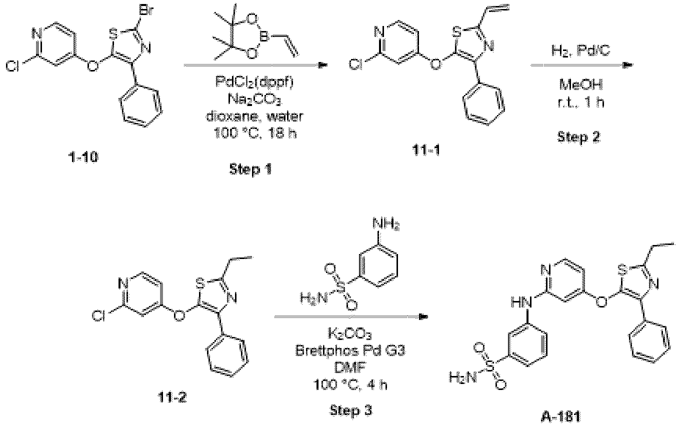

### (1) Preparation of the 5-((2-chloropyridin-4-yl)oxy)-4-phenyl-2-vinylthiazole (11-1)

To a solution of **1-10** (900 mg, 2.45 mmol) in 1,4-dioxane (22 mL) and water (2.2 mL) were added 4,4,5,5-tertramethyl-2-vinyl-1,3,2-dioxaborolane (754 mg, 4.90 mmol) and Na₂CO₃ (778 mg, 7.34 mmol) at room temperature. The mixture was degassed by purging and re-filled with Ar in several times. After addition of PdCl₂(dppf) (269 mg, 0.37mmol), the reaction mixture was heated at 100 °C for 18 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 10:1) to give the **11-1** (443 mg, 58%) as a yellow solid.

¹H-NMR (400 MHz, DMSO-*d*₆): *δ* 8.37 (1H, d, *J=* 5.6 Hz), 7.81 (2H, d, *J=* 7.6 Hz), 7.45-7.41 (3H, m), 7.37-7.33 (1H, m), 7.27 (1H, dd, *J=* 5.4, 1.8 Hz), 6.96 (1H, dd, *J=* 17.6, 11.2 Hz), 6.11 (1H, d, *J* = 17.2 Hz), 5.70 (1H, d, *J* = 10.8 Hz).

### (2) Preparation of 5-((2-chloropyridin-4-yl)oxy)-2-ethyl-4-phenylthiazole (11-2)

A mixture of **11-1** (443 mg, 1.41 mmol) and Pd/C (10 wt%, 150 mg, 0.14 mmol) in MeOH (14 mL) were stirred at room temperature for 1 hour under H₂ atmosphere (balloon). After filtration through a Celite pad, the filtrate was concentrated in *vacuo* to give the **11-2** (333 mg, 75%) as a yellow oil which was used without further purification.

¹H-NMR (400 MHz, DMSO-*d*₆): *δ* 8.33 (1H, d, *J* = 5.6 Hz), 7.77 (2H, d, *J* = 8.0 Hz), 7.39 (2H, t, *J=* 7.6 Hz), 7.33-7.38 (2H, m), 7.18 (1H, dd, *J=* 5.6, 1.6 Hz), 2.99 (2H, q, *J=* 7.7 Hz), 1.32 (3H, t, *J* = 7.6 Hz).

### (3) Preparation of A-181

To a solution of **11-2** (30.0 mg, 0.0950 mmol) in DMF (1.9 mL) were added 3-aminobenzenesulfonamide (24.0 mg, 0.14 mmol) and K₂CO₃ (39.0 mg, 0.284 mmol) at room temperature. The mixture was degassed by purging and re-filled with Ar in several times. After addition of Brettphos Pd G3 (17.0 mg, 0.02 mmol), the reaction mixture was heated at 100 °C for 4 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 1: 1) to give the **A-181** (21 mg, 49%) as a white solid.

The following **A-182** to **A-190** were synthesized using the procedure described for the synthesis of **A-181.**

### 26. Synthesis of A-191 to A-193

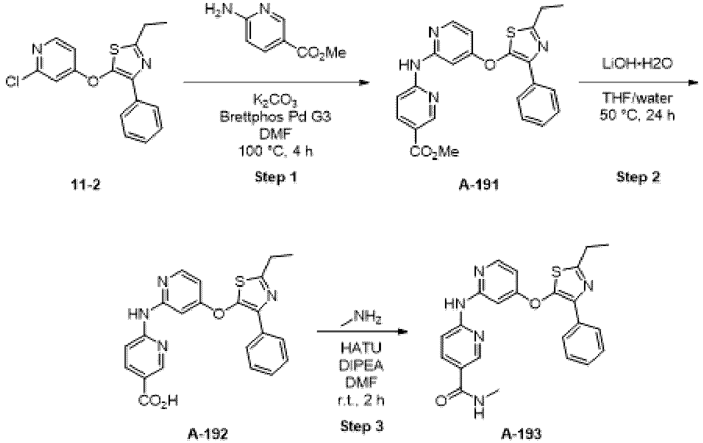

### (1) Preparation of A-191

**11-2** (200 mg, 0.63 mmol), methyl 6-aminonicotinate (96.0 mg, 0.63 mmol) and K₂CO₃ (87.0 mg, 0.63 mmol) in DMF (6.31 mL) was degassed by purging and re-filled with Ar in several times. After addition of BrettPhos Pd G3 (572 mg, 0.63 mmol), the reaction mixture was stirred at 100 °C for 4 hours. After cooled to room temperature, the reaction mixture was quenched with water and extracted with EtOAc. The separated organic layer was dried with MgSO₄, filtered off and concentrated in *vacuo.* The residue was purified by column chromatography on SiO₂ (Hexane:EtOAc = 1:1) to give the **A-191** (120 mg, 44%) as a yellow solid.

### (2) Preparation ofA-192

A solution of A-191 (70.0 mg, 0.16 mmol) in THF (1.29 mL) and Water (0.32 mL) was added LiOH·H₂O (39.0 mg, 1.62 mmol) at 0 °C. The reaction mixture was stirred at 50 °C for 24 hours. After cooling to 0 °C, the reaction mixture was acidified with aq. 1N HCl and concentrated in *vacuo* to give the **A-192** (90.0 mg, quant.) as a white solid, which was used for the next step without further purification.

### (3) Preparation ofA-193

A mixture of **A-192** (50.0 mg, 0.12 mmol), HATU (68.0 mg, 0.18 mmol), DIPEA (0.06 mL, 0.36 mmol) and methanamine (0.09 mL, 0.18 mmol) in DMF (1.19 mL) was stirred at room temperature for 2 hours. The reaction mixture was dissolved with EtOAc, washed with water and brine. The separated organic layer was dried with Na₂SO₄, filtered off and concentrated in *vacuo.* The residue was purified by column chromatography on SiO₂ (DCM:MeOH = 10:1) to give the **A-193** (43.0 mg, 83%) as a white solid.

The following **A-194** and **A-195** were synthesized using the procedure described for the synthesis of **A-192**

The following **A-196** to **A-215** were synthesized using the procedure described for the synthesis of **A-191** or **A-193**

### 27. Synthesis of A-216 and A-217

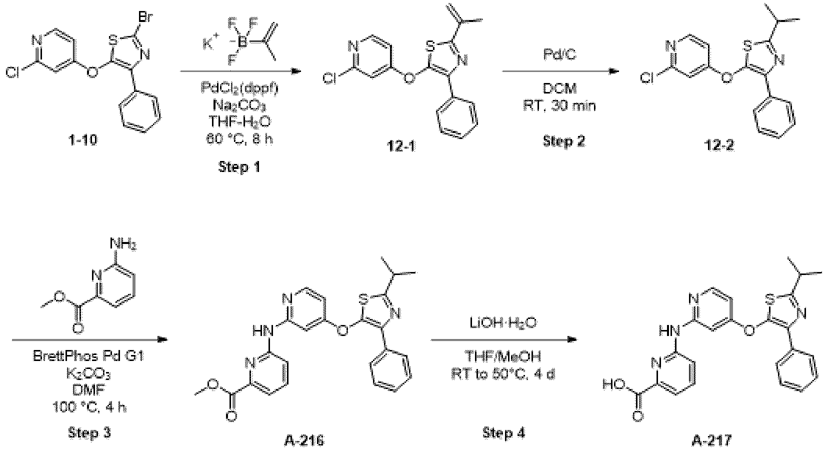

### (1) Preparation of 5-((2-chloropyridin-4-yl)oxy)-4-phenyl-2-(prop-1-en-2-yl)thiazole (12-1)

To a solution of **1-10** (40.0 mg, 0.11 mmol), potassium isopropenyltrifluoroborate (24.0 mg, 0.16 mmol), Na₂CO₃ (81.0 mg, 0.76 mmol), and PdCl₂(dppf) (8.9 mg, 0.01 mmol) was flushed with argon and the mixture was stirred at 60 °C for 8 hours. After being cooled to room temperature, the reaction mixture was quenched with water and extracted with EtOAc. The organic layers were dried over Na₂SO₄, filtered, and concentrated in *vacuo.* The residue was purified by column chromatography on SiO₂ (EtOAc:Hexane = 1:4) to afford the **12-1** (24.0 mg, 67%) as a yellow oil.

¹H-NMR (400 MHz, CDCl₃): *δ* 8.26 (1H, d, *J=* 6.0 Hz), 7.88-7.85 (2H, m), 7.39-7.35 (2H, m), 7.39-7.25 (1H, m), 7.02 (1H, d, *J=* 2.0 Hz), 6.95-6.93 (1H, m), 5.77 (1H, s), 5.37 (1H, d, *J =* 1.2 Hz), 2.26 (3H, s).

### (2) Preparation of 5-((2-chloropyridin-4-yl)oxy)-2-isopropyl-4-phenylthiazole (12-2)

A mixture of **12-1** (300 mg, 0.91 mmol) in DCM (10 mL) was added 10% Pd/C (97.0 mg, 0.09 mmol) and sitrred at room temperature under a H₂ atmosphere supplied from a latex balloon for 30 min. The mixture was filtered through Celite pad and washed with DCM. The filtrate was concentrated in *vacuo* to afford the **12-2** (170 mg, 56%) as a colorless oil.

¹H-NMR (400 MHz, CDCl₃): *δ* 8.26 (1H, d, *J=* 8.8 Hz), 7.83 (2H, d, *J=* 8.4 Hz), 7.35 (2H, t, *J* = 6.0 Hz), 7.27 (1H, t, *J* = 4.8 Hz), 7.00 (1H, d, *J* = 2.4 Hz), 6.92 (1H, dd, *J* = 2.4 Hz, 6.0 Hz), 3.31-3.24 (1H, m), 1.42 (6H, 5.2 Hz).

### (3) Preparation of A-216

To a solution of **12-2** (80.0 mg, 0.24 mmol) in DMF (2.4 mL) was added methyl 6-aminopicolinate (55.0 mg, 0.36 mmol) and K₂CO₃ (100 mg, 0.72 mmol) at room temperature. The mixture was degassed by purging and refilled with argon in several times. After addition of Brettphos Pd G3 (65.0 mg, 0.07 mmol), the reaction mixture was heated at 100 °C for 3 hours. After being cooled to room temperature, the reaction mixture was washed with water and extracted with EtOAc. The organic layer was dried over Na₂SO₄, filtered and concentrated in *vacuo.* The residue was purified by column chromatography on SiO₂ (Hexane:EtOAc = 3:1) to afford the **A-216** (41.0 mg, 38%) as a yellow solid.

### (4) Preparation ofA-217

A solution of **A-216** (41.0 mg, 0.09 mmol) in THF (0.9 mL) and water (0.9 mL) was added LiOH·H₂O (38.0 mg, 0.91 mmol) at 0 °C. The reaction mixture was stirred 50 °C for 18 hours. After cooling to 0 °C, the reaction mixture was acidified with aq. 1 N HCl solution and concentrated in *vacuo* to give the **A-217** (24.0 mg, 60%) as a yellow solid.

The following **A-218** to **A-229** were synthesized using the procedure described for the synthesis of **A-216.**

The following **A-230** to **A-232** were synthesized using the procedure described for the synthesis of **A-217.**

### 28. Synthesis of A-233

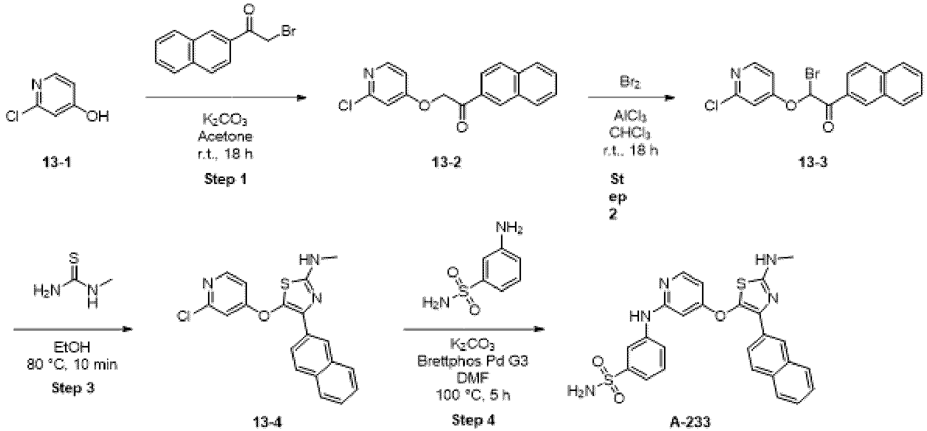

### (1) Preparation of 2-((2-chloropyridin-4-yl)oxy)-1-(naphthalen-2-yl)ethan-1-one (13-2)

A mixture of **13-1** (1.50 g, 11.58 mmol), 2-bromo-1-(naphthalene-2-yl)ethanone (3.46 g, 13.89 mmol) and K₂CO₃ (2.40 g, 17.37 mmol) in acetone (20 mL) was stirred for 18 hours at room temperature. The reaction mixture was washed with water and extracted with EtOAc. The organic layer was dried over Na₂SO₄, filtered, and concentrated in *vacuo* to afford the **13-2** (2.77 g, 80 %) as a yellow foam, which was used for the next step without further purification.

¹H-NMR (400 MHz, CDCl₃): *δ* 8.49 (1H, s), 8.21 (1H, d, *J* = 5.6 Hz), 8.01-7.86 (4H, m), 7.67-7.58 (2H, m), 6.88 (1H, d, *J* = 2.4 Hz), 6.82 (1H, dd, *J* = 2.4 Hz, 5.6 Hz), 5.49 (2H, s).

### (2) Preparation of 2-bromo-2-((2-chloropyridin-4-yl)oxy)-1-(naphthalen-2-yl)ethan-1-one (13-3)

To a solution of **13-2** (230 mg, 0.77 mmol) in CHCl₃ (8.0 mL) was added Br₂ (0.06 mL, 1.16 mmol) and AlCl₃ (21.0 mg, 0.15 mol) at 0 °C. The reaction mixture was stirred for 18 hours at room temperature. The reaction mixture was washed with saturated aq. NaHCO₃ solution at 0 °C and extracted with EtOAc. The organic layer was dried over Na₂SO₄, filtered, and concentrated in *vacuo* to afford the **13-3** (300 mg, quant.) as a yellow foam, which was used for the next step without further purification.

¹H-NMR (400 MHz, CDCl₃): *δ* 8.73 (1H, s), 8.40 (1H, d, *J=* 5.6 Hz), 8.14 (1H, d, *J* = 8.8 Hz), 8.03 -7.90 (3H, m), 7.69-7.59 (2H, m), 7.27 (1H, s), 7.16 (1H, s).

### (3) Preparation of 5-((2-chloropyridin-4-yl)oxy)-N-methyl-4-(naphthalen-2-yl)thiazol-2-amine (13-4)

A mixture of **13-3** (480 mg, 1.27 mmol), and 1-methylthiourea (115 mg, 1.27 mmol) in EtOH (13.0 mL) was stirred for 20 min at 75 °C. After being cooled at room temperature, the solvent was removed in *vacuo.* The residue was dissolved with EtOAc and washed with water. The organic layer was dried over Na₂SO₄, filtered, and concentrated in *vacuo.* The residue was purified by column chromatography on NH-SiO₂ (DCM) to afford the **13-4** (160 mg, 34 %) as a yellow solid.

¹H-NMR (400 MHz, CDCl₃): *δ* 8.25 (2H, d, *J=* 5.2 Hz), 7.85-7.76 (4H, m), 7.47-7.43 (2H, m), 7.08 (1H, d, *J* = 2.0 Hz), 7.00 (1H, dd, *J* = 2.4 Hz, 5.6 Hz), 5.59-5.57 (1H, m), 3.00 (3H, d, *J* = 4.8 Hz).

### (4) Preparation ofA-233

To a solution of **13-4** (80.0 mg, 0.22 mmol) in DMF (5.0 mL) was added K₂CO₃ (90.0 mg, 0.65 mmol), 3-aminobenzenesulfonamide (56.0 mg, 0.33 mmol) at room temperature. The mixture was degassed by purging and re-filled with argon several times. After addition of BrettPhos Pd G3 (39.0 mg, 0.04 mmol), the reaction mixture was heated at 100 °C for 5 hours. After being cooled at room temperature, the reaction mixture was washed with water and extracted with EtOAc. The organic layer was dried over Na₂SO₄, filtered, and concentrated in *vacuo.* The residue was purified by column chromatography on NH-SiO₂ (DCM:MeOH = 98:2) to afford the **A-233** (8.2 mg, 7%) as a beige solid.

### 29. Synthesis of A-234

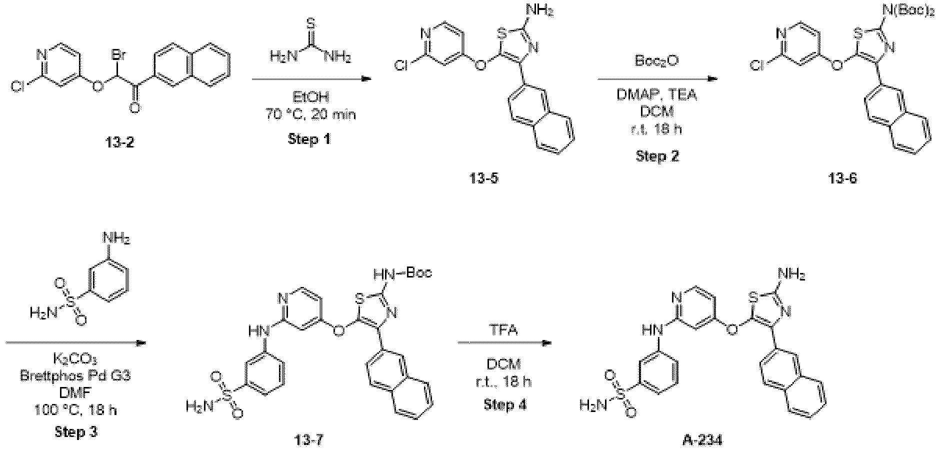

### (1) Preparation of 5-(2-chloropyridin-4-yloxy)-4-(naphthalen-2-yl)thiazol-2-amine (13-5)

A mixture of **13-2** (300 mg, 0.80 mmol), thiourea (61.0 mg, 0.80 mmol) in EtOH (7.9 mL) was heated for 20 min at 70 °C. After being cooled at room temperature, the solvent was removed in *vacuo.* The residue was dissolved with EtOAc and washed with water. The organic layer was dried over Na₂SO₄, filtered, and concentrated in *vacuo.* The residue was purified by column chromatography on NH-SiO₂ (DCM only) to afford the **13-5** (120 mg, 42%) as a yellow solid.

¹H-NMR (400 MHz, CDCl₃): *δ* 8.49 (1H, S), 8.21 (1H, d, *J* = 5.6 Hz), 7.90-8.02 (4H, m), 7.59-7.68 (2H, m), 6.89 (1H, d, *J* = 2.4 Hz), 6.82 (1H, dd, *J* = 2.4 Hz, 5.6 Hz), 5.50 (2H, s).

### (2) Preparation of di-tert-butyl 5-(2-chloropyridin-4-yloxy)-4-(naphthalen-2-yl)thiazol-2-ylcarbamate (13-6)

A mixture of **13-5** (300 mg, 0.85 mmol), Boc₂O (2.34 mL, 1.02 mmol), TEA (0.14 mL, 1.02 mmol), and DMAP (10.4 mg, 0.08 mmol) in DCM (30 mL) was stirred for 18 hours at room temperature. The reaction mixture was quenched with water and extracted with DCM. The organic layer was dried over Na₂SO₄, filtered, and concentrated in *vacuo.* The residue was purified by MPLC (Hexane/EtOAc) to afford the **13-6** (330 mg, 70 %) as a yellow foam.

¹H-NMR (400 MHz, CDCl₃): *δ* 8.29 (1H, s), 8.26 (1H, d, *J* = 5.6 Hz), 7.89 (1H, dd, *J* = 1.6 Hz, 8.4 Hz), 7.80-7.75 (3H, m), 7.45 (2H, dd, *J* = 2.8 Hz, 6.2 Hz), 7.06 (1H, d, *J* = 2.0 Hz), 7.06-6.99 (1H, m), 1.60 (18H, s).

### (3) Preparation of tert-butyl 4-(naphthalene-2-yl)-5-(2-(3-sulfamoylphenylamino)pyridin-4-yloxy)thiazol-2-ylcarbamate (13-7)

To a solution of **13-6** (200 mg, 0.36 mmol) in DMF (8 mL) was added K₂CO₃ (150 mg, 1.08 mmol), 3-aminobenzenesulfonamide (93.0 mg, 0.54 mmol) at room temperature. The mixture was degassed by purging and re-filled with argon several times. After the addition of BrettPhos Pd G3 (65.0 mg, 0.07 mmol), the reaction mixture was heated for 18 hours at 100 °C. After being cooled at room temperature, the reaction mixture was washed with water and extracted with EtOAc. The organic layer was dried over Na₂SO₄, filtered, and concentrated in *vacuo.* The residue was purified by column chromatography on SiO₂ (Hexane:EtOAc = 4:1) to afford the **13-7** (120 mg, 56%) as a dark brown solid.

MS Calcd.: 689.8; MS Found: 690.12 (M+H⁺).

### (4) Preparation ofA-234

To a solution of **13-7** (120 mg, 0.20 mmol) in DCM (5.0 mL) was added TFA (0.18 mL, 2.03 mmol) and stirred for 18 hours at room temperature. The reaction mixture was neutralized with saturated aq. NaHCO₃ solution and extracted with EtOAc. The organic layer was dried over Na₂SO₄, filtered, and concentrated in *vacuo.* The residue was purified by column chromatography on NH-SiO₂ (DCM:MeOH = 95:5) to afford the **A-234** (10.0 mg, 10%) as a beige solid.

### 30. Synthesis of A-235

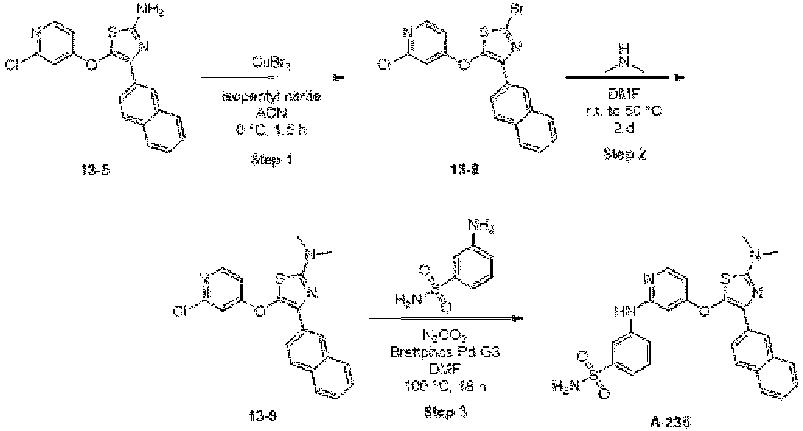

### (1) Preparation of 2-bromo-5-(2-chloropyridin-4-yloxy)-4-(naphtalen-2-yl)thiazole (13-8)

To a solution of **13-5** (9.49 g, 26.8 mmol) in ACN (90 mL) was added CuBr₂ (5.99 g, 26.8 mmol), and isopentyl nitrite (5.40 mL, 40.2 mmol) at 0 °C. The reaction mixture was stirred for 90 min at 0 °C. The reaction mixture was quenched with NH₄OH and extracted with EtOAc. The organic layer was dried over Na2SO4, filtered, and concentrated in *vacuo.* The residue was purified by column chromatography on SiO₂ (Hexane:EtOAc = 1:4) to afford the **13-8** (5.40 g, 48%) as a brown solid.

¹H-NMR (400 MHz, CDCl₃): *δ* 8.33 (1H, s), 8.29 (1H, dd, *J=* 6.0 Hz), 7.88-7.78 (4H, m), 7.50-7.45 (2H, m), 7.06 (1H, d, *J=* 2.5 Hz), 6.98-6.96 (1H, m).

### (2) Preparation of 5-(2-chloropyridin-4-yloxy)-N,N-dimethyl-4-(naphthalen-2-yl)thiazol-2-amine (13-9)

A mixture of **13-8** (100 mg, 0.24 mmol), and dimethyl amine (2M in THF, 0.18 mL, 0.36 mmol) in DMF (5 mL) was stirred for 2 days at room temperature to 50 °C. After being cooled at room temperature, the reaction mixture was washed with water and extracted with EtOAc. The organic layer was dried over Na₂SO₄, filtered, and concentrated in *vacuo.* The residue was purified by column chromatography on SiO₂ (Hexane:EtOAc = 3:2) to afford the **13-9** (23.0 mg, 25%) as a yellow oil.

¹H-NMR (400 MHz, CDCl₃): *δ* 8.30 (1H, s), 8.25 (1H, d, *J* = 6.0 Hz), 7.90 (1H, dd, *J* = 1.6 Hz, 8.6 Hz), 7.82-7.71 (3H, m), 7.46-7.41 (2H, m), 7.08 (1H, d, *J=* 2.0 Hz), 7.00 (1H, dd, *J* = 2.4 Hz, 6.0 Hz), 3.16 (6H, s).

### (3) Preparation ofA-235

To a solution of **13-9** (23.0 mg, 0.06 mmol) in DMF (2 mL) was added K₂CO₃ (33.0 mg, 0.24 mmol), 3-aminobenzenesulfonamide (16.0 mg, 0.09 mmol) at room temperautre. The mixture was degassed by purging and re-filled with argon several times. After addition of BrettPhos Pd G3 (11.0 mg, 0.01 mmol), the reaction mixture was heated for 18 hours at 100 °C. After being cooled at room temperature, the reaction mixture was washed with water and extracted with EtOAc. The organic layer was dried over Na₂SO₄, filtered, and concentrated in *vacuo.* The residue was purified by column chromatography on SiO₂ (Hexane:EtOAc = 1:1) to afford the **A-235** (5.8 mg, 18%) as a pale yellow solid.

### 31. Synthesis of A-236

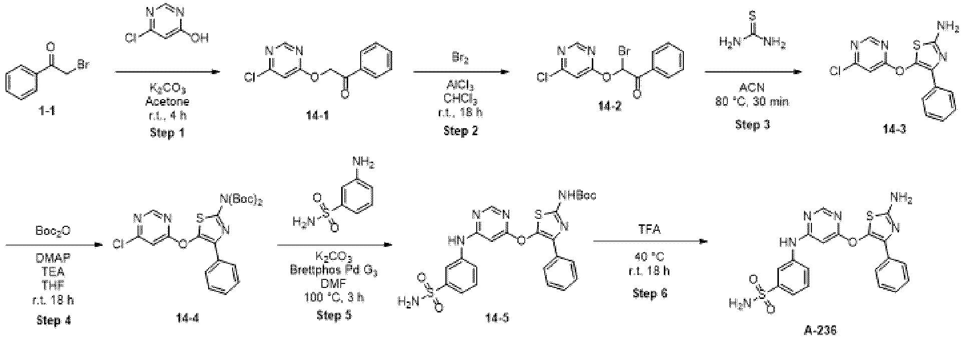

### (1) Preparation of 2-(6-cloropyrimidin-4-yloxy)-1-phenylethanone (14-1)

To a solution of **1-1** (5.03 g, 25.3 mmol) in acetone (46 mL) was added 6-choropyrimidin-4-ol (3.00 g, 23.0 mmol) and K₂CO₃ (4.76 g, 34.5 mmol) at room temperature. After stirred at room temperature for 4 hours, the reaction mixture was poured into water and extracted with EtOAc. The organic layer was dried over Na₂SO₄, filtered and concentrated in *vacuo.* The residue was purified by column chromatography on SiO₂ (Hexane:EtOAc = 1:2) to give the **14-1** (5.55 g, 97%) as a white solid.

¹H-NMR (400 MHz, CDCl₃): *δ* 8.01 (2H, d, *J* = 8.0 Hz), 7.96 (1H, s), 7.68 (1H, t, *J*= 7.4 Hz), 7.54 (2H, t, *J* = 7.8 Hz), 6.59 (1H, s), 5.36 (2H, s).

### (2) Preparation of 2-bromo-2-(6-chloropyrimidin-4-yloxy)-1-phenylethanone (14-2)

To a solution of **14-1** (5.55 g, 22.3 mmol) in CHCl₃ (74 mL) was added slowly AlCl₃ (1.19 g, 8.93 mmol) and Br₂ (1.73 mL, 33.5 mmol) at room temperature. The mixture was stirred at room temperature for 18 hours. The reaction mixture was quenched with sat. NaHCO₃ solution and extracted with EtOAc. The organic layer was dried over Na₂SO₄, filtered and concentrated in *vacuo.* The residue was purified by column chromatography on SiO₂ (Hexane:EtOAc = 5:1) to give the **14-2** (4.71 g, 64%) as a white solid.

¹H-NMR (400 MHz, CDCl₃): *δ* 8.53 (1H, s), 8.14 (1H, s), 8.08 (2H, d, *J* = 8.08 Hz), 7.70 (1H, t, *J* = 7.8 Hz), 7.56 (2H, t, *J* = 7.6 Hz), 6.53 (1H, s).

### (3) Preparation of 5-(6-chloropyrimidin-4-yloxy)-4-phenylthiazol-2-amine (14-3)

A mixture of **14-2** (4.70 g, 14.4 mmol) and thiourea (1.20 g, 15.8 mmol) in ACN (48 mL) was heated at 80 °C for 30 min. After cooled to room temperature, the reaction mixture was quenched with sat. NaHCO₃ solution and extracted with EtOAc. The organic layer was dried over Na₂SO₄, filtered and concentrated in *vacuo* to give the **14-3** (4.03 g, 92%) as a yellow solid, which was used for next step without further purification.

¹H-NMR (400 MHz, DMSO-*d*₆): *δ* 8.44 (1H, d, *J* = 14.4 Hz), 8.06 (1H, d, *J* = 8.0 Hz), 7.60 (1H, t, *J* = 7.6 Hz), 7.43 (1H, s), 7.34-7.30 (3H, m). Two protons from NH₂ were not observed.

### (4) Preparation of tert-butyl (tert-butoxycarbonyl)(5-((6-chloropyrimidin-4-yl)oxy)-4-phenylthiazol-2-yl)carbamate (14-4)

To a solution of **14-3** (1.00 g, 3.28 mmol) in THF (33 mL) was added Boc2O (2.15 g, 9.84 mmol), TEA (1.37 mL, 9.84 mmol) and DMAP (80.0 mg, 0.67 mmol) at room temperature. The mixture was stirred at room temperature for 18 hours. The reaction mixture was poured into water and extracted with EtOAc. The organic layer was dried over Na₂SO₄, filtered and concentrated in *vacuo.* The residue was purified by column chromatography on SiO₂ (Hexane:EtOAc = 5:1 to 3:1) to give the compound **14-4** (1.14 g, 69%) as a yellow solid.

¹H-NMR (400 MHz, CDCl₃): *δ* 7.84 (1H, s), 7.44-7.41 (2H, m), 7.36-7.32 (3H, m), 6.67 (1H, s), 1.58 (18H, s).

### (5) Preparation of tert-butyl 4-phenyl-5-(6-(3-sulfamoylphenylamino)pyrimidin-4-ylozy)-thizol-2-ylcarbamate (14-5)

To a solution of **14-4** (300 mg, 0.60 mmol) in DMF (6.0 mL) was added 3-aminobenzenesulfonamide (113 mg, 0.65 mmol) and K₂CO₃ (246 mg, 1.78 mmol) at room temperature. The mixture was degassed by purging and re-filled with Ar in several times. After addition of BrettPhos Pd G3 (162 mg, 0.18 mmol), the reaction mixture was heated at 100 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 1:3) to give the **14-5** (106 mg, 28%) as a yellow solid.

¹H-NMR (400 MHz, DMSO-*d*₆): *δ* 9.78 (1H, s), 8.30 (1H, s), 7.84 (1H, s), 7.59-7.57 (1H, m), 7.51-7.49 (2H, m), 7.36-7.33 (3H, m), 7.15 (2H, s), 6.98 (1H, s), 6.90 (1H, d, *J=* 7.6 Hz), 6.68 (1H, d, *J* = 6.8 Hz), 1.53 (9H, s).

* A proton from NH was not observed.

### (6) Preparation ofA-236

To a solution of **14-5** (106 mg, 0.17 mmol) in DCM (1.7 mL) was added TFA (0.13 mL, 1.65 mmol) at 0 °C. The mixture was stirred at room temperature for 18 hours. The reaction mixture was quenched with sat. NaHCO₃ solution and extracted with EtOAc. The organic layer was dried over Na₂SO₄, filtered and concentrated in *vacuo.* The residue was purified by column chromatography on NH-SiO₂ (DCM:MeOH = 20:1) to give the **A-236** (7.6 mg, 10%) as a pale yellow solid.

### 32. Synthesis of A-237

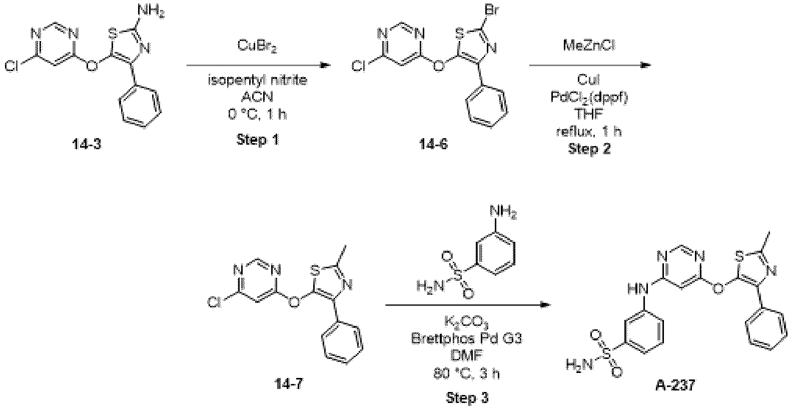

### (1) Preparation of the 2-bromo-5-(6-chloropyrimidin-4-yloxy)-4-phenylthiazole (14-6)

To a solution of **14-3** (400 mg, 1.31 mmol) in ACN (6.6 mL) wad added CuBr₂ (293 mg, 1.31 mmol) and isopentyl nitrite (0.26 mL, 1.97 mmol) at 0 °C. After stirred at room temperature for 1 hour, the reaction mixture was concentrated in *vacuo.* The residue was purified by column chromatography on SiO₂ (Hexane:EtOAc = 5:1) to give the **14-6** (284 mg, 59%) as a pale yellow solid.

¹H-NMR (400 MHz, CDCl₃): *δ* 7.81 (1H, s), 7.45-7.40 (5H, m), 6.69 (1H, s).

### (2) Preparation of the 5-(6-chloropyrimidin-4-yloxy)-2-methyl-4-phenylthiazole (14-7)

To a solution of **14-6** (284 mg, 0.77 mmol) in THF (7.7 mL) were added MeZnCl (2 M in THF, 0.96 mL, 1.93 mmol) and CuI (103 mg, 0.54 mmol) at room temperature. The mixture was degassed by purging and re-filled with Ar in several times. After addition of PdCl₂(dppf) (113 mg, 0.15 mmol), the reaction mixture was refluxed for 1 hour. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 1:1) to give the **14-7** (111 mg, 47%) as a yellow solid.

¹H-NMR (400 MHz, CDCl₃): *δ* 7.82 (1H, s), 7.45-7.43 (2H, m), 7.41-7.37 (3H, m), 6.67 (1H, s), 2.79 (3H, s).

### (3) Preparation ofA-237

To a solution of **14-7** (111 mg, 0.37 mmol) in DMF (3.7 mL) were added 3-aminobenzenesulfonamide (69.0 mg, 0.40 mmol) and K₂CO₃ (152 mg, 1.10 mmol) at room temperature. The mixture was degassed by purging and re-filled with Ar in several times. After addition of Brettphos Pd G3 (99.0 mg, 0.11 mmol), the reaction mixture was heated at 80 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 1:3) to give the **A-237** (38 mg, 24%) as a yellow solid.

### 33. Synthesis of A-238

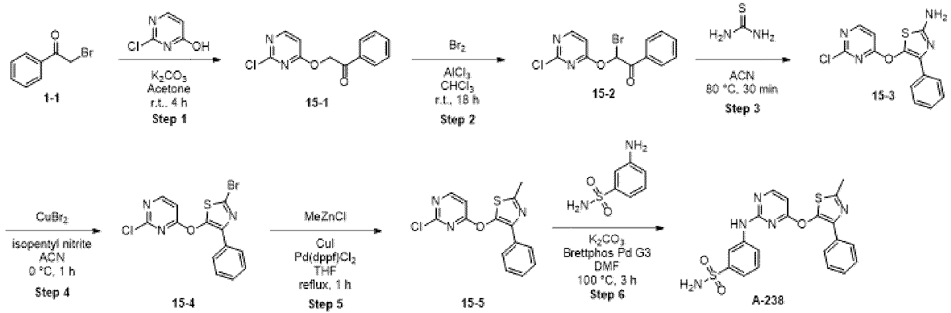

### (1) Preparation of 2-(2-chloropyrimidin-4-yloxy)-1-phenylethanone (15-1)

To a solution of **1-1** (1.68 g, 8.43 mmol) in acetone (15 mL) was added 2-chloropyridmidin-4-ol (1.00 g, 7.66 mmol) and K₂CO₃ (1.59 g, 11.5 mmol) at room temperature. After stirred at room temperature for 4 hours, the reaction mixture was poured into water and extracted with EtOAc. The organic layer was dried over Na₂SO₄, filtered and concentrated in *vacuo.* The residue was purified by column chromatography on SiO₂ (Hexane:EtOAc = 1:2) to give the **15-1** (1.69 g, 89%) as a white solid.

¹H-NMR (400MHz, CDCl₃): *δ* 8.37(1H, d, *J* = 5.2Hz), 7.97(2H, d, *J* = 8.0Hz), 7.65(1H, t, *J* = 7.2Hz), 7.53(2H , t, *J* = 7.8Hz), 6.89(1H, d, *J=* 5.6Hz), 5.71(2H, s).

### (2) Preparation of 2-bromo-2-(2-chloropyrimidin-4-yloxy)-1-phenylethanone (15-2)

To a solution of 15-1 (1.69 g, 6.80 mmol) in CHCl₃ (23 mL) was added slowly AlCl₃ (362 mg, 2.72 mmol) and Br₂ (0.525 mL, 10.2 mmol) at 0 °C. The mixture was stirred at room temperature for 18 hours. The reaction mixture was quenched with sat. NaHCO₃ solution and extracted with EtOAc. The organic layer was dried over Na₂SO₄, filtered and concentrated in *vacuo.* The residue was purified by column chromatography on SiO₂ (Hexane:EtOAc = 3:1) to give the **15-2** (1.55 g, 70%) as a white solid.

¹H-NMR (400MHz, CDCl₃): *δ* 8.50(1H, d, *J* = 5.6Hz), 8.11(2H, d, *J* = 7.6Hz), 8.06(1H, s), 7.66(1H, t, *J=* 7.4Hz), 7.54(2H, t, *J* = 7.8Hz), 6.99(1H, d, *J=* 5.6Hz).

### (3) Preparation of 5-(2-chloropyrimidin-4-yloxy)-4-phenylthiazol-2-amine (15-3)

A mixture of 15-2 (1.55 g, 4.73 mmol) and thiourea (396 mg, 5.21 mmol) in ACN (16 mL) was heated at 80 °C for 30 min. After cooled to room temperature, the reaction mixture was quenched with sat. NaHCO₃ solution and extracted with EtOAc. The organic layer was dried over Na₂SO₄, filtered and concentrated in *vacuo* to give the **15-3** (1.13 g, 78%) as a yellow solid, which was used for the next step without further purification.

¹H-NMR (400 MHz, DMSO-*d*₆): *δ* 8.67 (1H, d, *J=* 5.6 Hz), 7.67 (2H, d, *J=* 8.0 Hz), 7.37 (2H, d, *J=* 6.0 Hz), 7.32 (2H, d, *J=* 7.6 Hz), 7.12 (2H, s).

### (4) Preparation of 2-bromo-5-(2-chloropyrimidin-4-yloxy)-4-phenylthiazole (15-4)

To a solution of **15-3** (400 mg, 1.31 mmol) in ACN (6.6 mL) was added CuBr₂ (293 mg, 1.31 mmol) and isopentyl nitrite (0.26 mL, 1.97 mmol) at 0 °C. The mixture was stirred at 0 °C for 1 hour. The reaction mixture was poured into water and extracted with EtOAc. The organic layer was dried over Na₂SO₄, filtered and concentrated in *vacuo.* The residue was purified by column chromatography on SiO₂ (Hexane:EtOAc = 3:1) to give the **15-4** (159 mg, 33%) as a yellow solid.

¹H-NMR (400 MHz, DMSO-*d*₆): *δ* 8.76 (1H, d, *J=* 6.0 Hz), 7.86 (2H, d, *J=* 7.2 Hz), 7.56 (1H, d, *J* = 6.0 Hz), 7.46 (2H, t, *J* = 7.4 Hz), 7.38 (1H, t, *J* = 7.4 Hz).

### (5) Preparation of 5-(2-chloropyrimidin-4-yloxy)-2-methyl-4-phenylthiazole (15-5)

To a solution of **15-4** (159 mg, 0.43 mmol) in THF (4.3 mL) was added MeZnCl (2 M in THF, 0.54 mL, 1.08 mmol) and CuI (63.0 mg, 0.30 mmol) at room temperature. The mixture was degassed by purging and re-filled with Ar in several times. After addition of Pd(dppf)Cl₂ (63.0 mg, 0.09 mmol), the reaction mixture was refluxed for 1 hour. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 3:1) to give the **15-5** (57.0 mg, 44%) as a yellow solid.

¹H-NMR (400 MHz, DMSO-*d*₆): *δ* 8.72 (1H, d, *J=* 5.2 Hz), 7.81 (2H, d, *J=* 7.2 Hz), 7.47 (1H, d, *J=* 5.6 Hz), 7.41 (2H, t, *J=* 7.6 Hz), 7.31 (1H, t, *J=* 7.4 Hz), 2.67 (3H, s).

### (6) Preparation ofA-238

To a solution of the **15-5** (57.0 mg, 0.19 mmol) in DMF (1.9 mL) was added 3-aminobenzenesulfonamide (36.0 mg, 0.21 mmol) and K₂CO₃ (78.0 mg, 0.56 mmol) at room temperature. The mixture was degassed by purging and re-filled with Ar in several times. After addition of Brettphos Pd G3 (51.0 mg, 0.06 mmol), the reaction mixture was heated at 100 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on NH-SiO₂ (DCM:MeOH = 20:1) to give the **A-238** (8.8 mg, 11%) as a white solid.

### 34. Synthesis of A-239

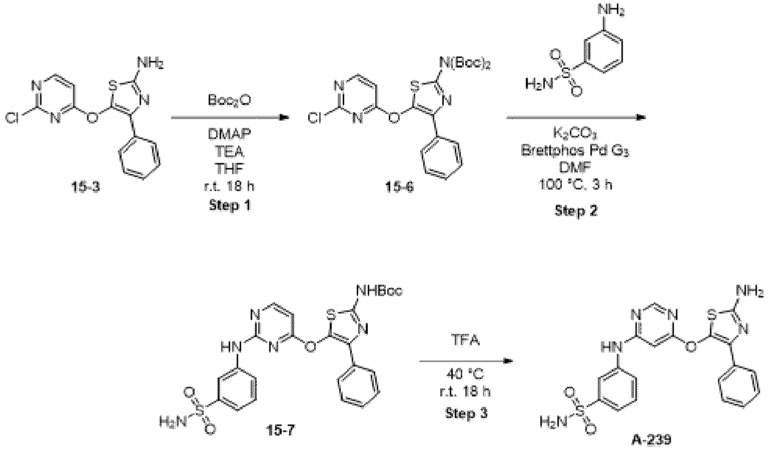

### (1) Preparation of tert-butyl (tert-butoxycarbonyl)(5-((2-chloropyrimidin-4-yl)oxy)-4-phenylthiazol-2-yl)carbamate (15-6)

To a solution of **15-3** (700 mg, 3.28 mmol) in THF (23 mL) was added Boc₂O (1.50 g, 6.89 mmol), TEA (0.96 mL, 6.89 mmol) and DMAP (56.0 mg, 0.46 mmol) at room temperature. The mixture was stirred at room temperature for 18 hours. The reaction mixture was poured into water and extracted with EtOAc. The organic layer was dried over Na₂SO₄, filtered and concentrated in *vacuo.* The residue was purified by column chromatography on SiO₂ (Hexane:EtOAc = 5:1 to 3:1) to give the compound **15-6** (339 mg, 29%) as a yellow solid.

Compound **15-6:** MS Calcd.: 504.99; MS Found: 504.78 (M+H⁺)

### (2) Preparation of tert-butyl 4-phenyl-5-(2-(3-sulfamoylphenylamino)pyrimidin-4-yloxy)-thizol-2-ylcarbamate (15-7)

To a solution of mixture compound **15-6** (339 mg, 0.67 mmol) in DMF (6.0 mL) was added 3-aminobenzenesulfonamide (127 mg, 0.74 mmol) and K₂CO₃ (278 mg, 2.01 mmol) at room temperature. The mixture was degassed by purging and re-filled with Ar in several times. After addition of Brettphos Pd G3 (183 mg, 0.20 mmol), the reaction mixture was heated at 100 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 1:1) to give the **15-7** (144 mg, 40%) as a beige solid.

¹H-NMR (400 MHz, DMSO-*d*₆): *δ* 11.6 (1H, s), 10.0 (1H, s), 8.45 (1H, d, *J=* 6.0 Hz), 8.08 (1H, s), 7.91 (1H, d, *J=* 6.8 Hz), 7.74 (2H, d, *J=* 7.06 Hz), 7.41-7.32 (4H, m), 7.31-7.25 (3H, m), 6.72 (1H, d, *J* = 5.2 Hz), 1.48 (9H, s).

### (3) Preparation of A-239

To a solution of **15-7** (144 mg, 0.27 mmol) in DCM (2.7 mL) was added TFA (0.31 mL, 4.00 mmol) at 0 °C. The mixture was stirred at room temperature for 18 hours. The reaction mixture was quenched with sat. NaHCO₃ solution and extracted with DCM. The organic layer was dried over Na₂SO₄, filtered and concentrated in *vacuo.* The residue was purified by column chromatography on NH-SiO₂ (DCM:MeOH = 10:1) to give the **A-239** (5.0 mg, 4%) as a white solid.

### 35. Synthesis of A-240

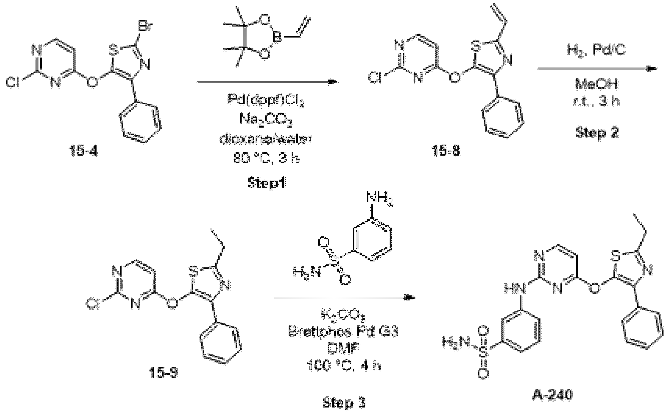

### (1) Preparation of 5-(2-chloropyrimidin-4-yloxy)-4-phenyl-2-vinylthiazole (15-8)

To a solution of the **15-4** (170 mg, 0.46 mmol) in dioxane (2.1 mL) and water (0.21 mL) was added 4,4,5,5-tetramethyl-2-vinyl-1,3,2-dioxaborolane (107 mg, 0.69 mmol) and Na₂CO₃ (147 mg, 1.38 mmol) at room temperature. The mixture was degassed by purging and re-filled with Ar in several times. After addition of Pd(dppf)Cl₂ (67.5 mg, 0.09 mmol), the reaction mixture was heated at 80 °C for 3 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 9:1) to give the **15-8** (59.0 mg, 41%) as an ivory oil.

¹H-NMR (400 MHz, CDCl₃): *δ* 8.53 (1H, d, *J=* 6.0 Hz), 7.87 (2H, d, *J=* 7.6 Hz), 7.44-7.40 (2H, m), 7.35-7.33 (1H, m), 7.01 (1H, d, *J=* 5.6 Hz), 6.92-6.85 (1H, m), 6.07-6.02 (1H, m), 5.61-5.58 (1H, m).

### (2) Preparation of 5-(2-chloropyrimidin-4-yloxy)-2-ethyl-4-phenylthizaole (15-9)

A mixture of **15-8** (59.0 mg, 0.19 mmol) and Pd/C (5 wt%, 9.94 mg, 0.09 mmol) in MeOH (3.7 mL) was stirred at room temperature for 3 hours under H₂ atmosphere (balloon). After filtrated through a Celite pad, the filtrate was concentrated in *vacuo* to give the **15-9** (51.0 mg, 86%) as a brown oil, which was used for next step without further purification.

¹H-NMR (400 MHz, CDCl₃): *δ* 8.51 (1H, d, *J=* 5.2 Hz), 7.85 (1H, d, *J=* 7.2 Hz), 7.42-7.38 (2H, m), 7.33-7.29 (1H, m), 6.97 (1H, d, *J=* 5.2 Hz), 3.04 (2H, q, *J=* 7.6 Hz), 1.44 (3H, t, *J* = 7.4 Hz).

### (3) Preparation of A-240

To a solution of **15-9** (51.0 mg, 0.16 mmol) in DMF (2.0 mL) were added 3-aminobenzenesulfonamide (33.2 mg, 0.19 mmol) and K₂CO₃ (66.5 mg, 0.48 mmol) at room temperature. The mixture was degassed by purging and re-filled with Ar in several times. After addition of BrettPhos Pd G3 (43.6 mg, 0.05 mmol), the reaction mixture was heated at 100 °C for 4 hours. After cooled to room temperature, the reaction mixture was quenched with water and extracted with EtOAc. The organic layer was dried over Na₂SO₄, filtered, and concentrated in *vacuo.* The residue was purified by column chromatography on NH-SiO₂ (EtOAc only) to give the **A-240** (8.5 mg, 12%) as an ivory solid.

The following **A-241** to **A-246** were synthesized using the procedure described for the synthesis of **A-240.**

### 36. Preparation of intermediates tert-butyl (5-((2-chloropyridin-4-yl)oxy)-4-(6-methylpyridin-2-yl)thiazol-2-yl)carbamate (16-6)

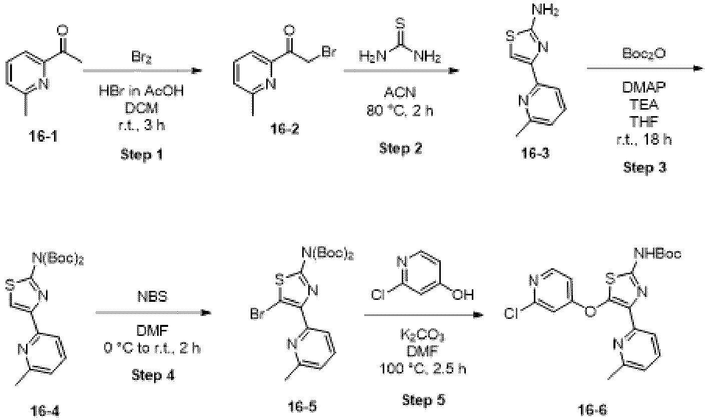

### (1) Preparation of the 2-bromo-1-(6-emthylpyridin-2-yl)ethanone (16-2)

To a solution of 1-(6-methylpyridin-2-yl)ethanone (**16-1**) (10.0 g, 74.0 mmol) in DCM (148 mL) were added HBr (33 wt% in AcOH, 24.35 mL, 148 mmol) and Br₂ (4.58 mL, 89.0 mmol) at 0 °C. The mixture was stirred for 3 hours at room temperature. The reaction mixture was neutralized with sat. NaHCO₃(aq) at 0 °C and extracted with EtOAc. The organic layer was dried over Na₂SO₄, filtered, and concentrated in *vacuo* to give the **16-2** (15.52 g, 98 %) as a yellow oil which was used for the next step without further purification.

¹H-NMR (400 MHz, DMSO-*d*₆): *δ* 7.90 (1H, t, *J=* 7.6 Hz), 7.81 (1H, d, *J=* 7.6 Hz), 7.55 (1H, d, *J=* 7.6 Hz), 4.99 (2H, s), 2.55 (3H, s).

### (2) Preparation of the 4-(6-methylpyridin-2-yl)thiazol-2-amine (16-3)

To a solution of **16-2** (15.52 g, 72.5 mmol) in acetonitrile (145 mL) were added thiourea (6.07 g, 80.0 mmol) at room temperature. The mixture was heated for 2 hours at 80 °C. After cooled to room temperature, the reaction mixture was neutralized with sat. NaHCO₃(aq) at 0 °C and extracted with EtOAc. The organic layer was dried over Na₂SO₄, filtered, and concentrated in *vacuo* to give the **16-3** (12.3 g, 89%) as a yellow solid which was used for the next step without further purification.

¹H-NMR (400 MHz, DMSO-*d*₆): *δ* 7.66 (1H, t, *J* = 7.8 Hz), 7.59 (1H, d, *J* = 7.6 Hz), 7.16 (1H, s), 7.09 (1H, d, *J=* 7.2 Hz), 7.05 (2H, s), 2.45 (3H, s).

### (3) Preparation of the tert-butoxycarbonyl)(4-(6-methylpyridin-2-yl)thiazol-2-yl)carbamate (16-4)

To a solution of **16-3** (12.3 g, 64.3 mmol) in THF (19 mL) were added Boc₂O (42.1 g, 193 mmol), DMAP (2.35 g, 19.29 mmol), and TEA (26.7 mL, 193 mmol) at room temperature. The mixture was stirred for 18 hours at room temperature. The reaction mixture was poured into water and extracted with EtOAc. The organic layer was dried over Na₂SO₄, filtered, and concentrated in *vacuo.* The residue was purified by column chromatography on SiO₂ (Hexane:EtOAc = 5:1) to give the **16-4** (15.78 g, 62%) as a pale yellow solid.

¹H-NMR (400 MHz, DMSO-*d*₆): *δ* 7.90 (1H, s, *J=* 7.6 Hz), 7.78 (1H, d, *J=* 8.0 Hz), 7.62 (1H, t, *J=* 8.0 Hz), 7.06 (1H, d, *J=* 7.6 Hz), 2.57 (3H, s), 1.53 (18H, s).

### (4) Preparation of the tert-butyl (5-bromo-4-(6-methylpyridin-2-yl)thiazol-2-yl)(tert-butoxycarbonyl)carbamate (16-5)

To a solution of **16-4** (15.7 g, 40.1 mmol) in DMF (201 mL) was added slowly NBS (7.85 g, 44.1 mmol) at 0 °C. The mixture was stirred at for 2 hours room temperature. The reaction mixture was quenched with sat. NaHCO₃(aq) and extracted with EtOAc. The organic layer was washed with brine several times and then dried over Na₂SO₄, filtered, and concentrated in *vacuo* to give the **16-5** (16.3 g, 86 %) as a brown oil which was used for the next step without further purification

¹H-NMR (400 MHz, DMSO-*d*₆): *δ* 7.77 (1H, t, *J=* 7.6 Hz), 7.61 (1H, d, *J=* 8.0 Hz), 7.24 (1H, d, *J=* 7.2 Hz), 2.51 (3H, s), 1.51 (18H, s).

### (5) Preparation of the tert-butyl 5-(2-chloropyridin-4-yloxy)-4-(6-methylpyridin-2-yl) thiazol-2-ylcarbamate (16-6)

A mixture of **16-5** (15.5 g, 33.0 mmol), 2-chloropyridin-4-ol (4.70 g, 36.2 mmol), and K₂CO₃ (9.11 g, 65.9 mmol) in DMF (165 mL) were stirred for 150 mins at 100 °C. The reaction was checked that consumed compound 5 by LC-MS once an hour. The mixture was poured into water and extracted with EtOAc. The organic layer washed with brine several times and then dried over Na₂SO₄, filtered, and concentrated in *vacuo.* The residue was purified by column chromatography on NH-SiO₂ (Hexane:EtOAc = 2:1) to give the **16-6** (6.4 g, 37%) as a yellow solid.

¹H-NMR (400 MHz, DMSO-*d*₆): *δ* 8.24 (1H, d, *J* = 5.6 Hz), 7.68 (1H, t, *J* = 7.6 Hz), 7.60 (1H, d, *J=* 7.2 Hz), 7.16 (1H, s), 7.08-7.04 (2H, m), 2.09 (3H, s), 1.47 (9H, s). * A proton from NH was not observed.

### 37. Synthesis of A-247

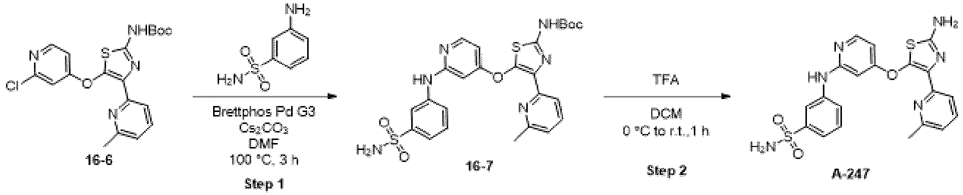

### (1) Preparation of the tert-butyl 4-(6-methylpyridin-2-yl)-5-(2-(3-sulfamoylphenylamino) pyridin-4-yloxy)thiazol-2-ylcarbamate (16-7)

To a solution of **16-6** (150 mg, 0.36mmol) in DMF (7.2 mL) were added 3-aminobenzenesulfonamide (92.0 mg, 0.54 mmol), and Cs₂CO₃ (350 mg, 1.07 mmol) at room temperature. The mixture was degassed by purging and re-filled with Ar in several times. After addition of Brettphos Pd G3 (97.0 mg, 0.11 mmol), the reaction mixture was heated at 100 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on NH-SiO₂ (only EtOAc to EtOAc:MeOH = 50:1) to give the **16-7** (44.0 mg, 22%) as a yellow solid.

¹H-NMR (400 MHz, DMSO-*d*₆): *δ* 9.31 (1H, s), 8.20 (1H, s), 8.10 (1H, d, *J* = 6.0 Hz), 7.80 (1H, d, *J=* 8.4 Hz), 7.70 (1H, t, *J=* 7.8 Hz), 7.60 (1H, d, *J=* 8.0 Hz), 7.40 (1H, t, *J=* 8.0 Hz), 7.30 (1H, d, *J=* 7.6 Hz), 7.27 (2H, s), 7.08 (1H, d, *J=* 7.6 Hz), 6.64 (1H, dd, *J* = 5.8, 2.2 Hz), 6.43 (1H, d, *J=* 2.0 Hz), 2.25 (3H, s), 1.50 (9H, s). * A proton from NH was not observed.

### (2) Preparation ofA-247

To a solution of **16-7** (44.0 mg, 0.08 mmol) in DCM (1.6 mL) were added TFA (0.122 mL, 1.59 mmol) at 0 °C. After stirred at room temperature for 1 hour, the reaction mixture was quenched with sat. NaHCO₃ and extracted with 5% MeOH/DCM. The organic layer was dried over Na₂SO₄, filtered, and concentrated in *vacuo.* The residue was purified by column chromatography on NH-SiO₂ (only EtOAc and EtOAc:MeOH = 10:1) to give the **A-247** (7.0 mg, 19%) as a pale orange solid.

The following **A-248** and **A-249** were synthesized using the procedure described for the synthesis of **A-247.**

### 38. Synthesis of A-250

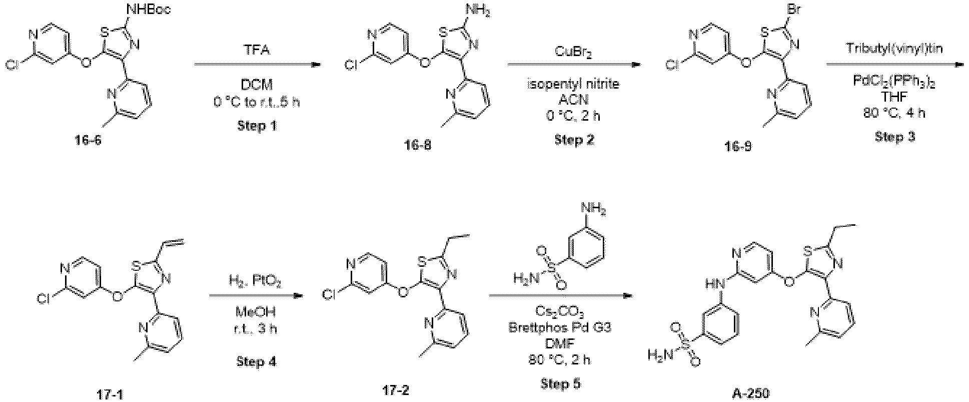

### (1) Preparation of the 5-(2-chloropyridin-4-yloxy)-4-(6-methylpyridin-2-yl)thiazol-2-amine (16-8)

To a solution of **16-6** (6.4 g, 15.28 mmol) in DCM (153 mL) was added TFA (23.54 mL, 306 mmol) at 0 °C. After stirred for 5 hours at room temperature, the reaction mixture was neutralized with sat. NaHCO₃(aq) at 0 °C and extracted with DCM. The organic layer was dried over Na₂SO₄, filtered, and concentrated in *vacuo* to give the **16-8** (4.3 g, 88%) as a red solid which was used for next step without further purification.

¹H-NMR (400 MHz, DMSO-*d*₆): *δ* 8.25 (1H, d, *J=* 5.6 Hz), 7.64 (1H, d, , *J=* 6.8 Hz), 7.54 (1H, d, , *J =* 8.0 Hz), 7.22 (2H, s), 7.11 (1H, s), 7.06 (1H, d, , *J=* 6.0 Hz), 7.01 (1H, d, , *J* = 7.6 Hz), 2.05 (3H, s).

### (2) Preparation of the 2-bromo-5-(2-chloropyridin-4-yloxy)-4-(6-methylpyridin-2-yl)thiazole (16-9)

To a solution of CuBr₂ (3.01 g, 13.49 mmol) and isophentyl nitrite (2.72 mL, 20.23 mmol) in acetonitrile (100 mL) was added 16-8 (4.3 g, 13.49 mmol) in acetonitrile (170 mL) at 0 °C. The reaction mixture stirred at room temperature for 2 hours and then quenched with NH₄OH (100 mL) and extracted with EtOAc. The organic layer was dried over Na₂SO₄, filtered and concentrated in *vacuo.* The residue was purified by column chromatography on SiO₂ (Hexane:EtOAc = 5:1 to 3:1) to give the **16-9** (1.57 g, 30%) as a yellow solid.

¹H-NMR (400 MHz, DMSO-*d*₆): *δ* 8.28 (1H, d, *J* = 5.6 Hz), 7.75 -7.68 (2H, m), 7.30 (1H, *d, J* = 2.0 Hz), 7.16 (1H, dd, *J* = 5.6, 2.4 Hz), 7.12 (1H, d, *J* = 7.2 Hz), 2.08 (3H, s).

### (3) Preparation of the 5-(2-chloropyridin-4-yloxy)-4-(6-methylpyridin-2-yl)-2-vinylthiazole (17-1)

To a solution of **16-9** (1.57 g, 4.10 mmol) in THF (41 mL) was added tributyl(vinyl)stannane (1.91 mL, 6.56 mmol) at room temperature. The mixture was degassed with Ar and then added PdCl₂(PPh₃)₂ ( 576 mg, 0.82 mmol). The mixture was heated at 80 °C for 4 hours. After cooled to room temperature, the reaction mixture was diluted with water and extracted with EtOAc. The organic layer was dried over Na₂SO₄, filtered and concentrated in *vacuo.* The residue was purified by column chromatography on SiO₂ (Hexane:EtOAc = 7:1 to 5:1) to give the **17-1** (510 mg, 37%) as a yellow solid.

¹H-NMR (400 MHz, DMSO-*d*₆): *δ* 8.27 (1H, d, *J* = 6.0 Hz), 7.78-7.70 (2H, m), 7.23 (1H, d, *J* = 2.0 Hz), 7.10 (2H, d, *J* = 6.4 Hz), 6.10 (1H, d, *J* = 17.6 Hz), 5.70 (1H, d, *J* = 10.8 Hz), 2.10 (3H, s).

### (4) Preparation of the 5-(2-chloropyridin-4-yloxy)-2-ethyl-4-(6-methylpyridin-2-yl)thiazole (17-2)

A mixture of 17-1 and PtO₂ (70.0 mg, 0.31 mmol) in MeOH (18 mL) was stirred at room temperature for 3 hours under H₂ atmosphere (balloon). After filtration through a Celite pad, the filtrate was concentrated in *vacuo.* The residue was purified by column chromatography on SiO₂ (EA only) to give the **17-2** (456 mg, 89%) as a yellow solid.

¹H-NMR (400 MHz, DMSO-*d*₆): *δ* 8.26 (1H, d, *J=* 6.0 Hz), 7.76-7.68 (2H, m), 7.16 (1H, d, *J* = 1.6 Hz), 7.08 (1H, d, *J* = 7.2 Hz), 7.05 (1H, dd, *J* = 6.0, 2.4 Hz), 3.01 (2H, q, *J*= 7.6 Hz), 2.09 (3H, s), 1.34 (3H, t, *J=* 7.8 Hz).

### (5) Preparation of A-250

To a solution of **17-2** (456 mg, 1.37 mmol) in DMF (13 mL) was added 3-aminobenzenesulfonamide (355 mg, 2.06 mmol) and Cs₂CO₃ (1.3 g, 4.12 mmol) at room temperature. The mixture was degassed by purging and re-filled with Ar in several times. After addition of BrettPhos Pd G3 (374 mg, 0.41 mmol), the reaction mixture was heated at 80 °C for 2 hours. After cooled to room temperature, the reaction mixture was quenched with water and extracted EA. The organic layer was purified by column chromatography on NH-SiO₂ (Hexane:EA = 1:2 to 1:4) to give the **A-250** (151 mg, 23%) as a white solid.

The following **A-251** to **A-273,** were synthesized using the procedure described for the step 5 synthesis of **A-250.**

### 39. Synthesis of A-274

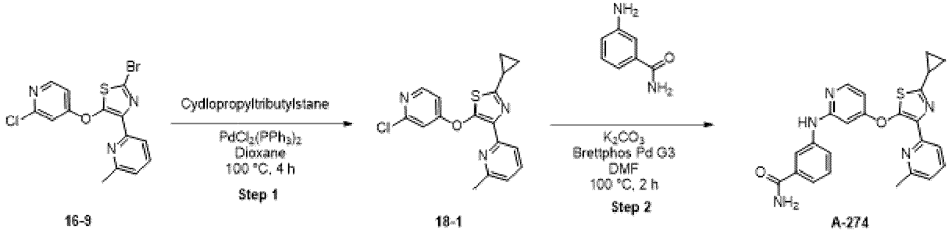

### (1) Preparation of 5-((2-chloropyridin-4-yl)oxy)-2-cyclopropyl-4-(6-methylpyridin-2-yl)thiazole (18-1)

To a solution of **16-9** (50 mg, 0.13 mmol) in Dioxane (3 mL) was added cyclopropyltributylstane (86.5 mg, 0.26 mmol), PdCl₂(PPh₃)₂ (18.3 mg, 0.03mmol) at room temperature. The mixture was heated at 100 °C for 4 hours. After cooled to room temperature, the reaction mixture was diluted with water and extracted with EtOAc. The organic layer was dried over Na2SO4, filtered and concentrated in *vacuo.* The residue was purified by column chromatography on SiO2 (Hexane:EtOAc = 3:1) to give the **18-1** (20.0 mg, 44.5%) as a yellow liquid.

MS Calcd.: 343.83; MS Found: 344.26 (M+H⁺).

### (2) Preparation ofA-274

To a solution of **18-1** (20.0 mg, 0.06 mmol) in DMF (1.0 mL) was added 3-aminobenzamide (10.0 mg, 0.07 mmol) and K₂CO₃ (24.1 mg, 0.18 mmol) at room temperature. The mixture was degassed by purging and re-filled with Ar in several times. After addition of Brettphos Pd G3 (21.0 mg, 0.02 mmol), the reaction mixture was heated at 100 °C for 2 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (DCM:MeOH = 10:1) to give the **A-274** (3.4 mg, 13.0%) as an ivory solid.

The following **A-275** to **A-282,** were synthesized using the procedure described for the synthesis of **A-274.**

### 40. Synthesis of A-283

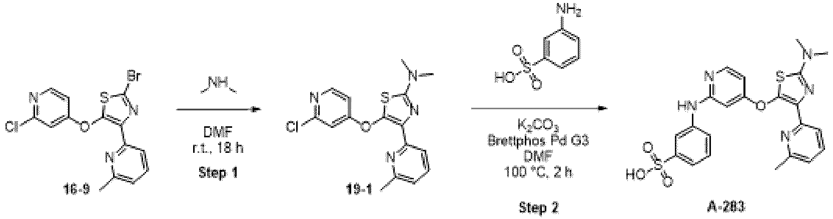

### (1) Preparation of 5-(2-chloropyridin-4-yloxy)-N,N-dimethyl-4-(6-methylpyridin-2-ylo)thiazol-2-amine (19-1)

To a solution of **16-9** (290 mg, 0.76 mmol) in DMF (3.0 mL) was added dimethylamine (2.0 M in THF, 0.57 mL, 1.14 mmol). The reaction mixture was stirred at room temperature for 18 hours. The reaction mixture was washed with water and extracted with EtOAc. The organic layer was dried over Na₂SO₄, filtered and concentrated in *vacuo* to afford the **19-1** (100 mg, 38%) as a yellow oil, which was used for the next step without further purification.

¹H-NMR (400 MHz, CDCl₃): *δ* 8.19 (1H, d, *J=* 5.6 Hz), 7.95 (1H, d, *J=* 6.0 Hz), 7.87 (1H, d, *J=* 7.6 Hz), 7.52 (1H, t, *J=* 8.0 Hz), 6.46 (1H, d, *J=* 2.4 Hz), 6.40-6.38 (1H, m), 3.11 (6H, s), 2.25 (3H, s).

### (2) Preparation ofA-283

To a solution of **19-1** (100 mg, 0.29 mmol) in DMF (3.0 mL) was added 3-aminobenzenesulfonamide (74.0 mg, 0.43 mmol) and K₂CO₃ (120 mg, 0.87 mmol) at room temperature. The mixture was degassed by purging and re-filled with argon several times. After addition of BrettPhos Pd G3 (52.0 mg, 0.06 mmol), the reaction mixture was heated at 100 °C for 2 hours. After being cooled to room temperature, the reaction mixture was quenched with water and extracted with EtOAc. The organic layers were dried over Na₂SO₄, filtered, and concentrated in *vacuo.* The residue was purified by column chromatography on NH-SiO₂ (DCM:MeOH = 98:2) to afford the **A-283** (55.4 mg, 40%) as an ivory solid.

The following **A-284** to **A-286,** were synthesized using the procedure described for the synthesis of **A-283.**

### 41. Synthesis of A-287

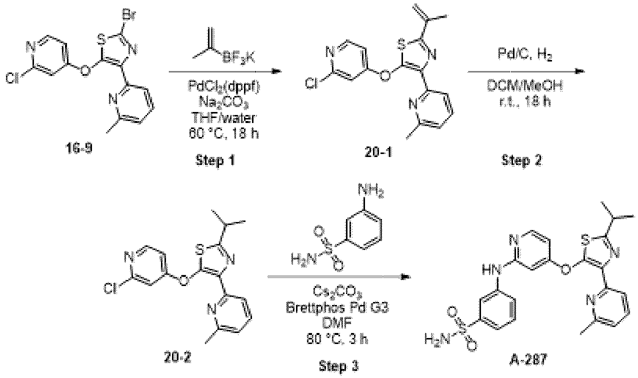

### (1) Preparation of the 5-(2-chloropyridin-4-yloxy)-4-(6-methylpyridin-2-yl)-2-(prop - 1-en-2yl)thioazole (20-1)

To a solution of **16-9** (400 mg, 1.05 mmol) in THF (5.2 mL) and water (5.2 mL) was added potassium isopropenyltrifluoroborate (232 mg, 1.57 mmol), and Na₂CO₃ (776 mg, 7.32 mmol) at room temperature. The mixture was degassed by purging and re-filled with Ar in several times. After addition of PdCl₂(dppf) (86.0 mg, 0.11 mmol), the reaction mixture was heated at 60 °C for 18 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 4:1) to give the **20-1** (133 mg, 37%) as a yellow oil.

¹H-NMR (400 MHz, DMSO-*d*₆): *δ* 8.27 (1H, d, *J=* 8.27 Hz), 7.80 (1H, d, *J=* 8.0 Hz), 7.73 (1H, t, *J=* 7.8 Hz), 7.22 (1H, s), 7.10 (2H, d, *J=* 7.6 Hz), 5.84 (1H, s), 5.50 (1H, s), 2.20 (3H, s), 2.09 (3H, s).

### (2) Preparation of the 5-(2-chloropyridin-4-yloxy)-2-isopropyl-4-(6-methylpyridin-2-yl) thiazole (20-2)

To a solution of **20-1** (173 mg, 0.503 mmol) in DCM (2.5 mL) and MeOH (2.5 mL) was added Pd/C (10 wt%, 54.0 mg, 0.05 mmol) at room temperature. The mixture was stirred at room temperature for 18 hours under H₂ atmosphere (balloon). After filtered through Celite pad, the filtrate was concentrated in *vacuo.* The residue was purified by column chromatography on SiO₂ (Heaxanes:EtOAc = 3:1) to afford the **20-2** (60.0 mg, 35%) as a colorless oil.

¹H-NMR (400 MHz, CDCl₃): *δ* 8.23 (1H, d, *J=* 5.6 Hz), 7.76 (1H, d, *J=* 7.6 Hz), 7.58 (1H, t, *J* = 7.6 Hz), 6.98-6.96 (2H, m), 6.90 (1H, dd, *J* = 5.8, 2.2 Hz), 3.31 (1H, sep., *J* = 6.8 Hz), 2.28 (3H, s), 1.45 (6H, d, *J=* 6.4 Hz).

### (3) Preparation ofA-287

To a solution of **20-2** (60.0 mg, 0.17 mmol) in DMF (3.5 mL) was added 3-aminobenzenesulfonamide (45.0 mg, 0.26 mmol), and Cs₂CO₃ (170 mg, 0.52 mmol) at room temperature. The mixture was degassed by purging and re-filled with Ar in several times. After addition of Brettphos Pd G3 (47.0 mg, 0.05 mmol), the reaction mixture was heated at 80 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on NH-SiO₂ (Hexane:EtOAc = 1:2) to give the **A-287** (22.2 mg, 27%) as a white solid.

The following **A-288** was synthesized using the procedure described for the synthesis of **A-287.**

### 42. Synthesis of A-289

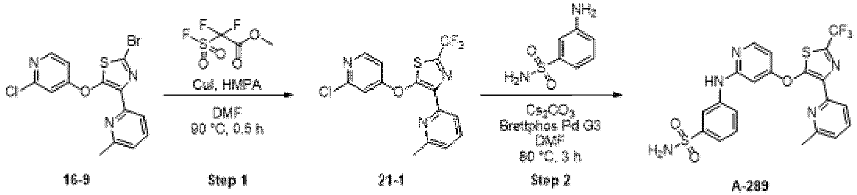

### (1) Preparation of the 5-(2-chloropyridin-4-yloxy)-4-(6-methylpyridin-2-yl)-2-(trifluoromethyl)thiazole (21-1)

To a solution of **16-9** (0.45 g, 1.18 mmol) in DMF (3 mL) was added CuI (403.1 mg, 2.12 mmol), HMPA (0.41 mL, 2.35 mmol), methyl 2,2-difluoro-2-(fluorosulfonyl)acetate (0.45 mL, 3.53 mmol) at room temperature. The mixture was heated at 90 °C for 0.5 hours. After cooled to room temperature, the reaction mixture was diluted with water and extracted with EtOAc. The organic layer was dried over Na₂SO₄, filtered and concentrated in *vacuo.* The residue was purified by column chromatography on SiO₂ (Hexane:EtOAc = 5:1) to give the **21-1** (50.0 mg, 11%) as a yellow liquid.

¹H-NMR (400 MHz, DMSO-*d*₆): *δ* 8.32 (1H, d, *J* = 6.0 Hz), 7.80 (2H, s), 7.43 (1H, d, *J* = 28.8 Hz), 7.21 (2H, dd, *J* = 20.2, 3.0 Hz), 2.14 (3H, s).

### (2) Preparation ofA-289

To a solution of **16-9** (38.0 mg, 0.10 mmol) in DMF (2.0 mL) was added 3-aminobenzenesulfonamide (26.0 mg, 0.15 mmol), and Cs₂CO₃ (100 mg, 0.31 mmol) at room temperature. The mixture was degassed by purging and re-filled with Ar in several times. After addition of BrettPhos Pd G3 (26.0 mg, 0.15 mmol), the reaction mixture was heated at 80 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on NH-SiO₂ (Hexane:EtOAc = 1:4) to give the **A-289** (12.0 mg, 23%) as a white solid.

The following **A-290** and **A-291** were synthesized using the procedure described for the synthesis of **A-289.**

### 43. Synthesis of A-292

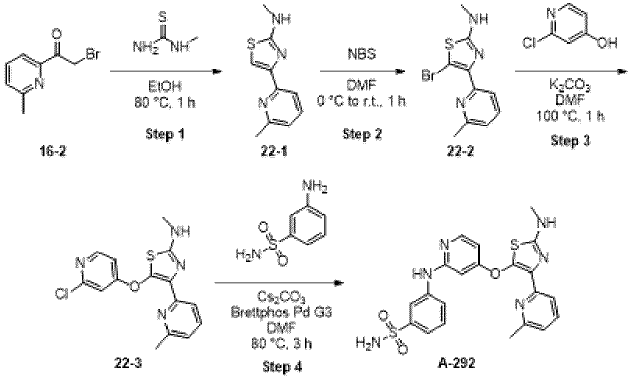

### (1) Preparation of the N-methyl-4-(6-methylpyridin-2-yl)thiazole-2-amine (22-1)

To a solution of **16-2** (2.00 g, 9.34 mmol) in EtOH (31 mL) was added 1-methylthiourea (842 mg, 9.34 mmol) at room temperature. The mixture was heated at 80 °C for 1 hour. After cooled to room temperature, the reaction mixture was concentrated in *vacuo* to give the **22-1** (1.70 g, 89%) as a yellow solid, which was used for next step without further purification.

¹H-NMR (400 MHz, CDCl₃): *δ* 7.66 (1H, d, *J=* 7.4 Hz), 7.61 (1H, t, *J* = 7.8 Hz), 7.26 (1H, d, *J* = 8.4 Hz), 7.05 (1H, d, *J* = 7.6 Hz), 3.00 (3H, s), 2.58 (3H, s). * A proton from NH was not observed.

### (2) Preparation of the 5-bromo-N-methyl-4-(6-methylpyridin-2-yl)thiazol-2-amine (22-2)

To a solution of **22-1** (2.00 g, 9.74 mmol) in DMF (49 mL) was added slowly NBS (1.73 g, 9.74 mmol) at 0 °C. The mixture was stirred at room temperature for 1 hour. The reaction mixture was quenched with sat. NaHCO₃ solution and extracted with EtOAc. The organic layer was dried over Na₂SO₄, filtered and concentrated in *vacuo.* The residue was triturated with diethyl ether and Hexane. The solid was filtered and dried to give the **22-2** (1.60 g, 58%) as a yellow solid.

¹H-NMR (400 MHz, CDCl₃): *δ* 7.68-7.55 (2H, m), 7.11 (1H, dd, *J=* 7.2, 1.6 Hz), 2.75 (3H, s), 2.64 (3H, s). * A proton from NH was not observed.

### (3) Preparation of the 5-(2-chloropyridin-4-yloxy)-N-methyl-4-(6-methylpyridin-2-yl) thiazol-2-amine (22-3)

A mixture **22-2** (1.00 g, 3.52 mmol), 2-chloropyridin-4-ol (501 mg, 3.87 mmol) and K₂CO₃ (501 mg, 3.87 mmol) in DMF (35 mL) was stirred at 100 °C for 1 hour. The reaction mixture was poured into water and extracted with EtOAc. The organic layer was dried over Na₂SO₄, filtered and concentrated in *vacuo.* The residue was purified by column chromatography on NH-SiO₂ (Hexane:EtOAc = 1:1) to give the **22-3** (542 mg, 46%) as a yellow solid.

MS Calcd.: 332.81; MS Found: 333.20 (M+H⁺).

### (4) Preparation of A-292

To a solution of **22-3** (250 mg, 0.75 mmol) in DMF (15 mL) was added 3-aminobenzenesulfonamide (194 mg, 1.13 mmol), and Cs₂CO₃ (734 mg, 2.25 mmol) at room temperature. The mixture was degassed by purging and re-filled with Ar in several times. After addition of BrettPhos Pd G3 (204 mg, 0.22 mmol), the reaction mixture was heated at 80 °C for 3 hours. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (DCM:MeOH = 10:1) to give the **A-292** (4.0 mg, 1%) as a pale yellow solid.

### 44. Synthesis of A-293 and A-294

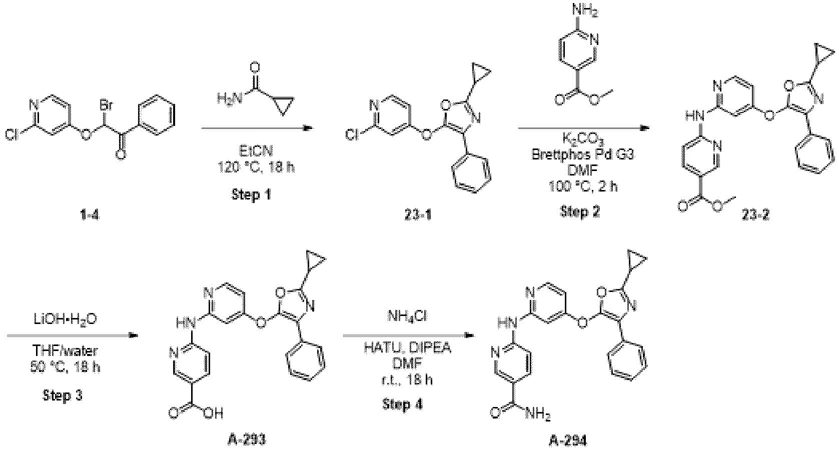

### (1) Preparation of 5-(2-chloropyridin-4-yloxy)-2-cyclopropyl-4-phenyloxazole (23-1)

To a solution of **1-4** (700 mg, 2.14 mmol) in EtCN (20 mL) was added cyclopropanecarboxamide (1.00 g, 11.8 mmol) at room temperature. The mixture was heated at 120 °C 18 hours. After cooled to room temperature, the reaction mixture was quenched with sat. NaHCO₃ solution and extracted with EtOAc. The organic layer was washed with brine, dried over Na₂SO₄, filtered and concentrated in *vacuo.* The residue was purified by column chromatography on SiO₂ (Hexane:EtOAc = 4:1) to give the 23-1 (180 mg, 27%) as a colorless oil.

¹H-NMR (400 MHz, CDCl₃): *δ* 8.32 (1H, d, *J =* 5.6 Hz), 7.67 (2H, d, *J =* 7.2 Hz), 7.36 (2H, t, *J =* 7.4 Hz), 7.28-7.25 (1H, m), 6.99 (1H, d, *J =* 2.0 Hz), 6.90 (1H, dd, *J =* 5.6, 2.0 Hz), 2.08-2.02 (1H, m), 1.14-1.05 (4H, m).

### (2) Preparation of methyl 6-(4-(2-cyclopropyl-4-phenyloxazol-5-yloxy)pyridin-2-ylamino) nicotinate (23-2)

To a solution of **23-1** (40.0 mg, 0.13 mmol) in DMF (5.0 mL) was added methyl 6-aminonicotinate (40.0 mg, 0.26 mmol) and K₂CO₃ (60.0 mg, 0.43 mmol) at room temperature. The mixture was degassed by purging and re-filled with Ar in several times. After addition of Brettphos Pd G3 (40.0 mg, 44.0 µmol), the reaction mixture was heated at 100 °C for 2 hours. After cooled to room temperature, the reaction mixture was quenched with water and extracted with EtOAc. The organic layer was dried over Na₂SO₄, filtered and concentrated in *vacuo.* The residue was purified by column chromatography on SiO₂ (Hexane:EtOAc = 4:1) to give the **23-2** (30.0 mg, 55%) as a yellow solid.

MS Calcd.: 428.15; MS Found: 429.2 (M+H⁺).

### (3) Preparation of A-293

To a solution of 23-2 (30.0 mg, 0.07 mmol) in THF (5.0 mL) and water (5.0 mL) was added LiOH·H₂O (50.0 mg, 2.09 mmol) at room temperature. The mixture was heated at 50 °C for 18 hours. After cooled to room temperature, the reaction mixture was concentrated in *vacuo.* The residue was acidified with 2 N aq. HCl solution. A precipitated solid was collected by filtration to afford the **A-293** (21.0 mg, 72%) as an ivory solid

### (4) Preparation of A-294

To a solution of **A-293** (15.0 mg, 36.0 µmol) in DMF (5.0 mL) was added HATU (30.0 mg, 79.0 µmol), NH₄Cl (50.0 mg, 0.94 mmol) and DIPEA (0.20 mL, 1.15 mmol) at room temperature. The mixture was stirred for 18 hours at room temperature. The reaction mixture was poured into water. The resulted solid was filtered and triturated with DCM and Hexane. The precipitated solid was collected by filtration and dried under vacuum to afford the **A-294** (4.0 mg, 19%) as a white solid.

The following **A-295** to **A-302** were synthesized using the procedure described for synthesis of the **A-293.**

### 45. Synthesis of A-303

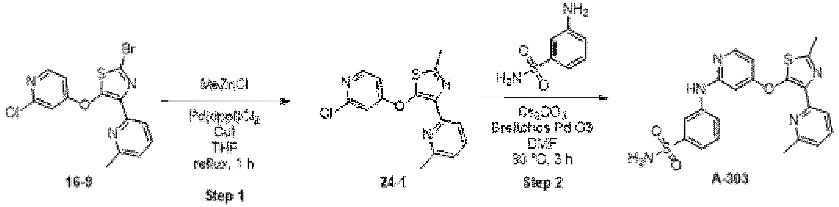

### (1) Preparation of the 5-(2-chloropyridin-4-yloxy)-2-methyl-4-(6-methylpyridin-2-yl) thiazole (24-1)

To a solution of **16-9** (200 mg, 0.52 mmol) in THF (5.2 mL) was added CuI (70.0 mg, 0.37 mmol) and Pd(dppf)Cl₂ (76.0 mg, 0.11 mmol) at room temperature. The mixture was degassed by purging and re-filled with Ar in several times. After addition of MeZnCl₂ (2 M in THF, 0.65 mL, 1.31 mmol), the reaction mixture was refluxed for 1 hour. After cooled to room temperature, the reaction mixture was quenched with water and extracted with EtOAc. The organic layer was dried over Na₂SO₄, filtered, and concentrated in *vacuo.* The residue was purified by column chromatography on SiO₂ (Hexane:EtOAc = 2:1) to give the **24-1** (90.0 mg, 54%) as a beige solid.

¹H-NMR (400 MHz, DMSO-*d*₆): *δ* 8.26 (1H, d, *J =* 5.8 Hz), 7.74-7.68 (2H, m), 7.16 (1H, d, *J =* 1.6 Hz), 7.08-7.04 (2H, m), 2.68 (3H, s), 2.08 (3H, s).

### (2) Preparation of A-303

To a solution of **24-1** (90.0 mg, 0.28 mmol) in DMF (5.7 mL) was added 3-aminobenzenesulfonamide (73.0 mg, 0.42 mmol), and Cs₂CO₃ (277 mg, 0.85 mmol) at room temperature. The mixture was degassed by purging and re-filled with Ar in several times. After addition of Brettphos Pd G3 (77.0 mg, 0.08 mmol), the reaction mixture was heated at 80 °C for 3 hours. After cooled to room temperature, the reaction mixture was quenched with water and extracted with EtOAc. The organic layer was dried over Na2SO4, filtered, and concentrated in *vacuo.* The residue was purified by column chromatography on SiO₂ (Hexane:EtOAc = 2:1) to give the **A-303** (5.0 mg, 4%) as a white solid.

The following **A-304** to **A-309** were synthesized using the procedure described for the synthesis of **A-303.**

The following **A-62** and **A-137** were synthesized using the procedure described for the synthesis of**A-1**.

The following **A-310** was synthesized using the procedure described for the synthesis of **A-50.**

The following **A-311** was synthesized using the procedure described for the synthesis of **A-191.**

The following **A-312** was synthesized using the procedure described for the synthesis of **A-193.**

### [Example 1] LanthaScreen Eu Kinase Binding Assay

4µL of 4X Test Compound in 4% DMSO and 4µL of 4X Kinase in Kinase buffer were added to wells (low volume, white 384-well plate (Greiner Cat. #784207)). The plate was shaken for 30 seconds, centrifuged for 10 seconds at 1000 rpm, and incubated at RT for 30 minutes. Then, 4µL of 4X Eu-anti-GST Antibody and 4µL of 4X Kinase Tracer 178 were added to the wells. The plate was shaken for 30 seconds, centrifuged for 10 seconds at 1000 rpm, and incubated at RT for 60 minutes. Fluorescence was measured by a plate reader, Biotek Synergy H1 (Vermont, USA) under the following conditions.
- Excitation: 340nm (30nm bandpass)
- Kinase tracer emission: 665nm (10nm bandpass)
- Eu Ab emission: 615nm (10nm bandpass)
- Emission ratio= 665nm(tracer)/615nm(Eu Ab)

The higher the binding affinity of compound to ALK5 kinase is, the less the emission ratio is.

**[Table 1]**

| IC50 | **< 100 nM (**)** | **> 100 nM (*)** |
|---|---|---|
| **Enzyme** | **A-1, A-2, A-3, A-4, A-5, A-9, A-10, A-14, A-39, A-40, A-41, A-42, A-43, A-44, A-45, A-46, A-48, A-49, A-50, A-51, A-52, A-53, A-54, A-55, A-56, A-57, A-58, A-59, A-60, A-61, A-63, A-64, A-65, A-66, A-68, A-69, A-70, A-71, A-91, A-92, A-93, A-104, A-105, A-107, A-109, A-110, A-111, A-112, A-113, A-114, A-115, A-116, A-117, A-118, A-119, A-120, A-121, A-122, A-124, A-125, A-126, A-127, A-128, A-129, A-132, A-133, A-134, A-135, A-136, A-138, A-139, A-140, A-141, A-142, A-143, A-144, A-145, A-146, A-147, A-148, A-149, A-150, A-151, A-152, A-153, A-154, A-155,A-156, A-157, A-158, A-159, A-160, A-161, A-163, A-164, A-165, A-166, A-167, A-168, A-169, A-170, A-171, A-174, A-175, A-181, A-182, A-183, A-184, A-185, A-186, A-187, A-188, A-189, A-190, A-192, A-193, A-194, A-195, A-220, A-221, A-223, A-224, A-225, A-226, A-227, A-228, A-229, A-230, A-231, A-232, A-238, A-247, A-248, A-249, A-250, A-251, A-252, A-253, A-255, A-256, A-257, A-258, A-259, A-260, A-261, A-262, A-263, A-264, A-265, A-266, A-267, A-268, A-269, A-270, A-271, A-274, A-275, A-276, A-277, A-278, A-279, A-280, A-283, A-284, A-285, A-286, A-287, A-288, A-289, A-290, A-291, A-292, A-293, A-294, A-295, A-303, A-304, A-309, A-310** | **A-47, A-72, A-172, A-173, A-176, A-177, A-178, A-179, A-180, A-217, A-218, A-219, A-222, A-233, A-234, A-235, A-254** |

### [Example 2] Assay for Evaluating Cellular Inhibition of TGF-β Signaling

4T1 (SBE-*luc2P*) transient pool that have SBE-*luc*2P (hygro) expression plasmid or HEK293 (SBE reporter) stable cells (BPS bioscience) were seeded at 6×10³ cells/well or 2.5×10⁴ cells/well in 96-well plate, respectively. 4T1 (SBE-*luc*2P) transient pool were pre-treated with novel compound synthesized in Example 1 above in 0.2% FBS at approximately 60% confluence at 37°C in 5% CO₂. Also, HEK293 (SBE reporter) stable cells were pre-treated with each test compounds of formula in 0.5% FBS at approximately 80% confluence at 37°C in 5% CO₂. After 2 hours, cells were treated with TGF-β1 (2ng/mL) for 24 h at 37°C in 5% CO₂. Cell lysates were prepared using Luciferase Assay (Promega) according to the manufacturer's instruction, and luminescence was measured by a plate reader, Biotek Synergy H1 (Vermont, USA). The results are shown in Table 2.

**[Table 2]**

| IC50 | **< 100 nM (**)** | **> 100 nM (*)** |
|---|---|---|
| **4T1 Cell** | **A-1, A-2, A-9, A-12, A-13, A-14, A-15, A-16, A-19, A-20, A-22, A-23, A-24, A-29, A-33, A-34, A-36, A-51, A-58, A-60, A-61, A-66, A-67, A-69, A-70, A-71, A-73, A-74, A-75, A-76, A-77, A-78, A-79, A-88, A-89, A-90, A-92, A-93, A-99, A-100, A-101, A-49, 104, A-105, A-106, A-107, A-108, A-111, A-112, A-113, A-114, A-116, A-117, A-119, A-122, A-123, A-124, A-125, A-126, A-127, A-128, A-129, A-130, A-132, A-95, 133, A-134, A-135, A-136, A-138, A-146, A-147, A-148, A-149, A-150, A-152, A-155, A-157, A-160, A-161, A-162, A-163, A-165, A-166, A-168, A-181, A-183, A-186, A-188, A-189, A-190, A-191, A-192, A-193, A-195, A-225, A-227, A-228, A-231, A-232, A-238, A-239, A-249, A-250, A-251, A-252, A-253, A-254, A-255, A-256, A-257, A-258, A-259, A-261, A-262, A-265, A-267, A-268, A-274, A-275, A-283, A-284, A-285, A-286, A-287, A-288, A-289, A-290, A-291, A-292, A-303, A-304, A-306, A-307, A-308, A-309, A-311** | **A-4, A-5, A-6, A-7, A-8, A-10, A-11, A-17, A-18, A-21, A-25, A-26, A-27, A-28, A-30, A-31, A-32, A-35, A-37, A-38, A-39, A-40, A-41, A-42, A-43, A-44, A-45, A-6, A-47, A-48, A-A-50, A-52, A-3, A-4, A-5, A-56, A-59, A-62, A-63, A-64, A-65, A-68, A-72, A-80, A-81, A-82, A-83, A-5, A-6, A-87, A-91, A-94, A-A-96, A-97, A-98, A-102, A-103, A-109, A-110, A-115, A-118, A-120, A-121, A-131, A-137, A-139, A-140, A-141, A-142, A-143, A-144, A-145, A-151, A-153, A-154, A-156, A-158, A-159, A-164, A-167, A-169, A-170, A-171, A-172, A-17, A-174, A-175, A-176, A-177, A-178, A-179, A-180, A-182, A-184, A-185, A-187, A-194, A-216, A-217, A-218, A-219, A-220, A-221, A-222, A-223, A-224, A-226, A-229, A-230, A-233, A-234, A-235, A-236, A-237, A-248, A-249, A-260, A-263, A-266, A-305, A-310** |
| **HEK 293 Cell** | **A-196, A-197, A-198, A-199, A-200, A-201, A-202, A-203, A-204, A-205, A-206, A-207, A-208, A-209, A-210, A-211, A-212, A-213, A-214, A-215, A-240, A-241, A-243, A-246, A-264, A-269, A-270, A-271, A-272, A-276, A-277, A-278, A-279, A-280, A-281, A-282, A-293, A-294, A-295, A-296, A-297, A-298, A-299, A-300, A-301, A-302, A-312** | **A-242, A-244, A-245, A-273** |

The foregoing description of the present disclosure is for illustrative purposes only, and those skilled in the art to which the present disclosure pertains will appreciate that the present disclosure may be easily modified into other specific forms without altering the technical spirit or essential features of the present disclosure. Therefore, it should be understood that the example embodiments described above are illustrative in all respects and not limited.

The scope of the present disclosure is indicated by the appended claims, and all modified or changed forms derived from the meaning and scope of the claims and their equivalent should be construed as being included in the scope of the present disclosure.

## Claims

1. A compound selected from a thiazole derivative compound represented by the following Chemical Formula 1, a pharmaceutically acceptable salt thereof, a solvate thereof, or a stereoisomer thereof: 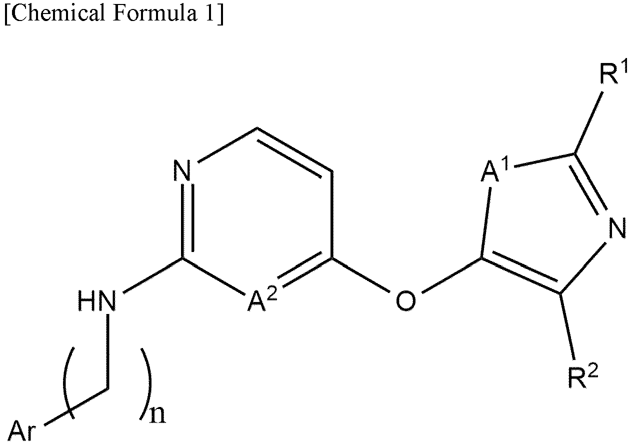
wherein, in the Chemical Formula 1,
A¹ is O or S,
A² is CH or N,
R¹ and R² are the same or different and are each one of (C1-C4)alkyl, (C1-C4)alkylene, (C1-C4)alkoxy, trifluoromethyl (CF₃), halo, NR¹¹R¹², unsubstituted or substituted (C3-C8)cycloalkyl, unsubstituted or substituted (C5-C10)aryl, and unsubstituted or substituted heteroaryl of 5 to 10 atoms including one or more heteroatoms selected from the group consisting of N, O, and S, the R¹¹ and R¹² are the same or different and are each any one of hydrogen, (C1-C5)alkyl, and (C3-C8)cycloalkyl, the substituted (C3-C8)cycloalkyl, substituted (C5-C10)aryl, or substituted heteroaryl of 5 to 10 atoms is substituted with (C1-C5)alkyl,
n is one of 0 to 3,
Ar is unsubstituted or substituted (C5-C10)aryl or unsubstituted or substituted heteroaryl of 5 to 10 atoms including one or more heteroatoms selected from the group consisting of N, O, and S, the substituted (C5-C10)aryl or substituted heteroaryl of 5 to 10 atoms is substituted with R³,
the R³ is -R³⁻¹ or -(C1-C5)alkyl-R³⁻¹,
the R³⁻¹ is any one of hydrogen, halogen, hydroxy, cyano, (C1-C5)alkyl, (C1-C5)alkoxy, -CONR³¹⁻¹R³¹⁻², -COOR³², -NHSO₂R³³, -SO₂R³⁴, -SO₂NR³⁵⁻¹R³⁵⁻², -COR₃⁶, -NH-(C1-C3)alkyl-R³⁷, 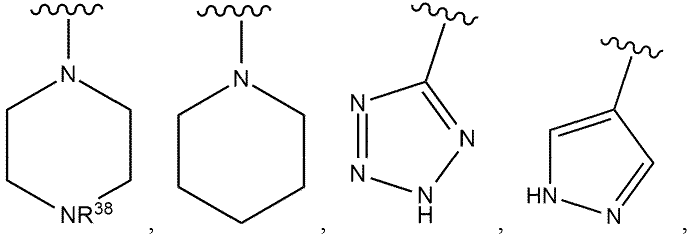
and 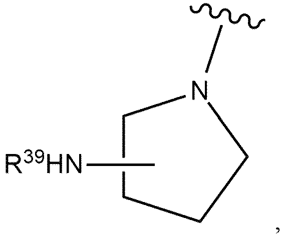
the R³¹⁻¹, R³¹⁻², R³², R³³, R³⁴, R³⁵⁻¹, and R³⁵⁻² are the same or different and are each hydrogen, hydroxy, trifluoromethyl, (C1-C5)alkyl, (C3-C8)cycloalkyl, -SO₂-(C1-C3)alkyl, - (C1-C3)alkyl-R³⁰¹, and 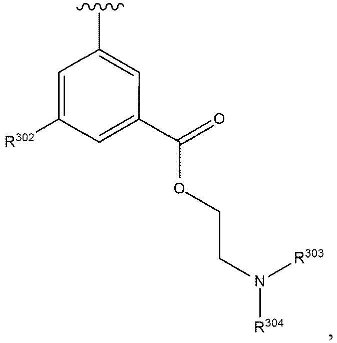
the R³⁰¹ is any one of (C1-C4)alkylamino, di[(C1~C4)alkyl]amino, (C3-C8)cycloalkyl, 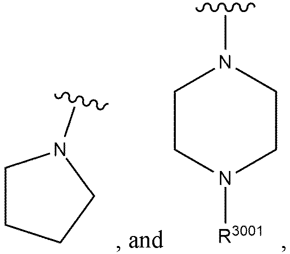
the R³⁰⁰¹ is hydrogen or (C1-CS)alkyl,
the R³⁰² is halogen,
the R³⁰³ and R³⁰⁴ are the same or different and are each hydrogen or (C1-C3)alkyl,
the R³⁶ and R³⁷ are the same or different and are each 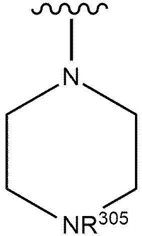 or 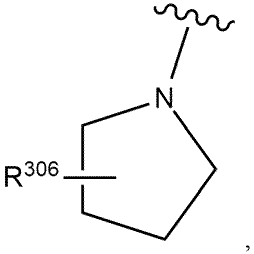
the R³⁰⁵ is hydrogen or (C1-C5)alkyl,
the R³⁰⁶ is any one of hydrogen, amino, (C1~C4)alkylamino, and di[(C1~C4)alkyl]amino, and
the R³⁸ and R³⁹ are the same or different and are each any one of hydrogen, (C1-C3)alkyl, and -(C1-C3)alkyl-(C1-C3)alkoxy.

2. The compound of claim 1, wherein, in the Chemical Formula 1,
R¹ is any one of (C1-C3)alkyl, (C1-C3)alkylene, methoxy, trifluoromethyl (CF₃), bromine (Br), (C3-C6)cycloalkyl, and NR¹¹R¹², the R¹¹ and R¹² are the same or different and are each any one of the hydrogen, (C1-C3)alkyl, and cyclopropyl,
R² is 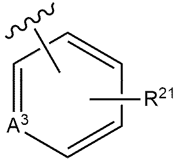 or naphthyl, the A³ is CH or N, the R²¹ is hydrogen or methyl,
n is one of 0 to 2,
Ar is any one of 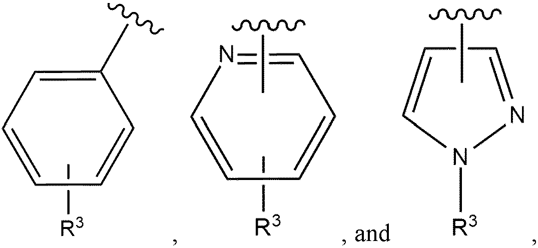 the R³ is -R³⁻¹ or -(C1-C3)alkyl-R³⁻¹, the R³⁻¹ is any one of hydrogen, fluorine, chlorine, hydroxy, cyano, (C1-C3)alkyl, (C1-C3)alkoxy, -CONR³¹⁻¹R³¹⁻², -COOR³², -NHSO₂R³³, -SO₂R³⁴, -SO₂NR³¹⁻¹R³⁵⁻², -COR₃⁶, -NH-(C1-C3)alkyl-R³⁷, 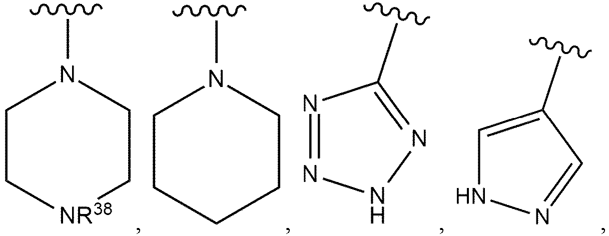 and 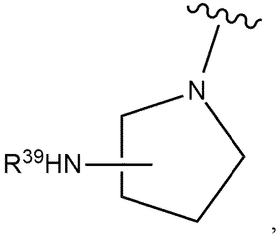
the R³¹⁻¹, R³¹⁻², R³², R³³, R³⁴, R³⁵⁻¹, and R³⁵⁻² are the same or different and are each hydrogen, hydroxy, trifluoromethyl, (C1-C5)alkyl, (C3-C8)cycloalkyl, -SO₂-(C1-C3)alkyl, -(C1-C3)alkyl-R³⁰¹, and 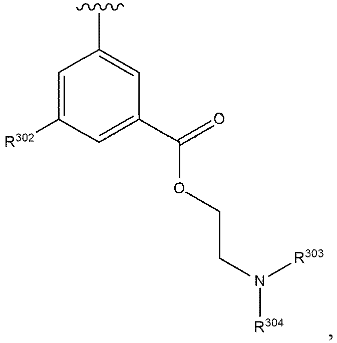 the R³⁰¹ is any one of methylamino, dimethylamino, cyclopropyl, 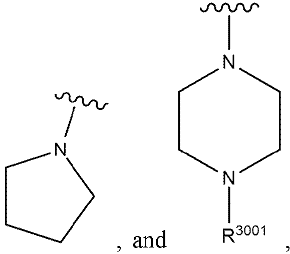
the R³⁰⁰¹ is hydrogen or (C1-C5)alkyl, the R³⁰² is fluorine, the R³⁰³ and R³⁰⁴ are the same or different and are each hydrogen or methyl, the R³⁶ and R³⁷ are the same or different and are each 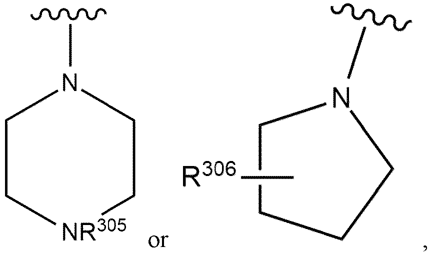 the R³⁰⁵ is (C1-C3)alkyl, the R³⁰⁶ is any one of hydrogen, amino, and methylamino, and the R³⁸ and R³⁹ are the same or different and are each any one of hydrogen, (C1-C3)alkyl, and -(C1-C3)alkyl-methoxy.

3. The compound of claim 1, wherein, in the Chemical Formula 1,
R¹ is any one of methyl, ethyl, isopropyl, methoxy, ethylene, trifluoromethyl (CF₃), bromine (Br), cyclopropyl, cyclopentyl, cyclohexyl, and NR¹¹R¹², the R¹¹ and R¹² are the same or different and are each any one of hydrogen, methyl, and cyclopropyl,
R² is one of 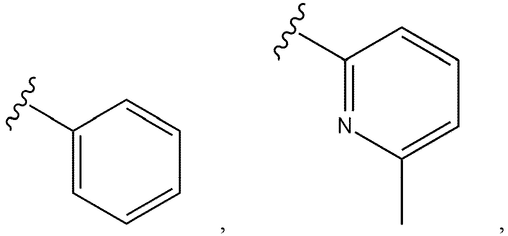
and 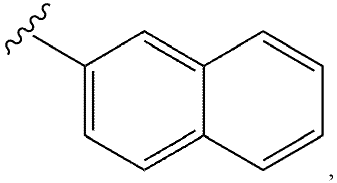
n is one of 0 to 2,
Ar is any one of 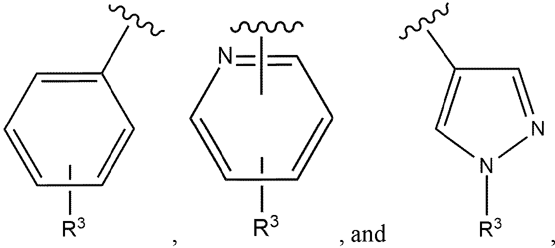 the R³ is -R³⁻¹ or -(C1-C3)alkyl-R³⁻¹, the R³⁻¹ is any one of hydrogen, fluorine, chlorine, hydroxy, cyano, methyl, methoxy, -CONR³¹⁻¹R³¹⁻², -COOR³², -NHSO₂R³³, -SO₂R³⁴, -SO₂NR³⁵⁻¹R³⁵⁻², -COR₃⁶,
-NH-(C1-C2)alkyl-R³⁷, 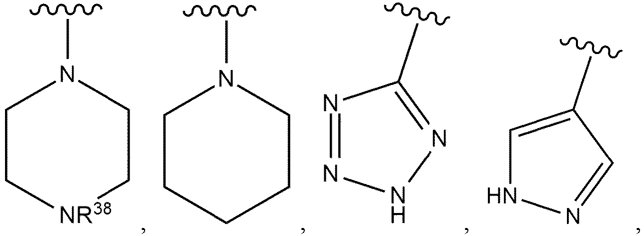 and 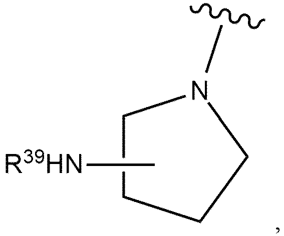
the R³¹⁻¹ and R³¹⁻² are the same or different and are each hydrogen, methyl, isopropyl, hydroxy, -SO₂CH₃, -(C1-C2)alkyl-R³⁰¹, and 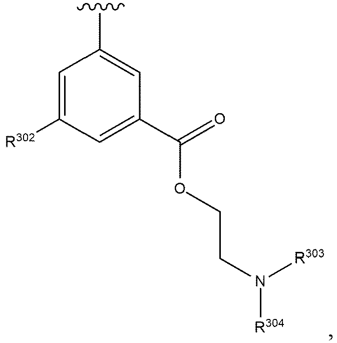 the R³⁰¹ is any one of dimethylamino, cyclopropyl, 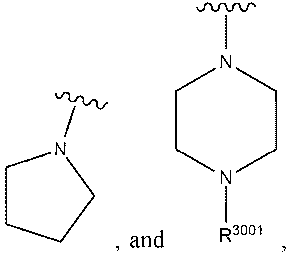 the R³⁰⁰¹ is one of methyl, ethyl, and isopropyl, the R³⁰² is fluorine, the R³⁰³ and R³⁰⁴ are methyl,
the R³² is one of hydrogen, methyl, and -(C1-C2)alkyl-dimethylamino,
the R³³ is one of methyl, ethyl, cyclopropyl, and trifluoromethyl,
the R³⁴ is one of hydroxy, methyl, ethyl, cyclopropyl, and trifluoromethyl,
the R³⁵⁻¹ and R³⁵⁻² are the same or different and are each one of hydrogen, methyl, and cyclopropyl,
the R³⁶ is 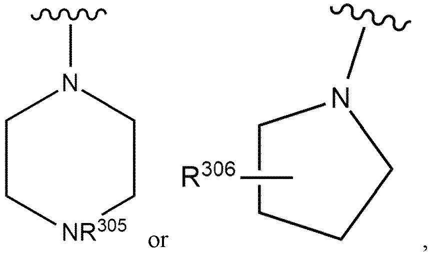 the R³⁰⁵ is methyl, the R³⁰⁶ is amino or methylamino,
the R³⁷ is 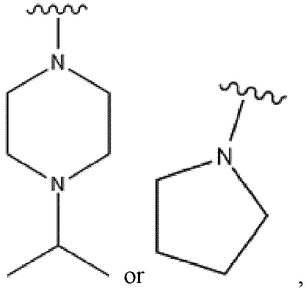
the R³⁸ is methyl or -CH₂CH₂OCH₃, and
the R³⁹ is hydrogen or methyl.

4. The thiazole derivative compound of claim 1, a stereoisomer thereof, a hydrate thereof, or a salt thereof, wherein the compound represented by Chemical Formula 1 is any one selected from the following group of compounds:
(1) 3-((4-((2-Amino-4-phenylthiazol-5-yl)oxy)pyridin-2-yl)amino)benzenesulfonamide
(2) 3-((4-((2-Amino-4-phenylthiazol-5-yl)oxy)pyridin-2-yl)amino)benzamide
(3) Methyl 3-((4-((2-amino-4-phenylthiazol-5-yl)oxy)pyridin-2-yl)amino)benzoate
(4) 4-((4-((2-Amino-4-phenylthiazol-5-yl)oxy)pyridin-2-yl)amino)benzenesulfonamide
(5) 4-((4-((2-Amino-4-phenylthiazol-5-yl)oxy)pyridin-2-yl)amino)benzamide
(6) 4-Phenyl-5-((2-(pyridin-3-ylamino)pyridin-4-yl)oxy)thiazol-2-amine
(7) 5-((2-((6-Fluoropyridin-3-yl)amino)pyridin-4-yl)oxy)-4-phenylthiazol-2-amine
(8) 4-Phenyl-5-((2-(pyridin-4-ylamino)pyridin-4-yl)oxy)thiazol-2-amine
(9) 6-((4-((2-Amino-4-phenylthiazol-5-yl)oxy)pyridin-2-yl)amino)nicotinamide
(10) 3-((4-((2-amino-4-phenylthiazol-5-yl)oxy)pyridin-2-yl)amino)-*N*,*N-*dimethylbenzamide
(11) 5-((4-((2-Amino-4-phenylthiazol-5-yl)oxy)pyridin-2-yl)amino)picolinamide
(12) 3-((4-((2-Amino-4-phenylthiazol-5-yl)oxy)pyridin-2-yl)amino)-*N-*methylbenzenesulfonamide
(13) 3-((4-((2-Amino-4-phenylthiazol-5-yl)oxy)pyridin-2-yl)amino)-*N*,*N-*dimethylbenzenesulfonamide
(14) *N*-(3-((4-((2-Amino-4-phenylthiazol-5-yl)oxy)pyridin-2-yl)amino)phenyl)methanesulfonamide
(15) 5-((2-((3-(Methylsulfonyl)phenyl)amino)pyridin-4-yl)oxy)-4-phenylthiazol-2-amine
(16) *N*-(3-((4-((2-Amino-4-phenylthiazol-5-yl)oxy)pyridin-2-yl)amino)phenyl)cyclopropanesulfonamide
(17) 4-Phenyl-5-((2-((3-((trifluoromethyl)sulfonyl)phenyl)amino)pyridin-4-yl)oxy)thiazol-2-amine
(18) 3-((4-((2-Amino-4-phenylthiazol-5-yl)oxy)pyridin-2-yl)amino)benzenesulfonic acid
(19) 5-((2-((3-((Methylsulfonyl)methyl)phenyl)amino)pyridin-4-yl)oxy)-4-phenylthiazol-2-amine
(20) 3-((4-((2-Amino-4-phenylthiazol-5-yl)oxy)pyridin-2-yl)amino)-*N-*cyclopropylbenzenesulfonamide
(21) 5-((2-((3-Fluorobenzyl)amino)pyridin-4-yl)oxy)-4-phenylthiazol-2-amine
(22) 2-((4-((2-Amino-4-phenylthiazol-5-yl)oxy)pyridin-2-yl)amino)isonicotinamide
(23) 3-((4-((2-Amino-4-phenylthiazol-5-yl)oxy)pyridin-2-yl)amino)-*N-*methylbenzamide
(24) 5-((2-((2-Methoxypyridin-4-yl)amino)pyridin-4-yl)oxy)-4-phenylthiazol-2-amine
(25) 5-((2-(Benzylamino)pyridin-4-yl)oxy)-4-phenylthiazol-2-amine
(26) 4-Phenyl-5-((2-((pyridin-4-ylmethyl)amino)pyridin-4-yl)oxy)thiazol-2-amine
(27) 5-((2-((2-Chloropyridin-4-yl)amino)pyridin-4-yl)oxy)-4-phenylthiazol-2-amine
(28) 4-Phenyl-5-((2-((pyridin-3-ylmethyl)amino)pyridin-4-yl)oxy)thiazol-2-amine
(29) 3-((4-((2-Amino-4-phenylthiazol-5-yl)oxy)pyridin-2-yl)amino)phenyl)methanesulfonamide
(30) 5-((2-((2-Fluoropyridin-4-yl)amino)pyridin-4-yl)oxy)-4-phenylthiazol-2-amine
(31) 5-((2-((6-Methoxypyridin-3-yl)amino)pyridin-4-yl)oxy)-4-phenylthiazol-2-amine
(32) 5-((4-((2-Amino-4-phenylthiazol-5-yl)oxy)pyridin-2-yl)amino)nicotinamide
(33) 6-((4-((2-Amino-4-phenylthiazol-5-yl)oxy)pyridin-2-yl)amino)picolinamide
(34) 5-((2-((3-(Ethylsulfonyl)phenyl)amino)pyridin-4-yl)oxy)-4-phenylthiazol-2-amine
(35) 5-((2-((6-Chloropyridin-3-yl)amino)pyridin-4-yl)oxy)-4-phenylthiazol-2-amine
(36) *N*-(3-((4-((2-Amino-4-phenylthiazol-5-yl)oxy)pyridin-2-yl)amino)phenyl)-1,1,1-trifluoromethanesulfonamide
(37) 5-((2-((1-Methyl-1*H*-pyrazol-4-yl)amino)pyridin-4-yl)oxy)-4-phenylthiazol-2-amine
(38) 5-((2-((1*H*-Pyrazol-4-yl)amino)pyridin-4-yl)oxy)-4-phenylthiazol-2-amine
(39) 4-((4-((2-amino-4-phenylthiazol-5-yl)oxy)pyridin-2-yl)amino)benzoic acid
(40) 3-((4-((2-Amino-4-phenylthiazol-5-yl)oxy)pyridin-2-yl)amino)benzoic acid
(41) 2-(3-((4-((2-amino-4-phenylthiazol-5-yl)oxy)pyridin-2-yl)amino)phenyl)propan-2-ol
(42) 2-(4-((4-((2-Amino-4-phenylthiazol-5-yl)oxy)pyridin-2-yl)amino)pyridin-2-yl)propan-2-ol
(43) 2-(5-((4-((2-Amino-4-phenylthiazol-5-yl)oxy)pyridin-2-yl)amino)pyridin-2-yl)propan-2-ol
(44) 2-(3-((4-((2-(Cyclopropylamino)-4-phenylthiazol-5-yl)oxy)pyridin-2-yl)amino)phenyl)propan-2-ol
(45) 3-((4-((2-(Cyclopropylamino)-4-phenylthiazol-5-yl)oxy)pyridin-2-yl)amino)benzenesulfonamide
(46) 3-((4-((2-(Cyclopropylamino)-4-phenylthiazol-5-yl)oxy)pyridin-2-yl)amino)benzamide
(47) 4-((4-((2-(Cyclopropylamino)-4-phenylthiazol-5-yl)oxy)pyridin-2-yl)amino)benzamide
(48) 3-((4-((2-(Cyclopropylamino)-4-phenylthiazol-5-yl)oxy)pyridin-2-yl)amino)benzoic acid
(49) 4-((4-((2-(Cyclopropylamino)-4-phenylthiazol-5-yl)oxy)pyridin-2-yl)amino)benzoic acid
(50) 2-(3-((4-((2-(Methylamino)-4-phenylthiazol-5-yl)oxy)pyridin-2-yl)amino)phenyl)propan-2-ol
(51) 3-((4-((2-(Methylamino)-4-phenylthiazol-5-yl)oxy)pyridin-2-yl)amino)benzenesulfonamide
(52) 3-((4-((2-(Methylamino)-4-phenylthiazol-5-yl)oxy)pyridin-2-yl)amino)benzamide
(53) 4-((4-((2-(Methylamino)-4-phenylthiazol-5-yl)oxy)pyridin-2-yl)amino)benzenesulfonamide
(54) 2-(4-((4-((2-(Methylamino)-4-phenylthiazol-5-yl)oxy)pyridin-2-yl)amino)pyridin-2-yl)propan-2-ol
(55) 2-(5-((4-((2-(Methylamino)-4-phenylthiazol-5-yl)oxy)pyridin-2-yl)amino)pyridin-2-yl)propan-2-ol
(56) 4-((4-((2-(Methylamino)-4-phenylthiazol-5-yl)oxy)pyridin-2-yl)amino)benzoic acid
(57) 3-((4-((2-(Methylamino)-4-phenylthiazol-5-yl)oxy)pyridin-2-yl)amino)benzoic acid
(58) 3-((4-((2-Methyl-4-phenylthiazol-5-yl)oxy)pyridin-2-yl)amino)benzenesulfonamide
(59) 2-(4-((4-((2-Methyl-4-phenylthiazol-5-yl)oxy)pyridin-2-yl)amino)pyridin-2-yl)propan-2-ol
(60) 3-((4-((2-Methyl-4-phenylthiazol-5-yl)oxy)pyridin-2-yl)amino)benzamide
(61) 2-(3-((4-((2-Methyl-4-phenylthiazol-5-yl)oxy)pyridin-2-yl)amino)phenyl)propan-2-ol
(62) 5-((2-((1-(Methylsulfonyl)-1*H*-pyrazol-4-yl)amino)pyridin-4-yl)oxy)-4-phenylthiazol-2-amine
(63) 4-((4-((2-Methyl-4-phenylthiazol-5-yl)oxy)pyridin-2-yl)amino)benzamide
(64) 4-((4-((2-Methyl-4-phenylthiazol-5-yl)oxy)pyridin-2-yl)amino)benzenesulfonamide
(65) 2-(5-((4-((2-Methyl-4-phenylthiazol-5-yl)oxy)pyridin-2-yl)amino)pyridin-2-yl)propan-2-ol
(66) 2-Methyl-2-(3-((4-((2-methyl-4-phenylthiazol-5-yl)oxy)pyridin-2-yl)amino)phenyl)propanoic acid
(67) *N*,*N*-Dimethyl-3-((4-((2-methyl-4-phenylthiazol-5-yl)oxy)pyridin-2-yl)amino)benzamide
(68) 3-((4-((2-Methyl-4-phenylthiazol-5-yl)oxy)pyridin-2-yl)amino)benzenesulfonic acid
(69) *N*-Methyl-3-((4-((2-methyl-4-phenylthiazol-5-yl)oxy)pyridin-2-yl)amino)benzamide
(70) 4-((2-Methyl-4-phenylthiazol-5-yl)oxy)-*N*-(3-(methylsulfonyl)phenyl)pyridin-2-amine
(71) *N-*(3-((4-((2-Methyl-4-phenylthiazol-5-yl)oxy)pyridin-2-yl)amino)phenyl)methanesulfonamide
(72) 4-((2-Methyl-4-phenylthiazol-5-yl)oxy)-*N*-(3-((trifluoromethyl)sulfonyl)phenyl)pyridin-2-amine
(73) *N*-Methyl-3-((4-((2-methyl-4-phenylthiazol-5-yl)oxy)pyridin-2-yl)amino)benzenesulfonamide
(74) *N*,*N*-Dimethyl-3-((4-((2-methyl-4-phenylthiazol-5-yl)oxy)pyridin-2-yl)amino)benzenesulfonamide
(75) *N*-(3-((4-((2-Methyl-4-phenylthiazol-5-yl)oxy)pyridin-2-yl)amino)phenyl)cyclopropanesulfonamide
(76) *N*-Cyclopropyl-3-((4-((2-methyl-4-phenylthiazol-5-yl)oxy)pyridin-2-yl)amino)benzenesulfonamide
(77) *N*-(3-(Ethylsulfonyl)phenyl)-4-((2-methyl-4-phenylthiazol-5-yl-yl)oxy)pyridin-2-amine
(78) 3-((4-((2-Methyl-4-phenylthiazol-5-yl)oxy)pyridin-2-yl)amino)phenyl)methanesulfonamide
(79) 4-((2-Methyl-4-phenylthiazol-5-yl)oxy)-*N*-(3-((methylsulfonyl)methyl)phenyl)pyridin-2-amine
(80) 4-((2-Methyl-4-phenylthiazol-5-yl)oxy)-*N*-(pyridin-4-ylmethyl)pyridin-2-amine
(81) 4-((2-Methyl-4-phenylthiazol-5-yl)oxy)-*N*-(pyridin-3-ylmethyl)pyridin-2-amine
(82) 4-((2-Methyl-4-phenylthiazol-5-yl)oxy)-*N*-(pyridin-3-yl)pyridin-2-amine
(83) *N*-(6-Fluoropyridin-3-yl)-4-((2-methyl-4-phenylthiazol-5-yl)oxy)pyridin-2-amine
(84) *N*-(6-Chloropyridin-3-yl)-4-((2-methyl-4-phenylthiazol-5-yl)oxy)pyridin-2-amine
(85) *N*-(6-Methoxypyridin-3-yl)-4-((2-methyl-4-phenylthiazol-5-yl)oxy)pyridin-2-amine
(86) 4-((2-Methyl-4-phenylthiazol-5-yl)oxy)-*N*-(pyridin-4-yl)pyridin-2-amine
(87) *N*-(2-Fluoropyridin-4-yl)-4-((2-methyl-4-phenylthiazol-5-yl)oxy)pyridin-2-amine
(88) *N*-(2-Methoxypyridin-4-yl)-4-((2-methyl-4-phenylthiazol-5-yl)oxy)pyridin-2-amine
(89) *N-*(2-Chloropyridin-4-yl)-4-((2-methyl-4-phenylthiazol-5-yl)oxy)pyridin-2-amine
(90) 6-((4-((2-Methyl-4-phenylthiazol-5-yl)oxy)pyridin-2-yl)amino)nicotinamide
(91) 5-((4-((2-Methyl-4-phenylthiazol-5-yl)oxy)pyridin-2-yl)amino)picolinamide
(92) 5-((4-((2-Methyl-4-phenylthiazol-5-yl)oxy)pyridin-2-yl)amino)nicotinamide
(93) 2-((4-((2-Methyl-4-phenylthiazol-5-yl)oxy)pyridin-2-yl)amino)isonicotinamide
(94)*N*-Benzyl-4-((2-methyl-4-phenylthiazol-5-yl)oxy)pyridin-2-amine
(95) *N*-(4-Fluorobenzyl)-4-((2-methyl-4-phenylthiazol-5-yl)oxy)pyridin-2-amine
(96) *N*-(3-Fluorobenzyl)-4-((2-methyl-4-phenylthiazol-5-yl)oxy)pyridin-2-amine
(97) 4-((2-Methyl-4-phenylthiazol-5-yl)oxy)-*N*-(1-(methylsulfonyl)-1*H*-pyrazol-4-yl)pyridin-2-amine
(98) *N*-(1-(Cyclopropylsulfonyl)-1*H*-pyrazol-4-yl)-4-((2-methyl-4-phenylthiazol-5-yl)oxy)pyridin-2-amine
(99) *N*-(1-Methyl-1*H*-pyrazol-4-yl)-4-((2-methyl-4-phenylthiazol-5-yl)oxy)pyridin-2-amine
(100) 4-((2-Methyl-4-phenylthiazol-5-yl)oxy)-*N*-(1*H*-pyrazol-4-yl)pyridin-2-amine
(101) 6-((4-((2-Methyl-4-phenylthiazol-5-yl)oxy)pyridin-2-yl)amino)picolinamide
(102) 4-((4-((2-Methyl-4-phenylthiazol-5-yl)oxy)pyridin-2-yl)amino)picolinamide
(103) *N*-(4-Methoxyphenyl)-4-((2-methyl-4-phenylthiazol-5-yl)oxy)pyridin-2-amine
(104) 4-((2-Methyl-4-phenylthiazol-5-yl)oxy)-*N*-(4-(piperazin-1-yl)phenyl)pyridin-2-amine
(105) 4-((2-Methyl-4-phenylthiazol-5-yl)oxy)-*N*-(4-(2-(piperidin-1-yl)ethoxy)phenyl)pyridin-2-amine
(106) *N*-(3-Methoxy-4-(4-methylpiperazin-1-yl)phenyl)-4-((2-methyl-4-phenylthiazol-5-yl)oxy)pyridin-2-amine
(107) 1,1,1-Trifluoro-*N*-(3-((4-((2-methyl-4-phenylthiazol-5-yl)oxy)pyridin-2-yl)amino)phenyl)methanesulfonamide
(108) *N*-(4-(4-(2-Methoxyethyl)piperazin-1-yl)phenyl)-4-((2-methyl-4-phenylthiazol-5-yl)oxy)pyridin-2-amine
(109) 3-((4-((2-Methyl-4-phenylthiazol-5-yl)oxy)pyridin-2-yl)amino)benzoic acid
(110) 4-((4-((2-Methyl-4-phenylthiazol-5-yl)oxy)pyridin-2-yl)amino)benzoic acid
(111) 2-(3-((4-((2-Methyl-4-phenylthiazol-5-yl)oxy)pyridin-2-yl)amino)phenyl)acetonitrile
(112) *N*-(3-((1*H*-tetrazol-5-yl)methyl)phenyl)-4-((2-methyl-4-phenylthiazol-5-yl)oxy)pyridin-2-amine
(113) 3-(3-((4-((2-Methyl-4-phenylthiazol-5-yl)oxy)pyridin-2-yl)amino)phenyl)propanenitrile
(114) *N*-(3-(2-(2*H*-Tetrazol-5-yl)ethyl)phenyl)-4-((2-methyl-4-phenylthiazol-5-yl)oxy)pyridin-2-amine
(115) 2-(3-((4-((2-Cyclopropyl-4-phenylthiazol-5-yl)oxy)pyridin-2-yl)amino)phenyl)propan-2-ol
(116) 3-((4-((2-Cyclopropyl-4-phenylthiazol-5-yl)oxy)pyridin-2-yl)amino)benzenesulfonamide
(117) 3-((4-((2-Cyclopropyl-4-phenylthiazol-5-yl)oxy)pyridin-2-yl)amino)benzamide
(118) 4-((4-((2-Cyclopropyl-4-phenylthiazol-5-yl)oxy)pyridin-2-yl)amino)benzenesulfonamide
(119) 4-((4-((2-Cyclopropyl-4-phenylthiazol-5-yl)oxy)pyridin-2-yl)amino)benzamide
(120) 2-(4-((4-((2-Cyclopropyl-4-phenylthiazol-5-yl)oxy)pyridin-2-yl)amino)pyridin-2-yl)propan-2-ol
(121) 2-(5-((4-((2-Cyclopropyl-4-phenylthiazol-5-yl)oxy)pyridin-2-yl)amino)pyridin-2-yl)propan-2-ol
(122) *N*-(3-((4-((2-Cyclopropyl-4-phenylthiazol-5-yl)oxy)pyridin-2-yl)amino)phenyl)ethanesulfonamide
(123) *N*-(3-((4-((2-Cyclopropyl-4-phenylthiazol-5-yl)oxy)pyridin-2-yl)amino)phenyl)-1,1,1-trifluoromethanesulfonamide
(124) 6-((4-((2-Cyclopropyl-4-phenylthiazol-5-yl)oxy)pyridin-2-yl)amino)picolinamide
(125) 6-((4-((2-Cyclopropyl-4-phenylthiazol-5-yl)oxy)pyridin-2-yl)amino)-*N-*methylpicolinamide
(126) 4-((4-((2-Cyclopropyl-4-phenylthiazol-5-yl)oxy)pyridin-2-yl)amino)-*N-*methylbenzamide
(127) *N*-(6-((4-((2-Cyclopropyl-4-phenylthiazol-5-yl)oxy)pyridin-2-yl)amino)pyridin-3-yl)methanesulfonamide
(128) *N*-(4-((4-((2-Cyclopropyl-4-phenylthiazol-5-yl)oxy)pyridin-2-yl)amino)phenyl)methanesulfonamide
(129) 6-((4-((2-Cyclopropyl-4-phenylthiazol-5-yl)oxy)pyridin-2-yl)amino)nicotinamide
(130) Methyl 6-((4-((2-cyclopropyl-4-phenylthiazol-5-yl)oxy)pyridin-2-yl)amino)nicotinate
(131) Methyl 6-((4-((2-cyclopropyl-4-phenylthiazol-5-yl)oxy)pyridin-2-yl)amino)picolinate
(132) 6-((4-((2-Cyclopropyl-4-phenylthiazol-5-yl)oxy)pyridin-2-yl)amino)nicotinic acid
(133) 6-((4-((2-Cyclopropyl-4-phenylthiazol-5-yl)oxy)pyridin-2-yl)amino)-*N-*methylnicotinamide
(134) 6-((4-((2-Cyclopropyl-4-phenylthiazol-5-yl)oxy)pyridin-2-yl)amino)picolinic acid
(135) 3-((4-((2-Cyclopropyl-4-phenylthiazol-5-yl)oxy)pyridin-2-yl)amino)benzoic acid
(136) 4-((4-((2-Cyclopropyl-4-phenylthiazol-5-yl)oxy)pyridin-2-yl)amino)benzoic acid
(137) 6-((4-((2-Cyclopropyl-4-phenylthiazol-5-yl)oxy)pyridin-2-yl)amino)picolinic acid
(138) 6-((4-((2-Cyclopropyl-4-phenylthiazol-5-yl)oxy)pyridin-2-yl)amino)-*N-*hydroxynicotinamide
(139) 4-((4-((2-Cyclopropyl-4-phenylthiazol-5-yl)oxy)pyridin-2-yl)amino)-*N-*hydroxybenzamide
(140) 6-((4-((2-Cyclopropyl-4-phenylthiazol-5-yl)oxy)pyridin-2-yl)amino)-*N-*(methylsulfonyl)nicotinamide
(141) 2-(3-((4-((2-Methoxy-4-phenylthiazol-5-yl)oxy)pyridin-2-yl)amino)phenyl)propan-2-ol
(142) 3-((4-((2-Methoxy-4-phenylthiazol-5-yl)oxy)pyridin-2-yl)amino)benzenesulfonamide
(143) 3-((4-((2-Methoxy-4-phenylthiazol-5-yl)oxy)pyridin-2-yl)amino)benzamide
(144) 3-((4-((2-Methoxy-4-phenylthiazol-5-yl)oxy)pyridin-2-yl)amino)benzoic acid
(145) 3-((4-((4-Phenyl-2-(trifluoromethyl)thiazol-5-yl)oxy)pyridin-2-yl)amino)benzenesulfonamide
(146) 6-((4-((4-Phenyl-2-(trifluoromethyl)thiazol-5-yl)oxy)pyridin-2-yl)amino)nicotinamide
(147) 4-((4-((4-Phenyl-2-(trifluoromethyl)thiazol-5-yl)oxy)pyridin-2-yl)amino)benzamide
(148) *N*-Methyl-4-((4-((4-phenyl-2-(trifluoromethyl)thiazol-5-yl)oxy)pyridin-2-yl)amino)benzamide
(149) 6-((4-((4-Phenyl-2-(trifluoromethyl)thiazol-5-yl)oxy)pyridin-2-yl)amino)nicotinic acid
(150) *N*-Methyl-6-((4-((4-phenyl-2-(trifluoromethyl)thiazol-5-yl)oxy)pyridin-2-yl)amino)nicotinamide
(151) 3-((4-((4-Phenyl-2-(trifluoromethyl)thiazol-5-yl)oxy)pyridin-2-yl)amino)benzoic acid
(152) 4-((4-((4-Phenyl-2-(trifluoromethyl)thiazol-5-yl)oxy)pyridin-2-yl)amino)benzoic acid
(153) 2-(3-((4-((2-(Dimethylamino)-4-phenylthiazol-5-yl)oxy)pyridin-2-yl)amino)phenyl)propan-2-ol
(154) 3-((4-((2-(Dimethylamino)-4-phenylthiazol-5-yl)oxy)pyridin-2-yl)amino)benzenesulfonamide
(155) 3-((4-((2-(Dimethylamino)-4-phenylthiazol-5-yl)oxy)pyridin-2-yl)amino)benzamide
(156) 4-((4-((2-(Dimethylamino)-4-phenylthiazol-5-yl)oxy)pyridin-2-yl)amino)benzenesulfonamide
(157) 4-((4-((2-(Dimethylamino)-4-phenylthiazol-5-yl)oxy)pyridin-2-yl)amino)benzamide
(158) 2-(4-((4-((2-(Dimethylamino)-4-phenylthiazol-5-yl)oxy)pyridin-2-yl)amino)pyridin-2-yl)propan-2-ol
(159) 2-(5-((4-((2-(Dimethylamino)-4-phenylthiazol-5-yl)oxy)pyridin-2-yl)amino)pyridin-2-yl)propan-2-ol
(160) 6-((4-((2-(Dimethylamino)-4-phenylthiazol-5-yl)oxy)pyridin-2-yl)amino)picolinamide
(161) 6-((4-((2-(Dimethylamino)-4-phenylthiazol-5-yl)oxy)pyridin-2-yl)amino)-*N-*methylpicolinamide
(162) Methyl 6-((4-((2-(Dimethylamino)-4-phenylthiazol-5-yl)oxy)pyridin-2-yl)amino)picolinate
(163) 6-((4-((2-(Dimethylamino)-4-phenylthiazol-5-yl)oxy)pyridin-2-yl)amino)nicotinamide
(164) 4-((4-((2-(Dimethylamino)-4-phenylthiazol-5-yl)oxy)pyridin-2-yl)amino)-*N-*methylbenzamide
(165) 6-((4-((2-(Dimethylamino)-4-phenylthiazol-5-yl)oxy)pyridin-2-yl)amino)nicotinic acid
(166) 6-((4-((2-(Dimethylamino)-4-phenylthiazol-5-yl)oxy)pyridin-2-yl)amino)-*N-*methylnicotinamide
(167) 3-((4-((2-(Dimethylamino)-4-phenylthiazol-5-yl)oxy)pyridin-2-yl)amino)benzoic acid
(168) 4-((4-((2-(Dimethylamino)-4-phenylthiazol-5-yl)oxy)pyridin-2-yl)amino)benzoic acid
(169) 6-((4-((2-(Dimethylamino)-4-phenylthiazol-5-yl)oxy)pyridin-2-yl)amino)picolinic acid
(170) Methyl 3-((4-((2-bromo-4-phenylthiazol-5-yl)oxy)pyridin-2-yl)amino)benzoate
(171) 3-((4-((2-Bromo-4-phenylthiazol-5-yl)oxy)pyridin-2-yl)amino)benzoic acid
(172) 3-((4-((2-Bromo-4-phenylthiazol-5-yl)oxy)pyridin-2-yl)amino)benzamide
(173) 6-((4-((2-Cyclopentyl-4-phenylthiazol-5-yl)oxy)pyridin-2-yl)amino)nicotinamide
(174) 6-((4-((2-Cyclopentyl-4-phenylthiazol-5-yl)oxy)pyridin-2-yl)amino)nicotinic acid
(175) 4-((4-((2-Cyclopentyl-4-phenylthiazol-5-yl)oxy)pyridin-2-yl)amino)benzoic acid
(176) 6-((4-((2-Cyclopentyl-4-phenylthiazol-5-yl)oxy)pyridin-2-yl)amino)-*N-*methylnicotinamide
(177) 6-((4-((2-Cyclohexyl-4-phenylthiazol-5-yl)oxy)pyridin-2-yl)amino)nicotinamide
(178) 6-((4-((2-Cyclohexyl-4-phenylthiazol-5-yl)oxy)pyridin-2-yl)amino)nicotinic acid
(179) 4-((4-((2-Cyclohexyl-4-phenylthiazol-5-yl)oxy)pyridin-2-yl)amino)benzoic acid
(180) 6-((4-((2-Cyclohexyl-4-phenylthiazol-5-yl)oxy)pyridin-2-yl)amino)-*N-*methylnicotinamide
(181) 3-((4-((2-Ethyl-4-phenylthiazol-5-yl)oxy)pyridin-2-yl)amino)benzenesulfonamide
(182) 2-(3-((4-((2-Ethyl-4-phenylthiazol-5-yl)oxy)pyridin-2-yl)amino)phenyl)propan-2-ol
(183) 3-((4-((2-Ethyl-4-phenylthiazol-5-yl)oxy)pyridin-2-yl)amino)benzamide
(184) 4-((4-((2-Ethyl-4-phenylthiazol-5-yl)oxy)pyridin-2-yl)amino)benzenesulfonamide
(185) 4-((4-((2-Ethyl-4-phenylthiazol-5-yl)oxy)pyridin-2-yl)amino)benzamide
(186) 2-(4-((4-((2-Ethyl-4-phenylthiazol-5-yl)oxy)pyridin-2-yl)amino)pyridin-2-yl)propan-2-ol
(187) 2-(5-((4-((2-Ethyl-4-phenylthiazol-5-yl)oxy)pyridin-2-yl)amino)pyridin-2-yl)propan-2-ol
(188) *N*-(3-((4-((2-Ethyl-4-phenylthiazol-5-yl)oxy)pyridin-2-yl)amino)phenyl)ethanesulfonamide
(189) 4-((4-((2-Ethyl-4-phenylthiazol-5-yl)oxy)pyridin-2-yl)amino)-*N-*methylbenzamide
(190) 6-((4-((2-Ethyl-4-phenylthiazol-5-yl)oxy)pyridin-2-yl)amino)nicotinamide
(191) 6-((4-((2-Ethyl-4-phenylthiazol-5-yl)oxy)pyridin-2-yl)amino)nicotinate
(192) 6-((4-((2-Ethyl-4-phenylthiazol-5-yl)oxy)pyridin-2-yl)amino)nicotinic acid
(193) 6-((4-((2-Ethyl-4-phenylthiazol-5-yl)oxy)pyridin-2-yl)amino)-*N-*methylnicotinamide
(194) 3-((4-((2-Ethyl-4-phenylthiazol-5-yl)oxy)pyridin-2-yl)amino)benzoic acid
(195) 4-((4-((2-Ethyl-4-phenylthiazol-5-yl)oxy)pyridin-2-yl)amino)benzoic acid
(196) 6-((4-((2-Ethyl-4-phenylthiazol-5-yl)oxy)pyridin-2-yl)amino)-*N-*isopropylnicotinamide
(197) 6-((4-((2-Ethyl-4-phenylthiazol-5-yl)oxy)pyridin-2-yl)amino)pyridin-3-yl)(4-methylpiperazin-1-yl)methanone
(198) 3-Aminopyrrolidin-1-yl)(6-(4-(2-ethyl-4-phenylthiazol-5-yloxy) pyridin-2-ylamino)pyridin-3-yl)methanone
(199) 6-((4-((2-Ethyl-4-phenylthiazol-5-yl)oxy)pyridin-2-yl)amino)-*N*-(2-(pyrrolidin-1-yl)ethyl)nicotinamide
(200) 6-((4-((2-Ethyl-4-phenylthiazol-5-yl)oxy)pyridin-2-yl)amino)pyridin-3-yl)(3-(methylamino)pyrrolidin-1-yl)methanone
(201) 6-((4-((2-Ethyl-4-phenylthiazol-5-yl)oxy)pyridin-2-yl)amino)-*N*-(2-(4-methylpiperazin-1-yl)ethyl)nicotinamide
(202) 6-((4-((2-Ethyl-4-phenylthiazol-5-yl)oxy)pyridin-2-yl)amino)-*N*-(2-(4-i sopropylpiperazin-1 -yl)ethyl)nicotinamide
(203) 6-((4-((2-Ethyl-4-phenylthiazol-5-yl)oxy)pyridin-2-yl)amino)-*N*-(2-(4-ethylpiperazin-1-yl)ethyl)nicotinamide
(204) 5-(3-Aminopyrrolidin-1-yl)-*N*-(4-((2-ethyl-4-phenylthiazol-5-yl)oxy)pyridin-2-yl)pyridin-2-amine
(205) *N*¹-(4-((2-Ethyl-4-phenylthiazol-5-yl)oxy)pyridin-2-yl)-*N*⁴-(2-(4-isopropylpiperazin-1-yl)ethyl)benzene-1,4-diamine
(206) *N*¹-(4-((2-Ethyl-4-phenylthiazol-5-yl)oxy)pyridin-2-yl)-*N*⁴-(2-(4-methylpiperazin-1-yl)ethyl)benzene-1,4-diamine
(207) *N*¹-(4-((2-Ethyl-4-phenylthiazol-5-yl)oxy)pyridin-2-yl)-*N*⁴-(2-(4-ethylpiperazin-1-yl)ethyl)benzene-1,4-diamine
(208) *N*¹-(4-((2-Ethyl-4-phenylthiazol-5-yl)oxy)pyridin-2-yl)-*N*⁴-(2-(pyrrolidin-1-yl)ethyl)benzene-1,4-diamine
(209) *N*²-(4-((2-Ethyl-4-phenylthiazol-5-yl)oxy)pyiidin-2-yl)-*N*⁵-(2-(4-methylpiperazin-1-yl)ethyl)pyridine-2, 5-diamine
(210) *N*²-(4-((2-Ethyl-4-phenylthiazol-5-yl)oxy)pyridin-2-yl)-*N*⁵-(2-(pyrrolidin-1-yl)ethyl)pyridine-2,5-diamine
(211) *N*²-(4-((2-Ethyl-4-phenylthiazol-5-yl)oxy)pyiidin-2-yl)-*N*⁵-(2-(4-ethylpiperazin-1-yl)ethyl)pyridine-2,5-diamine
(212) *N*²-(4-((2-Ethyl-4-phenylthiazol-5-yl)oxy)pyiidin-2-yl)-*N*⁵-(2-(4-isopropylpiperazin-1-yl)ethyl)pyridine-2, 5-diamine
(213) *N*-(5-(1H-Pyrazol-4-yl)pyridin-2-yl)-4-((2-ethyl-4-phenylthiazol-5-yl)oxy)pyridin-2-amine
(214) *N*-(4-(3-Aminopyrrolidin-1-yl)phenyl)-4-((2-ethyl-4-phenylthiazol-5-yl)oxy)pyridin-2-amine
(215) 4-((2-Ethyl-4-phenylthiazol-5-yl)oxy)-*N*-(4-(3-(methylamino)pyrrolidin-1-yl)phenyl)pyridin-2-amine
(216) Methyl 6-((4-((2-isopropyl-4-phenylthiazol-5-yl)oxy)pyridin-2-yl)amino)picolinate
(217) 6-((4-((2-Isopropyl-4-phenylthiazol-5-yl)oxy)pyridin-2-yl)amino)picolinic acid
(218) 3-((4-((2-Isopropyl-4-phenylthiazol-5-yl)oxy)pyridin-2-yl)amino)benzenesulfonamide
(219) 2-(3-((4-((2-Isopropyl-4-phenylthiazol-5-yl)oxy)pyridin-2-yl)amino)phenyl)propan-2-ol
(220) 3-((4-((2-Isopropyl-4-phenylthiazol-5-yl)oxy)pyridin-2-yl)amino)benzamide
(221) 4-((4-((2-Isopropyl-4-phenylthiazol-5-yl)oxy)pyridin-2-yl)amino)benzenesulfonamide
(222) 4-((4-((2-Isopropyl-4-phenylthiazol-5-yl)oxy)pyridin-2-yl)amino)benzamide
(223) 2-(4-((4-((2-Isopropyl-4-phenylthiazol-5-yl)oxy)pyridin-2-yl)amino)pyridin-2-yl)propan-2-ol
(224) 2-(5-((4-((2-Isopropyl-4-phenylthiazol-5-yl)oxy)pyridin-2-yl)amino)pyridin-2-yl)propan-2-ol
(225) 6-((4-((2-Isopropyl-4-phenylthiazol-5-yl)oxy)pyridin-2-yl)amino)-A-methylpicolinamide
(226) 6-((4-((2-Isopropyl-4-phenylthiazol-5-yl)oxy)pyridin-2-yl)amino)picolinamide
(227) 6-((4-((2-Isopropyl-4-phenylthiazol-5-yl)oxy)pyridin-2-yl)amino)nicotinamide
(228) 6-((4-((2-Isopropyl-4-phenylthiazol-5-yl)oxy)pyridin-2-yl)amino)-A-methylnicotinamide
(229) 4-((4-((2-Isopropyl-4-phenylthiazol-5-yl)oxy)pyridin-2-yl)amino)-A-methylbenzamide
(230) 3-((4-((2-Isopropyl-4-phenylthiazol-5-yl)oxy)pyridin-2-yl)amino)benzoic acid
(231) 4-((4-((2-Isopropyl-4-phenylthiazol-5-yl)oxy)pyridin-2-yl)amino)benzoic acid
(232) 6-((4-((2-Isopropyl-4-phenylthiazol-5-yl)oxy)pyridin-2-yl)amino)nicotinic acid
(233) 3-((4-((2-(Methylamino)-4-(naphthalen-2-yl)thiazol-5-yl)oxy)pyridin-2-yl)amino)benzenesulfonamide
(234) 3-((4-((2-Amino-4-(naphthalen-2-yl)thiazol-5-yl)oxy)pyridin-2-yl)amino)benzenesulfonamide
(235) 3-((4-((2-(Dimethylamino)-4-(naphthalen-2-yl)thiazol-5-yl)oxy)pyridin-2-yl)amino)benzenesulfonamide
(236) 3-((6-((2-Amino-4-phenylthiazol-5-yl)oxy)pyrimidin-4-yl)amino)benzenesulfonamide
(237) 3-((6-((2-Methyl-4-phenylthiazol-5-yl)oxy)pyrimidin-4-yl)amino)benzenesulfonamide
(238) 3-((4-((2-Methyl-4-phenylthiazol-5-yl)oxy)pyrimidin-2-yl)amino)benzenesulfonamide
(239) 3-((6-((2-Amino-4-phenylthiazol-5-yl)oxy)pyrimidin-4-yl)amino)benzenesulfonamide
(240) 3-((6-((2-Amino-4-phenylthiazol-5-yl)oxy)pyrimidin-4-yl)amino)benzenesulfonamide
(241) 6-((4-((2-Ethyl-4-phenylthiazol-5-yl)oxy)pyrimidin-2-yl)amino)picolinamide
(242) 2-((4-((2-Ethyl-4-phenylthiazol-5-yl)oxy)pyrimidin-2-yl)amino)isonicotinamide
(243) 3-((4-((2-Ethyl-4-phenylthiazol-5-yl)oxy)pyrimidin-2-yl)amino)benzamide
(244) 6-((4-((2-Ethyl-4-phenylthiazol-5-yl)oxy)pyrimidin-2-yl)amino)nicotinic acid
(245) 6-((4-((2-Ethyl-4-phenylthiazol-5-yl)oxy)pyrimidin-2-yl)amino)nicotinamide
(246) 4-((4-((2-Ethyl-4-phenylthiazol-5-yl)oxy)pyrimidin-2-yl)amino)benzoic acid
(247) 3-((4-((2-Amino-4-(6-methylpyridin-2-yl)thiazol-5-yl)oxy)pyridin-2-yl)amino)benzenesulfonamide
(248) 4-((4-((2-Amino-4-(6-methylpyridin-2-yl)thiazol-5-yl)oxy)pyridin-2-yl)amino)benzoic acid
(249) 3-((4-((2-Amino-4-(6-methylpyridin-2-yl)thiazol-5-yl)oxy)pyridin-2-yl)amino)benzoic acid
(250) 3-((4-((2-Ethyl-4-(6-methylpyridin-2-yl)thiazol-5-yl)oxy)pyridin-2-yl)amino)benzenesulfonamide
(251) 6-((4-((2-Ethyl-4-(6-methylpyridin-2-yl)thiazol-5-yl)oxy)pyridin-2-yl)amino)nicotinamide
(252) 6-((4-((2-Ethyl-4-(6-methylpyridin-2-yl)thiazol-5-yl)oxy)pyridin-2-yl)amino)nicotinic acid
(253) 6-((4-((2-Ethyl-4-(6-methylpyridin-2-yl)thiazol-5-yl)oxy)pyridin-2-yl)amino)-N-methylnicotinamide
(254) 6-((4-((2-Ethyl-4-(6-methylpyridin-2-yl)thiazol-5-yl)oxy)pyridin-2-yl)amino)picolinic acid
(255) 6-((4-((2-Ethyl-4-(6-methylpyridin-2-yl)thiazol-5-yl)oxy)pyridin-2-yl)amino)picolinamide
(256) 6-((4-((2-Ethyl-4-(6-methylpyridin-2-yl)thiazol-5-yl)oxy)pyridin-2-yl)amino)-N-methylpicolinamide
(257) 3-((4-((2-Ethyl-4-(6-methylpyridin-2-yl)thiazol-5-yl)oxy)pyridin-2-yl)amino)benzoic acid
(258) 3-((4-((2-Ethyl-4-(6-methylpyridin-2-yl)thiazol-5-yl)oxy)pyridin-2-yl)amino)benzamide
(259) 3-((4-((2-Ethyl-4-(6-methylpyridin-2-yl)thiazol-5-yl)oxy)pyridin-2-yl)amino)-*N*-methylbenzamide
(260) 5-((4-((2-Ethyl-4-(6-methylpyridin-2-yl)thiazol-5-yl)oxy)pyridin-2-yl)amino)nicotinic acid
(261) 5-((4-((2-Ethyl-4-(6-methylpyridin-2-yl)thiazol-5-yl)oxy)pyridin-2-yl)amino)nicotinamide
(262) 5-((4-((2-Ethyl-4-(6-methylpyridin-2-yl)thiazol-5-yl)oxy)pyridin-2-yl)amino)-*N*-methylnicotinamide
(263) 4-((4-((2-Ethyl-4-(6-methylpyridin-2-yl)thiazol-5-yl)oxy)pyridin-2-yl)amino)picolinic acid
(264) 4-((4-((2-Ethyl-4-(6-methylpyridin-2-yl)thiazol-5-yl)oxy)pyridin-2-yl)amino)picolinamide
(265) 4-((4-((2-Ethyl-4-(6-methylpyridin-2-yl)thiazol-5-yl)oxy)pyridin-2-yl)amino)-*N*-methylpicolinamide
(266) 2-((4-((2-Ethyl-4-(6-methylpyridin-2-yl)thiazol-5-yl)oxy)pyridin-2-yl)amino)isonicotinic acid
(267) 2-((4-((2-Ethyl-4-(6-methylpyridin-2-yl)thiazol-5-yl)oxy)pyridin-2-yl)amino)isonicotinamide
(268) 2-((4-((2-Ethyl-4-(6-methylpyridin-2-yl)thiazol-5-yl)oxy)pyridin-2-yl)amino)-*N*-methylisonicotinamide
(269)*N*-(2-(Dimethylamino)ethyl)-6-((4-((2-ethyl-4-(6-methylpyridin-2-yl)thiazol-5-yl)oxy)pyridin-2-yl)amino)nicotinamide
(270) *N*-(Cyclopropylmethyl)-6-((4-((2-ethyl-4-(6-methylpyridin-2-yl)thiazol-5-yl)oxy)pyridin-2-yl)amino)nicotinamide
(271) 2-(Dimethylamino)ethyl6-((4-((2-ethyl-4-(6-methylpyridin-2-yl)thiazol-5-yl)oxy)pyridin-2-yl)amino)nicotinate
(272) *N*-(3-((4-((2-ethyl-4-(6-methylpyridin-2-yl)thiazol-5-yl)oxy)pyridin-2-yl)amino)phenyl)methanesulfonamide
(273) 2-(Dimethylamino)ethyl3-(6-((4-((2-ethyl-4-(6-methylpyridin-2-yl)thiazol-5-yl)oxy)pyridin-2-yl)amino)nicotinamido)-5-fluorobenzoate
(274) 3-((4-((2-Cyclopropyl-4-(6-methylpyridin-2-yl)thiazol-5-yl)oxy)pyridin-2-yl)amino)benzamide
(275) 6-((4-((2-Ethyl-4-(6-methylpyridin-2-yl)thiazol-5-yl)oxy)pyridin-2-yl)amino)nicotinamide
(276) 2-((4-((2-Cyclopropyl-4-(6-methylpyridin-2-yl)thiazol-5-yl)oxy)pyridin-2-yl)amino)isonicotinamide
(277) 3-((4-((2-Cyclopropyl-4-(6-methylpyridin-2-yl)thiazol-5-yl)oxy)pyridin-2-yl)amino)benzenesulfonamide
(278) 6-((4-((2-Cyclopropyl-4-(6-methylpyridin-2-yl)thiazol-5-yl)oxy)pyridin-2-yl)amino)picolinamide
(279) 6-((4-((2-Cyclopropyl-4-(6-methylpyridin-2-yl)thiazol-5-yl)oxy)pyridin-2-yl)amino)-*N*-methylpicolinamide
(280) 2-((4-((2-Cyclopropyl-4-(6-methylpyridin-2-yl)thiazol-5-yl)oxy)pyridin-2-yl)amino)-*N*-methylisonicotinamide
(281) 3-((4-((2-Cyclopropyl-4-(6-methylpyridin-2-yl)thiazol-5-yl)oxy)pyridin-2-yl)amino)-*N*-methylbenzamide
(282) 6-((4-((2-Cyclopropyl-4-(6-methylpyridin-2-yl)thiazol-5-yl)oxy)pyridin-2-yl)amino)-*N*-methylnicotinamide
(283) 3-((4-((2-(Dimethylamino)-4-(6-methylpyridin-2-yl)thiazol-5-yl)oxy)pyridin-2-yl)amino)benzenesulfonamide
(284) 6-((4-((2-(Dimethylamino)-4-(6-methylpyridin-2-yl)thiazol-5-yl)oxy)pyridin-2-yl)amino)nicotinamide
(285) 6-((4-((2-(Dimethylamino)-4-(6-methylpyridin-2-yl)thiazol-5-yl)oxy)pyridin-2-yl)amino)nicotinic acid
(286) 6-((4-((2-(Dimethylamino)-4-(6-methylpyridin-2-yl)thiazol-5-yl)oxy)pyridin-2-yl)amino)-*N*-methylnicotinamide
(287) 3-((4-((2-Isopropyl-4-(6-methylpyridin-2-yl)thiazol-5-yl)oxy)pyridin-2-yl)amino)benzenesulfonamide
(288) 6-((4-((2-Isopropyl-4-(6-methylpyridin-2-yl)thiazol-5-yl)oxy)pyridin-2-yl)amino)nicotinamide
(289) 3-((4-((4-(6-Methylpyridin-2-yl)-2-(trifluoromethyl)thiazol-5-yl)oxy)pyridin-2-yl)amino)benzenesulfonamide
(290) 6-((4-((2-Isopropyl-4-(6-methylpyridin-2-yl)thiazol-5-yl)oxy)pyridin-2-yl)amino)nicotinamide
(291) 3-((4-((4-(6-Methylpyridin-2-yl)-2-(trifluoromethyl)thiazol-5-yl)oxy)pyridin-2-yl)amino)benzamide
(292) 3-((4-((2-(Methylamino)-4-(6-methylpyridin-2-yl)thiazol-5-yl)oxy)pyridin-2-yl)amino)benzenesulfonamide
(293) 6-((4-((2-Cyclopropyl-4-phenyloxazol-5-yl)oxy)pyridin-2-yl)amino)nicotinic acid
(294) 6-((4-((2-Cyclopropyl-4-phenyloxazol-5-yl)oxy)pyridin-2-yl)amino)nicotinamide
(295) 4-((4-((2-Cyclopropyl-4-phenyloxazol-5-yl)oxy)pyridin-2-yl)amino)benzoic acid
(296) 3-((4-((2-Cyclopropyl-4-phenyloxazol-5-yl)oxy)pyridin-2-yl)amino)benzamide
(297) 3-((4-((2-Cyclopropyl-4-phenyloxazol-5-yl)oxy)pyridin-2-yl)amino)-*N-*methylbenzamide
(298) 3-((4-((2-Cyclopropyl-4-phenyloxazol-5-yl)oxy)pyridin-2-yl)amino)benzenesulfonamide
(299) 6-((4-((2-Cyclopropyl-4-phenyloxazol-5-yl)oxy)pyridin-2-yl)amino)picolinamide
(300) 3-((4-((2-Cyclopropyl-4-phenyloxazol-5-yl)oxy)pyridin-2-yl)amino)benzoic acid
(301) 6-((4-((2-Cyclopropyl-4-phenyloxazol-5-yl)oxy)pyridin-2-yl)amino)picolinic acid
(302) 6-((4-((2-Cyclopropyl-4-phenyloxazol-5-yl)oxy)pyridin-2-yl)amino)-*N-*methylpicolinamide
(303) 3-((4-((2-Methyl-4-(6-methylpyridin-2-yl)thiazol-5-yl)oxy)pyridin-2-yl)amino)benzenesulfonamide
(304) 2-(3-((4-((2-Methyl-4-phenylthiazol-5-yl)oxy)pyridin-2-yl)amino)phenyl)acetonitrile
(305) *N*-(3-((1*H*-Tetrazol-5-yl)methyl)phenyl)-4-((2-methyl-4-(6-methylpyridin-2-yl)thiazol-5-yl)oxy)pyridin-2-amine
(306) 3-(3-((4-((2-Methyl-4-(6-methylpyridin-2-yl)thiazol-5-yl)oxy)pyridin-2-yl)amino)phenyl)propanenitrile
(307) *N*-(3-(2-(2*H*-Tetrazol-5-yl)ethyl)phenyl)-4-((2-methyl-4-(6-methylpyridin-2-yl)thiazol-5-yl)oxy)pyridin-2-amine
(308) Methyl 2-methyl-2-(3-((4-((2-methyl-4-(6-methylpyridin-2-yl)thiazol-5-yl)oxy)pyridin-2-yl)amino)phenyl)propanoate
(309) 2-Methyl-2-(3-((4-((2-methyl-4-(6-methylpyridin-2-yl)thiazol-5-yl)oxy)pyridin-2-yl)amino)phenyl)propanoic acid
(310) 4-((4-((2-(Methylamino)-4-phenylthiazol-5-yl)oxy)pyridin-2-yl)amino)benzamide
(311) N (3-((4-((2-Ethyl-4-phenylthiazol-5-yl)oxy)pyridin-2-yl)amino)phenyl)-1,1,1-trifluoromethanesulfonamide and
(312) 4-((2-Ethyl-4-phenylthiazol-5-yl)oxy)-*N*-(5-(3-(methylamino)pyrrolidin-1-yl)pyridin-2-yl)pyridin-2-amine.

5. A pharmaceutical composition for treating or preventing a cancer disease, comprising a compound selected from the thiazole derivative compound according to claim 1, a pharmaceutically acceptable salt thereof, a solvate thereof, or a stereoisomer thereof as an active ingredient.

6. The pharmaceutical composition of claim 5, wherein the cancer disease is selected from the group consisting of lung cancer, breast cancer, ovarian cancer, uterine cancer, pancreatic cancer, lung cancer, stomach cancer, liver cancer, colorectal cancer, skin cancer, head or neck cancer, brain cancer, laryngeal cancer, prostate cancer, bladder cancer, esophageal cancer, thyroid cancer, kidney cancer, and rectal cancer.

7. The pharmaceutical composition of claim 5, wherein the cancer disease is a transforming growth factor beta (TGFβ)-related cancer disease.

8. The pharmaceutical composition of claim 5, wherein the pharmaceutical composition selectively inhibits transforming growth factor beta receptor 1 (TGFβR1).

9. A health functional food composition for ameliorating or preventing a cancer disease, comprising a compound selected from the thiazole derivative compound according to claim 1, a pharmaceutically acceptable salt thereof, a solvate thereof, or a stereoisomer thereof as an active ingredient.

10. The health functional food composition of claim 9, wherein the cancer disease is selected from the group consisting of lung cancer, breast cancer, ovarian cancer, uterine cancer, pancreatic cancer, lung cancer, stomach cancer, liver cancer, colorectal cancer, skin cancer, head or neck cancer, brain cancer, laryngeal cancer, prostate cancer, bladder cancer, esophageal cancer, thyroid cancer, kidney cancer, and rectal cancer.

11. A reagent composition for suppressing expression of transforming growth factor beta (TGFβ), comprising a compound selected from the thiazole derivative compound according to claim 1, a pharmaceutically acceptable salt thereof, a solvate thereof, or a stereoisomer thereof as an active ingredient.

12. The reagent composition of claim 11, wherein the reagent composition selectively inhibits transforming growth factor beta receptor 1 (TGFβR1).
